(19) Europäisches Patentamt
European Patent Office
Office européen des brevets

(11) EP 0 982 401 A2

(12) EUROPEAN PATENT APPLICATION

(43) Date of publication:
**01.03.2000 Bulletin 2000/09**

(21) Application number: **98310694.9**

(22) Date of filing: **23.12.1998**

(51) Int. Cl.[7]: **C12N 15/31**, C07K 14/40, A61K 31/70, A61K 38/16, C07K 16/14, G01N 33/50, C12Q 1/68

(84) Designated Contracting States:
**AT BE CH CY DE DK ES FI FR GB GR IE IT LI LU MC NL PT SE**
Designated Extension States:
**AL LT LV MK RO SI**

(30) Priority: **14.08.1998 GB 9817796**

(71) Applicant:
**JANSSEN PHARMACEUTICA N.V.**
**2340 Beerse (BE)**

(72) Inventors:
- **Contreras, Roland Henri**
  **9000 Gent (BE)**
- **Nelissen, Bart**
  **2340 Beerse (BE)**
- **De Backer, Marianne Denise**
  **2340 Beerse (BE)**
- **Luyten, Walter Herman Maria Louis**
  **2340 Beerse (BE)**
- **Viaene, Jasmine Elsa**
  **9000 Gent (BE)**
- **Logghe, Marc George**
  **9000 Gent (BE)**

(74) Representative:
**Baldock, Sharon Claire et al**
**BOULT WADE TENNANT,**
**27 Furnival Street**
**London EC4A 1PQ (GB)**

(54) **Drug targets in Candida albicans**

(57) Nucleic acid molecules encoding polypeptides that are critical for survival and growth of the yeast Candida albicans are disclosed. Also provided are methods of identifying compounds which selectively modulate expression or activity of such polypeptides comprising the steps of (a) contacting a compound to be tested with one or more *Candida albicans* cells having a mutation in a nucleic acid molecule according to the invention which mutation results in overexpression or underexpression of said polypeptides in addition to contacting one or more wild type *Candida albicans* cells with said compound, and (b) monitoring the growth and/or activity of said mutated cell compared to said wild type; wherein differential growth or activity of said one or more mutated Candida cells is indicative of selective action of said compound on a polypeptide or another polypeptide in the same or a parallel pathway.

EP 0 982 401 A2

SEQUENCE LISTING

(1) GENERAL INFORMATION:

(i) APPLICANT:
(A) NAME: Janssen Pharmaceutica
(B) STREET: Turnhoutseweg 30
(C) CITY: Beerse
(E) COUNTRY: Belgium
(F) POSTAL CODE (ZIP): B-2340
(G) TELEPHONE: +32 (0)14/60.21.11
(H) TELEFAX: +32 (0) 14/60.28.41

(ii) TITLE OF INVENTION: DRUG TARGETS IN CANDIDA ALBICANS

(iii) NUMBER OF SEQUENCES: 72

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(vi) PRIOR APPLICATION DATA:
(A) APPLICATION NUMBER: GB 9817796.7
(B) FILING DATE: 14-AUG-1998

(2) INFORMATION FOR SEQ ID NO: 1:

(i) SEQUENCE CHARACTERISTICS:
(A) LENGTH: 255 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 1:

```
AACGTTCGTG CAAAAGGCTA TACTGGTGAT ATCCACGCAG ATGAAGAGCA AGTTTAATCA    60
ACTCTTTGTC AATTAATGCT GTACTTGTTT TCATTTTATT TGCTGGCATT TAAAGAATAC   120
CCATAGTTCA GAAAATAAAA TTGAAAAATT TAAAAAAAAA CGCAATATCA TTCATTTTTT   180
TTGTTTTTTT GACAATAATA TTAATATGTA GTTACCAATG TTTTTAGATT TTATATGTTT   240
TGAAAAAATA GTTTG                                                    255
```

(2) INFORMATION FOR SEQ ID NO: 2:

(i) SEQUENCE CHARACTERISTICS:
(A) LENGTH: 648 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 2:

AACCTCTTAT TCGGTTCTAG TGTCTCAATT GGTTATCCAT TAACATCTAT TCCCAACTCC 60
ATCATTATTG GCAATAAATA AATGGGTGTT ATATCTATTG GTAATAACTA AACTGGTGTC 120
AATTCAATTC CAATATGGTC ATGACAATTG AAAGTGTTAC TGTTCTGGTT TACATATTCT 180
ACAGGTTACA ACTATTGATT GGTTAGAAGT TTGGTTTCAA CATCACCTGT TGCTAAGAAT 240
AAATGTTGGT CATATCAATT GAATCATTTG TTGGTGTTAT GGTAAGTAAA TGCTGGTTAT 300
ATCTATTATC TACAACCACC AAGTGATAAA TGCTGAACCG TAGTCACCAA CTGTTATGCT 360
GGTTGTATCT ATTGACTAAA ACTACCCTAG GGATAAATGC TGAACCGTGG TTACCAACTG 420
TTATGCTGGT TGTATCTATT AACTGCAACC ACCAAATGAT AAATGCTGAA CCATAATTAC 480
CAACTGTTAC ATTGCTGGTA CTACATTAAG AATAAATGCT GCATCTACAA GTACCACCTG 540
TTGTGTTAAT AAATGCTGCA CCTGCTAGTA CAACTGTTGC TGGTCATGAT AGTTACTACA 600
CATTACACAC CAGACAGTGG CAAACAAGGT TATGTAGAAA CCAACGTT 648

(2) INFORMATION FOR SEQ ID NO: 3:

(i) SEQUENCE CHARACTERISTICS:
(A) LENGTH: 904 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 3:

AACTGTCCTG TGAAGACGAA CATCACAACC ACAATCATGG TCATAACCAA AATCACAATC 60
ATGTTGCTCC TATTCCTACA ACAGCTGGAC AATCATTAAA TAATAAAATT GATACATCTA 120
AAGTGACAGC TCTCAACATG GCCAACTCTG CTGACGATCT AGCAAAAGTT TTCAAAGATT 180
CGACTAAAAA ATATCAAATC AAACCAATTA TCAAATCAGA CAGTGATGAA CAAATGATTA 240
TCAACATTCC ATTTCTTAAT GGTAGTGTCA AATTGTATTC GATAATTCTA CGTACCAATG 300
GGGATTTGTA TTGTCCCAAA ACAATAAAAT TATTCAAAAA TGACACATCA ATTGATTTTG 360
ATAATGTGGA TTCGAAGAAA CCAATACAGG TGTTAACTCA TCCTCAAGTT GGTGTTGCTA 420
ATAATGATAG CGATGATCTT CCAGAGTTTT TGGAATCAAA TAACGATGAC GATTTTGTCG 480
AACATTATGT GTCTCGACAT AAATTCACTG GGGTAAATCA ATTGACAATA TTTATTGAAG 540
ATATTTATGA TGAAGGAGAA GAAGAGTGTC ATTTACATTC AATTGAATTG AGAGGGGAAT 600
TCACTGAATT AAACAAAGAC CCTGTCATTA CATTATATGA ACTGGCTGCT AATCCTGCTG 660
ATCATAAGAA TTTAACGATT GTTGAAAATC AAAATCTAGC ATAAAACAAA GAAGTGAAAG 720
GTATCAGATA AGCTGGTTAC ATTACAATTG ATCTAATTTA GAATCTCAAG GTATTTAAAT 780
TTGCCGTTTT GCGATAATAT AACATGGTCA AGAACGTTGA ATCGATTACG TTAATGGTTT 840
AGCTAATTGA TTTTTAGGAT CGAGTATTTA GAGTGAATAA ACAATAAACA AGAATGATGA 900
ATTG 904

(2) INFORMATION FOR SEQ ID NO: 4:

(i) SEQUENCE CHARACTERISTICS:
(A) LENGTH: 232 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 4:

```
Ser Cys Glu Asp Glu His His Asn His Asn His Gly His Asn Gln Asn
1               5                   10                  15

His Asn His Val Ala Pro Ile Pro Thr Thr Ala Gly Gln Ser Leu Asn
            20                  25                  30

Asn Lys Ile Asp Thr Ser Lys Val Thr Ala Leu Asn Met Ala Asn Ser
        35                  40                  45

Ala Asp Asp Leu Ala Lys Val Phe Lys Asp Ser Thr Lys Lys Tyr Gln
    50                  55                  60

Ile Lys Pro Ile Ile Lys Ser Asp Ser Asp Glu Gln Met Ile Ile Asn
65                  70                  75                  80

Ile Pro Phe Leu Asn Gly Ser Val Lys Leu Tyr Ser Ile Ile Leu Arg
                85                  90                  95

Thr Asn Gly Asp Leu Tyr Cys Pro Lys Thr Ile Lys Leu Phe Lys Asn
            100                 105                 110

Asp Thr Ser Ile Asp Phe Asp Asn Val Asp Ser Lys Lys Pro Ile Gln
        115                 120                 125

Val Leu Thr His Pro Gln Val Gly Val Ala Asn Asn Asp Ser Asp Asp
    130                 135                 140

Leu Pro Glu Phe Leu Glu Ser Asn Asn Asp Asp Asp Phe Val Glu His
145                 150                 155                 160

Tyr Val Ser Arg His Lys Phe Thr Gly Val Asn Gln Leu Thr Ile Phe
                165                 170                 175

Ile Glu Asp Ile Tyr Asp Glu Gly Glu Glu Glu Cys His Leu His Ser
            180                 185                 190

Ile Glu Leu Arg Gly Glu Phe Thr Glu Leu Asn Lys Asp Pro Val Ile
        195                 200                 205

Thr Leu Tyr Glu Ser Ala Ala Asn Pro Ala Asp His Lys Asn Leu Thr
    210                 215                 220

Ile Val Glu Asn Gln Asn Leu Ala
225                 230
```

(2) INFORMATION FOR SEQ ID NO: 5:

(i) SEQUENCE CHARACTERISTICS:
(A) LENGTH: 608 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 5:

```
AACCTACAAA AGACTCACAT GTGCTGTACA ATAAATTTCT GGATAAGCAT ATAAGTGATG     60
AGCAACTATC ACACTTACTC GACAATCATA AACCCAATCT AGTGACTACC ACAACTTTAA    120
TTGATTCTAT CAAAGAAAGT GAACTGTTAT ATAATACCAT GGACAGTTTG ATGATAAAAT    180
CCATCAATTT TCCTGCAGCC ATGTACCAGT CAAATGACAA CAATTCACAA TCACCAATCG    240
AGTATTTATC TAACAGAGTA AAATTGCTCA CACAAGAGTT ATACGAAGAT TCAGTCAAAT    300
ATGGCAAGTT TCTACAGAGT GGTAATAATC ATATATATCA ATTACGAAGT AGGATTTTAC    360
AGACCTTTGA TCAGTTGTCA GAGAGTCACT ATTCTTTAAA TGAACTATAT AATAAAGACA    420
TGTCTTACGC AGAAACATTA CACGGATCTT TCAAGAAATG GGATCAACAA AGAAATAAAG    480
TATTGTCCAA AGTGAAATCT ATAAAAAGTG ATACAAGCAA ACATGGAGCC AAATTATTCA    540
CCTTATTAGA TGAAGTTAAT GATGTTGATG ACGAGATCAA ACTTTTGGAA GCAAAACTAC    600
AGCAGGTT                                                             608
```

(2) INFORMATION FOR SEQ ID NO: 6:

(i) SEQUENCE CHARACTERISTICS:
(A) LENGTH: 1497 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 6:

```
GATATCTGCA GAATTCGGCT TCTCTCTCAT CTTCACACAA TGCATTTTAC AAGTAGCCTA     60
CTAGCCACCT TGATATGGTT TACATTACCG GTTCAAAGTT TGAATACTGA ATCTAGGACA    120
ACTTCAAATA ACACAATATC AATACTTACA AACCATTTTC AAATACTAAA GGATTTGCTA    180
CCATATAGCA AAACTTCTAA ACCGCAAATC AAGGAATCCA GACCGTTGAT TAAAGTTCTG    240
AGAGATGGAG TGCCAATAAA TTTCCACAGG GCTCCGGCTA TAATAATGAA ATCGAACAAA    300
ACAGACGATT TAGTCAGGAA TAGCAATAAA ACAATGGTGC TAACTGAAAT AAAAACGATT    360
ACTGAATTTG CAACTACCAC TGTTTCCCCT ACACAAGAAT TTCAAGCACT ACAGATAAAC    420
CTTAACACGT TATCAATAGA GACTTCAACA CCAACATTCC AATCCCATGA CTTTCCACCG    480
ATTACCATTG AAGACACACC CAAAACACTA GAACCAGAAG AATCGTCAGA TGCTTTGCAG    540
AGGGATGCAT TTGATCAAAT TAAGAAACTA GAAAAATTGG TATTGGATTT GAGACTTGAA    600
ATGAAAGAGC AACAAAAGAG TTTCAACGAT CAATTAGTGG ATATATATAC CGCAAGAAGT    660
ATTGTTCCAA TTTATACTAC ACATATCGTC ACTTCGGCGA TTCCATCGTA TGTACCAAAA    720
GAAGAAGTAA TGGTTTCACA TGATACTGCA CCAATTGTAA GTCGTCCTAG AACAGATATT    780
```

CCAGTATCTC AACGAATTGA TACTATCTCA AAACATAAAA TGAATGGAAA AAATATATTG 840

AACAACAATC CTCCGCCCAA TTCAGTTTTA ATAGTTCCTC AGTTTCAGTT CCATGAAAGA 900

ATGGCCACCA AAACCGAAGT AGCTTATATG AAACCAAAAA TTGTCTGGAC CAACTTTCCA 960

ACCACTACTG CAACGTCAAT GTTTGACAAT TTTATTTTAA AAAATCTTGT TGACGAAACG 1020

GATTCTGAAA TTGATAGTGG TGAAACTGAA TTGTCTGACG ATTATTATTA CTATTATAGT 1080

TACGAAGATG ATGGTAAAGA AGACGATAGT GATGAGATTA CGGCTCAAAT ACTATTATCC 1140

AATTCAGAAT TAGGCACGAA GACGCCAAAT TTTGAGGATC CTTTTGAACA AATCAATATT 1200

GAAGACAATA AAGTAATATC TGTTAATACA CCAAAGACAA AGAAACCTAC TACAACAGTA 1260

TTTGGCACTT CTACTAGTGC ATTATCAACT TTTGAAAGTA CAATATTTGA AATTCCCAAA 1320

TTCTTTTATG GTAGCAGAAG AAAACAACTG AGCTCATTCA AAAATAAGAA CAGTACAATC 1380

AAATTTGATG TGTTTGATTG GATATTTGAA AGTGGTACTA CCAATGAGAA AGTACATGGA 1440

TTAGTGTTGG TGTCTAGTGG TGTTCTACTA GGAACTTGTC TATTGTTCAT TTTGTAG 1497

(2) INFORMATION FOR SEQ ID NO: 7:

(i) SEQUENCE CHARACTERISTICS:
(A) LENGTH: 485 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 7:

```
Met His Phe Thr Ser Ser Leu Leu Ala Thr Leu Ile Trp Phe Thr Leu
1               5                   10                  15

Pro Val Gln Ser Leu Asn Thr Glu Ser Arg Thr Thr Ser Asn Asn Thr
            20                  25                  30

Ile Ser Ile Leu Thr Asn His Phe Gln Ile Leu Lys Asp Leu Leu Pro
        35                  40                  45

Tyr Ser Lys Thr Ser Lys Pro Gln Ile Lys Glu Ser Arg Pro Leu Ile
    50                  55                  60

Lys Val Ser Arg Asp Gly Val Pro Ile Asn Phe His Arg Ala Pro Ala
65                  70                  75                  80

Ile Ile Met Lys Ser Asn Lys Thr Asp Asp Leu Val Arg Asn Ser Asn
                85                  90                  95

Lys Thr Met Val Leu Thr Glu Ile Lys Thr Ile Thr Glu Phe Ala Thr
            100                 105                 110

Thr Thr Val Ser Pro Thr Gln Glu Phe Gln Ala Leu Gln Ile Asn Leu
        115                 120                 125

Asn Thr Leu Ser Ile Glu Thr Ser Thr Pro Thr Phe Gln Ser His Asp
    130                 135                 140

Phe Pro Pro Ile Thr Ile Glu Asp Thr Pro Lys Thr Leu Glu Pro Glu
145                 150                 155                 160

Glu Ser Ser Asp Ala Leu Gln Arg Asp Ala Phe Asp Gln Ile Lys Lys
```

```
                165                 170                 175
Leu Glu Lys Leu Val Leu Asp Leu Arg Leu Glu Met Lys Glu Gln Gln
            180                 185                 190

Lys Ser Phe Asn Asp Gln Leu Val Asp Ile Tyr Thr Ala Arg Ser Ile
        195                 200                 205

Val Pro Ile Tyr Thr Thr His Ile Val Thr Ser Ala Ile Pro Ser Tyr
    210                 215                 220

Val Pro Lys Glu Glu Val Met Val Ser His Asp Thr Ala Pro Ile Val
225                 230                 235                 240

Ser Arg Pro Arg Thr Asp Ile Pro Val Ser Gln Arg Ile Asp Thr Ile
                245                 250                 255

Ser Lys His Lys Met Asn Gly Lys Asn Ile Leu Asn Asn Asn Pro Pro
            260                 265                 270

Pro Asn Ser Val Leu Ile Val Pro Gln Phe Gln Phe His Glu Arg Met
        275                 280                 285

Ala Thr Lys Thr Glu Val Ala Tyr Met Lys Pro Lys Ile Val Trp Thr
    290                 295                 300

Asn Phe Pro Thr Thr Thr Ala Thr Ser Met Phe Asp Asn Phe Ile Leu
305                 310                 315                 320

Lys Asn Leu Val Asp Glu Thr Asp Ser Glu Ile Asp Ser Gly Glu Thr
                325                 330                 335

Glu Leu Ser Asp Asp Tyr Tyr Tyr Tyr Tyr Ser Tyr Glu Asp Asp Gly
            340                 345                 350

Lys Glu Asp Asp Ser Asp Glu Ile Thr Ala Gln Ile Leu Leu Ser Asn
        355                 360                 365

Ser Glu Leu Gly Thr Lys Thr Pro Asn Phe Glu Asp Pro Phe Glu Gln
    370                 375                 380

Ile Asn Ile Glu Asp Asn Lys Val Ile Ser Val Asn Thr Pro Lys Thr
385                 390                 395                 400

Lys Lys Pro Thr Thr Thr Val Phe Gly Thr Ser Thr Ser Ala Leu Ser
                405                 410                 415

Thr Phe Glu Ser Thr Ile Phe Glu Ile Pro Lys Phe Phe Tyr Gly Ser
            420                 425                 430

Arg Arg Lys Gln Ser Ser Ser Phe Lys Asn Lys Asn Ser Thr Ile Lys
        435                 440                 445

Phe Asp Val Phe Asp Trp Ile Phe Glu Ser Gly Thr Thr Asn Glu Lys
    450                 455                 460

Val His Gly Leu Val Leu Val Ser Ser Gly Val Leu Leu Gly Thr Cys
465                 470                 475                 480

Leu Leu Phe Ile Leu
                485
```

(2) INFORMATION FOR SEQ ID NO: 8:

(i) SEQUENCE CHARACTERISTICS:
(A) LENGTH: 1651 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 8:

```
GAGCTCTTCC AGAGGCAACA AGCGGAAGAA GCACAACGAA AGAAGGAATT TGAACAAAAG      60
GCCGAATTCA TCAAAGCATC ATTACTTGAA ATGCGCCGAA GAGAAATAGA GAGGCGGAAA     120
CAGCAAAAGG AAAGGGAACA AAGACAAAAG GAGCACGAAG CAAAGAGGGA TATCAGGATA     180
CAACAACTTT CAGAGCAGGA TTCACGGAGT AATCAAACTA AAGAAGAAGA GGAAGTGTTC     240
AAGAAGGCCC GGTCTACTAA TTCGGGAGCA GACGAGACTG GTTTGATGTC AGATAAAGAG     300
TTTGATGATT CTGCATATTC ACCCGATTAT TTGTTTGAAG AGAATTTGTG GAATAAACCA     360
AATCATCCAG ATACAAATCA TAAAACCAAA AAATATACTG AGAATGTGGT TGAAAATCTA     420
GATTCTCCAC CAAATGATAC ATCTGCGTAC AATTCAAGTT TTCATGATGA AACTAATATT     480
CAAAATGAGA TCCAAATACC AGAAAATGAC GAGTATGTAC CACAGATGAA AGCTACATCC     540
AGTGTCAATA ATACCACCAT CCCTGCACAA AGAAGACATG AGTCACTTTC CACTTCTGAA     600
AACAAAAGAA GGAAATTTGA AACAGCCGAC GTTGGGGTTG ATGGGTTAGA TTCCCCAGTG     660
CGGGCACAAC CAGAAATATC TGGAAAATCC AAGTCTCCGA TAATCCCTGA TGTAATACTT     720
TTACTGGACG AAGAGACTGA AACTCCTGAA GCAAATGCTG TGCAGGACAA TAGTACATAT     780
ATTCCTCAGG GGTCTTTAGG ACACGAATTT AGAAATATTT TGGAAGAGCA TCCACGTCAA     840
GTAAAGAATA AACAAAATTC TGGTGTTGCT TTTGCATTTC CGAATGCTTC CAAGAATACC     900
GAAAACAAAC TCCACTCTAA TTTCAAAGAT AAAGATGAAG GAATAATTGA TGTTGAAGCT     960
TACGTACCTG ATGTCAAAGC AGCAACTTCA AACACCACCC CAGCAACAGG ACAAACATCA    1020
GCAAGGTCGG AAAAACTGCC ACCCTTACCT ACTCATATTC CAAATCCATC GACCATGAAT    1080
GAAGCTCGAC CTCATCCAAC AACTCCACAT AAAAGATCAA AAGTCATTTT CGATTTAAAA    1140
GATTTAGAAC AAAAGTTAGG TAATGATATT GAGGATTTGG ATTTTAAGGA TATGTATGAG    1200
AGTTTGCCTG ACCATTCAAG TAAGGCAACA CCTAAAGACG ATATTTTAAC CCGTTCTAAA    1260
AGAAGACTTT ATACATATAC CGATGGAACA TCAAAGGCTG AAACGTTATC TACACCAATG    1320
AACAAAAATC CTGTTCGTGG ACATAGTACC AAGAAAAAGC TTAGTATGTT GGACATGCAT    1380
GCGTCTTCTA AAATTCAAAG TCTTTTACCT CCACAACCGC CACAAATGTC AATTGATCCT    1440
TCTGTTTCCA AGCAAGTGTG GGCTAAATAC GTTGATGCAA TCTTGACTTA TCAAAGAGAA    1500
TTTTTCAATT ATAAAAAAGT GATTGTTCAA TACCAAATGG AACGGATAAA CAAAGACCTT    1560
GAACATTTTG ACGATATAAA TGATGGTTCA CACACTGAGA ATTTGGATAC TTTCAAGCAT    1620
TGTTTAGAAC AAGATTATTT GGTTAGTTGA C                                   1651
```

(2) INFORMATION FOR SEQ ID NO: 9:

(i) SEQUENCE CHARACTERISTICS:
(A) LENGTH: 463 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 9:

```
AACCTGTTGA CGCGTTGTCT TTTTCTACCC CACGTTTAAC AATCTTGCCA GTCAATTCAC      60
TAGCCAAATA AACTTTAGAC TCACAACTCT AACACTGACT CGCCCCCCCC TGTTTAAACT     120
CTAAATTACT TCACAGAGCC TTTACTACCT TAATTTAAGA TTATCTATTG TTTCTGTTCT     180
TTTGCAATCA CCCTGACTCG TTTTTTTTTC AGCCAGTTTT TTCGTAAAAT CTGACCAAAA     240
ATTTACAACT CTAATTTAAA ACTCTAAATA ACAATTAAAA CTCAATTCAG ACAAGTCCTT     300
CTGCTCATTC TGAGTCTTCT CTATTGTCTT TTGACTTTTT GTGTGTGACT ATTTTCATGA     360
TCACCCCGTT TCTTGCATTT TTTTCAGTCA ACTTTTTCTC AAAATCAAGC CAAAAAAACA     420
CATTTAACTG CCTATACAAC GCAAACCTAT TCAAAACAAG GTT                       463
```

(2) INFORMATION FOR SEQ ID NO: 10:

(i) SEQUENCE CHARACTERISTICS:
(A) LENGTH: 582 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 10:

```
AACCTCCCCG TTAACCACTT CTAGGTATAC CATTTCATCT GACTGAATAA CTGGTTAGTC      60
GATTTGTTGT TGAAGAAAAG TGACCACCTA GTTTTTTCTG CCAACATTTT TTGCGATGAG     120
CCGTCGACGC GTTGTCTTTT TCTACCCCAC GTTTAACAAT CTTGCCAGTC AATTCCCTAG     180
CCAAATAAAC TTTAGACTCA CAACTCTAAC ACTGACTCGT GCCCCCCTGT TTAAACTCTA     240
AATTACTTCA CAGAGCCTTT ACTACCTTAA TTTAAGATTA TCTATTGTTT CTGTTTTTTT     300
GCAATCACCC TGACTCGTTT TTTTTTCAGC CAGTTTTTTC GTAAAATCTG ACCAAAAATT     360
TACAACTCTA ATTTAAAACT CTAAATAACA ATTAAAACTC AATTCAGACA AGTCCTTCTG     420
CTCATTCTGA GTCTTCTCTA TTGTCTTTTG ACTTTTTGTG TGTGACTATT TTCATGATCA     480
CCCCGTTTCT TGCATTTTTT TCAGTCAACT TTTTCTCAAA ATCAAGCCAA AAAAACACAC     540
CTTTAACTAC CTATACAACG CAAACCTATT CAAAACAAGG TT                        582
```

(2) INFORMATION FOR SEQ ID NO: 11:

(i) SEQUENCE CHARACTERISTICS:
(A) LENGTH: 1066 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(ix) FEATURE:
(A) NAME/KEY: misc_feature
(B) LOCATION:183
(D) OTHER INFORMATION:/note= "W = A or T"

(ix) FEATURE:
(A) NAME/KEY: misc_feature
(B) LOCATION:564
(D) OTHER INFORMATION:/note= "Y = C or T"

(xi) SEQUENCE DESCRIPTION: SEQ ID NO: 11:

```
AACCATAAAT ATGCCAAGAT TTAAACAAGT TGATGTATTC ACCAATGTCA AATATTTGGG    60
TAATCCAGTT GCCGTTATTT ATGATAGTGA TAATTTAACC ACTCAAGAAA TGCAAAAAAT   120
TGCTCGATGG ACAAATTTAT CAGAAACAAC ATTTATATTG ACTCCAAAAT CATCAATTGC   180
TGWTTATAGT ATTAGAATTT TCACTTCTGG TGGGAATGAA TTACCATTTG CTGGTCATCC   240
TACTTTAGGT ACTGCATTTG CATTATTGGA AGATGGTAAA ATAAAACCAA ATGACAATGG   300
ACAAATAATT CAAGAATGTG GTGCTGGATT AGTGAAAATA TCCGTTGAAA AAACACCTAA   360
TAATAATAGT AATGAGTTGC CGTTTTTGTT ATCTTTTGAA TTACCATATT TCAAATTTCA   420
TGAAATTGAT GACAAAGTAA TCGAGGAATT ACAACATTCA TGGAATGGAA CCAATATTAT   480
TGGTAAACCG GTACTTATTG ATGCTGGTCC AAAATGGGCA GTTTTCCAAC TTGGCTCCGG   540
TAAAGAAGTA TTAGACTTGA ATGYTGATTT AGCACAAATT GAGAGATTAA GTTTAGAAAA   600
TGGTTGGACA GGAATTGGTG TCTTTGGAAA ACATAATGAA AATGGTGATT CGGTCGAATT   660
GAGAAATATT GCTCCTGCTG TTGGAGTCGC TGAAGATCCT GCTTGTGGAA GTGGATCAGG   720
TGCTATTGGA GCATATTTGG CAAATCACGT TTTCAATGAA AAGGAAAAAT TTACAATTGA   780
TATTTCTCAA GGTAAACCAA TTGAAAGAGA TGCTAAGATT CAAGTTAAAG TTAATCGTCT   840
TACCACCAAA AATGGTGATT TATCTATTCA TGTTGGTGGT CATGCCATCA CTTGTTTCGA   900
AGGTACTTAT TCTATTTAAA ACTTGATATA ATTCTTGAGT TATATCTAAT TTATCTAATT   960
CACTTGTCCC TGGAGTAGTT TGATCTAATT GATGTAATTT ATTTAATAAA TCACGTTCTA  1020
AATCAGTTTG TTTAGATAAA TCATTTAATA AATCATCTTC AGCATT                 1066
```

(2) INFORMATION FOR SEQ ID NO: 12:

(i) SEQUENCE CHARACTERISTICS:
(A) LENGTH: 302 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 12:

```
Met Pro Arg Phe Lys Gln Val Asp Val Phe Thr Asn Val Lys Tyr Leu
1               5                   10                  15

Gly Asn Pro Val Ala Val Ile Tyr Asp Ser Asp Asn Leu Thr Thr Gln
```

```
                20                  25                  30

Glu Met Gln Lys Ile Ala Arg Trp Thr Asn Leu Ser Glu Thr Thr Phe
        35                  40                  45

Ile Leu Thr Pro Lys Ser Ser Ile Ala Xaa Tyr Ser Ile Arg Ile Phe
    50                  55                  60

Thr Ser Gly Gly Asn Glu Leu Pro Phe Ala Gly His Pro Thr Leu Gly
65                  70                  75                  80

Thr Ala Phe Ala Leu Leu Glu Asp Gly Lys Ile Lys Pro Asn Asp Asn
                85                  90                  95

Gly Gln Ile Ile Gln Glu Cys Gly Ala Gly Leu Val Lys Ile Ser Val
            100                 105                 110

Glu Lys Thr Pro Asn Asn Asn Ser Asn Glu Leu Pro Phe Leu Leu Ser
        115                 120                 125

Phe Glu Leu Pro Tyr Phe Lys Phe His Glu Ile Asp Asp Lys Val Ile
    130                 135                 140

Glu Glu Leu Gln His Ser Trp Asn Gly Thr Asn Ile Ile Gly Lys Pro
145                 150                 155                 160

Val Leu Ile Asp Ala Gly Pro Lys Trp Ala Val Phe Gln Leu Gly Ser
                165                 170                 175

Gly Lys Glu Val Leu Asp Leu Asn Xaa Asp Leu Ala Gln Ile Glu Arg
            180                 185                 190

Leu Ser Leu Glu Asn Gly Trp Thr Gly Ile Gly Val Phe Gly Lys His
        195                 200                 205

Asn Glu Asn Gly Asp Ser Val Glu Leu Arg Asn Ile Ala Pro Ala Val
    210                 215                 220

Gly Val Ala Glu Asp Pro Ala Cys Gly Ser Gly Ser Gly Ala Ile Gly
225                 230                 235                 240

Ala Tyr Leu Ala Asn His Val Phe Asn Glu Lys Glu Lys Phe Thr Ile
                245                 250                 255

Asp Ile Ser Gln Gly Lys Pro Ile Glu Arg Asp Ala Lys Ile Gln Val
            260                 265                 270

Lys Val Asn Arg Leu Thr Thr Lys Asn Gly Asp Leu Ser Ile His Val
        275                 280                 285

Gly Gly His Ala Ile Thr Cys Phe Glu Gly Thr Tyr Ser Ile
    290                 295                 300
```

(2) INFORMATION FOR SEQ ID NO: 13:

(i) SEQUENCE CHARACTERISTICS:
(A) LENGTH: 2829 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 13:

```
ATGACGGAAA CTGTGATAGA AAAGAAAAGA AAGGTTGATT TAAATGCCTC AGGTATTACA        60
```

```
AAACAACCAA AAGCTTCTAA AATCTTCAGT CCATTCAGAG TTTTAGGGAA TGTTACAGAC   120
TCAACTCCTT TTGCCATGGG GACATTAGGT TCAACATTTT ATGCTGTCAC TTCTGTTGGC   180
AGATCTTTCC AAATTTATGA CTTGGCTACA TTACATTTAT TGTTTGTTTC CCAAACTCAA   240
ACTCCTTCAA GAATTACAAG TTTGGCTGCA CACCATCACT ATGTCTATGC ATCTTATGGT   300
GATCGTATTG GTATTTTTAG ACGTGGTAGA TTAGAGCATG AATTGGTTTG TGAAGGGAAC   360
TCTACAGTTA ACCAATTATT AGTATTTGGA GAATACCTTA TTGCTACCAC ATTAGAAGGT   420
GATATTTTCG TATTTAGAAA AACTGAAGGA AAGAAATTCC CAACTGAATT ATACACTACA   480
ATCAGAATAA TTAATTCTTT AGTTGAAGGA GAAATTGTGG GATTAATTCA TCCACCTACG   540
TATTTAAATA AAGTAATTGT TGCTACTACT CAATCTGTGT TTGTTATAAA TGTGAGAACT   600
GGCAAATTAT TATACAAATC CCGGGAATTA CAATTCGAAG GCGAAAAGAT TTCATCAATC   660
GAAGCTGCTC CAGTTTTGGA TGTAATTGCT GTTGGTACAT CTAATGGAAA TGTATTTTTA   720
TTCAACATTA AAAAGGGGAA AGTGTTGGGC CAAAAAATTA TTACTTCTGG AACTGAATCT   780
TCTTCGAAAG TTGCCTCGAT CTCTTTTAGA ACAGATGGAG CACCTCATTT GGTTGCTGGT   840
TTGAATAACG GGGACTTATA TTTCTACGAT TTAGACAAGA AATCACGTGT TCATGTTTTG   900
AGAAATGCCC ATAAAGAGAC TCATGGGGGT GTTGCAAACG CCAAATTTTT GAATGGTCAA   960
CCAATAGTAT TATCAAATGG TGGTGATAAT CATTTGAAAG AATTTGTTTT TGATCCTAAT  1020
TTAACCACTT CGAATTCATC CATTGTTCCT CCTCCAAGAC ATCTCAGATC TAGAGGTGGG  1080
CATTCAGCAC CACCAGTAGC TATTGAATTT CCTCAAGAAG ATAAAACCCA TTTTTTATTG  1140
AGTGCTTCTA GAGATAAAAC ATTTTGGACA TTCTCTTTGA GAAAAGATGC TCAAGCACAG  1200
GAAATGTCTC AAAGATTGCA AAAATCTAAG GATGGTAAAA GACAGGCTGG ACAAGTTGTT  1260
TCTATGAGAG AGAAATTCCC AGAAATCATT TCCATTTCAT CCTCTTATGC CAGAGAAGGT  1320
GATTGGGAAA ATATCATAAC CGCCCACAAG GATGAAACTT TTGCGAGAAC ATGGGATTCA  1380
AGAAATAAAA GAGTCGGTAG ACATTTGTTA AACACTATTG ATGGTGGCAT TGTGAAATCT  1440
GTATGTGTGT CTCAGTGTGG TAATTTTGGT TTAGTGGGAT CATCACTGGG TGGTATTGGA  1500
TCATACAACC TTCAAAGTGG ATTGTTGCGT AAAAAATATG TTTTACATAA ACAAGCTGTC  1560
ACCGGTTTAG CAATTGATGG AATGAATAGA AAAATGGTTA GTTGTGGTTT AGATGGAATT  1620
GTGGGATTCT ATGATTTTGG AAAGTCTGTC TATTTAGGCA AATTACAACT TGAAGCACCT  1680
ATAACATCCA TGATATATCA CAAACTGTCT GATCTTGTTG CTTGTGCCTT GGATGATTTG  1740
TCCATAGTTG TTATTGACGT GACTACTCAA AAAGTCATAA GAATATTATA TGGTCATACC  1800
AACAGAATTT CAGGAATGGA TTTCTCGCCT GATGGGAGAT GGATAGTTTC AGTTGCATTG  1860
GACTCCACTT TGCGAACTTG GGACTTGCCA ACTGGTGGTT GTATTGATGG GGTGATTTTA  1920
CCAATTGTGG CAACTGCAGT TAAATTTTCT CCTATTGGTG ATATCTTAGC GACAACACAT  1980
GTCTCTGGAA ATGGTGTATC CTTATGGACT AATCGTGCCC AGTTCAAGCC TGTGTCCACC  2040
AGACACGTAG AAGAAGATGA GTTTTCAACT ATTTTATTAC CAAATGCTTC TGGAGATGGC  2100
GGTTCAACAA TGCTAGACGG GTTTTTGGAC GAGGATTCTA ATGAAGACGG CACTATTGAT  2160
GAACAGTATA CATCTGCTGC TCAAATTGAT GCATCCTTGA TTACTTTATC ATCAGAGCCA  2220
```

```
AGATCAAAAT TCAACACTTT ATTGCATTTG GATACCATTA AACAACAAAG CAAACCGAAA    2280
GAAGCACCTA AAAAACCAGA AAATGCACCT TTCTTTTTAC AATTGACTGG ACAAGCAGTT    2340
GGTGATAGGG CATCGGTTGC TGAAGGCAAA ACTTCAGAAC AAACAAATAA CACTGTTGAA    2400
GAAACCAACA GCAAATTGCG TAAATTGGAT ACAAACGGTA ACCACGCATT TGAAAGTGAA    2460
TTCACAAAAC TATTAAGGGA AGCTGGAGAG AGTGGACAAT TTGAAAGATT TTTGACTTAC    2520
TTACTTAACT TATCTCCTGC TGTATTGGAC TTGGAAATTA GATCACTTAA TTCATTTGTT    2580
CCATTGACTG AAATGACAAA TTTTATTCAA GCTTTAAATG CTGGTTTGAA ATCAAACGCA    2640
AATTATGAAA TATGGGAAAC TTTATATGCC ATGTTTTTCA ACATACATGG TGATGTTATC    2700
CATCAGTTTG AAAATGAAAC TAGTCTTCAT GAAGCTTTGG AAGAATACAG ACAGTTAAAT    2760
GATGAAAAGA ATAACAAAAT GGATTCTTTA GTGAAATATT GTGCTAGTAT CGTAAGTTTT    2820
ATTAGTTAG                                                            2829
```

(2) INFORMATION FOR SEQ ID NO: 14:

(i) SEQUENCE CHARACTERISTICS:
(A) LENGTH: 942 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 14:

```
Met Thr Glu Thr Val Ile Glu Lys Lys Arg Lys Val Asp Leu Asn Ala
1               5                   10                  15

Ser Gly Ile Thr Lys Gln Pro Lys Ala Ser Lys Ile Phe Ser Pro Phe
            20                  25                  30

Arg Val Leu Gly Asn Val Thr Asp Ser Thr Pro Phe Ala Met Gly Thr
        35                  40                  45

Leu Gly Ser Thr Phe Tyr Ala Val Thr Ser Val Gly Arg Ser Phe Gln
    50                  55                  60

Ile Tyr Asp Leu Ala Thr Leu His Leu Leu Phe Val Ser Gln Thr Gln
65                  70                  75                  80

Thr Pro Ser Arg Ile Thr Ser Leu Ala Ala His His His Tyr Val Tyr
                85                  90                  95

Ala Ser Tyr Gly Asp Arg Ile Gly Ile Phe Arg Arg Gly Arg Leu Glu
            100                 105                 110

His Glu Leu Val Cys Glu Gly Asn Ser Thr Val Asn Gln Leu Leu Val
        115                 120                 125

Phe Gly Glu Tyr Leu Ile Ala Thr Thr Leu Glu Gly Asp Ile Phe Val
    130                 135                 140

Phe Arg Lys Thr Glu Gly Lys Lys Phe Pro Thr Glu Leu Tyr Thr Thr
145                 150                 155                 160

Ile Arg Ile Ile Asn Ser Leu Val Glu Gly Glu Ile Val Gly Leu Ile
                165                 170                 175
```

His Pro Pro Thr Tyr Leu Asn Lys Val Ile Val Ala Thr Thr Gln Ser
180 185 190

Val Phe Val Ile Asn Val Arg Thr Gly Lys Leu Leu Tyr Lys Ser Arg
195 200 205

Glu Leu Gln Phe Glu Gly Glu Lys Ile Ser Ser Ile Glu Ala Ala Pro
210 215 220

Val Leu Asp Val Ile Ala Val Gly Thr Ser Asn Gly Asn Val Phe Leu
225 230 235 240

Phe Asn Ile Lys Lys Gly Lys Val Leu Gly Gln Lys Ile Ile Thr Ser
245 250 255

Gly Thr Glu Ser Ser Ser Lys Val Ala Ser Ile Ser Phe Arg Thr Asp
260 265 270

Gly Ala Pro His Leu Val Ala Gly Leu Asn Asn Gly Asp Leu Tyr Phe
275 280 285

Tyr Asp Leu Asp Lys Lys Ser Arg Val His Val Leu Arg Asn Ala His
290 295 300

Lys Glu Thr His Gly Gly Val Ala Asn Ala Lys Phe Leu Asn Gly Gln
305 310 315 320

Pro Ile Val Leu Ser Asn Gly Gly Asp Asn His Leu Lys Glu Phe Val
325 330 335

Phe Asp Pro Asn Leu Thr Thr Ser Asn Ser Ser Ile Val Pro Pro Pro
340 345 350

Arg His Leu Arg Ser Arg Gly Gly His Ser Ala Pro Pro Val Ala Ile
355 360 365

Glu Phe Pro Gln Glu Asp Lys Thr His Phe Leu Leu Ser Ala Ser Arg
370 375 380

Asp Lys Thr Phe Trp Thr Phe Ser Leu Arg Lys Asp Ala Gln Ala Gln
385 390 395 400

Glu Met Ser Gln Arg Leu Gln Lys Ser Lys Asp Gly Lys Arg Gln Ala
405 410 415

Gly Gln Val Val Ser Met Arg Glu Lys Phe Pro Glu Ile Ile Ser Ile
420 425 430

Ser Ser Ser Tyr Ala Arg Glu Gly Asp Trp Glu Asn Ile Ile Thr Ala
435 440 445

His Lys Asp Glu Thr Phe Ala Arg Thr Trp Asp Ser Arg Asn Lys Arg
450 455 460

Val Gly Arg His Leu Leu Asn Thr Ile Asp Gly Gly Ile Val Lys Ser
465 470 475 480

Val Cys Val Ser Gln Cys Gly Asn Phe Gly Leu Val Gly Ser Ser Ser
485 490 495

Gly Gly Ile Gly Ser Tyr Asn Leu Gln Ser Gly Leu Leu Arg Lys Lys
500 505 510

Tyr Val Leu His Lys Gln Ala Val Thr Gly Leu Ala Ile Asp Gly Met
515 520 525

Asn Arg Lys Met Val Ser Cys Gly Leu Asp Gly Ile Val Gly Phe Tyr
530 535 540

Asp Phe Gly Lys Ser Val Tyr Leu Gly Lys Leu Gln Leu Glu Ala Pro
545 550 555 560

```
Ile Thr Ser Met Ile Tyr His Lys Ser Ser Asp Leu Val Ala Cys Ala
            565                 570                 575

Leu Asp Asp Leu Ser Ile Val Val Ile Asp Val Thr Thr Gln Lys Val
        580                 585                 590

Ile Arg Ile Leu Tyr Gly His Thr Asn Arg Ile Ser Gly Met Asp Phe
    595                 600                 605

Ser Pro Asp Gly Arg Trp Ile Val Ser Val Ala Leu Asp Ser Thr Leu
610                 615                 620

Arg Thr Trp Asp Leu Pro Thr Gly Gly Cys Ile Asp Gly Val Ile Leu
625                 630                 635                 640

Pro Ile Val Ala Thr Ala Val Lys Phe Ser Pro Ile Gly Asp Ile Leu
            645                 650                 655

Ala Thr Thr His Val Ser Gly Asn Gly Val Ser Leu Trp Thr Asn Arg
        660                 665                 670

Ala Gln Phe Lys Pro Val Ser Thr Arg His Val Glu Glu Asp Glu Phe
    675                 680                 685

Ser Thr Ile Leu Leu Pro Asn Ala Ser Gly Asp Gly Gly Ser Thr Met
690                 695                 700

Leu Asp Gly Phe Leu Asp Glu Asp Ser Asn Glu Asp Gly Thr Ile Asp
705                 710                 715                 720

Glu Gln Tyr Thr Ser Ala Ala Gln Ile Asp Ala Ser Leu Ile Thr Leu
            725                 730                 735

Ser Ser Glu Pro Arg Ser Lys Phe Asn Thr Leu Leu His Leu Asp Thr
        740                 745                 750

Ile Lys Gln Gln Ser Lys Pro Lys Glu Ala Pro Lys Lys Pro Glu Asn
    755                 760                 765

Ala Pro Phe Phe Leu Gln Leu Thr Gly Gln Ala Val Gly Asp Arg Ala
770                 775                 780

Ser Val Ala Glu Gly Lys Thr Ser Glu Gln Thr Asn Asn Thr Val Glu
785                 790                 795                 800

Glu Thr Asn Ser Lys Leu Arg Lys Leu Asp Thr Asn Gly Asn His Ala
            805                 810                 815

Phe Glu Ser Glu Phe Thr Lys Leu Leu Arg Glu Ala Gly Glu Ser Gly
        820                 825                 830

Gln Phe Glu Arg Phe Leu Thr Tyr Leu Leu Asn Leu Ser Pro Ala Val
    835                 840                 845

Leu Asp Leu Glu Ile Arg Ser Leu Asn Ser Phe Val Pro Leu Thr Glu
850                 855                 860

Met Thr Asn Phe Ile Gln Ala Leu Asn Ala Gly Leu Lys Ser Asn Ala
865                 870                 875                 880

Asn Tyr Glu Ile Trp Glu Thr Leu Tyr Ala Met Phe Phe Asn Ile His
            885                 890                 895

Gly Asp Val Ile His Gln Phe Glu Asn Glu Thr Ser Leu His Glu Ala
        900                 905                 910

Leu Glu Glu Tyr Arg Gln Leu Asn Asp Glu Lys Asn Asn Lys Met Asp
    915                 920                 925

Ser Leu Val Lys Tyr Cys Ala Ser Ile Val Ser Phe Ile Ser
930                 935                 940
```

(2) INFORMATION FOR SEQ ID NO: 15:

(i) SEQUENCE CHARACTERISTICS:
(A) LENGTH: 725 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 15:

```
AACCTGGCAA TTAACTGCCC GGCAAGTGAT AGCAGGAGAT AGGTGTGTAT AGATTATAAT      60
GGAACGCCGA TTTTTGCAGT ATCACGCGTA ATAAGGACAG CAGTTGGACA TCGGTACATG     120
AGAGAGCAAT GTAAGTCTTG ATAGTAATGA GCCGTGTTGA AGTAGTATTT TAATCTAATT     180
TTACTCAAAA AAGGACAATG GAGATCTGGA GATAACAGCA CACTAATCGG TTCTAGACAT     240
AGACTAAGCC TGAAAGGGGG TACTACAGCT TGTTTTGAAA AGGTTTGCGT TGTATAGGCA     300
GTTAAATGTG TGTTTTTTTT GGGTAGAATT TGAGAAAAAG TTGACTGAAA AAAATGCAAG     360
AAACGGGGTG ATCATGAAAA TAGACACACA CAAAAAGTCA AAAAACAATG GAAAAGCTTC     420
AGAATAAGCA GTAGGAGGTG TCTGAATTGA GTTTGTATTG TTATTTAGAG TTTTAAATTA     480
GAGTTGTAAA TTTTTGGGTA GAATTTACGA AAAAGTCGAA CAAAAAAACG ACAAGTCAGG     540
GTGATTGCAA AAAAACAGAA ACAATAGATA ATCTTAAATT AAGGTAGTAG AGGCTCTGTG     600
AAGTAATTTA GAGTTTAAAC AGGGGGGCAC GAGTCAGTGT TAGAGTTGTG AAGTTTATTT     660
GGCTAGTGAA TTGACTGGCA AGATTGTTAA ACGTGGGGTA GAAAAAGACA ACGCATCGAC     720
AGGTT                                                                 725
```

(2) INFORMATION FOR SEQ ID NO: 16:

(i) SEQUENCE CHARACTERISTICS:
(A) LENGTH: 1144 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 16:

```
CCATGATATA GAAATTGGTG GGTCAACGTA CTATCAAATT AACATAAAAC TACCACTTCG      60
GTCATTCACG ATAAAGAAAC GGTACCTGGA ATTCCAGCAA TTGGTGCTGG ACTTGAGTCG     120
TAATCTAGGC ATTGATAGTC GAGATTTTCC ATATGAATTA CCTGGGAAAC GGATCAACTG     180
GCTTAACAAG ACCAGTATTG TTGAGGAGAG AAAAGTGGGA CTTGCAGAAT TTCTCAATAA     240
CCTCATTCAA GACTCAACAC TTCAGAATGA ACGAGAAGTG TTGTCGTTTT TGCAATTGCC     300
GTCTAATTTT AGATTCACCA AGGATATGTT ACAGAATAAT CGAGCAGACT TGGATTCTGT     360
```

```
GCAAAATAAC TGGTACGATG TATATCGTAA GTTGAAACTG GATATACTCA ACGAATCGTC     420
TAGCAGCATT AGTGAACAGA TACATATTCG TGATCGCATT AGTCGGGTCT ACCAACCACG     480
GATTCTCGAC TTGGTCAGGG CTATTGGTAC AGATAAAGAA GAGGCCCTAA AGAAGAAGCA     540
GTTGGTTTCC CAATTACAAG AGAGTATAGA TAATTTGTTA GTACAGGAAG TTCCCCGATC     600
AAAGAGGGTG TTGGGTGGAG CAGTTAAGGA AACGCCAGAG ACATTACCAT TAAACAATAA     660
AGAACTTCTT CAACACCAAG TACAAATTCA TCAAAACCAA GACAAAGAAC TAGACCAGCT     720
TAGGGTGTTA ATTGCCCGGC AGAAACAGAT TGGCGAGCTA ATTAATGCAG AAGTAGAGGA     780
ACAGAATGAA ATGTTGGATA GGTTTAATGA AGAGGTCGAC TACACGTCCA GCAAAATCAA     840
GCAAGCAAGA CGCAGAGCTA AGAAGATATT ATAGTAATTT GTTCGCTACT TCGATATTAT     900
CTGCCATTGA CGTTATTCTT GCAGGTTGGC CCAATTGTTC GTTTGAAAGT TTTTCGAGGT     960
CTTCAGCGTC TAATGCCCTA TCTGAGCTCT CGCCATCGAG TTTCCAAAAC CCGCCGATAT    1020
TTTGAAAGAA TCTTTGAATG CCAAACCGTC GTGGCGGGAA CGATCTGCCT GCGTTGGCCA    1080
AGTTGAATAT GCTAGGGTGG TACTGTAAAT AGAAGACAGA TCCAATAAAC GTTCCTATAA    1140
ATGC                                                                 1144
```

(2) INFORMATION FOR SEQ ID NO: 17:

(i) SEQUENCE CHARACTERISTICS:
(A) LENGTH: 290 amino acids
(B) TYPE: amino acid
(C) STRANDEDNESS:
(D) TOPOLOGY: linear

(ii) MOLECULE TYPE: peptide

(iii) HYPOTHETICAL: NO

(xi) SEQUENCE DESCRIPTION: SEQ ID NO: 17:

```
His Asp Ile Glu Ile Gly Gly Ser Thr Tyr Tyr Gln Ile Asn Ile Lys
1               5                   10                  15

Leu Pro Leu Arg Ser Phe Thr Ile Lys Lys Arg Tyr Ser Glu Phe Gln
            20                  25                  30

Gln Leu Val Ser Asp Leu Ser Arg Asn Leu Gly Ile Asp Ser Arg Asp
        35                  40                  45

Phe Pro Tyr Glu Leu Pro Gly Lys Arg Ile Asn Trp Leu Asn Lys Thr
    50                  55                  60

Ser Ile Val Glu Glu Arg Lys Val Gly Leu Ala Glu Phe Leu Asn Asn
65                  70                  75                  80

Leu Ile Gln Asp Ser Thr Leu Gln Asn Glu Arg Glu Val Leu Ser Phe
                85                  90                  95

Leu Gln Leu Pro Ser Asn Phe Arg Phe Thr Lys Asp Met Leu Gln Asn
            100                 105                 110

Asn Arg Ala Asp Leu Asp Ser Val Gln Asn Asn Trp Tyr Asp Val Tyr
        115                 120                 125

Arg Lys Leu Lys Ser Asp Ile Leu Asn Glu Ser Ser Ser Ser Ile Ser
    130                 135                 140
```

```
Glu Gln Ile His Ile Arg Asp Arg Ile Ser Arg Val Tyr Gln Pro Arg
145                 150                 155                 160

Ile Leu Asp Leu Val Arg Ala Ile Gly Thr Asp Lys Glu Glu Ala Leu
                165                 170                 175

Lys Lys Lys Gln Leu Val Ser Gln Leu Gln Glu Ser Ile Asp Asn Leu
            180                 185                 190

Leu Val Gln Glu Val Pro Arg Ser Lys Arg Val Leu Gly Gly Ala Val
        195                 200                 205

Lys Glu Thr Pro Glu Thr Leu Pro Leu Asn Asn Lys Glu Leu Leu Gln
    210                 215                 220

His Gln Val Gln Ile His Gln Asn Gln Asp Lys Glu Leu Asp Gln Leu
225                 230                 235                 240

Arg Val Leu Ile Ala Arg Gln Lys Gln Ile Gly Glu Leu Ile Asn Ala
                245                 250                 255

Glu Val Glu Glu Gln Asn Glu Met Leu Asp Arg Phe Asn Glu Glu Val
            260                 265                 270

Asp Tyr Thr Ser Ser Lys Ile Lys Gln Ala Arg Arg Arg Ala Lys Lys
        275                 280                 285

Ile Leu
    290
```

(2) INFORMATION FOR SEQ ID NO: 18:

(i) SEQUENCE CHARACTERISTICS:
(A) LENGTH: 2736 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(ix) FEATURE:
(A) NAME/KEY: misc_feature
(B) LOCATION:11
(D) OTHER INFORMATION:/note= "N = G or A or T or C"

(ix) FEATURE:
(A) NAME/KEY: misc_feature
(B) LOCATION:2723..2724
(D) OTHER INFORMATION:/note= "N = A or T or C or G"

(ix) FEATURE:
(A) NAME/KEY: misc_feature
(B) LOCATION:2714..2715
(D) OTHER INFORMATION:/note= "N = A or T or C or G"

(ix) FEATURE:
(A) NAME/KEY: misc_feature
(B) LOCATION:2710
(D) OTHER INFORMATION:/note= "N = A or T or C or G"

(ix) FEATURE:
(A) NAME/KEY: misc_feature
(B) LOCATION:2706..2707
(D) OTHER INFORMATION:/note= "N = A or T or C or G"

(xi) SEQUENCE DESCRIPTION: SEQ ID NO: 18:

```
ATGGAAAAAA NTTTGGCGAG TGTAAAGTTG TACACCGATT TGGAGTGTGT TTTTAATTCA        60
```

```
AACTATCCAA CAAGAATTGT TTGGGGTGCT TCTTACAATT TTGGAATTCA ACAGATGATG    120
GCAAACTTTG ATCGGTTTTC AAAACCACCA GTGGATCCAT CTACAAAATT AGGATTTTGG    180
GATAAGTTAA AGTATATCTT ACATGGTAAA TGCCAAATCA GAACTAGGAA AAGTTTAGAA    240
GTTGCATTTA AAGGATCAAG AGATCCGTAT GATTTGTTCA CGACTGCAGG CGGGTTTGTA    300
TTGTCATTTA GAAAGAATGT TGTCTGGGAC ATCAATAAAG ACGATAATTC GAAAAATTAC    360
TTCGATATCA CGGCAGATAA AGTTTCCTGG TATATTCCAA ACTATTTAGC AGGACCATTA    420
TTGGCTTGGA CAAGAAGTAG TAAAAATTCA ATTTATTTAC CAAATTCACC AAATGTGGTT    480
AATTCTTGCT TTGCATATTA CCTTCAAGAT TTTACTGGAC AAGCTGATTT TGATCATGCT    540
GCCCGAGTAT TTGAAAGAAA TGTGGTCAAT CTTAGTGGAG GAATTCATTT TCAAGTTGGG    600
TTTCTACTTG AACGTAAAGA TACAAATGGT AAGAGAACCG ATGAATTCAA ACCTCATTAC    660
GAAGTGCAGT TGTTTGATCC CAAGTATTGT GAGAAAGGAC ATGACTCTTA TGCTGGGTTC    720
CGAAGTCAAT TTATACATAT GGCTATCTCA TTGGAATCAA CAAACAGTTC AAGTTATAAT    780
ACAATCCATC TTAGTCCTGG TACTTTCCAA CAGTTTTTCG ATTGGTGGAA GTTATTTGCT    840
AGTAATATGC AGTTACCTAT TAGACGTGGC AAAATGTTTG GAGAAGCAAA AGAATCTGTC    900
AAGTTTTCGC AACATTTATT CACAAACAAG TTTTCTTTCA TGTTGAAATC TTTGTTTATT    960
GCTCATGTTT ATCGAGACGA AATTGTTGAT ATCAATAACG ATAGAATAGA AAGTATTGGT   1020
TTAAGAGCCA AAGTAGATGA TTTTATGGTT GATTTACATC AAAGAAAAGA GCCAGCAACC   1080
CTTTACCATG AAGAATTATC TAAGAATGAG AAGGTGATGA AAATGAATTT TGATTTAGGA   1140
GAAGTCGTTT TATCAGGAAT AGACTTACGT GTCATGCATG TTTCATTTCT CCAAAATTTA   1200
TACACTCAAT CACATTCCAA TTCAGGTGAC GCTAAATCAA CTTATAATAT TTACGACAAT   1260
GATCATCGAT GGTTTGATAT TATGGATTTC CAAGAGGCAT TTTTGACATC AATTAAGGAT   1320
TGTGTCAGGA CAGTTGATAT TTATCCATTG ATGTATTTAC AAAGATTCTT TTATGAAAGA   1380
GATACACATG GTGGCAAGTC TGAGGATGAG ACTGCATTTG GAAAAGAAGT TATTCATAAA   1440
TGTAATTTGG GTGCCATGAA TCCCTTGGAA ACAAGATTGA ATGTATTGGT TCAAAGACTT   1500
AACGCTCTAC AAGAACAAGT CAAAAAATTG TCCAAAACAT CTGCTCCAGA ACCTGTAGCA   1560
GATTTGAAAA AACGAATTCT GTTTTTGCAA AAAGAGATTA GCACAACCAA AGCTGGCGTT   1620
AAGTCGAAAA TGCGTCGTAC ATCCACTATA AATGGTATGA ATAATTCTGA AAATTACCAC   1680
AATAAGTTTA CTTTCTATAA CATGCTTCTT AAATGGAATT TCAATTGTCG GAATTTGACA   1740
TTGAAATACA TACATTTTGT GAAATTGAAA TCACAACTTC GAAATTACTT GTCACACAAG   1800
TCCATTGAAA CACTTGAAAA AATGATGGAT AGTGTAAATG CATACAACGA TAAGGACGAT   1860
TTGTCATCGA CGTCAGAAAT AATCCGTCGT TTCACACTGG AAGGGGTTAA ATCACAGACA   1920
TCTACCAGCA AAGATATCAC TTCACAACAG AAACTTGACA ATTTCAACAC AATATTACGA   1980
GAGACCAGAC CAGACGAAAA AGTGGTTGAG GATTATTTGA TTGACGTGAT CGCACCTCAA   2040
ATTCAATTAC AAAGTGAGGA TTATCCTGAT TCTGTTGTGC TCATCTCTAC ACCATCTATT   2100
AAAGGTAAAA TTTTGTCCAT TAGGGATTCC AGGAATAATG CAAACCAAAT CTTGTTAGAA   2160
ACTAGGTATG GTATTTTACT AAAAGATGCC AATGTTTTTG TATTAAACAA AGAGGATATT   2220
```

GTAGGGTGTC CAGATATGTT AAGTATTAGT AATCCATATG GAGCTAAATC TAATTGGCCA 2280

CCATGGCTAG GAACAGAAAT AACCCAAAAT GGTAAATGGG CTGGAGCCAA CAACTTATTG 2340

ATTGAAAAGC TTTCTGTTAT GACAATGTGT TATGAAAGTG AAATTTTGTC AAGCAAGCTT 2400

TCTCCAAATG CACAAGATCT GGATCAAGAA GAGCAAGAAA ATTACAATGA TGATAATTCG 2460

AAACAGGCTC CTCTTCGACT TGGTATTGAT ATGCCTTCTG TGGTGATTAC ATCTACATCA 2520

AGTCAATACT TTACCTTATA TGTTATCATA GTGAGCTTGT TGTTTTATAG CGAGCCTATG 2580

AGTAAAGTGA TCCACAAGAA AATCGAAAAG ATGAAGTTTT CTATTGATTT CGAAGATTTG 2640

GGTGCTCTTA CTAGCAGATT AACGAAAATG CAGCAACATC ATAAATTGTT GAAAGTATTG 2700

TCTAANNACN AATNNTTTCC CGNNCGGGGG AATTAA 2736

(2) INFORMATION FOR SEQ ID NO: 19:

(i) SEQUENCE CHARACTERISTICS:
(A) LENGTH: 911 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 19:

```
Met Glu Lys Xaa Leu Ala Ser Val Lys Leu Tyr Thr Asp Leu Glu Cys
1               5                   10                  15

Val Phe Asn Ser Asn Tyr Pro Thr Arg Ile Val Trp Gly Ala Ser Tyr
            20                  25                  30

Asn Phe Gly Ile Gln Gln Met Met Ala Asn Phe Asp Arg Phe Ser Lys
        35                  40                  45

Pro Pro Val Asp Pro Ser Thr Lys Leu Gly Phe Trp Asp Lys Leu Lys
    50                  55                  60

Tyr Ile Leu His Gly Lys Cys Gln Ile Arg Thr Arg Lys Ser Leu Glu
65                  70                  75                  80

Val Ala Phe Lys Gly Ser Arg Asp Pro Tyr Asp Leu Phe Thr Thr Ala
                85                  90                  95

Gly Gly Phe Val Leu Ser Phe Arg Lys Asn Val Val Trp Asp Ile Asn
            100                 105                 110

Lys Asp Asp Asn Ser Lys Asn Tyr Phe Asp Ile Thr Ala Asp Lys Val
        115                 120                 125

Ser Trp Tyr Ile Pro Asn Tyr Leu Ala Gly Pro Leu Leu Ala Trp Thr
    130                 135                 140

Arg Ser Ser Lys Asn Ser Ile Tyr Leu Pro Asn Ser Pro Asn Val Val
145                 150                 155                 160

Asn Ser Cys Phe Ala Tyr Tyr Leu Gln Asp Phe Thr Gly Gln Ala Asp
                165                 170                 175

Phe Asp His Ala Ala Arg Val Phe Glu Arg Asn Val Val Asn Leu Ser
            180                 185                 190
```

```
Gly Gly Ile His Phe Gln Val Gly Phe Leu Leu Glu Arg Lys Asp Thr
        195                 200                 205

Asn Gly Lys Arg Thr Asp Glu Phe Lys Pro His Tyr Glu Val Gln Leu
    210                 215                 220

Phe Asp Pro Lys Tyr Cys Glu Lys Gly His Asp Ser Tyr Ala Gly Phe
225                 230                 235                 240

Arg Ser Gln Phe Ile His Met Ala Ile Ser Leu Glu Ser Thr Asn Ser
                245                 250                 255

Ser Ser Tyr Asn Thr Ile His Leu Ser Pro Gly Thr Phe Gln Gln Phe
            260                 265                 270

Phe Asp Trp Trp Lys Leu Phe Ala Ser Asn Met Gln Leu Pro Ile Arg
        275                 280                 285

Arg Gly Lys Met Phe Gly Glu Ala Lys Glu Ser Val Lys Phe Ser Gln
    290                 295                 300

His Leu Phe Thr Asn Lys Phe Ser Phe Met Leu Lys Ser Leu Phe Ile
305                 310                 315                 320

Ala His Val Tyr Arg Asp Glu Ile Val Asp Ile Asn Asn Asp Arg Ile
                325                 330                 335

Glu Ser Ile Gly Leu Arg Ala Lys Val Asp Asp Phe Met Val Asp Leu
            340                 345                 350

His Gln Arg Lys Glu Pro Ala Thr Leu Tyr His Glu Glu Leu Ser Lys
        355                 360                 365

Asn Glu Lys Val Met Lys Met Asn Phe Asp Leu Gly Glu Val Val Leu
    370                 375                 380

Ser Gly Ile Asp Leu Arg Val Met His Val Ser Phe Leu Gln Asn Leu
385                 390                 395                 400

Tyr Thr Gln Ser His Ser Asn Ser Gly Asp Ala Lys Ser Thr Tyr Asn
                405                 410                 415

Ile Tyr Asp Asn Asp His Arg Trp Phe Asp Ile Met Asp Phe Gln Glu
            420                 425                 430

Ala Phe Leu Thr Ser Ile Lys Asp Cys Val Arg Thr Val Asp Ile Tyr
        435                 440                 445

Pro Leu Met Tyr Leu Gln Arg Phe Phe Tyr Glu Arg Asp Thr His Gly
    450                 455                 460

Gly Lys Ser Glu Asp Glu Thr Ala Phe Gly Lys Glu Val Ile His Lys
465                 470                 475                 480

Cys Asn Leu Gly Ala Met Asn Pro Leu Glu Thr Arg Leu Asn Val Leu
                485                 490                 495

Val Gln Arg Leu Asn Ala Leu Gln Glu Gln Val Lys Lys Leu Ser Lys
            500                 505                 510

Thr Ser Ala Pro Glu Pro Val Ala Asp Leu Lys Lys Arg Ile Ser Phe
        515                 520                 525

Leu Gln Lys Glu Ile Ser Thr Thr Lys Ala Gly Val Lys Ser Lys Met
    530                 535                 540

Arg Arg Thr Ser Thr Ile Asn Gly Met Asn Asn Ser Glu Asn Tyr His
545                 550                 555                 560

Asn Lys Phe Thr Phe Tyr Asn Met Leu Leu Lys Trp Asn Phe Asn Cys
                565                 570                 575
```

```
Arg Asn Leu Thr Leu Lys Tyr Ile His Phe Val Lys Leu Lys Ser Gln
            580                 585                 590

Leu Arg Asn Tyr Leu Ser His Lys Ser Ile Glu Thr Leu Glu Lys Met
        595                 600                 605

Met Asp Ser Val Asn Ala Tyr Asn Asp Lys Asp Asp Leu Ser Ser Thr
    610                 615                 620

Ser Glu Ile Ile Arg Arg Phe Thr Ser Glu Gly Val Lys Ser Gln Thr
625                 630                 635                 640

Ser Thr Ser Lys Asp Ile Thr Ser Gln Gln Lys Leu Asp Asn Phe Asn
                645                 650                 655

Thr Ile Leu Arg Glu Thr Arg Pro Asp Glu Lys Val Val Glu Asp Tyr
            660                 665                 670

Leu Ile Asp Val Ile Ala Pro Gln Ile Gln Leu Gln Ser Glu Asp Tyr
        675                 680                 685

Pro Asp Ser Val Val Leu Ile Ser Thr Pro Ser Ile Lys Gly Lys Ile
    690                 695                 700

Leu Ser Ile Arg Asp Ser Arg Asn Asn Ala Asn Gln Ile Leu Leu Glu
705                 710                 715                 720

Thr Arg Tyr Gly Ile Leu Leu Lys Asp Ala Asn Val Phe Val Leu Asn
                725                 730                 735

Lys Glu Asp Ile Val Gly Cys Pro Asp Met Leu Ser Ile Ser Asn Pro
            740                 745                 750

Tyr Gly Ala Lys Ser Asn Trp Pro Pro Trp Leu Gly Thr Glu Ile Thr
        755                 760                 765

Gln Asn Gly Lys Trp Ala Gly Ala Asn Asn Leu Leu Ile Glu Lys Leu
    770                 775                 780

Ser Val Met Thr Met Cys Tyr Glu Ser Glu Ile Leu Ser Ser Lys Leu
785                 790                 795                 800

Ser Pro Asn Ala Gln Asp Ser Asp Gln Glu Glu Gln Glu Asn Tyr Asn
                805                 810                 815

Asp Asp Asn Ser Lys Gln Ala Pro Leu Arg Leu Gly Ile Asp Met Pro
            820                 825                 830

Ser Val Val Ile Thr Ser Thr Ser Ser Gln Tyr Phe Thr Leu Tyr Val
        835                 840                 845

Ile Ile Val Ser Leu Leu Phe Tyr Ser Glu Pro Met Ser Lys Val Ile
    850                 855                 860

His Lys Lys Ile Glu Lys Met Lys Phe Ser Ile Asp Phe Glu Asp Leu
865                 870                 875                 880

Gly Ala Leu Thr Ser Arg Leu Thr Lys Met Gln Gln His His Lys Leu
                885                 890                 895

Leu Lys Val Leu Ser Xaa Xaa Xaa Xaa Phe Pro Xaa Arg Gly Asn
            900                 905                 910
```

(2) INFORMATION FOR SEQ ID NO: 20:

(i) SEQUENCE CHARACTERISTICS:
(A) LENGTH: 626 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 20:

```
ATTCTTTGTT TGTTTGTTGA TTTTTGATCT CTTGTCTAGA ATCACTCATT AATATTTGAT     60
TCAGGGTTTT GATTTGCTAA ATAAGGGGTC TATTAGGAGG ATATTATATA TAATGTGATG    120
TGGCGAAAAA AAAAAACAAG ATCTACTACT CTGTTGGATT TATTTGTGAT GGCGATTGAA    180
GAGAAAACAC GTCTTTTTAA CGCGTTTTTT TATTTTTTGG AGAAGCAAAT TTCAAGCAAA    240
GACTCTTATT GTGTTGCTTT TGATCCATTC AAATTTTGTA TTACTTTTCA TTAGAACTAT    300
AACTGTTCAT TATCAATGAC GTATACATGT CTGGTTCCTG TTATGTATTG TAATTTTAGT    360
TAATTATAAG CCGTATATTG GTAGTATTCC TCTGTACTCA CAATGGAATT GGTCTTTCAA    420
CAGCAACAAG TGTTATTTTC CCTGAATGTA GAAAATGAAA GGTAGTGTTT ACATATAGTT    480
GGAAATCAAG CCTCTGAAAT GAATCACAAT ATAATAACAA TTTGTAGTTG CAGAGAAAAA    540
CAATTCAAGT TGACGGGTAG TTTTTTTTTT TTCACTGCAT TTTTCAACGA AAACTAAATA    600
AAATTTCGCT GATATTGATA AAGTAT                                         626
```

(2) INFORMATION FOR SEQ ID NO: 21:

(i) SEQUENCE CHARACTERISTICS:
(A) LENGTH: 652 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 21:

```
ATGGCGTCAA TTTCTGTTCC AATTGAAAAA GGATCATTTC ACGATGGAGA TGGATTCAAT     60
CAACATCATT TAGGAGACCC AGTTATTTCA GGACCTCCCT ATATTATTAA ATTATTAAAC    120
TTACCCGTCA CAGCTAATGA TTCATTTGTC CAAGACTTGT TTCAAAGCAG ATTTACCCCA    180
TATGTCAAAT TTAAAATTGT AACAGACCCC GCATCAAATA TTTTGGAGAC TCATGTCATT    240
AGACAAGTGG CTTTTGTGGA ATTGGAATCG GCCAGTGATA TGTCAAAAGC TTTAAAATGG    300
CATGATTTGT ATTATAAGAC AAATAGAAGA GTAACTGTTG AAGTGGCAGA TTTTAATGAT    360
TTTCAAAATT GTATTAAATT CAATCAAGAA CATGAACGTG AAATTATGCA AATCCAACAA    420
GAATTCATTG CTCAGAAACA ACAACAACGG CAACCCAGAC ATATGGCTCT TTTAGATGAA    480
TTTGAAAGAA ACCAGCGCGG TCCTGGATCA CCCTTGCATC AAAACCATGA TCACCACAAT    540
CCCCACCCAC AACAACAACA ACACCATCAT TTCAATCCTA ATTTAAACAG ACCTTCAGGT    600
AGATCAAGTC TTCCAATAGA TGAAACGTCT CATTCAAGAA GACTTTCTTT TG            652
```

(2) INFORMATION FOR SEQ ID NO: 22:

(i) SEQUENCE CHARACTERISTICS:

```
          (A) LENGTH: 217 amino acids
          (B) TYPE: amino acid
          (C) STRANDEDNESS:
          (D) TOPOLOGY: unknown

    (ii) MOLECULE TYPE: peptide

   (iii) HYPOTHETICAL: NO


    (xi) SEQUENCE DESCRIPTION: SEQ ID NO: 22:

     Met Ala Ser Ile Ser Val Pro Ile Glu Lys Gly Ser Phe His Asp Gly
     1               5                   10                  15

     Asp Gly Phe Asn Gln His His Leu Gly Asp Pro Val Ile Ser Gly Pro
                 20                  25                  30

     Pro Tyr Ile Ile Lys Leu Leu Asn Leu Pro Val Thr Ala Asn Asp Ser
             35                  40                  45

     Phe Val Gln Asp Leu Phe Gln Ser Arg Phe Thr Pro Tyr Val Lys Phe
         50                  55                  60

     Lys Ile Val Thr Asp Pro Ala Ser Asn Ile Leu Glu Thr His Val Ile
     65                  70                  75                  80

     Arg Gln Val Ala Phe Val Glu Leu Glu Ser Ala Ser Asp Met Ser Lys
                     85                  90                  95

     Ala Leu Lys Trp His Asp Leu Tyr Tyr Lys Thr Asn Arg Arg Val Thr
                 100                 105                 110

     Val Glu Val Ala Asp Phe Asn Asp Phe Gln Asn Cys Ile Lys Phe Asn
             115                 120                 125

     Gln Glu His Glu Arg Glu Ile Met Gln Ile Gln Gln Glu Phe Ile Ala
         130                 135                 140

     Gln Lys Gln Gln Gln Arg Gln Pro Arg His Met Ala Leu Leu Asp Glu
     145                 150                 155                 160

     Phe Glu Arg Asn Gln Arg Gly Pro Gly Ser Pro Leu His Gln Asn His
                     165                 170                 175

     Asp His His Asn Pro His Pro Gln Gln Gln Gln His His His Phe Asn
                 180                 185                 190

     Pro Asn Leu Asn Arg Pro Ser Gly Arg Ser Ser Leu Pro Ile Asp Glu
             195                 200                 205

     Thr Ser His Ser Arg Arg Leu Ser Phe
         210                 215

(2) INFORMATION FOR SEQ ID NO: 23:

     (i) SEQUENCE CHARACTERISTICS:
          (A) LENGTH: 1513 base pairs
          (B) TYPE: nucleic acid
          (C) STRANDEDNESS: single
          (D) TOPOLOGY: linear

    (ii) MOLECULE TYPE: cDNA

   (iii) HYPOTHETICAL: NO

    (ix) FEATURE:
          (A) NAME/KEY: misc_feature
          (B) LOCATION:1492
```

(D) OTHER INFORMATION:/note= "N = A or G or C or T"

(xi) SEQUENCE DESCRIPTION: SEQ ID NO: 23:

```
GTAGTTTGTG AAGAAATTGA AACAATCGGA AAACAACAAT ATCAAACTGA TGCCCAATAA     60
CACTGTATGT ACCTAGATGG ATTACCAAGA TCTACTACAT AAAATAATAA AGGAGTTCCA    120
CTCACTCAAA GAGTTCAAAC CATGGGATAG CAGTGTTTTG TATGAGACGT TACTACGATC    180
AGTATTAACT ACTTTGATCG AACTTTTGGG CATAGACAAT CCACCCAGTT ATCTACACCT    240
CACCACCAAC AATGATAGTA TAGGTGATTT GAAAATAAAA TACTATGGAA ATGCATTAAG    300
CAAGTCAATC AACGGTCATA GCATGTTGCA ATATCTTGAA TCAAAGCATG TATCGATATT    360
ACAGGCCGTG GTTGAGATTA TTAATACGCG ATCATATAGA ATCAAAGAGT CTTATTCTGC    420
TGTTTTCAAA GACGTTTCTC ATTTATTTGA AAAACTACTA AAGGAAAGAT ATGAAGCTGA    480
ATCTAATCTA GAGGATTATA TATTGCAGTG CTTGATGTAC GAGACCCAAT TTTACCAAGG    540
AATTGTTGAT AATGTTTTAA CTGCCGATGA CACCGAAAAA TTGGCTAGTT TTTTGGGGAC    600
ACGACTATCT GAAGAAGATT CGATGTTTAG CTATAGGGAT ATAGATTATC CACTAGAGTT    660
AAACATTAAT AATGAATCTC TTGAAAAGAT ATATAAAATT TTCTTAGGAG TCATTGGCAC    720
CAAAAGATTC GATATCAAGG AGGTTGCGTC TGCTGTTGTT GGTGTGTATA AACGACACCA    780
GAGAATAGAT CATTTTGAAA AGTTGGATTC AGATGAGATT TTGGGAAAGT TTTTCAGAAA    840
TATATTGCCA CAACTGTTCC AGAGTGTGAC AAATAAGGTT TTCCGGGAAT TTCACAAAGA    900
GGTAGATGAC CCACCATCGG ACGTGCTAGA CCAGCTAGAT AATATTGTTG ATGACTTTAT    960
TGCGGTTGGA ATTGAAGGGG TAGATTTGGG CTTTCCGGCT TTGTTCAGAC ACTACATAAA   1020
ATTCATGAAC GAAATTTTTC CCACTGTGGT CGAGGATGCT GACCGCGATT TTGTTGCAAG   1080
AATTAATAGT TTAATTGCTC AAGTCTTGGA GTTTAAAGAC GATGAAAAAT CCTGTGATAT   1140
CAATCAAGTG GTATCTGAAT TTGTTTCATT ACAAAGTTTG CTACTTAAGA ATAACTATCT   1200
TTCACCATCT ACATTATTGA TGCGTGCAAG TACTCACGAT TACTATAAAA ATTTACAGAT   1260
CGTGAAAATA ACCTTTGATG GATGGAATGA GAATTCAAAG AGGATATTGA AATTGGAGAA   1320
CAGCGGCTTT TTACAAAGCA AGACATTGCC AAAGTATTTA AAATTATGGT ACTCAAAAAG   1380
TATGAAGTTG AATGAATTAT GTAACCGGGT AGATGAATTT TATAATGGAG AACTTTGTCG   1440
GAAAGTTTTG GGCATTGTTG GGAGGGTCAC AACCAAAATG TCTATAAATC CNCAAAAATG   1500
GGAGGGTTGC TGA                                                      1513
```

(2) INFORMATION FOR SEQ ID NO: 24:

(i) SEQUENCE CHARACTERISTICS:
(A) LENGTH: 478 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 24:

```
Met Asp Tyr Gln Asp Leu Leu His Lys Ile Ile Lys Glu Phe His Ser
1               5                   10                  15

Leu Lys Glu Phe Lys Pro Trp Asp Ser Ser Val Leu Tyr Glu Thr Leu
            20                  25                  30

Leu Arg Ser Val Leu Thr Thr Leu Ile Glu Leu Leu Gly Ile Asp Asn
        35                  40                  45

Pro Pro Ser Tyr Leu His Leu Thr Thr Asn Asn Asp Ser Ile Gly Asp
    50                  55                  60

Leu Lys Ile Lys Tyr Tyr Gly Asn Ala Leu Ser Lys Ser Ile Asn Gly
65                  70                  75                  80

His Ser Met Leu Gln Tyr Leu Glu Ser Lys His Val Ser Ile Leu Gln
                85                  90                  95

Ala Val Val Glu Ile Ile Asn Thr Arg Ser Tyr Arg Ile Lys Glu Ser
            100                 105                 110

Tyr Ser Ala Val Phe Lys Asp Val Ser His Leu Phe Glu Lys Leu Leu
        115                 120                 125

Lys Glu Arg Tyr Glu Ala Glu Ser Asn Leu Glu Asp Tyr Ile Leu Gln
    130                 135                 140

Cys Leu Met Tyr Glu Thr Gln Phe Tyr Gln Gly Ile Val Asp Asn Val
145                 150                 155                 160

Leu Thr Ala Asp Asp Thr Glu Lys Leu Ala Ser Phe Leu Gly Thr Arg
                165                 170                 175

Leu Ser Glu Glu Asp Ser Met Phe Ser Tyr Arg Asp Ile Asp Tyr Pro
            180                 185                 190

Leu Glu Leu Asn Ile Asn Asn Glu Ser Leu Glu Lys Ile Tyr Lys Ile
        195                 200                 205

Phe Leu Gly Val Ile Gly Thr Lys Arg Phe Asp Ile Lys Glu Val Ala
    210                 215                 220

Ser Ala Val Val Gly Val Tyr Lys Arg His Gln Arg Ile Asp His Phe
225                 230                 235                 240

Glu Lys Leu Asp Ser Asp Glu Ile Leu Gly Lys Phe Phe Arg Asn Ile
                245                 250                 255

Leu Pro Gln Ser Phe Gln Ser Val Thr Asn Lys Val Phe Arg Glu Phe
            260                 265                 270

His Lys Glu Val Asp Asp Pro Pro Ser Asp Val Leu Asp Gln Leu Asp
        275                 280                 285

Asn Ile Val Asp Asp Phe Ile Ala Val Gly Ile Glu Gly Val Asp Leu
    290                 295                 300

Gly Phe Pro Ala Leu Phe Arg His Tyr Ile Lys Phe Met Asn Glu Ile
305                 310                 315                 320

Phe Pro Thr Val Val Glu Asp Ala Asp Arg Asp Phe Val Ala Arg Ile
                325                 330                 335

Asn Ser Leu Ile Ala Gln Val Leu Glu Phe Lys Asp Asp Glu Lys Ser
            340                 345                 350

Cys Asp Ile Asn Gln Val Val Ser Glu Phe Val Ser Leu Gln Ser Leu
        355                 360                 365

Leu Leu Lys Asn Asn Tyr Leu Ser Pro Ser Thr Leu Leu Met Arg Ala
```

```
370                 375                 380

Ser Thr His Asp Tyr Tyr Lys Asn Leu Gln Ile Val Lys Ile Thr Phe
385                 390                 395                 400

Asp Gly Trp Asn Glu Asn Ser Lys Arg Ile Leu Lys Leu Glu Asn Ser
                405                 410                 415

Gly Phe Leu Gln Ser Lys Thr Leu Pro Lys Tyr Leu Lys Leu Trp Tyr
            420                 425                 430

Ser Lys Ser Met Lys Leu Asn Glu Leu Cys Asn Arg Val Asp Glu Phe
        435                 440                 445

Tyr Asn Gly Glu Leu Cys Arg Lys Val Leu Gly Ile Val Gly Arg Val
    450                 455                 460

Thr Thr Lys Met Ser Ile Asn Xaa Gln Lys Trp Glu Gly Cys
465                 470                 475
```

(2) INFORMATION FOR SEQ ID NO: 25:

(i) SEQUENCE CHARACTERISTICS:
(A) LENGTH: 436 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 25:

```
AGTTATGTCT CATACATACA ACACAGATGA GGACATGTGT TTAAATGATA AATTGAAATA      60

TTTGTACGAT TTATAATCGC TTTATCGTGA CAATTTCGAA TACTGGTACT TTCTACTCTA     120

TTTGACAAAA ATTTGCAAAA AATTGGGGAA AAAAATCCTG TTGCATTTTC GAGACCATCA     180

GTTGCAACCA ATCTGAATAT ATTTTGACAC TTCAATAAAT CTAGTGAAAC TAGTCGTCTA     240

CTTTTTAATT CTAATCATCT CATAGTATAT CAAGCAAAGA CTTACTATGC GTTTATCAAA     300

TTTAAGAAAA TGTAGACAGT ACGAAAATAC ACGAGTTTCC CAATCTTTGA ACTTGAAAAG     360

ATAGTAATAC CGAGATTGGC CAAATCCTAG CCATAGTCCG TTCATACAAA TTCATGAACA     420

ACATCTACAT AAGTAA                                                     436
```

(2) INFORMATION FOR SEQ ID NO: 26:

(i) SEQUENCE CHARACTERISTICS:
(A) LENGTH: 717 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 26:

```
CTTTCTTTCG AATTAGATTC AATCTTTTCC AATTTTGCTT GTACACTTGC TAGTTTGAAT      60
```

TTACGTTTTT CCTCTTTACG TTGTTTCACA ATGGCTGCAC GTTCTTCAAA ATTTATTCCC 120

TTCTTCTTGG TTGGTCTTAT ATCGTTCTCA TCTTCAGGCT TCCTCTCCTC TTGTAACCCT 180

TCTTTTTCTA ATAGTTTGAA ATAGTTCTTT CTTAATCTAG CCCTATGGGT TAATGCACGT 240

TTTATATCTT GAGACTTGGC TTCTCGACGA TCTATAAATT TCTTTTTTGA TTTAAATGAA 300

TTTTTATTAT TTGGATGCAT TGTTGTGGAG GTGTATTTGA TAGGTTGATA ACTAGAAATA 360

AAAACTATGT GAAAGAACAA AATGCCAATC ACTAAAAAAA ATTTAAGATG AGTATGAAAT 420

CAAAACTTTA CGACATCTTT GCGACATGCA CATTATGAGC GACATTTTGA TTCGATACCA 480

GAAATAGACA GATTTAGACA GGGTCTATAA CAGAGAAATC AACAATTAAC TGGTATCAAC 540

CTTAAGATTA AAAATGGTCT ATGGCGATAT GAACTGTTGT GATGAAAAAC AATATATTTG 600

GAAATACTTC TTTTCATTTG ACAATTTTTT ATAAAATTTT GGCAACAATT TTGTACCTAA 660

AAATTCTTTT GTCTTCAAAA GTGAAATGTA ATATAGAAAT ACTATTACAA CCAAACA 717

(2) INFORMATION FOR SEQ ID NO: 27:

(i) SEQUENCE CHARACTERISTICS:
(A) LENGTH: 667 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 27:

TTTAGTTTTA TATTGATGAT GTTTTTAAGT GCTTGTTTAT CATGGTGGAT GGAAATTAGA 60

ATGAGTAAAT TGAATGGAAA ATCACTGCAA CACCAACAAC AACCACTGGT GGATACGAAA 120

ATTTAGTGTA CAAATTTCTG CCAAAAAAAT ACAATAAAAA CCGCTTATAG TCTTCTACTG 180

ACATAACAAC ACAAGTCAAT AAATCAACAA CTCATAAACA ATGTAGACTT AATACTATCG 240

CTTAATTATT TAAACTATAA TAAATACCCT ATAGTATTAT GCCTTTGTCA ATGTGTGTAG 300

AATTTGGTTA TTACATATCC ATGTGTAATA TATATGTTGA TCAAAAAACG CGATCTTCTC 360

TTTGGTGTAG TGTGTTACAC AAAAAATTCA CTAGTCTAGG TCACATGATA ATCACGTGAA 420

AATCAAAAAT TTGTTGAAAT TGAATTTCCT CAATTTTGAA ATTTTGTTTG AAATTTTTTT 480

TTTGCTTTAC AAAAAGACTC CATTTTGTTT TCCATTTCAC AACCAATTAC TTAATTCCTC 540

TTTTTCATAA TTAATAACTA TCATTACTTA CAACTACAAA CAACTACGAT CATTTCCTAA 600

GAAAAAGCAA CGAGGGCGAA TTGAGACATT AATCCCCTTT ATTTTATCAT CATGCCTTAT 660

ACAGAAC 667

(2) INFORMATION FOR SEQ ID NO: 28:

(i) SEQUENCE CHARACTERISTICS:
(A) LENGTH: 165 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 28:

AACTATTGCC AATGGTAAAT ATGCCAGTGA AATCGAGAAT TTTAATAAGT CGGTCCCTCT 60

TAAGGTCCCA TTCAAATTCA CTAATGCACA ATTGGATCTT TATGCTGCTA GCACACATAA 120

CCAAGAGCCA ATATCCTAGT AACGACGCAC CATAGTAGAC CGAAT 165

(2) INFORMATION FOR SEQ ID NO: 29:

(i) SEQUENCE CHARACTERISTICS:
(A) LENGTH: 207 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(ix) FEATURE:
(A) NAME/KEY: misc_feature
(B) LOCATION:120
(D) OTHER INFORMATION:/note= "N = A or C or G or T"

(ix) FEATURE:
(A) NAME/KEY: misc_feature
(B) LOCATION:129
(D) OTHER INFORMATION:/note= "N = A or T or C or G"

(ix) FEATURE:
(A) NAME/KEY: misc_feature
(B) LOCATION:162
(D) OTHER INFORMATION:/note= "N = A or T or C or G"

(ix) FEATURE:
(A) NAME/KEY: misc_feature
(B) LOCATION:178
(D) OTHER INFORMATION:/note= "N = A or T or C or G"

(ix) FEATURE:
(A) NAME/KEY: misc_feature
(B) LOCATION:194
(D) OTHER INFORMATION:/note= "N = A or T or C or G"

(ix) FEATURE:
(A) NAME/KEY: misc_feature
(B) LOCATION:195
(D) OTHER INFORMATION:/note= "N = A or T or C or G"

(ix) FEATURE:
(A) NAME/KEY: misc_feature
(B) LOCATION:199
(D) OTHER INFORMATION:/note= "N = A or T or C or G"

(ix) FEATURE:
(A) NAME/KEY: misc_feature
(B) LOCATION:203
(D) OTHER INFORMATION:/note= "N = A or T or C or G"

(xi) SEQUENCE DESCRIPTION: SEQ ID NO: 29:

ATGAAGATTT CACCAGAGAC AGTAAATAAA CTACAACTGG ATGCATCGTG TATAAGAAAC 60

```
ATCTGTATTT TAGCACATGT CGACCACGGT AAAACCTCAT TGAGTGACTC ATTATTAGCN     120
ACCAATGGNA TCATTTCCCA ACGTATGGCA GGTAAAGTTA GNTATCTTGA TTCGAGANGA     180
GATGAACAAT TGANNGGTNT AANCATG                                         207
```

(2) INFORMATION FOR SEQ ID NO: 30:

(i) SEQUENCE CHARACTERISTICS:
(A) LENGTH: 69 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 30:

```
Met Lys Ile Ser Pro Glu Thr Val Asn Lys Leu Gln Ser Asp Ala Ser
1               5                   10                  15

Cys Ile Arg Asn Ile Cys Ile Leu Ala His Val Asp His Gly Lys Thr
            20                  25                  30

Ser Leu Ser Asp Ser Leu Leu Xaa Thr Asn Xaa Ile Ile Ser Gln Arg
        35                  40                  45

Met Ala Gly Lys Val Xaa Tyr Leu Asp Ser Arg Xaa Asp Glu Gln Leu
    50                  55                  60

Xaa Gly Xaa Xaa Met
65
```

(2) INFORMATION FOR SEQ ID NO: 31:

(i) SEQUENCE CHARACTERISTICS:
(A) LENGTH: 2510 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(ix) FEATURE:
(A) NAME/KEY: misc_feature
(B) LOCATION:2481
(D) OTHER INFORMATION:/note= "N = A or T or C or G"

(xi) SEQUENCE DESCRIPTION: SEQ ID NO: 31:

```
AAGTCATGCG ATTGCAACAA GGATCACAAG AACCAGAAGT TCACGAACAT TTGATTAATT      60
TGATTGATTC ACCTGGGCAT ATTGACTTTT CGTCTGAAGT GAGTACTTCT TCGAGATTAT     120
GTGATGGTGC AGTTGTTTTG GTCGATGTCG TCGAAGGTGT CTGCTCACAA ACAGTCAACG     180
TTCTACGCCA ATGTTGGATT GATAAGTTGA AGCCATTACT AGTTATTAAC AAAATTGATA     240
GGTTAATCAC AGAATGGAAA TTGTCTCCCT TGGAGGCATA CCAACACATT TCCAGAATTA     300
TAGAACAAGT AAACTCTGTG ATTGGGTCAT TTTTTGCTGG TGATAGACTA GAAGATGACT     360
TGAATTGGCG TGAGGCTGGT TCTGTCGGGG AGTTTATCGA GAAGAGTGAT GAAGACTTGT     420
```

ATTTCACACC TGAAAAGAAT AATGTAATAT TTGCCTCGGC AATAGATGGA TGGGCATTTT 480
CAGTCAATAC ATTTGCCAAA ATATACCTGA AAAAATTAGG GTTCTCTCAA CAAGCATTGT 540
CAAAAACTCT CTGGGGAGAC TTTTACTTGG ATATGAAAAA TAAAAAAATC ATCCCTGGTA 600
AAAAATTGAA AAATAATAGT AACAGTTTGA AGCCATTATT TGTTTCGTTG ATTTTGGACC 660
AGGTTTGGGC TGTTTATGAA AACTGTGTTA TTGAAAGAAA TCAAGACAAG TTGGAAAAAA 720
TCATTGAGAA ATTAGGGGCC AAAATCACCC CTCGTGATTT GCGATCCAAA GATTACAAGA 780
ACTTGCTAAA CTTGATTATG TCTCAGTGGA TTCCTTTGAG TCATGCCATA TTGGGGTCAG 840
TGATTGAATA CTTGCCAAGC CCCATTGTTG CTCAGCGTGA AAGAATAGAC AAGATTTTGG 900
ATGAAACGAT TTATAGTGCA GTGGATTCAG AACTGAGATA AATCCAAACT AGTCGACCCT 960
TCATTTGTCA AGGCGATGCA GGAATGTGAT AGTTCACACC CGGAAACCCA TACAATAGCA 1020
TATGTATCAA AATTGTTGTC AATCCCCAAT GAAGACTTAC CCAAAGCTAG TAATGCCGCT 1080
ACTGGAGGAT TGACGGCCGA TGAAATCCAA GAACGAGGAA GAATTGCTCG AGAATTAGCC 1140
AAAAAGGCAT CTGAAGCAGC TGCTTTGGCA CAAGAAGGTT CCAAAAATGA AGATGAGTTT 1200
GCCATTAAAC CCAAGAAAGA TCCATTTGAA TGGGAATTTG AGGAGGACGA TTTTGAGAAT 1260
GAGGAAGATG AGAGCGATGC AAACGCAGTT GAAGAATCAA CTGAAACCAT AGTGGGTTTC 1320
ACTCGTATTT ATTCTGGATC GTTATCTAGA GGCCAAAAGC TCACGGTAAT TGGACCCAAA 1380
TACGACCCTT CATTACCTAG AGACCATCAA ACCAACTTTG AACAAATAAC CAATGAAGTT 1440
GAAATTAAAG ACTTGTTTTT AATCATGGGA CGAGAATTAG TGAGAATGGA AAAAGTCCTG 1500
CGGGTAATAT TGTTGGGGTT GTTGGATTGG ATACGCCGTG CTTAAGAATG CCACAATTTG 1560
CTCACCGTTA CCTGAAGATA AACCATACAT TAATTTAGCT TCAACATCAA CCTTGATCCA 1620
CAATAAACCA ATTATGAAAA TAGCAGTTGA ACCAACAAAC CCAATAAAAC TAGCAAAATT 1680
GGAACGAGGA TTAGATTTAT TGGCCAAAGC CGACCCGGTT TTGGAATGGT ATGTCGACGA 1740
CGAGTCAGGT GAATTGATTG TTTGTGTTGC TGGAGAATTG CATCTAGAAC GATGCTTGAA 1800
AGATTTAGAA GAGAGATTCG CTAAGGGTTG TGAAGTTACC GTCAAAGAGC CAGTCATTCC 1860
CTTCAGAGAG GGGTTGGCAG ATGACAAAAT CAGTACCAAC ACCAATAATA ACAACGACGA 1920
CAATGAAGAT CATGAATTAG ATGAAAACGA AGATGAGCTT GCTGATTTAG AGTTTGATAT 1980
TTCTCCGTTG CCATTAGAAG TGACTCAGTT TTTAATTGAG AATGAAACGA TTATTGCCGA 2040
AATTGTCAAC AACAAGCAAG ATACTCATGA AATTAGAAAC GATTTTATTG AAAAATTTGC 2100
CACTATTATT GATAATTCTA ATTTGGCTAC ACAATTTCCA GACACCAAGT CTTTTATCAA 2160
CAATATAATT TGCTTTGGAC CTAAACGTGT TGGGCCTAAT ATTTTCATTG AAGATTATGG 2220
GTTAAACAAA TTTAGACATC TACTTGGTGA ATCTGCCACT GAATCTCGAT TTGTTTATGA 2280
GAATAATGTG TTCAATGGGG TTCAATTGGT ATTCAATGGG GGTCCGTTAG CATCAGAGCC 2340
AATGCAAGGT ATTATTGTTA GACTTAAGAA GGCAGAAAAA AGAGAAGTTG ACGAGGATAA 2400
GATAGTCAAC CCTGGTAAAA TAATCACACA GACTCGTGAC TTGATTTACA AGCGGTTTTT 2460
GCAAAAATCA CCACGCTTGT NCCTTGCAAT GTATACGTGT GAAATCCAAG 2510

(2) INFORMATION FOR SEQ ID NO: 32:

(i) SEQUENCE CHARACTERISTICS:
(A) LENGTH: 310 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 32:

```
Val Met Arg Leu Gln Gln Gly Ser Gln Glu Pro Glu Val His Glu His
1               5                   10                  15

Leu Ile Asn Leu Ile Asp Ser Pro Gly His Ile Asp Phe Ser Ser Glu
            20                  25                  30

Val Ser Thr Ser Ser Arg Leu Cys Asp Gly Ala Val Val Leu Val Asp
        35                  40                  45

Val Val Glu Gly Val Cys Ser Gln Thr Val Asn Val Leu Arg Gln Cys
    50                  55                  60

Trp Ile Asp Lys Leu Lys Pro Leu Leu Val Ile Asn Lys Ile Asp Arg
65                  70                  75                  80

Leu Ile Thr Glu Trp Lys Leu Ser Pro Leu Glu Ala Tyr Gln His Ile
                85                  90                  95

Ser Arg Ile Ile Glu Gln Val Asn Ser Val Ile Gly Ser Phe Phe Ala
            100                 105                 110

Gly Asp Arg Leu Glu Asp Asp Leu Asn Trp Arg Glu Ala Gly Ser Val
        115                 120                 125

Gly Glu Phe Ile Glu Lys Ser Asp Glu Asp Leu Tyr Phe Thr Pro Glu
    130                 135                 140

Lys Asn Asn Val Ile Phe Ala Ser Ala Ile Asp Gly Trp Ala Phe Ser
145                 150                 155                 160

Val Asn Thr Phe Ala Lys Ile Tyr Ser Lys Lys Leu Gly Phe Ser Gln
                165                 170                 175

Gln Ala Leu Ser Lys Thr Leu Trp Gly Asp Phe Tyr Leu Asp Met Lys
            180                 185                 190

Asn Lys Lys Ile Ile Pro Gly Lys Lys Leu Lys Asn Asn Ser Asn Ser
        195                 200                 205

Leu Lys Pro Leu Phe Val Ser Leu Ile Leu Asp Gln Val Trp Ala Val
    210                 215                 220

Tyr Glu Asn Cys Val Ile Glu Arg Asn Gln Asp Lys Leu Glu Lys Ile
225                 230                 235                 240

Ile Glu Lys Leu Gly Ala Lys Ile Thr Pro Arg Asp Leu Arg Ser Lys
                245                 250                 255

Asp Tyr Lys Asn Leu Leu Asn Leu Ile Met Ser Gln Trp Ile Pro Leu
            260                 265                 270

Ser His Ala Ile Leu Gly Ser Val Ile Glu Tyr Leu Pro Ser Pro Ile
        275                 280                 285

Val Ala Gln Arg Glu Arg Ile Asp Lys Ile Leu Asp Glu Thr Ile Tyr
    290                 295                 300
```

```
Ser Ala Val Asp Ser Glu
305                 310
```

(2) INFORMATION FOR SEQ ID NO: 33:

(i) SEQUENCE CHARACTERISTICS:
(A) LENGTH: 188 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 33:

```
Asp Lys Ser Lys Leu Val Asp Pro Ser Phe Val Lys Ala Met Gln Glu
1               5                   10                  15

Cys Asp Ser Ser His Pro Glu Thr His Thr Ile Ala Tyr Val Ser Lys
            20                  25                  30

Leu Leu Ser Ile Pro Asn Glu Asp Leu Pro Lys Ala Ser Asn Ala Ala
        35                  40                  45

Thr Gly Gly Leu Thr Ala Asp Glu Ile Gln Glu Arg Gly Arg Ile Ala
    50                  55                  60

Arg Glu Leu Ala Lys Lys Ala Ser Glu Ala Ala Ala Leu Ala Gln Glu
65                  70                  75                  80

Gly Ser Lys Asn Glu Asp Glu Phe Ala Ile Lys Pro Lys Lys Asp Pro
                85                  90                  95

Phe Glu Trp Glu Phe Glu Glu Asp Asp Phe Glu Asn Glu Glu Asp Glu
            100                 105                 110

Ser Asp Ala Asn Ala Val Glu Glu Ser Thr Glu Thr Ile Val Gly Phe
        115                 120                 125

Thr Arg Ile Tyr Ser Gly Ser Leu Ser Arg Gly Gln Lys Leu Thr Val
    130                 135                 140

Ile Gly Pro Lys Tyr Asp Pro Ser Leu Pro Arg Asp His Gln Thr Asn
145                 150                 155                 160

Phe Glu Gln Ile Thr Asn Glu Val Glu Ile Lys Asp Leu Phe Leu Ile
                165                 170                 175

Met Gly Arg Glu Leu Val Arg Met Glu Lys Val Ser
            180                 185
```

(2) INFORMATION FOR SEQ ID NO: 34:

(i) SEQUENCE CHARACTERISTICS:
(A) LENGTH: 336 amino acids
(B) TYPE: amino acid
(C) STRANDEDNESS:
(D) TOPOLOGY: unknown

(ii) MOLECULE TYPE: cDNA

(iii) HYPOTHETICAL: NO

(xi) SEQUENCE DESCRIPTION: SEQ ID NO: 34:

```
Gly Asn Ile Val Gly Val Val Gly Leu Asp Xaa Ala Val Leu Lys Asn
1               5                   10                  15

Ala Thr Ile Cys Ser Pro Leu Pro Glu Asp Lys Pro Tyr Ile Asn Leu
            20                  25                  30

Ala Ser Thr Ser Thr Leu Ile His Asn Lys Pro Ile Met Lys Ile Ala
        35                  40                  45

Val Glu Pro Thr Asn Pro Ile Lys Leu Ala Lys Leu Glu Arg Gly Leu
    50                  55                  60

Asp Leu Leu Ala Lys Ala Asp Pro Val Leu Glu Trp Tyr Val Asp Asp
65                  70                  75                  80

Glu Ser Gly Glu Leu Ile Val Cys Val Ala Gly Glu Leu His Leu Glu
                85                  90                  95

Arg Cys Leu Lys Asp Leu Glu Glu Arg Phe Ala Lys Gly Cys Glu Val
            100                 105                 110

Thr Val Lys Glu Pro Val Ile Pro Phe Arg Glu Gly Leu Ala Asp Asp
        115                 120                 125

Lys Ile Ser Thr Asn Thr Asn Asn Asn Asn Asp Asp Asn Glu Asp His
    130                 135                 140

Glu Leu Asp Glu Asn Glu Asp Glu Leu Ala Asp Leu Glu Phe Asp Ile
145                 150                 155                 160

Ser Pro Leu Pro Leu Glu Val Thr Gln Phe Leu Ile Glu Asn Glu Thr
                165                 170                 175

Ile Ile Ala Glu Ile Val Asn Asn Lys Gln Asp Thr His Glu Ile Arg
            180                 185                 190

Asn Asp Phe Ile Glu Lys Phe Ala Thr Ile Ile Asp Asn Ser Asn Leu
        195                 200                 205

Ala Thr Gln Phe Pro Asp Thr Lys Ser Phe Ile Asn Asn Ile Ile Cys
    210                 215                 220

Phe Gly Pro Lys Arg Val Gly Pro Asn Ile Phe Ile Glu Asp Tyr Gly
225                 230                 235                 240

Leu Asn Lys Phe Arg His Leu Leu Gly Glu Ser Ala Thr Glu Ser Arg
                245                 250                 255

Phe Val Tyr Glu Asn Asn Val Phe Asn Gly Val Gln Leu Val Phe Asn
            260                 265                 270

Gly Gly Pro Leu Ala Ser Glu Pro Met Gln Gly Ile Ile Val Arg Leu
        275                 280                 285

Lys Lys Ala Glu Lys Arg Glu Val Asp Glu Asp Lys Ile Val Asn Pro
    290                 295                 300

Gly Lys Ile Ile Thr Gln Thr Arg Asp Leu Ile Tyr Lys Arg Phe Leu
305                 310                 315                 320

Gln Lys Ser Pro Arg Leu Xaa Leu Ala Met Tyr Thr Cys Glu Ile Gln
                325                 330                 335
```

(2) INFORMATION FOR SEQ ID NO: 35:

(i) SEQUENCE CHARACTERISTICS:
- (A) LENGTH: 841 base pairs
- (B) TYPE: nucleic acid
- (C) STRANDEDNESS: single

(D) TOPOLOGY: linear

(ii) MOLECULE TYPE: cDNA

(iii) HYPOTHETICAL: NO

(ix) FEATURE:
(A) NAME/KEY: misc_feature
(B) LOCATION:8
(D) OTHER INFORMATION:/note= "N = A or T or C or G"

(ix) FEATURE:
(A) NAME/KEY: misc_feature
(B) LOCATION:9
(D) OTHER INFORMATION:/note= "N = A or T or G or C"

(ix) FEATURE:
(A) NAME/KEY: misc_feature
(B) LOCATION:18
(D) OTHER INFORMATION:/note= "N = A or T or C or G"

(xi) SEQUENCE DESCRIPTION: SEQ ID NO: 35:

```
CGCGAAGNNT CAATCATNTC AGAAGAAATG AAAGAAGGTA CTCCGTTCTT TACTATTGTG      60
GCAAGAATCC CTGTGATTGA GGCATTTGGG TTTTCCGAGG ATATTAGAAA GAAGACATCC     120
GGGGCAGCTA GTCCTCAATT AGTTTTTGAT GGGTATGATA TGTTAGATAT CGATCCATTT     180
TGGGTTCCAC ATACTGAAGA AGAATTAGAA GAATTGGGTG AATTTGCAGA AAGAGAAAAT     240
GTTGCTAGAA GATATATGAA TAATATCAGA AGAAGAAAAG GGTTATTTGT TGATGAGAAA     300
GTCGTCAAAA ATGCTGAAAA GCAAAGAACT TTGAAAAGAG ATTAGATTAT CCAGTAAAAC     360
AGGCAATATG TGTGAAATTG TTACAGAAAA GACAGATACG ATGTGGCCAT TATTTGTTTA     420
ATATTCAACA ACAAGTAAAT GTATTGATAT AGATGTATAA TATAGTCAAA TGTTGAGACT     480
ATCCGAATAG ACATAGACAC ACAACTCAGC CTGTCAGGGC TGTTTATTAA GTTGTGATGT     540
ATACTAAAAT CCATCCACAC TTCTCGTAAT TGTAGGGAAG AATTACAAAA AAGATCACAT     600
AAAAATAATA ATTCTATCAC ACTTTGAAAA TTTGATTGAA GGTGTTACTA GTATTGTTTC     660
AACATTACTC TTTTCAAACA ACGAGATCCA AATACTGCAC AATCTTCAAA CGAACGGAGT     720
TACATCACTA TAGTTTTCTA TTGTTGTAAG ATCAATACAG ACAAAAAGAA AGTGTAGCAT     780
AAATAATTGA TTGCAATTTG CCAAACTAGA AAACAAAGAG GAAAAAAAGA AAAAAATTTC     840
A                                                                     841
```

(2) INFORMATION FOR SEQ ID NO: 36:

(i) SEQUENCE CHARACTERISTICS:
(A) LENGTH: 114 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 36:

```
Arg Glu Xaa Ser Ile Xaa Ser Glu Glu Met Lys Glu Gly Thr Pro Phe
1               5                   10                  15

Phe Thr Ile Val Ala Arg Ile Pro Val Ile Glu Ala Phe Gly Phe Ser
            20                  25                  30

Glu Asp Ile Arg Lys Lys Thr Ser Gly Ala Ala Ser Pro Gln Leu Val
        35                  40                  45

Phe Asp Gly Tyr Asp Met Leu Asp Ile Asp Pro Phe Trp Val Pro His
    50                  55                  60

Thr Glu Glu Glu Leu Glu Glu Leu Gly Glu Phe Ala Glu Arg Glu Asn
65                  70                  75                  80

Val Ala Arg Arg Tyr Met Asn Asn Ile Arg Arg Arg Lys Gly Leu Phe
                85                  90                  95

Val Asp Glu Lys Val Val Lys Asn Ala Glu Lys Gln Arg Thr Leu Lys
            100                 105                 110

Arg Asp
```

(2) INFORMATION FOR SEQ ID NO: 37:

(i) SEQUENCE CHARACTERISTICS:
(A) LENGTH: 564 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 37:

```
AACCTAAAAA TGGCTAAGTT CATCAAATCT GGTAAAGTTG CTATTGTTGT AAGAGGTCGT      60
TACGCTGGTA AAAAAGTAGT CATTGTGAAA CCACATGATG AAGGTACCAA ATCTCACCCA     120
TTCCCACATG CCATTGTCGC TGGTATTGAA AGAGCTCCAT TGAAGGTTAC CAAGAAGATG     180
GATGCTAAAA AAGTTACCAA AAGAACTAAA GTCAAGCCAT TTGTTAAATT AGTAAACTAC     240
AACCATTTAA TGCCAACTAG ATACTCATTG GATGTTGAAT CATTCAAATC TGCTGTCACT     300
TCTGAAGCTT TAGAAGAACC ATCTCAAAGA GAAGAAGCTA AAAAAGTTGT CAAGAAGGCT     360
TTTGAAGAAA AACATCAAGC TGGTAAGAAC AAATGGTTCT TCCAAAAATT ACACTTTTAA     420
GAAAGGAACC ACCTTTATTT GAATGTTTGT AATATAGGTT GAATCAGAGA GACAAAGTAG     480
AAGAAAATAC AAAAAAGAGA GTATATCTGT ATAGTATAAT TTAATGGGGG TCTAATTTAC     540
TTACCACTTT ATTCGTGCAT TATT                                            564
```

(2) INFORMATION FOR SEQ ID NO: 38:

(i) SEQUENCE CHARACTERISTICS:
(A) LENGTH: 136 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 38:

```
Met Ala Lys Phe Ile Lys Ser Gly Lys Val Ala Ile Val Val Arg Gly
1               5                   10                  15

Arg Tyr Ala Gly Lys Lys Val Val Ile Val Lys Pro His Asp Glu Gly
            20                  25                  30

Thr Lys Ser His Pro Phe Pro His Ala Ile Val Ala Gly Ile Glu Arg
        35                  40                  45

Ala Pro Leu Lys Val Thr Lys Lys Met Asp Ala Lys Lys Val Thr Lys
    50                  55                  60

Arg Thr Lys Val Lys Pro Phe Val Lys Leu Val Asn Tyr Asn His Leu
65                  70                  75                  80

Met Pro Thr Arg Tyr Ser Leu Asp Val Glu Ser Phe Lys Ser Ala Val
                85                  90                  95

Thr Ser Glu Ala Leu Glu Glu Pro Ser Gln Arg Glu Glu Ala Lys Lys
            100                 105                 110

Val Val Lys Lys Ala Phe Glu Glu Lys His Gln Ala Gly Lys Asn Lys
        115                 120                 125

Trp Phe Phe Gln Lys Leu His Phe
    130                 135
```

(2) INFORMATION FOR SEQ ID NO: 39:

(i) SEQUENCE CHARACTERISTICS:
(A) LENGTH: 1192 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 39:

```
TTTGAAACGA TTAAGTCCAA TCAAACAATC TTATTCAAAA GTACTCGCAA TACGTACAAT     60
GTCAATTCCA TCTACTCAGT ACGGATTTTT TTATAATAAA GCTAGTGGTC TTAATTTGAA    120
AAAAGACTTG CCGGTTAACA AGCCAGGTGC TGGTCAATTG CTTTTAAAGG TTGATGCAGT    180
TGGCCTTTGT CATTCAGATT TACATGTTCT CTATGAAGGT TTGGATTGTG GTGATAATTA    240
TGTGATGGGC CACGAAATTG CTGGGACTGT TGCTGAACTA GGTGAAGAGG TGAGTGAGTT    300
TGCAGTTGGA GATCGTGTCG CTTGTGTCGG CCCCAATGGA TGTGGTCTTT GTAAACACTG    360
TCTTACTGGT AACGATAATG TTTGTACCAA GTCGTTTTTG GATTGGTTTG GATTGGGTTA    420
CAATGGAGGT TACGAGCAAT TTTTGTTAGT CAAGAGACCA AGAAACTTGG TCAAGATCCC    480
TGACAATGTT ACTTCCGAGG AAGCTGCAGC TATTACGGAT GCCGTATTGA CTCCTTACCA    540
TGCTATCAAG TCTGCAGGTG TTGGTCCAGC AAGTAATATA TTAATTATCG GAGCTGGTGG    600
ATTAGGAGGT AACGCTATTC AAGTTGCAAA AGCATTTGGT GCGAAGGTTA CTGTTTTGGA    660
```

```
TAAAAAGGAT AAGGCAAGAG ACCAAGCTAA GGCCTTTGGA GCTGACCAGG TTTACAGTGA     720
ATTACCAGAC AGCGTTTTAC CTGGGTCATT CAGTGCTTGT TTTGATTTTG TTTCGGTTCA     780
GGCAACATAC GATTTGTGTC AAAAGTATTG TGAGCCAAAG GGTACTATTG TTCCCGTAGG     840
TCTAGGTGCA ACTTCGCTTA ACATAAATCT TGCTGATTTA GATCTTCGTG AAATTACCGT     900
CAAGGGCTCA TTCTGGGGTA CCCTGATGGA TTTAAGAGAA GCATTTGAAT TGGCTGCACA     960
GGGAAAGGTC AAACCAAATG TTGCTCATGC TCCATTGTCA GAATTGCCTA AGTATATGGA    1020
GAAGTTGAGA GCCGGTGGTT ATGAAGGAAG AGTCGTGTTT AATCCATAAT ACTGAAAAGT    1080
GAAGAAACCA TCAATAATAG CTTGGTGAGT ATGTATGGGA AATATTCATT TATGTATGTA    1140
GGTCATTTAT ATGTGTGTAA TGATTTCTAA TCTGAATTTC GTACAATTCT TT            1192
```

(2) INFORMATION FOR SEQ ID NO: 40:

(i) SEQUENCE CHARACTERISTICS:
(A) LENGTH: 336 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 40:

```
Met Ser Ile Pro Ser Thr Gln Tyr Gly Phe Phe Tyr Asn Lys Ala Ser
1               5                   10                  15

Gly Leu Asn Leu Lys Lys Asp Leu Pro Val Asn Lys Pro Gly Ala Gly
            20                  25                  30

Gln Leu Leu Leu Lys Val Asp Ala Val Gly Leu Cys His Ser Asp Leu
        35                  40                  45

His Val Leu Tyr Glu Gly Leu Asp Cys Gly Asp Asn Tyr Val Met Gly
    50                  55                  60

His Glu Ile Ala Gly Thr Val Ala Glu Leu Gly Glu Glu Val Ser Glu
65                  70                  75                  80

Phe Ala Val Gly Asp Arg Val Ala Cys Val Gly Pro Asn Gly Cys Gly
                85                  90                  95

Leu Cys Lys His Cys Leu Thr Gly Asn Asp Asn Val Cys Thr Lys Ser
            100                 105                 110

Phe Leu Asp Trp Phe Gly Leu Gly Tyr Asn Gly Gly Tyr Glu Gln Phe
        115                 120                 125

Leu Leu Val Lys Arg Pro Arg Asn Leu Val Lys Ile Pro Asp Asn Val
    130                 135                 140

Thr Ser Glu Glu Ala Ala Ala Ile Thr Asp Ala Val Leu Thr Pro Tyr
145                 150                 155                 160

His Ala Ile Lys Ser Ala Gly Val Gly Pro Ala Ser Asn Ile Leu Ile
                165                 170                 175

Ile Gly Ala Gly Gly Leu Gly Gly Asn Ala Ile Gln Val Ala Lys Ala
            180                 185                 190

Phe Gly Ala Lys Val Thr Val Leu Asp Lys Lys Asp Lys Ala Arg Asp
```

```
            195                 200                 205

        Gln Ala Lys Ala Phe Gly Ala Asp Gln Val Tyr Ser Glu Leu Pro Asp
            210                 215                 220

        Ser Val Leu Pro Gly Ser Phe Ser Ala Cys Phe Asp Phe Val Ser Val
        225                 230                 235                 240

        Gln Ala Thr Tyr Asp Leu Cys Gln Lys Tyr Cys Glu Pro Lys Gly Thr
                        245                 250                 255

        Ile Val Pro Val Gly Leu Gly Ala Thr Ser Leu Asn Ile Asn Leu Ala
                    260                 265                 270

        Asp Leu Asp Leu Arg Glu Ile Thr Val Lys Gly Ser Phe Trp Gly Thr
                275                 280                 285

        Ser Met Asp Leu Arg Glu Ala Phe Glu Leu Ala Ala Gln Gly Lys Val
            290                 295                 300

        Lys Pro Asn Val Ala His Ala Pro Leu Ser Glu Leu Pro Lys Tyr Met
        305                 310                 315                 320

        Glu Lys Leu Arg Ala Gly Gly Tyr Glu Gly Arg Val Val Phe Asn Pro
                        325                 330                 335
```

(2) INFORMATION FOR SEQ ID NO: 41:

(i) SEQUENCE CHARACTERISTICS:
(A) LENGTH: 2021 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(ix) FEATURE:
(A) NAME/KEY: misc_feature
(B) LOCATION:1270
(D) OTHER INFORMATION:/note= "R = A or G"

(ix) FEATURE:
(A) NAME/KEY: misc_feature
(B) LOCATION:1395
(D) OTHER INFORMATION:/note= "R = A or G"

(xi) SEQUENCE DESCRIPTION: SEQ ID NO: 41:

```
ATGGAAAAAA TTGACATTAA TACAAATTCA AACAAAATCC AACAAGCATA CGATAAAGTT      60
GTTAGAGGAG ACCCAAATGC AACATTCGTC GTTTATTCTG TTGACAAAAA CGCCACTATG     120
GACGTCACTG AAACAGGGGA CGGATCATTA GAGGATTTTG TTGAACATTT TACTGATGGA     180
CAAGTTCAAT TTGGTTTAGC CAGGGTTACT GTTCCAGGAT CTGACGTTTC CAAAAACATC     240
TTGTTAGGAT GGTGTCCTGA CAGTGCTCCA GCAAAAATTGA GATTGTCATT TGCCAATAAT    300
TTTGCTGATG TGTCCAGAGT ATTGAGCGGA TACCATGTGC AAATTACTGC AAGGGATCAA     360
GATGATTTAG ACGTGAATGA ATTCTTGAAT AGAGTTGGTG CTGCTGCTGG TGCAAGATAT     420
TCCACTCAAA CTTCCGGACT CAAAAAACCA TCCCCTGCTG CACCTAAACC TACTTCAAAA     480
CCTGTTGTTG CTAAATCTAG TTCTGCTTCA AAACCTTCAT TTGTACCCAA ATCTACTGGG     540
AAGCCTGTTG CTCCAGCTAA GCCAAAACCA AAGAACATCA CCAAGGATGC TGGTTGGGGT     600
```

```
GATGCTGAAG ACGTTGAGGA AAGAGACTTT GACAAGAAAC CTTTGGATAA CGTTCCATCG    660
GCATATAAAC CAACAAAGGT TAACATTGAC GAATTGAGAA AACAAAAATC AGATACAACT    720
AGCTCAACTC CTAAAACATT CAAATCTGAA CCACAAGAAG AAAAGAATGA CGATGATGGG    780
CAATCCAAAC CTTTATCGGA AAGGATGAAA GCCTATGATC AACCATCAAG TAGTGATGGA    840
AGATTGACTT CTTTACCAAA ACCAAAGATT GGACATTCTG TTGCCGATAA ATATAAAGCT    900
AGTGCATCTG GGAATGGTGC TGCTCCTGCG TTTGGTGCTA AACCAGCATT TGGTACACAA    960
TCAGTTGATT CAAGAAAGGA TAAATTGGTA GGTGGTTTGT CGAGAGATTT TGGTGCTGAA   1020
AATGGAAAAA CTCCGGCACA AATTTGGGCT GAAAAAAGGG GAAAATACAA AACAGTGGCC   1080
TCCGATGAGA AAGAAACTAA CTCAAGTGAA AAAGTTGATG AGCCAGAGGA ACATCATGCT   1140
GCCGACTTGG CCAAAAAATT TGAAGAAAAG GCAAATATTG CTGGCGATAC TCCTTCCTTG   1200
CCAACTAGAA ACTTACCACC AGCACCACCA GCACGAGAAA CCGCAATTCC ATCTAACGAA   1260
AAAGACAAAR AAGAAAAGGA AGAGGAAGAA CAAGCTCCAG CACCATCTTT GCCTACTAGA   1320
AACTTACCAC CACCGTCACA AAGACAACCT GAGCCCGAAC CAGAACCAGA AGAAGAGGAG   1380
GAAGAAGAAG AAGARGAGGC TCCTGCTCCA AGCTTACCAG CAAGAAATCT CCCACCAGCA   1440
CCAAAAGCAG AAGCAGAAGA ATCAAAAAAA CAGTCAACCA CAGCCACCGC AGAGTATGAT   1500
TACGAAAAGG ACGAAGATAA TGAAATTGGA TTCTCCGAAG GTGACTTGAT TATTGATATT   1560
GAATTTGTGG ATGACGATTG GTGGCAAGGT AAACATGCTA AAACTGGTGA AGTTGGTTTG   1620
TTTCCTGCCA CTTATGTGTC ATTAAATGAA AAAGCTGCTG ACAAAGAAGA GGAAGCCCCA   1680
GCTCCAGCTC CAGCGCCATC ATTACCTTCT AGAGAAGAAA CACAAGCAGC ACCAGCATTA   1740
CCAAGTAGAT CAGAGCAAAA ACCAGAATCA AAAACTGCTA CAGCTGAATA CGATTACGAA   1800
AAGGACGAAG ACAATGAAAT TGGTTTTTCA GAAGGTGATT TGATTGTTGA AATCGAATTT   1860
GTTGACGATG ATTGGTGGCA AGGAAAACAT TCCAAGACAG GAGAAGTCGG ATTGTTCCCT   1920
GCTAACTATG TTGTCTTGAA TGAGTAGATT TAGTATAAAC AATATTCGTT TTTTTTTTAT   1980
ATGAATCTAT AATATAAATA CAAAGAAAAG ATAAATTGGT G                       2021
```

(2) INFORMATION FOR SEQ ID NO: 42:

(i) SEQUENCE CHARACTERISTICS:
(A) LENGTH: 648 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 42:

```
Met Glu Lys Ile Asp Ile Asn Thr Asn Ser Asn Lys Ile Gln Gln Ala
1               5                   10                  15

Tyr Asp Lys Val Val Arg Gly Asp Pro Asn Ala Thr Phe Val Val Tyr
            20                  25                  30
```

Ser Val Asp Lys Asn Ala Thr Met Asp Val Thr Glu Thr Gly Asp Gly
35 40 45

Ser Leu Glu Asp Phe Val Glu His Phe Thr Asp Gly Gln Val Gln Phe
50 55 60

Gly Leu Ala Arg Val Thr Val Pro Gly Ser Asp Val Ser Lys Asn Ile
65 70 75 80

Leu Leu Gly Trp Cys Pro Asp Ser Ala Pro Ala Lys Leu Arg Leu Ser
85 90 95

Phe Ala Asn Asn Phe Ala Asp Val Ser Arg Val Leu Ser Gly Tyr His
100 105 110

Val Gln Ile Thr Ala Arg Asp Gln Asp Asp Leu Asp Val Asn Glu Phe
115 120 125

Leu Asn Arg Val Gly Ala Ala Ala Gly Ala Arg Tyr Ser Thr Gln Thr
130 135 140

Ser Gly Leu Lys Lys Pro Ser Pro Ala Ala Pro Lys Pro Thr Ser Lys
145 150 155 160

Pro Val Val Ala Lys Ser Ser Ser Ala Ser Lys Pro Ser Phe Val Pro
165 170 175

Lys Ser Thr Gly Lys Pro Val Ala Pro Ala Lys Pro Lys Pro Lys Asn
180 185 190

Ile Thr Lys Asp Ala Gly Trp Gly Asp Ala Glu Asp Val Glu Glu Arg
195 200 205

Asp Phe Asp Lys Lys Pro Leu Asp Asn Val Pro Ser Ala Tyr Lys Pro
210 215 220

Thr Lys Val Asn Ile Asp Glu Leu Arg Lys Gln Lys Ser Asp Thr Thr
225 230 235 240

Ser Ser Thr Pro Lys Thr Phe Lys Ser Glu Pro Gln Glu Glu Lys Asn
245 250 255

Asp Asp Asp Gly Gln Ser Lys Pro Leu Ser Glu Arg Met Lys Ala Tyr
260 265 270

Asp Gln Pro Ser Ser Ser Asp Gly Arg Leu Thr Ser Leu Pro Lys Pro
275 280 285

Lys Ile Gly His Ser Val Ala Asp Lys Tyr Lys Ala Ser Ala Ser Gly
290 295 300

Asn Gly Ala Ala Pro Ala Phe Gly Ala Lys Pro Ala Phe Gly Thr Gln
305 310 315 320

Ser Val Asp Ser Arg Lys Asp Lys Leu Val Gly Gly Leu Ser Arg Asp
325 330 335

Phe Gly Ala Glu Asn Gly Lys Thr Pro Ala Gln Ile Trp Ala Glu Lys
340 345 350

Arg Gly Lys Tyr Lys Thr Val Ala Ser Asp Glu Lys Glu Thr Asn Ser
355 360 365

Ser Glu Lys Val Asp Glu Pro Glu Glu His His Ala Ala Asp Leu Ala
370 375 380

Lys Lys Phe Glu Glu Lys Ala Asn Ile Ala Gly Asp Thr Pro Ser Leu
385 390 395 400

Pro Thr Arg Asn Leu Pro Pro Ala Pro Pro Ala Arg Glu Thr Ala Ile
405 410 415

```
Pro Ser Asn Glu Lys Asp Lys Xaa Glu Lys Glu Glu Glu Glu Gln Ala
            420                 425                 430

Pro Ala Pro Ser Leu Pro Thr Arg Asn Leu Pro Pro Pro Ser Gln Arg
        435                 440                 445

Gln Pro Glu Pro Glu Pro Glu Pro Glu Glu Glu Glu Glu Glu Glu Glu
    450                 455                 460

Xaa Glu Ala Pro Ala Pro Ser Leu Pro Ala Arg Asn Leu Pro Pro Ala
465                 470                 475                 480

Pro Lys Ala Glu Ala Glu Glu Ser Lys Lys Gln Ser Thr Thr Ala Thr
                485                 490                 495

Ala Glu Tyr Asp Tyr Glu Lys Asp Glu Asp Asn Glu Ile Gly Phe Ser
            500                 505                 510

Glu Gly Asp Leu Ile Ile Asp Ile Glu Phe Val Asp Asp Asp Trp Trp
        515                 520                 525

Gln Gly Lys His Ala Lys Thr Gly Glu Val Gly Leu Phe Pro Ala Thr
    530                 535                 540

Tyr Val Ser Leu Asn Glu Lys Ala Ala Asp Lys Glu Glu Glu Ala Pro
545                 550                 555                 560

Ala Pro Ala Pro Ala Pro Ser Leu Pro Ser Arg Glu Glu Thr Gln Ala
                565                 570                 575

Ala Pro Ala Leu Pro Ser Arg Ser Glu Gln Lys Pro Glu Ser Lys Thr
            580                 585                 590

Ala Thr Ala Glu Tyr Asp Tyr Glu Lys Asp Glu Asp Asn Glu Ile Gly
        595                 600                 605

Phe Ser Glu Gly Asp Leu Ile Val Glu Ile Glu Phe Val Asp Asp Asp
    610                 615                 620

Trp Trp Gln Gly Lys His Ser Lys Thr Gly Glu Val Gly Leu Phe Pro
625                 630                 635                 640

Ala Asn Tyr Val Val Leu Asn Glu
                645
```

(2) INFORMATION FOR SEQ ID NO: 43:

(i) SEQUENCE CHARACTERISTICS:
(A) LENGTH: 1340 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 43:

```
ATGTGTGACG TCGTATTAGG ATCTCAATGG GGGGATGAAG GTAAAGGTAA ATTAGTCGAT    60

TTATTATGTG ATGATATCGA TGTTTGTGCC AGGTGTCAAG GTGGTAACAA TGCTGGCCAC   120

ACAATTGTTG TTGGTAAAGT CAAGTATGAC TTCCACATGT TACCTTCTGG TTTGGTCAAT   180

CCTAAATGTC AAAACTTAGT TGGATCTGGT GTTGTTATCC ACGTTCCTTC CTTCTTTGCT   240

GAATTGGAAA ACTTGGAAGC AAAAGGGTTA GATTGTCGTG ATAGATTGTT TGTTTCATCT   300
```

```
AGAGCTCATT TGGTCTTTGA CTTCCATCAA CGTACTGATA AATTGAAAGA AGCTGAATTA     360
TCAACCAATA AGAAATCAAT AGGTACTACC GGTAAAGGTA TTGGTCCAAC TTACTCAACC     420
AAGGCAAGTA GATCAGGTAT CAGAGTCCAC CATTTAGTCA ACCCTGATCC AGAAGCTTGG     480
GAAGAATTCA AAACTAGATA TTTGAGATTA GTCGAGAGTA GACAAAAAAG ATACGGTGAA     540
TTTGAATATG ATCCTAAGGA AGAATTGGCA AGATTTGAAA AATACCGTGA AACCTTGAGA     600
CCATTCGTCG TCGACTCCGT CAACTTCATG CACGAAGCTA TTGCTGCCAA TAAAAAAATC     660
TTGGTTGAAG GTGCTAATGC GTTAATGTTG GATATTGATT TCGGTACTTA TCCATACGTC     720
ACTTCTTCAT CAACTGGTAT TGGTGGTGTT TTGACTGGGT TGGGTATTCC TCCAAGAACC     780
ATCAGAAATG TCTATGGTGT TGTTAAAGCC TACACCACTA GAGTTGGTGA GGGTCCATTC     840
CCAACAGAAC AATTGAACAA GGTAGGTGAA ACTTTGCAAG ATGTTGGTGC CGAATATGGT     900
GTTACTACTG GAAGAAAAAG AAGATGTGGT TGGTTGGATT TGGTTGTGTT GAAATATTCC     960
AACCTGATCA ACGGATACAC TTCTTTGAAC ATCACCAAAT TGGATGTTTT GGATAAATTC    1020
AAGGAAATTG AAGTTGGTGT TGCTTATAAA TTGAATGGAA AAGAGTTGCC AAGTTTCCCT    1080
GAAGATTTGA TTGATTTAGC TAAAGTCGAG GTTGTGTATA AGAAATTCCC AGGTTGGGAA    1140
CAAGATATCA CCGGTATCAA GAAATATGAA GACTTGCCAG AAAACGCTAA GAACTATCTT    1200
AAATTCATTG AAGATTACTT GCAAGTTCCA ATCCAATGGG TAGGTACCGG TCCAGCTAGA    1260
GATTCTATGT TAGAAAAGAA GATTTAGTTG TACACATGCT ACGGAAGACG ATTAGATTTG    1320
TTTTATTAGA TTAATAACCT                                                1340
```

(2) INFORMATION FOR SEQ ID NO: 44:

(i) SEQUENCE CHARACTERISTICS:
(A) LENGTH: 428 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 44:

```
Met Cys Asp Val Val Leu Gly Ser Gln Trp Gly Asp Glu Gly Lys Gly
1               5                   10                  15

Lys Leu Val Asp Leu Leu Cys Asp Asp Ile Asp Val Cys Ala Arg Cys
            20                  25                  30

Gln Gly Gly Asn Asn Ala Gly His Thr Ile Val Val Gly Lys Val Lys
        35                  40                  45

Tyr Asp Phe His Met Leu Pro Ser Gly Leu Val Asn Pro Lys Cys Gln
    50                  55                  60

Asn Leu Val Gly Ser Gly Val Val Ile His Val Pro Ser Phe Phe Ala
65                  70                  75                  80

Glu Leu Glu Asn Leu Glu Ala Lys Gly Leu Asp Cys Arg Asp Arg Leu
                85                  90                  95

Phe Val Ser Ser Arg Ala His Leu Val Phe Asp Phe His Gln Arg Thr
```

```
                100                     105                     110

Asp Lys Leu Lys Glu Ala Glu Leu Ser Thr Asn Lys Lys Ser Ile Gly
        115                     120                     125

Thr Thr Gly Lys Gly Ile Gly Pro Thr Tyr Ser Thr Lys Ala Ser Arg
    130                     135                     140

Ser Gly Ile Arg Val His His Leu Val Asn Pro Asp Pro Glu Ala Trp
145                 150                     155                 160

Glu Glu Phe Lys Thr Arg Tyr Leu Arg Leu Val Glu Ser Arg Gln Lys
                165                     170                     175

Arg Tyr Gly Glu Phe Glu Tyr Asp Pro Lys Glu Glu Leu Ala Arg Phe
            180                     185                     190

Glu Lys Tyr Arg Glu Thr Leu Arg Pro Phe Val Val Asp Ser Val Asn
        195                     200                     205

Phe Met His Glu Ala Ile Ala Ala Asn Lys Lys Ile Leu Val Glu Gly
    210                     215                     220

Ala Asn Ala Leu Met Leu Asp Ile Asp Phe Gly Thr Tyr Pro Tyr Val
225                 230                     235                 240

Thr Ser Ser Ser Thr Gly Ile Gly Gly Val Leu Thr Gly Leu Gly Ile
                245                     250                     255

Pro Pro Arg Thr Ile Arg Asn Val Tyr Gly Val Val Lys Ala Tyr Thr
            260                     265                     270

Thr Arg Val Gly Glu Gly Pro Phe Pro Thr Glu Gln Leu Asn Lys Val
        275                     280                     285

Gly Glu Thr Leu Gln Asp Val Gly Ala Glu Tyr Gly Val Thr Thr Gly
    290                     295                     300

Arg Lys Arg Arg Cys Gly Trp Leu Asp Leu Val Val Leu Lys Tyr Ser
305                 310                     315                 320

Asn Ser Ile Asn Gly Tyr Thr Ser Leu Asn Ile Thr Lys Leu Asp Val
                325                     330                     335

Leu Asp Lys Phe Lys Glu Ile Glu Val Gly Val Ala Tyr Lys Leu Asn
            340                     345                     350

Gly Lys Glu Leu Pro Ser Phe Pro Glu Asp Leu Ile Asp Leu Ala Lys
        355                     360                     365

Val Glu Val Val Tyr Lys Lys Phe Pro Gly Trp Glu Gln Asp Ile Thr
    370                     375                     380

Gly Ile Lys Lys Tyr Glu Asp Leu Pro Glu Asn Ala Lys Asn Tyr Leu
385                 390                     395                 400

Lys Phe Ile Glu Asp Tyr Leu Gln Val Pro Ile Gln Trp Val Gly Thr
                405                     410                     415

Gly Pro Ala Arg Asp Ser Met Leu Glu Lys Lys Ile
            420                     425
```

(2) INFORMATION FOR SEQ ID NO: 45:

(i) SEQUENCE CHARACTERISTICS:
- (A) LENGTH: 2481 base pairs
- (B) TYPE: nucleic acid
- (C) STRANDEDNESS: single
- (D) TOPOLOGY: linear

(ii) MOLECULE TYPE: cDNA

(iii) HYPOTHETICAL: NO

(xi) SEQUENCE DESCRIPTION: SEQ ID NO: 45:

```
ATGACTGGTG AAGAAGATAA AAAACAACAT TTTGATGCTT CTGGTGCTTC TGCTGTAGAT      60
GATAAAACAG CAACTGCAAT TTTAAGAAGA AAAAAGAAAG ATAATGCCTT GGTCGTTGAT     120
GACGCCACCA ACGATGACAA TTCTGTCATA ACCATGTCGT CAAACACAAT GGAATTGTTA     180
CAATTATTCC GTGGTGATAC AGTCTTGGTG AAAGGTAAGA AGAGAAAGGA CACAGTGTTG     240
ATCGTTTTAG CTGATGATGA TATGCCTGAT GGCGTTGCTA GAGTTAACAG ATGTGTTCGT     300
AACAATTTGC GTGTCAGATT GGGAGATATC GTTACTGTCC ATCCATGTCC TGATATTAAA     360
TATGCCAACA GAATCTCAGT ATTGCCAATT GCTGATACTG TTGAAGGTAT TAATGGTTCC     420
TTATTCGACC TTTACTTGAA GCCATATTTT GTTGAAGCCT ATAGACCAGT GAGAAAAGGT     480
GATTTATTCA CTGTGAGGGG TGGTATGAGA CAAGTAGAAT TCAAAGTTGT TGAAGTTGAC     540
CCTGAAGAAA TTGCAATTGT TGCTCAAGAT ACCATTATTC ATTGTGAAGG AGAACCTATT     600
AATCGTGAAG ATGAAGAAAA TAGCTTGAAT GAAGTGGGTT ACGACGATAT TGGAGGTTGT     660
AAGAAACAAA TGGCCCAAAT TAGAGAATTG GTTGAATTGC CTTTAAGACA TCCACAATTA     720
TTCAAATCGA TTGGTATTAA GCCACCAAAG GGTATTTTGA TGTATGGTCC ACCTGGTACC     780
GGTAAAACCA TTATGGCAAG AGCAGTGGCC AATGAAACAG GTGCCTTCTT TTTCTTAATA     840
AATGGTCCAG AAATTATGTC TAAAATGGCT GGTGAGTCTG AATCCAATTT AAGAAAAGCT     900
TTTGAAGAGG CTGAAAAGAA TTCTCCTTCC ATTATTTTCA TTGATGAGAT TGACTCTATT     960
GCCCCAAAGA GAGACAAAAC TAATGGTGAA GTAGAAAGAA GAGTTGTTTC TCAATTGTTA    1020
ACCCTTATGG ATGGTATGAA GGCCAGATCT AATGTAGTTG TTATTGCTGC TACTAACAGA    1080
CCAAATTCTA TTGATCCTGC TTTGAGAAGA TTTGGAAGAT TCGACAGAGA AGTTGACATT    1140
GGTGTTCCGG ATGCTGAAGG ACGTTTAGAG ATTTTGAGAA TCCACACAAA GAATATGAAA    1200
TTGGCTGATG ATGTTGACTT GGAAGCCATC GCTTCTGAAA CACATGGTTT CGTTGGTGCT    1260
GATATTGCTT CATTATGTTC AGAAGCTGCT ATGCAACAAA TCCGTGAAAA GATGGATCTT    1320
ATCGACTTGG AAGAAGAAAC CATTGATACT GAAGTGTTGA ACTCTTTGGG TGTCACTCAA    1380
GACAACTTCA GATTTGCTCT CGGAAACTCC AACCCATCTG CCTTGCGTGA AACTGTTGTT    1440
GAAAATGTTA ATGTCACTTG GGATGATATT GGTGGTTTGG ACAACATTAA GAATGAATTA    1500
AAAGAAACCG TGGAGTATCC TGTTTTACAT CCAGATCAAT ACCAAAAATT CGGATTGGCA    1560
CCAACAAAAG GTGTTTTGTT CTTTGGTCCA CCAGGTACTG GTAAGACACT TTTGGCCAAG    1620
GCTGTTGCTA CTGAAGTTTC TGCTAATTTC ATTTCTGTCA AAGGTCCAGA ATTGTTGAGT    1680
ATGTGGTATG GTGAATCTGA GTCTAATATC CGTGATATAT TTGACAAGGC CAGAGCTGCT    1740
GCTCCTACTG TGGTGTTTTT GGATGAATTG GACTCCATTG CCAAAGCTAG AGGTGGTTCT    1800
CACGGTGATG CTGGTGGTGC CTCCGACAGA GTGGTCAATC AATTGTTGAC TGAAATGGAC    1860
GGTATGAATG CTAAGAAGAA TGTGTTTGTC ATTGGTGCCA CTAACAGACC AGATCAAATT    1920
GATCCTGCAT TATTGAGACC AGGTAGATTG GATCAATTAA TTTATGTCCC ATTGCCAGAT    1980
```

```
GAGCCAGCTA GATTGTCTAT TTTACAAGCT CAATTGAGAA ACACTCCATT AGAACCTGGT    2040
TTGGACTTGA ACGAAATTGC CAAGATCACT CACGGTTTCT CGGGTGCAGA TTTGTCTTAT    2100
ATTGTTCAAA GATCTGCTAA ATTTGCTATT AAAGACTCTA TTGAAGCCCA AGTAAAGATT    2160
AACAAGATTA AAGAAGAAAA AGAAAAGGTG AAAACTGAAG ATGTTGATAT GAAGGTAGAT    2220
GAAGTTGAAG AAGAAGACCC TGTGCCTTAC ATTACCAGAG CTCACTTTGA AGAGGCTATG    2280
AAGACCGCAA AAAGATCTGT TTCAGACGCT GAATTACGTC GTTATGAGTC TTACGCTCAA    2340
CAATTGCAAG CCTCAAGAGG TCAATTTTCT AGCTTTAGAT TCAATGAAAA TGCTGGTGCC    2400
ACTGATAATG GTTCAGCAGC AGGTGCCAAC TCAGGTGCAG CTTTCGGAAA CGTTGAAGAG    2460
GAAGACGATT TGTACAGTTG A                                              2481
```

(2) INFORMATION FOR SEQ ID NO: 46:

(i) SEQUENCE CHARACTERISTICS:
(A) LENGTH: 826 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 46:

```
Met Thr Gly Glu Glu Asp Lys Lys Gln His Phe Asp Ala Ser Gly Ala
1               5                   10                  15

Ser Ala Val Asp Asp Lys Thr Ala Thr Ala Ile Leu Arg Arg Lys Lys
            20                  25                  30

Lys Asp Asn Ala Leu Val Val Asp Asp Ala Thr Asn Asp Asp Asn Ser
        35                  40                  45

Val Ile Thr Met Ser Ser Asn Thr Met Glu Leu Leu Gln Leu Phe Arg
    50                  55                  60

Gly Asp Thr Val Leu Val Lys Gly Lys Lys Arg Lys Asp Thr Val Leu
65                  70                  75                  80

Ile Val Leu Ala Asp Asp Asp Met Pro Asp Gly Val Ala Arg Val Asn
                85                  90                  95

Arg Cys Val Arg Asn Asn Leu Arg Val Arg Leu Gly Asp Ile Val Thr
            100                 105                 110

Val His Pro Cys Pro Asp Ile Lys Tyr Ala Asn Arg Ile Ser Val Leu
        115                 120                 125

Pro Ile Ala Asp Thr Val Glu Gly Ile Asn Gly Ser Leu Phe Asp Leu
    130                 135                 140

Tyr Leu Lys Pro Tyr Phe Val Glu Ala Tyr Arg Pro Val Arg Lys Gly
145                 150                 155                 160

Asp Leu Phe Thr Val Arg Gly Gly Met Arg Gln Val Glu Phe Lys Val
                165                 170                 175

Val Glu Val Asp Pro Glu Glu Ile Ala Ile Val Ala Gln Asp Thr Ile
            180                 185                 190
```

```
Ile His Cys Glu Gly Glu Pro Ile Asn Arg Glu Asp Glu Glu Asn Ser
        195                 200                 205

Leu Asn Glu Val Gly Tyr Asp Asp Ile Gly Gly Cys Lys Lys Gln Met
    210                 215                 220

Ala Gln Ile Arg Glu Leu Val Glu Leu Pro Leu Arg His Pro Gln Leu
225                 230                 235                 240

Phe Lys Ser Ile Gly Ile Lys Pro Pro Lys Gly Ile Leu Met Tyr Gly
                245                 250                 255

Pro Pro Gly Thr Gly Lys Thr Ile Met Ala Arg Ala Val Ala Asn Glu
            260                 265                 270

Thr Gly Ala Phe Phe Phe Leu Ile Asn Gly Pro Glu Ile Met Ser Lys
        275                 280                 285

Met Ala Gly Glu Ser Glu Ser Asn Leu Arg Lys Ala Phe Glu Glu Ala
    290                 295                 300

Glu Lys Asn Ser Pro Ser Ile Ile Phe Ile Asp Glu Ile Asp Ser Ile
305                 310                 315                 320

Ala Pro Lys Arg Asp Lys Thr Asn Gly Glu Val Glu Arg Arg Val Val
                325                 330                 335

Ser Gln Leu Leu Thr Leu Met Asp Gly Met Lys Ala Arg Ser Asn Val
            340                 345                 350

Val Val Ile Ala Ala Thr Asn Arg Pro Asn Ser Ile Asp Pro Ala Leu
        355                 360                 365

Arg Arg Phe Gly Arg Phe Asp Arg Glu Val Asp Ile Gly Val Pro Asp
    370                 375                 380

Ala Glu Gly Arg Leu Glu Ile Leu Arg Ile His Thr Lys Asn Met Lys
385                 390                 395                 400

Leu Ala Asp Asp Val Asp Leu Glu Ala Ile Ala Ser Glu Thr His Gly
                405                 410                 415

Phe Val Gly Ala Asp Ile Ala Ser Leu Cys Ser Glu Ala Ala Met Gln
            420                 425                 430

Gln Ile Arg Glu Lys Met Asp Leu Ile Asp Leu Glu Glu Glu Thr Ile
        435                 440                 445

Asp Thr Glu Val Leu Asn Ser Leu Gly Val Thr Gln Asp Asn Phe Arg
    450                 455                 460

Phe Ala Leu Gly Asn Ser Asn Pro Ser Ala Leu Arg Glu Thr Val Val
465                 470                 475                 480

Glu Asn Val Asn Val Thr Trp Asp Asp Ile Gly Gly Leu Asp Asn Ile
                485                 490                 495

Lys Asn Glu Leu Lys Glu Thr Val Glu Tyr Pro Val Leu His Pro Asp
            500                 505                 510

Gln Tyr Gln Lys Phe Gly Leu Ala Pro Thr Lys Gly Val Leu Phe Phe
        515                 520                 525

Gly Pro Pro Gly Thr Gly Lys Thr Leu Leu Ala Lys Ala Val Ala Thr
    530                 535                 540

Glu Val Ser Ala Asn Phe Ile Ser Val Lys Gly Pro Glu Leu Leu Ser
545                 550                 555                 560

Met Trp Tyr Gly Glu Ser Glu Ser Asn Ile Arg Asp Ile Phe Asp Lys
                565                 570                 575
```

```
Ala Arg Ala Ala Ala Pro Thr Val Val Phe Leu Asp Glu Leu Asp Ser
            580                 585                 590

Ile Ala Lys Ala Arg Gly Gly Ser His Gly Asp Ala Gly Gly Ala Ser
        595                 600                 605

Asp Arg Val Val Asn Gln Leu Leu Thr Glu Met Asp Gly Met Asn Ala
    610                 615                 620

Lys Lys Asn Val Phe Val Ile Gly Ala Thr Asn Arg Pro Asp Gln Ile
625                 630                 635                 640

Asp Pro Ala Leu Leu Arg Pro Gly Arg Leu Asp Gln Leu Ile Tyr Val
                645                 650                 655

Pro Leu Pro Asp Glu Pro Ala Arg Leu Ser Ile Leu Gln Ala Gln Leu
            660                 665                 670

Arg Asn Thr Pro Leu Glu Pro Gly Leu Asp Leu Asn Glu Ile Ala Lys
        675                 680                 685

Ile Thr His Gly Phe Ser Gly Ala Asp Leu Ser Tyr Ile Val Gln Arg
    690                 695                 700

Ser Ala Lys Phe Ala Ile Lys Asp Ser Ile Glu Ala Gln Val Lys Ile
705                 710                 715                 720

Asn Lys Ile Lys Glu Glu Lys Glu Lys Val Lys Thr Glu Asp Val Asp
                725                 730                 735

Met Lys Val Asp Glu Val Glu Glu Glu Asp Pro Val Pro Tyr Ile Thr
            740                 745                 750

Arg Ala His Phe Glu Glu Ala Met Lys Thr Ala Lys Arg Ser Val Ser
        755                 760                 765

Asp Ala Glu Leu Arg Arg Tyr Glu Ser Tyr Ala Gln Gln Leu Gln Ala
    770                 775                 780

Ser Arg Gly Gln Phe Ser Ser Phe Arg Phe Asn Glu Asn Ala Gly Ala
785                 790                 795                 800

Thr Asp Asn Gly Ser Ala Ala Gly Ala Asn Ser Gly Ala Ala Phe Gly
                805                 810                 815

Asn Val Glu Glu Glu Asp Asp Leu Tyr Ser
            820                 825
```

(2) INFORMATION FOR SEQ ID NO: 47:

(i) SEQUENCE CHARACTERISTICS:
(A) LENGTH: 1918 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 47:

```
TTTTTTTTTC TCCCTCTCTC TCGTTCAGAT TCTGTAGAAT TGATTGGTTG AGAGTAAAAG    60
TCAGACTTTT TTTTTTGCTC TCCATCTAGT GGGACAAATA AGAAGTTTAA CAAAGAACGA   120
CAAAAAAATCC TCACCAGAAG AAAAAAAAAT CAATTTTCAC AGGTAAAGTT GTACGGACAG  180
CACGACAGAC ACAAAACTAA AGTAAATCCA TGAGGAAAAA AGTAAAAAAA AAAAAATTGT   240
```

```
TCACCACAAC TTCAAGAGCC ATTAAAACCA AAAATTTGGA ATATAAATTT CAACTGATTT     300
CTTGCTGGAT TTTTTTGTAT ATATTTGCAA TTGATTTCCT TTTACTTTTT TTTTTTCCAT     360
TTCTTCTTTT CCTTTTTCCA TCTTTTAAGT TTCTTTTAGA ATATAGTATA TTTATCAAAC     420
AATGTCTGCA TTCAGATCAA TTCAACGTTC AACCAACGTA GCCAAGAGCA CTTTCAAAAA     480
CAGCATCAGA ACATATGCTT CTGCTGAACC AGTATGTATT CACTTTTTTG AGGATCCGGG     540
CAATGTGCTT GGGATTTTAC TTTTAACGTA TATACAAAGA TAATTTACTA ACTTGCTTTC     600
TTAGACCTTA AAACAAAGAT TGGAAGAAAT CTTGCCAGCC AAAGCTGAAG AAGTTAAACA     660
ATTCAAAAAA GAACACGGTA AAACTGTCAT TGGTGAAGTT TTATTAGAAC AAGCTTACGG     720
TGGTATGAGA GGTATCAAAG GTTTAGTTTG GGAAGGTTCT GTTTTGGACC CAATTGAAGG     780
TATCCGTTTC AGAGGAAGAA CCATCCCAGA CATTCAAAAA GAATTGCCAA AAGCACCAGG     840
TGGTGAAGAA CCATTACCAG AAGCTCTTTT CTGGTTGTTG TTGACTGGTG AAGTTCCAAC     900
TGACGCCCAA ACTAAGGCTT TATCCGAAGA ATTTGCTGCT AGATCAGCAT TACCAAAGCA     960
CGTTGAAGAA TTGATCGACA GATCTCCATC TCACTTGCAC CCAATGGCTC AATTCTCCAT    1020
TGCCGTTACT GCTTTGGAAT CTGAATCCCA ATTTGCCCAA GCTTATGCTA AAGGTGCCAA    1080
CAAATCCGAA TACTGGAAAT ACACTTACGA AGATTCCATC GATTTGTTAG CTAAATTGCC    1140
AACCATTGCT GCTAAGATTT ACAGAAACGT TTTCCACGAT GGTAAATTGC CAGCTGCCAT    1200
TGACTCCAAA TTGGATTACG GTGCTAACTT GGCCAGTTTG TTAGGTTTTG GTGACAACAA    1260
GGAATTTGTT GAATTAATGA GATTGTACCT TACCATCCAC TCTGACCACG AAGGTGGTAA    1320
CGTCTCTGCA CACACCACCC ACTTGGTTGG TTCCGCTTTA TCTTCCCCAT TCTTGTCATT    1380
AGCTGCTGGT TTGAATGGTT TAGCTGGTCC ATTACACGGT AGAGCTAACC AAGAAGTTTT    1440
GGAATGGTTG TTCAAATTAA GAGAAGAATT AAACGGTGAC TACTCCAAGG AAGCCATTGA    1500
AAAATACTTG TGGGAAACCT TGAACTCCGG TAGAGTTGTC CCAGGTTACG GTCACGCTGT    1560
CTTGAGAAAG ACCGATCCAA GATACACTGC TCAAAGAGAA TTTGCTCTTA AACATATGCC    1620
AGACTACGAA TTGTTCAAAT TGGTTTCAAA CATTTACGAA GTCGCTCCAG GTGTTTTAAC    1680
CAAACACGGT AAGACCAAGA ACCCATGGCC AAATGTGGAC TCCCACTCTG GTGTCTTGTT    1740
ACAATACTAC GGTTTGACTG AACAATCTTT CTACACTGTC TTGTTCGGTG TTTCCAGAGC    1800
CTTTGGTGTC TTGCCACAAT TGATCTTGGA CCGTGGTATC GGTATGCCAA TTGAAAGACC    1860
AAAATCTTTC TCCACTGAAA AATACATTGA ATTGGTCAAA AACATCAACA AAGCTTAA      1918
```

(2) INFORMATION FOR SEQ ID NO: 48:

(i) SEQUENCE CHARACTERISTICS:
- (A) LENGTH: 466 amino acids
- (B) TYPE: amino acid
- (C) STRANDEDNESS:
- (D) TOPOLOGY: unknown

(ii) MOLECULE TYPE: peptide

(iii) HYPOTHETICAL: NO

(xi) SEQUENCE DESCRIPTION: SEQ ID NO: 48:

```
Met Ser Ala Phe Arg Ser Ile Gln Arg Ser Thr Asn Val Ala Lys Ser
1               5                   10                  15

Thr Phe Lys Asn Ser Ile Arg Thr Tyr Ala Ser Ala Glu Pro Thr Leu
            20                  25                  30

Lys Gln Arg Leu Glu Glu Ile Leu Pro Ala Lys Ala Glu Glu Val Lys
        35                  40                  45

Gln Phe Lys Lys Glu His Gly Lys Thr Val Ile Gly Glu Val Leu Leu
    50                  55                  60

Glu Gln Ala Tyr Gly Gly Met Arg Gly Ile Lys Gly Leu Val Trp Glu
65                  70                  75                  80

Gly Ser Val Leu Asp Pro Ile Glu Gly Ile Arg Phe Arg Gly Arg Thr
                85                  90                  95

Ile Pro Asp Ile Gln Lys Glu Leu Pro Lys Ala Pro Gly Gly Glu Glu
            100                 105                 110

Pro Leu Pro Glu Ala Leu Phe Trp Leu Leu Leu Thr Gly Glu Val Pro
        115                 120                 125

Thr Asp Ala Gln Thr Lys Ala Leu Ser Glu Glu Phe Ala Ala Arg Ser
    130                 135                 140

Ala Leu Pro Lys His Val Glu Glu Leu Ile Asp Arg Ser Pro Ser His
145                 150                 155                 160

Leu His Pro Met Ala Gln Phe Ser Ile Ala Val Thr Ala Leu Glu Ser
                165                 170                 175

Glu Ser Gln Phe Ala Gln Ala Tyr Ala Lys Gly Ala Asn Lys Ser Glu
            180                 185                 190

Tyr Trp Lys Tyr Thr Tyr Glu Asp Ser Ile Asp Leu Leu Ala Lys Leu
        195                 200                 205

Pro Thr Ile Ala Ala Lys Ile Tyr Arg Asn Val Phe His Asp Gly Lys
    210                 215                 220

Leu Pro Ala Ala Ile Asp Ser Lys Leu Asp Tyr Gly Ala Asn Leu Ala
225                 230                 235                 240

Ser Leu Leu Gly Phe Gly Asp Asn Lys Glu Phe Val Glu Leu Met Arg
                245                 250                 255

Leu Tyr Leu Thr Ile His Ser Asp His Glu Gly Gly Asn Val Ser Ala
            260                 265                 270

His Thr Thr His Leu Val Gly Ser Ala Leu Ser Ser Pro Phe Leu Ser
        275                 280                 285

Leu Ala Ala Gly Leu Asn Gly Leu Ala Gly Pro Leu His Gly Arg Ala
    290                 295                 300

Asn Gln Glu Val Leu Glu Trp Leu Phe Lys Leu Arg Glu Glu Leu Asn
305                 310                 315                 320

Gly Asp Tyr Ser Lys Glu Ala Ile Glu Lys Tyr Leu Trp Glu Thr Leu
                325                 330                 335

Asn Ser Gly Arg Val Val Pro Gly Tyr Gly His Ala Val Leu Arg Lys
            340                 345                 350

Thr Asp Pro Arg Tyr Thr Ala Gln Arg Glu Phe Ala Leu Lys His Met
        355                 360                 365

Pro Asp Tyr Glu Leu Phe Lys Leu Val Ser Asn Ile Tyr Glu Val Ala
```

```
    370                 375                 380

Pro Gly Val Leu Thr Lys His Gly Lys Thr Lys Asn Pro Trp Pro Asn
385                 390                 395                 400

Val Asp Ser His Ser Gly Val Leu Leu Gln Tyr Tyr Gly Leu Thr Glu
                405                 410                 415

Gln Ser Phe Tyr Thr Val Leu Phe Gly Val Ser Arg Ala Phe Gly Val
            420                 425                 430

Leu Pro Gln Leu Ile Leu Asp Arg Gly Ile Gly Met Pro Ile Glu Arg
        435                 440                 445

Pro Lys Ser Phe Ser Thr Glu Lys Tyr Ile Glu Leu Val Lys Asn Ile
    450                 455                 460

Asn Lys
465
```

(2) INFORMATION FOR SEQ ID NO: 49:

(i) SEQUENCE CHARACTERISTICS:
(A) LENGTH: 678 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 49:

```
TTTTCTGATT ATCATGTTAT TTGGTTAGCT AAACGGAATA ATGGGATAAT GGAAGCTGAA    60
TATCGATTAT ATTTATTAGT TATCACTTTA ATCATTTCAC CCGTAGGGTT AATTATGTTT   120
GGTGTTGGTG CCGCTAGAGA ATGGCCATGG CAAGTGATTT ATGTTGGATT AGGTTTCATT   180
GGGTTTGGTT GGGGATCAAT TGGTGATACT TCAATGTCTT ATTTAATGGA TGCTTATCCT   240
GATATTGTCA TTCAAGGAAT GGTGGGAGTA AGTATTATTA ATAATACTTT GGCTTGTATT   300
TTCACTTTTG CTTGTTCTTA TTGGTTAGAT GGATCAGGAA CACAAAACAC ATATATTGCC   360
TTGTCAATTA TTGATTTTGC TACCATAGCA TTGGTTTTCC CCTTTTTATA TTATGGTAAA   420
ACATTTAGAA GGAAAACTAA AAGACTTTAT GTTTCAATGG TTGAATTGAC TCAAGGGATG   480
GGATAAGAGA GTGAGTGGTA AAAGAATTTT ATTAATGATA CATTTATTAT TAGAATTACT   540
ACTATGGAAA TCCGAGTCTG TGTTTTTTTT AGAAGTATAT TTTAGACGTA TTTAGAGTTG   600
TTTTTCTCCT TTGTACTTTA TTTAGCATTT TATAATATAT TAATTCAAGT TGCATTAATA   660
TATATAAATA AAAAAACT                                                 678
```

(2) INFORMATION FOR SEQ ID NO: 50:

(i) SEQUENCE CHARACTERISTICS:
(A) LENGTH: 159 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 50:

```
Ser Asp Tyr His Val Ile Trp Leu Ala Lys Arg Asn Asn Gly Ile Met
1               5                   10                  15

Glu Ala Glu Tyr Arg Leu Tyr Leu Leu Val Ile Thr Leu Ile Ile Ser
            20                  25                  30

Pro Val Gly Leu Ile Met Phe Gly Val Gly Ala Ala Arg Glu Trp Pro
        35                  40                  45

Trp Gln Val Ile Tyr Val Gly Leu Gly Phe Ile Gly Phe Gly Trp Gly
    50                  55                  60

Ser Ile Gly Asp Thr Ser Met Ser Tyr Leu Met Asp Ala Tyr Pro Asp
65                  70                  75                  80

Ile Val Ile Gln Gly Met Val Gly Val Ser Ile Ile Asn Asn Thr Leu
                85                  90                  95

Ala Cys Ile Phe Thr Phe Ala Cys Ser Tyr Trp Leu Asp Gly Ser Gly
            100                 105                 110

Thr Gln Asn Thr Tyr Ile Ala Leu Ser Ile Ile Asp Phe Ala Thr Ile
        115                 120                 125

Ala Leu Val Phe Pro Phe Leu Tyr Tyr Gly Lys Thr Phe Arg Arg Lys
    130                 135                 140

Thr Lys Arg Leu Tyr Val Ser Met Val Glu Leu Thr Gln Gly Met
145                 150                 155
```

(2) INFORMATION FOR SEQ ID NO: 51:

(i) SEQUENCE CHARACTERISTICS:
(A) LENGTH: 1480 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(ix) FEATURE:
(A) NAME/KEY: misc_feature
(B) LOCATION:1060
(D) OTHER INFORMATION:/note= "R = A or G"

(ix) FEATURE:
(A) NAME/KEY: misc_feature
(B) LOCATION:1063
(D) OTHER INFORMATION:/note= "Y = C or T"

(ix) FEATURE:
(A) NAME/KEY: misc_feature
(B) LOCATION:1123
(D) OTHER INFORMATION:/note= "Y = C or T"

(xi) SEQUENCE DESCRIPTION: SEQ ID NO: 51:

```
TTTGGATTTT CAATTACAAG ATATTTTGCA TCATGTTGAA AGCAAATGGT TTGGTGGGTT      60

TATTTCAGGT ATTTTCACTA ATGACAATGA CGTTGAAAAT GAATCCAAGA ACGTGTTTCA     120

TAAATTCAAA CAAGATTTAA TGAAAATTTT GAAAGATTGT TTAACCGTAA GTGACGATAA     180
```

```
ATCGAATATA GAGAGGTTTC TTCAGTTTAA TGAATTTATT TATTACTGCT TTTACTCAAT     240
GGAGGAATAT AATTATGAAT TGGTTGATGA TTTGATAAAA TTTATAACTA TAAATATGAA     300
TTCTCATGGC AGAATAGTTA ATTTTGGCAC TAATGTTAAA ATTAATAAAT TACACGAATT     360
AATTAAGAAT TTGATTGATA AAGTTAATAA AAACAAACAA AATGTGACTA GCAACAACAA     420
AAACAACAAC AACAACAACA GCAACAACAA CAGCAACAGC AACAATTCCC AACATATTGT     480
TTTGATACCT AATGCCAACT GTTCCAATTT CCCATGGGAA TCGATGGAAT TTCTTCGTAG     540
TAAATCAATT TCAAGAATGC CATCAATTCA TATGTTACTT GATCTAGTCA AATCAAACAC     600
CAATAACAAG AACAAGTTAA TGTTTGTTGA TAAATCTAAT TTGTATTATT TGATTAATCC     660
CAGTGGTGAT TTAATTCGAT CAGAAAATCG ATTCAAAAAA CTATTTGAAT CAAATCATTT     720
ATGGAGAGGG GAAATTGGAA AATTATCAAG TAATGAACAT GAAGATTATC AAGATTCAAT     780
ATTATGTGAA ATCTTGAAAA GTCATTTATT TGTTTATATT GGTCATGGTG GTTGTGATCA     840
ATATATTAAA GTATCAAAAT TATTTAAAAA ATGTGGCAAT AATCAAGATT TACTGAATAA     900
ATTACCTCCT AGTTTATTGT TAGGTTGTTC ATCAGTTAAA TTAGATAATT GTAATTATAA     960
CTATAATTCC AGTATGTTAC AACCACTGGG TAATATTTAT AATTGGTTGA ACTGTAAATC    1020
GTCAATGATA CTCGGGAATC TATGGGATGT TACTGATAAR GAYATTGATA TTTTTACACT    1080
TTCATTACTA CAAAAATGGG GGTTAATAGA TGATTATAAT GGYAGTGGCC ATGATTATGG    1140
TATGAAGAAA TTGGATTTGA CTAATTGTGT TGTTCAAAGT CGAAGTAAAT GTACTTTGAA    1200
ATACTTGAAT GGATCAGCAC CTGTGGTTTA TGGTCTACCA ATGTATTTAA AATAGACATT    1260
CTGTTTGCAT ATAAGTTTAT ATATTTTAAT AATAAGAAAA AGAGCATAAT TTGGATCTTG    1320
ATTTTGTATT GTTTGGTTTG TTATGAACAA ATTTTGCACC CAATCACTAT CGAACTTTCT    1380
TTTTTAAACA GAGAACATTT AATCAACATT TATGTTACAT TTAAGCGTTT AAATACATAT    1440
TTGTGTTAGA TAGTTATATA ATGTTTGATG CAAACATACA                          1480
```

(2) INFORMATION FOR SEQ ID NO: 52:

(i) SEQUENCE CHARACTERISTICS:
(A) LENGTH: 417 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 52:

```
Leu Asp Phe Gln Leu Gln Asp Ile Leu His His Val Glu Ser Lys Trp
1               5                   10                  15

Phe Gly Gly Phe Ile Ser Gly Ile Phe Thr Asn Asp Asn Asp Val Glu
            20                  25                  30

Asn Glu Ser Lys Asn Val Phe His Lys Phe Lys Gln Asp Leu Met Lys
        35                  40                  45

Ile Leu Lys Asp Cys Leu Thr Val Ser Asp Asp Lys Ser Asn Ile Glu
```

```
            50                  55                  60

        Arg Phe Leu Gln Phe Asn Glu Phe Ile Tyr Tyr Cys Phe Tyr Ser Met
        65                  70                  75                  80

        Glu Glu Tyr Asn Tyr Glu Leu Val Asp Asp Leu Ile Lys Phe Ile Thr
                        85                  90                  95

        Ile Asn Met Asn Ser His Gly Arg Ile Val Asn Phe Gly Thr Asn Val
                    100                 105                 110

        Lys Ile Asn Lys Leu His Glu Leu Ile Lys Asn Leu Ile Asp Lys Val
                115                 120                 125

        Asn Lys Asn Lys Gln Asn Val Thr Ser Asn Asn Lys Asn Asn Asn Asn
            130                 135                 140

        Asn Asn Ser Asn Asn Asn Ser Asn Ser Asn Asn Ser Gln His Ile Val
        145                 150                 155                 160

        Leu Ile Pro Asn Ala Asn Cys Ser Asn Phe Pro Trp Glu Ser Met Glu
                        165                 170                 175

        Phe Leu Arg Ser Lys Ser Ile Ser Arg Met Pro Ser Ile His Met Leu
                    180                 185                 190

        Leu Asp Leu Val Lys Ser Asn Thr Asn Asn Lys Asn Lys Leu Met Phe
                195                 200                 205

        Val Asp Lys Ser Asn Leu Tyr Tyr Leu Ile Asn Pro Ser Gly Asp Leu
            210                 215                 220

        Ile Arg Ser Glu Asn Arg Phe Lys Lys Leu Phe Glu Ser Asn His Leu
        225                 230                 235                 240

        Trp Arg Gly Glu Ile Gly Lys Leu Ser Ser Asn Glu His Glu Asp Tyr
                        245                 250                 255

        Gln Asp Ser Ile Leu Cys Glu Ile Leu Lys Ser His Leu Phe Val Tyr
                    260                 265                 270

        Ile Gly His Gly Gly Cys Asp Gln Tyr Ile Lys Val Ser Lys Leu Phe
                275                 280                 285

        Lys Lys Cys Gly Asn Asn Gln Asp Leu Ser Asn Lys Leu Pro Pro Ser
            290                 295                 300

        Leu Leu Leu Gly Cys Ser Ser Val Lys Leu Asp Asn Cys Asn Tyr Asn
        305                 310                 315                 320

        Tyr Asn Ser Ser Met Leu Gln Pro Ser Gly Asn Ile Tyr Asn Trp Leu
                        325                 330                 335

        Asn Cys Lys Ser Ser Met Ile Leu Gly Asn Leu Trp Asp Val Thr Asp
                    340                 345                 350

        Xaa Xaa Ile Asp Ile Phe Thr Leu Ser Leu Leu Gln Lys Trp Gly Leu
                355                 360                 365

        Ile Asp Asp Tyr Asn Xaa Ser Gly His Asp Tyr Gly Met Lys Lys Leu
            370                 375                 380

        Asp Leu Thr Asn Cys Val Val Gln Ser Arg Ser Lys Cys Thr Leu Lys
        385                 390                 395                 400

        Tyr Leu Asn Gly Ser Ala Pro Val Val Tyr Gly Leu Pro Met Tyr Leu
                        405                 410                 415

        Lys
```

(2) INFORMATION FOR SEQ ID NO: 53:

(i) SEQUENCE CHARACTERISTICS:
(A) LENGTH: 1443 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 53:

```
CTTCTTTTAG AGACAATGCA GTGGTTTTCT TACCAGATGC ATGACCCCCA CCCAATAAAA     60
CTATAATCGA TCTATTCACA GTATTTGATG CCATTTTGAT GGTGATGAAT GATGTGATGT    120
GATGCTCATC TTATTGGGAG TTTCAAAAAA AAAAGTTACA CTCGAAAAAA AAAAAATAGC    180
ATTATAAATA GAAGCTTTAC TATCTTATAG AACAAAACAA AAAACACTAT CTTCTAATTA    240
ATAATGGATG ATTTTGATAG AGATTTAGAT AATGAGTTGG AATTTAGTCA TAAATCAACG    300
AAAGGAATAA AGGTTCATCG CACTTTTGAA AGTATGAATT TGAAACCTGA TCTTTTGAAA    360
GGAATATATG CCTATGGATT TGAAGCACCA TCTGCTATTC AATCTAGGGC TATTATGCAG    420
ATCATCAGTG GTAGAGACAC AATAGCACAG GCACAATCTG GAACTGGTAA AACTGCTACT    480
TTTTCTATTG GTATGCTTGA GGTTATAGAT ACTAAATCAA AAGAGTGTCA AGCACTTATC    540
TTGTCTCCTA CTAGAGAGTT GGCAATTCAA ATACAAAATG TGGTCATGCA TTTAGGAGAT    600
TATATGAACA TTCACACCCA TGCCTGTATT GGTGGGAAAA ATGTCGGTGA GGATGTTAAG    660
AAATTGCAGC AAGGGCAACA AATAGTTAGT GGGACACCAG GTAGAGTGAT TGATGTGATA    720
AAAAGAAGAA ATCTACAAAC TAGAAATATC AAGGTTCTTA TTTTAGATGA AGCTGATGAA    780
CTTTTTACAA AAGGGTTTAA AGAACAGATC TACGAAATCT ACAAACATTT ACCACCTTCG    840
GTTCAAGTAG TAGTTGTTAG TGCCACTTTG CCACGTGAAG TATTGGAGAT GACAAGTAAG    900
TTTACCACTG ATCCAGTGAA AATCTTGGTG AAGAGGGATG AGATTTCGCT TCTGGGAATC    960
AAACAATATT ATGTTCAATG TGAACGTGAA GATTGGAAGT TTGATACACT ATGTGATTTG   1020
TATGACAACC TTACAATAAC TCAAGCAGTG ATATTTTGTA ATACCAAATT GAAGGTGAAT   1080
TGGCTTGCTG ATCAAATGAA AAAGCAAAAC TTTACTGTTG TGGCAATGCA TGGTGATATG   1140
AAACAAGATG AACGAGATTC AATTATGAAC GATTTTAGAA GGGGGAATTC AAGAGTATTA   1200
ATATCTACAG ATGTTTGGGC AAGAGGTATT GATGTCCAAC AAGTCTCGTT GGTAATAAAT   1260
TATGATTTGC CCACCGATAA GGAAAACTAT ATTCATAGAA TTGGACGATC AGGTAGATTT   1320
GGTAGAAAGG GAACAGCTAT AAACTTGATA ACTAAAGATG ATGTGGTCAC TTTAAAAGAA   1380
TTGGAGAAAT ATTATTCAAC GAAAATTAAG GAAATGCCAA TGAATATTAA TGATATAATG   1440
TAA                                                                 1443
```

(2) INFORMATION FOR SEQ ID NO: 54:

(i) SEQUENCE CHARACTERISTICS:
(A) LENGTH: 399 amino acids
(B) TYPE: amino acid
(C) STRANDEDNESS:

(D) TOPOLOGY: unknown

(ii) MOLECULE TYPE: peptide

(iii) HYPOTHETICAL: NO

(xi) SEQUENCE DESCRIPTION: SEQ ID NO: 54:

```
Met Asp Asp Phe Asp Arg Asp Leu Asp Asn Glu Leu Glu Phe Ser His
1               5                   10                  15

Lys Ser Thr Lys Gly Ile Lys Val His Arg Thr Phe Glu Ser Met Asn
            20                  25                  30

Leu Lys Pro Asp Leu Leu Lys Gly Ile Tyr Ala Tyr Gly Phe Glu Ala
        35                  40                  45

Pro Ser Ala Ile Gln Ser Arg Ala Ile Met Gln Ile Ile Ser Gly Arg
    50                  55                  60

Asp Thr Ile Ala Gln Ala Gln Ser Gly Thr Gly Lys Thr Ala Thr Phe
65                  70                  75                  80

Ser Ile Gly Met Leu Glu Val Ile Asp Thr Lys Ser Lys Glu Cys Gln
                85                  90                  95

Ala Leu Ile Leu Ser Pro Thr Arg Glu Leu Ala Ile Gln Ile Gln Asn
            100                 105                 110

Val Val Met His Leu Gly Asp Tyr Met Asn Ile His Thr His Ala Cys
        115                 120                 125

Ile Gly Gly Lys Asn Val Gly Glu Asp Val Lys Lys Leu Gln Gln Gly
    130                 135                 140

Gln Gln Ile Val Ser Gly Thr Pro Gly Arg Val Ile Asp Val Ile Lys
145                 150                 155                 160

Arg Arg Asn Leu Gln Thr Arg Asn Ile Lys Val Leu Ile Leu Asp Glu
                165                 170                 175

Ala Asp Glu Leu Phe Thr Lys Gly Phe Lys Glu Gln Ile Tyr Glu Ile
            180                 185                 190

Tyr Lys His Leu Pro Pro Ser Val Gln Val Val Val Val Ser Ala Thr
        195                 200                 205

Leu Pro Arg Glu Val Leu Glu Met Thr Ser Lys Phe Thr Thr Asp Pro
    210                 215                 220

Val Lys Ile Leu Val Lys Arg Asp Glu Ile Ser Leu Ser Gly Ile Lys
225                 230                 235                 240

Gln Tyr Tyr Val Gln Cys Glu Arg Glu Asp Trp Lys Phe Asp Thr Leu
                245                 250                 255

Cys Asp Leu Tyr Asp Asn Leu Thr Ile Thr Gln Ala Val Ile Phe Cys
            260                 265                 270

Asn Thr Lys Leu Lys Val Asn Trp Leu Ala Asp Gln Met Lys Lys Gln
        275                 280                 285

Asn Phe Thr Val Val Ala Met His Gly Asp Met Lys Gln Asp Glu Arg
    290                 295                 300

Asp Ser Ile Met Asn Asp Phe Arg Arg Gly Asn Ser Arg Val Leu Ile
305                 310                 315                 320

Ser Thr Asp Val Trp Ala Arg Gly Ile Asp Val Gln Gln Val Ser Leu
```

```
                325                 330                 335
Val Ile Asn Tyr Asp Leu Pro Thr Asp Lys Glu Asn Tyr Ile His Arg
            340                 345                 350
Ile Gly Arg Ser Gly Arg Phe Gly Arg Lys Gly Thr Ala Ile Asn Leu
        355                 360                 365
Ile Thr Lys Asp Asp Val Val Thr Leu Lys Glu Leu Glu Lys Tyr Tyr
    370                 375                 380
Ser Thr Lys Ile Lys Glu Met Pro Met Asn Ile Asn Asp Ile Met
385                 390                 395
```

(2) INFORMATION FOR SEQ ID NO: 55:

(i) SEQUENCE CHARACTERISTICS:
(A) LENGTH: 1020 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 55:

```
AACGTTGGCC TGGCCCAGTT AATTCCGTTT CCAAGCAAAT GAATGTCGAT ACCGACATCA     60
TCACGTTGAC CCGTTTTATT TTACAAGAAC AGCAAACTGT TGCTCCCACC GCCACCGGTG    120
AGTTGTCGTT GTTGTTGAAT GCGCTTCAAT TTGCATTCAA GTTTATTGCC CACAATATCA    180
GAAGAGCTGA GTTGGTCAAC CTTATTGGTG TTTCTGGCTC TGCCAACTCT ACCGGTGATG    240
TTCAGAAGAA ATTGGATGTG ATTGGTGATG AGATCTTTAT CAATGCCATG AGATCTTCCA    300
ACAACGTCAA GGTTTTGGTT TCTGAAGAGC AAGAAGACCT TATTGTGTTC CCAGGTGGTG    360
GCACATATGC TGTTTGTACT GATCCAATTG ATGGGTCGTC CAATATCGAT GCTGGTGTTT    420
CTGTTGGTAC GATTTTTGGT GTGTACAAGT TGCAAGAGGG GTCTACTGGT GGCATCAGCG    480
ATGTCTTGCG TCCTGGTAAG GAGATGGTCG CTGCGGGGTA CACCATGTAC GGTGCATCTG    540
CCCATTTGGC ATTGACTACA GGTCACGGTG TCAATCTTTT TACTTTGGAT ACTCAGTTGG    600
GTGAATTTAT CTTGACCCAT CCAAACTTGA AGTTGCCAGA TACTAAGAAC ATCTACTCGT    660
TGAATGAAGG GTACTCGAAC AAATTCCCAG AATACGTTCA AGATTATCTG AAGGACATTA    720
AAAAGGAAGG GTACAGTTTG AGATACATTG GACTGATGGT TGCTGATGTC CATCGTACTC    780
TTTTGTATGG TGGTATTTTT GCTTACCCTA CATTAAAGTT GAGAGTGTTG TATGAATGTT    840
TCCCCATGGC CTTGTTGATG GAACAAGCAG GCGGTTCTGC TGTCACCATC AAGGGTGAGA    900
GGATCTTGGA TATCTTGCCA AAAGGTATAC ACGACAAGAG TTCTATTGTG TTGGGATCCA    960
AGGGTGAAGT TGAAAAGTAT TTAAAGCATG TACCAAAATA GATTATGTAG AAAATTTATG   1020
```

(2) INFORMATION FOR SEQ ID NO: 56:

(i) SEQUENCE CHARACTERISTICS:
(A) LENGTH: 320 amino acids
(B) TYPE: amino acid
(C) STRANDEDNESS:

(D) TOPOLOGY: unknown

(ii) MOLECULE TYPE: peptide

(iii) HYPOTHETICAL: NO

(xi) SEQUENCE DESCRIPTION: SEQ ID NO: 56:

```
Met Asn Val Asp Thr Asp Ile Ile Thr Leu Thr Arg Phe Ile Leu Gln
1               5                   10                  15

Glu Gln Gln Thr Val Ala Pro Thr Ala Thr Gly Glu Leu Ser Leu Leu
            20                  25                  30

Leu Asn Ala Leu Gln Phe Ala Phe Lys Phe Ile Ala His Asn Ile Arg
        35                  40                  45

Arg Ala Glu Leu Val Asn Leu Ile Gly Val Ser Gly Ser Ala Asn Ser
    50                  55                  60

Thr Gly Asp Val Gln Lys Lys Leu Asp Val Ile Gly Asp Glu Ile Phe
65                  70                  75                  80

Ile Asn Ala Met Arg Ser Ser Asn Asn Val Lys Val Leu Val Ser Glu
                85                  90                  95

Glu Gln Glu Asp Leu Ile Val Phe Pro Gly Gly Gly Thr Tyr Ala Val
            100                 105                 110

Cys Thr Asp Pro Ile Asp Gly Ser Ser Asn Ile Asp Ala Gly Val Ser
        115                 120                 125

Val Gly Thr Ile Phe Gly Val Tyr Lys Leu Gln Glu Gly Ser Thr Gly
    130                 135                 140

Gly Ile Ser Asp Val Leu Arg Pro Gly Lys Glu Met Val Ala Ala Gly
145                 150                 155                 160

Tyr Thr Met Tyr Gly Ala Ser Ala His Leu Ala Leu Thr Thr Gly His
                165                 170                 175

Gly Val Asn Leu Phe Thr Leu Asp Thr Gln Leu Gly Glu Phe Ile Leu
            180                 185                 190

Thr His Pro Asn Leu Lys Leu Pro Asp Thr Lys Asn Ile Tyr Ser Leu
        195                 200                 205

Asn Glu Gly Tyr Ser Asn Lys Phe Pro Glu Tyr Val Gln Asp Tyr Ser
    210                 215                 220

Lys Asp Ile Lys Lys Glu Gly Tyr Ser Leu Arg Tyr Ile Gly Ser Met
225                 230                 235                 240

Val Ala Asp Val His Arg Thr Leu Leu Tyr Gly Gly Ile Phe Ala Tyr
                245                 250                 255

Pro Thr Leu Lys Leu Arg Val Leu Tyr Glu Cys Phe Pro Met Ala Leu
            260                 265                 270

Leu Met Glu Gln Ala Gly Gly Ser Ala Val Thr Ile Lys Gly Glu Arg
        275                 280                 285

Ile Leu Asp Ile Leu Pro Lys Gly Ile His Asp Lys Ser Ser Ile Val
    290                 295                 300

Leu Gly Ser Lys Gly Glu Val Glu Lys Tyr Leu Lys His Val Pro Lys
305                 310                 315                 320
```

(2) INFORMATION FOR SEQ ID NO: 57:

(i) SEQUENCE CHARACTERISTICS:
(A) LENGTH: 825 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 57:

```
AACCCCACCT TCAAAGACAA AGAAGATTTC GTCAAGCAAA CGAATGTCAG AGCAGAAAAG     60
AACCAAGAAC TAATCAAATT TGCCCGTGAC AACCTTAACC ATTTACCATT CACCGAAAAA    120
GACGGAGGTG CATGGGAAAA CTATGAACGA ATGATCAGTG GTATGCTCTA CAACTGTTTA    180
CAAAAAGAAT TGGAAACAAC ACGTATGTCT TGCAGAGACT ACATGTTGGA CTACGGCAGT    240
TTCAGAACTA GAGATTATAA AACAACCCAA GAATTTCTTG ATGCAAAATA CAAACATTTA    300
GAAAGTTTCA TTGGACATGT TGGCAAAAAT GCATTTATGG AATATCCAAT CTATTTTGAT    360
TATGGGTTTA ACACTTATTT GGGTGATAAT TTCTATTCCA ATTACAATTT GACAATTTTG    420
GATGTTTCCA TAGTCAGAAT TGGTAATAAT GTCAAGTGTG GTCCCAATGT ATCTATCCTT    480
ACCCCAACAC ACCCAGTGGA TCCCACTTTG CGCTATGATC AATTGGAAAA TGCCTTGCCT    540
GTGACGGTGG GTAACGGGGT CTGGTTGTGT GGAAGCTGTA CCATTCTTGG TGGGGTGACA    600
GTAGGTGATG GCAGCATTGT GGCTGCTGGT GCAGTTGTCA ACAAGGACGT TCCACCAAAC    660
ACTGTAGTTG CGGGAGTTCC TGCTAGGGTA GTTAAGCAGC TAGAACCTAG AGACCCTAAC    720
TTTGACACTA TGGCAGTTTT GAAACAATAT GGTATGGGTT ATATAGATTA GTAATTAGAT    780
TTGATGTAAT GTACACGACT ACACTATTTG CTGGTGTCTG TTTTT                    825
```

(2) INFORMATION FOR SEQ ID NO: 58:

(i) SEQUENCE CHARACTERISTICS:
(A) LENGTH: 206 amino acids
(B) TYPE: amino acid
(C) STRANDEDNESS: single
(D) TOPOLOGY: linear

(ii) MOLECULE TYPE: peptide

(iii) HYPOTHETICAL: NO

(xi) SEQUENCE DESCRIPTION: SEQ ID NO: 58:

```
Met Ile Ser Gly Met Leu Tyr Asn Cys Leu Gln Lys Glu Leu Glu Thr
1               5                   10                  15

Thr Arg Met Ser Cys Arg Asp Tyr Met Leu Asp Tyr Gly Ser Phe Arg
            20                  25                  30

Thr Arg Asp Tyr Lys Thr Thr Gln Glu Phe Leu Asp Ala Lys Tyr Lys
        35                  40                  45

His Leu Glu Ser Phe Ile Gly His Val Gly Lys Asn Ala Phe Met Glu
```

```
                50                  55                  60

            Tyr Pro Ile Tyr Phe Asp Tyr Gly Phe Asn Thr Tyr Leu Gly Asp Asn
            65                  70                  75                  80

            Phe Tyr Ser Asn Tyr Asn Leu Thr Ile Leu Asp Val Ser Ile Val Arg
                            85                  90                  95

            Ile Gly Asn Asn Val Lys Cys Gly Pro Asn Val Ser Ile Leu Thr Pro
                        100                 105                 110

            Thr His Pro Val Asp Pro Thr Leu Arg Tyr Asp Gln Leu Glu Asn Ala
                    115                 120                 125

            Leu Pro Val Thr Val Gly Asn Gly Val Trp Leu Cys Gly Ser Cys Thr
                130                 135                 140

            Ile Leu Gly Gly Val Thr Val Gly Asp Gly Ser Ile Val Ala Ala Gly
            145                 150                 155                 160

            Ala Val Val Asn Lys Asp Val Pro Pro Asn Thr Val Val Ala Gly Val
                            165                 170                 175

            Pro Ala Arg Val Val Lys Gln Leu Glu Pro Arg Asp Pro Asn Phe Asp
                        180                 185                 190

            Thr Met Ala Val Leu Lys Gln Tyr Gly Met Gly Tyr Ile Asp
                    195                 200                 205
```

(2) INFORMATION FOR SEQ ID NO: 59:

(i) SEQUENCE CHARACTERISTICS:
(A) LENGTH: 1380 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 59:

```
AATTACAATC TGGTTTGTTA CTACCATATC CCATTAGTGT TATTGTCATT GTAGATATTG      60
ATAATGGTTA AAGGATTGGT TTTCATTTTT TGTGTAATGA ATGAGCCAAA ATAAAAAATC     120
AATTCGATGC GATGCAATGA AGTTTAATAA AATTTTTTTT TTTCTTTATT TCTTTTAATC     180
AACCCATCAA TCATTAAATT GAATCAATAC CTACCATTAA CATACTTCTA TATACATATA     240
TATATATAAC AAAATATCAT GGGGAAGATA ACAACTAGTG ATACTAAAAC AAAACAACGT     300
CATAATCCAT TATTAAAAGA TATTTCATCC CAAGGTGGGA ATTTAAGAAC CGTTCCAAGA     360
TCATCATCAT CATCATCATC ACAAAAGAAG AAATCATCAA AGAAACAAAG ACATAACGAT     420
GAAGACGACG AAGAAAATGG TGGCGGTGAA GGATTTTTAG ATGCTTCTAG TTCAAGAAAG     480
ATTTTACAAT TGGCAAAAGA ACAACAAGAT GAACTTGAAC AAGAAGATGA AATACAAAAT     540
AAACCTTCAT TTGCTCAATC ATTTAAAAAT CAACAAATAG ATAGTGAAGA AGAAGAAGAG     600
GAAGATGAGT ATTCAGATTT TGAAGAAGAA GAAGAAGTTG AAGAGATAGT ATATGATGAA     660
GAAGATGCAG AAGTTGATCC CAAAGATGCA GAATTATTTA ATAAATATTT CCAATCCAAC     720
GGTGAAGCTA ATAATAATGA TGATGATAAT TCATTTCAAC CAACAATAAA TTTAGCTGAT     780
```

```
AAAATCTTAG CCAAAATTCA AGAAAAAGAA TCCCAACAAC AACAACAACA ACAAAGCTCT      840
CCAGATAATA GTAATGAAGA TGCCGTATTG TTACCACCAA AAGTCATTTT AGCTTATGAA      900
AAAATTGGTC AAATTTTATC AACTTATACT CATGGGAAAT TACCTAAATT ATTTAAAATT      960
TTACCAAGTT TAAAAAATTG GCAAGATGTA TTATACGTGA CAAATCCAAA TAGTTGGACT     1020
CCTCATGCCA CATATGAAGC AACTAAATTA TTTGTGTCGA ATTTATCAAG TAATGAAGCT     1080
ACAGTTTTCA TTGAAACTAT CTTGTTGCCA CGATTCCGTG ATTCTATTGA AAATTCCGAT     1140
GATCATTCAT TAAATTATCA TATTTATCGA GCATTAAAAA AATCATTATA TAAACCAGGA     1200
GCTTTTTTCA AAGGGTTCTT GTTACCTTTA GTCGATGGTT ATTGTTCTGT ACGTGAAGCC     1260
ACTATTGCTG CTTCAGTGTT AACTAAAGTT TCTGTCCCTG TTTTACATTC ATGTCATTAT     1320
TGTGGCGTAC TGATGAATAA AAAACGAGAA TCACCTGTAT TTGTCCTACG GCGAATATAA     1380
```

(2) INFORMATION FOR SEQ ID NO: 60:

(i) SEQUENCE CHARACTERISTICS:
(A) LENGTH: 373 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 60:

```
Met Gly Lys Ile Thr Thr Ser Asp Thr Lys Thr Lys Gln Arg His Asn
1               5                   10                  15

Pro Leu Leu Lys Asp Ile Ser Ser Gln Gly Gly Asn Leu Arg Thr Val
            20                  25                  30

Pro Arg Ser Ser Ser Ser Ser Ser Ser Gln Lys Lys Lys Ser Ser Lys
        35                  40                  45

Lys Gln Arg His Asn Asp Glu Asp Asp Glu Glu Asn Gly Gly Gly Glu
    50                  55                  60

Gly Phe Leu Asp Ala Ser Ser Ser Arg Lys Ile Leu Gln Leu Ala Lys
65                  70                  75                  80

Glu Gln Gln Asp Glu Leu Glu Gln Glu Asp Glu Ile Gln Asn Lys Pro
                85                  90                  95

Ser Phe Ala Gln Ser Phe Lys Asn Gln Gln Ile Asp Ser Glu Glu Glu
            100                 105                 110

Glu Glu Glu Asp Glu Tyr Ser Asp Phe Glu Glu Glu Glu Glu Val Glu
        115                 120                 125

Glu Ile Val Tyr Asp Glu Glu Asp Ala Glu Val Asp Pro Lys Asp Ala
    130                 135                 140

Glu Leu Phe Asn Lys Tyr Phe Gln Ser Asn Gly Glu Ala Asn Asn Asn
145                 150                 155                 160

Asp Asp Asp Asn Ser Phe Gln Pro Thr Ile Asn Leu Ala Asp Lys Ile
                165                 170                 175

Leu Ala Lys Ile Gln Glu Lys Glu Ser Gln Gln Gln Gln Gln Gln Gln
```

```
                180                           185                           190

        Ser Ser Pro Asp Asn Ser Asn Glu Asp Ala Val Leu Leu Pro Pro Lys
                195                 200                 205

        Val Ile Leu Ala Tyr Glu Lys Ile Gly Gln Ile Leu Ser Thr Tyr Thr
            210                 215                 220

        His Gly Lys Leu Pro Lys Leu Phe Lys Ile Leu Pro Ser Leu Lys Asn
        225                 230                 235                 240

        Trp Gln Asp Val Leu Tyr Val Thr Asn Pro Asn Ser Trp Thr Pro His
                        245                 250                 255

        Ala Thr Tyr Glu Ala Thr Lys Leu Phe Val Ser Asn Leu Ser Ser Asn
                    260                 265                 270

        Glu Ala Thr Val Phe Ile Glu Thr Ile Leu Leu Pro Arg Phe Arg Asp
                275                 280                 285

        Ser Ile Glu Asn Ser Asp Asp His Ser Leu Asn Tyr His Ile Tyr Arg
            290                 295                 300

        Ala Leu Lys Lys Ser Leu Tyr Lys Pro Gly Ala Phe Phe Lys Gly Phe
        305                 310                 315                 320

        Leu Leu Pro Leu Val Asp Gly Tyr Cys Ser Val Arg Glu Ala Thr Ile
                        325                 330                 335

        Ala Ala Ser Val Leu Thr Lys Val Ser Val Pro Val Leu His Ser Cys
                    340                 345                 350

        His Tyr Cys Gly Val Ser Met Asn Lys Lys Arg Glu Ser Pro Val Phe
                355                 360                 365

        Val Leu Arg Arg Ile
            370
```

(2) INFORMATION FOR SEQ ID NO: 61:

(i) SEQUENCE CHARACTERISTICS:
(A) LENGTH: 823 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 61:

```
AACCAACAAT GAGTCAAGTC GCTCCAAAGT GGTACCAATC AGAAGACGTT CCAGCTCCAA      60
AACAAACCAG AAAGACTGCT CGTCCACAAA AATTACGTGC CTCTTTAGTC CCAGGTACCG     120
TTTTAATTTT ATTGGCCGGT AGATTCAGAG GTAAAAGAGT TGTTTACTTG AAGAACTTGG     180
AAGACAACAC CTTATTGGTT TCTGGTCCAT TCAAAGTCAA TGGTGTTCCA TTGAGAAGAG     240
TTAACGCTAG ATACGTTATC GCCACCTCCA CCAAAGTCAA CGTTTCTGGT GTTGATGTTT     300
CTAAATTCAA CGTCGAATAC TTTGCTAGAG AAAAATCTTC TAAATCTAAA AAATCCGAAG     360
CTGAATTCTT CAATGAATCT CAACCAAAGA AAGAAATCAA AGCTGAAAGA GTTGCTGACC     420
AAAAATCTGT CGATGCTGCT TTATTAAGTG AAATCAAAAA GACCCCATTA TTGAAACAAT     480
ACTTGGCCGC TTCATTCTCT TTGAAGAACG GTGACAGACC ACACTTGTTA AAATTTTAAT     540
```

```
TTAGGTGAAA TTAATATTTT GCAAACATGT TCATGATAAA TAACAATGTG GCTTTTAAAG     600
CAATGGATGG GATATGGTTA AGAGGATGTC TTTATATTTT GAGTTTTATA TATGGGTACT     660
TTGTTTAATA ATGGAAGGTA TTGGCTCAGA TGAACTTCAA AATGGAGATT ACTTTTTTCT     720
TTTACTTTTA CAATATTTTC GTCTATTTGC TGTTTAAGCT GCAAAAACAA ATTTTTAATC     780
GGTGTATCTT AACTCTTATT CATTTTGTAT ATTTAATACA TAT                       823
```

(2) INFORMATION FOR SEQ ID NO: 62:

(i) SEQUENCE CHARACTERISTICS:
(A) LENGTH: 176 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 62:

```
Met Ser Gln Val Ala Pro Lys Trp Tyr Gln Ser Glu Asp Val Pro Ala
1               5                   10                  15

Pro Lys Gln Thr Arg Lys Thr Ala Arg Pro Gln Lys Leu Arg Ala Ser
            20                  25                  30

Leu Val Pro Gly Thr Val Leu Ile Leu Leu Ala Gly Arg Phe Arg Gly
        35                  40                  45

Lys Arg Val Val Tyr Leu Lys Asn Leu Glu Asp Asn Thr Leu Leu Val
    50                  55                  60

Ser Gly Pro Phe Lys Val Asn Gly Val Pro Leu Arg Arg Val Asn Ala
65                  70                  75                  80

Arg Tyr Val Ile Ala Thr Ser Thr Lys Val Asn Val Ser Gly Val Asp
                85                  90                  95

Val Ser Lys Phe Asn Val Glu Tyr Phe Ala Arg Glu Lys Ser Ser Lys
            100                 105                 110

Ser Lys Lys Ser Glu Ala Glu Phe Phe Asn Glu Ser Gln Pro Lys Lys
        115                 120                 125

Glu Ile Lys Ala Glu Arg Val Ala Asp Gln Lys Ser Val Asp Ala Ala
    130                 135                 140

Leu Leu Ser Glu Ile Lys Lys Thr Pro Leu Leu Lys Gln Tyr Leu Ala
145                 150                 155                 160

Ala Ser Phe Ser Leu Lys Asn Gly Asp Arg Pro His Leu Leu Lys Phe
                165                 170                 175
```

(2) INFORMATION FOR SEQ ID NO: 63:

(i) SEQUENCE CHARACTERISTICS:
(A) LENGTH: 415 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 63:

```
AACATTAAAG CAAGATGGAA AACGATAAAG GTCAATTAGT TGAATTATAC GTCCCAAGAA      60
AATGTTCTGC TACCAACAGA ATCATTAAAG CCAAAGATCA CGCTTCTGTT CAAATCTCAA     120
TTGCTAAAGT TGATGAAGAC GGTAGAGCTA TTGCTGGTGA AAACATCACT TACGCTTTAA     180
GTGGTTACGT TAGAGGTAGA GGTGAAGCTG ATGACTCATT AAACAGATTG GCTCAACAAG     240
ACGGTTTATT GAAGAACGTC TGGTCTTACT CTCGTTAAGA GAATAGAAGA ATAGACAAAA     300
TTGATAATTG GGTATTTTAA GAAATTACTT TTTTTATATT GCAAATTAAT TTTAATCTTT     360
CTTCTGTGTA TATTTAATGT CTTAACATAA TAAAAAAAAA GAATAGAAAT GGTTT          415
```

(2) INFORMATION FOR SEQ ID NO: 64:

(i) SEQUENCE CHARACTERISTICS:
(A) LENGTH: 87 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 64:

```
Met Glu Asn Asp Lys Gly Gln Leu Val Glu Leu Tyr Val Pro Arg Lys
1               5                   10                  15

Cys Ser Ala Thr Asn Arg Ile Ile Lys Ala Lys Asp His Ala Ser Val
            20                  25                  30

Gln Ile Ser Ile Ala Lys Val Asp Glu Asp Gly Arg Ala Ile Ala Gly
        35                  40                  45

Glu Asn Ile Thr Tyr Ala Leu Ser Gly Tyr Val Arg Gly Arg Gly Glu
    50                  55                  60

Ala Asp Asp Ser Leu Asn Arg Leu Ala Gln Gln Asp Gly Leu Leu Lys
65                  70                  75                  80

Asn Val Trp Ser Tyr Ser Arg
                85
```

(2) INFORMATION FOR SEQ ID NO: 65:

(i) SEQUENCE CHARACTERISTICS:
(A) LENGTH: 1519 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(ix) FEATURE:
(A) NAME/KEY: misc_feature
(B) LOCATION:749

(D) OTHER INFORMATION:/note= "N = A or T or C or G"

(xi) SEQUENCE DESCRIPTION: SEQ ID NO: 65:

```
ACCATGTGTC AAATTGCTTG GTCGTGTCCT TTCACCACAC ATTTTTTTGG ATTAAATTTC      60
TCGCACGCTC AAAAAATGAC TTCGACAAAA AGCAATGCCA CTCTTCCTAC AATTAATTCC     120
CTCCGCCCCT TCCTTTTCAT ATACTATCTC CCTTCCTTCT TCCTTCTCCT TTTATTTTTT     180
CAATTATTAC AATCTTATGT CATTTAAAGG ATTCAAAAAG GGTGTCCTTA GGGCCCCACA     240
GACAATGCGT CAGAAATTCA ACATGGGAGA AATCACCCAA GATGCTGTTT ATCTCGATGC     300
TGAAAGAAGA TTCAAAGAAA TCGAAACGGA AACAAAAAAG TTGAGTGAAG AATCCAAGAA     360
ATATTTCAAT GCTGTCAATG GGATGTTAGA TGAACAAATT GATTTTGCCA AAGCCGTGGC     420
TGAGATTTAT AAACCAATCA GTGGTAGATT ATCGGACCCC AGTGCTACGG TACCAGAAGA     480
TAACCCACAA GGTATTGAAG CATCGGAACT GTACCAAGCA GTGGTTAAAG ATCTCAAAGA     540
TACCTTAAAA CCCGATTTGG AATTGATTGA AAAAAGAATT GTTGAACCAG CACAAGAATT     600
ATTGAAGATT ATACAAGCTA TAAGGAAAAT GTCAGTGAAA AGAGACCATA AACAATTGGA     660
TTTGGATCGT CATAAGAGAA ATTTTTCTAA ATATGAACTG AAGAAAGAAA GAACTGTTAA     720
AGATGAAGAA AAAATGTTCA GTGCTCAANC AGAAGTAGAA ATTGCTCAAC AAGAGTACGA     780
TTATTATAAT GATTTGTTAA AGAATGAATT GCCAGTTTTG TTTCAAATGC AAAGTGATTT     840
TATCAAACCA TTGTTTGTTT CATTCTATTA CATGCAGTTG AATATTTTCT ACACATTATA     900
CACTAGAATG GAAGAGTTGA AAATTCCATA TTTTGATTTG TCTACTGATA TTGTCGAAGC     960
TTATACTGCC AAGAAGGGGA ACATTGAGGA ACAAACCGAT GCTATTGGAA TCACTCATTT    1020
CAAAGTCGGG CATGCCAAAT CCAAATTGGA AGCCACTAAA AGAAGACATG CTGCTATGAA    1080
TAGTCCACCT CCTACCGGTG CCAGCTCTAT TGCATCTACA GGTACTGGTG GTGAATTACC    1140
TGCATACTCC CCAGGAGGTT ACAACCAACC ATATGGTGAT AGCAAGTATC AACCACCATC    1200
TTCTCCAGCA ACATACCAAT CTCCAGTAGT AGCAGCCACT GCTCAATCTC CAGCTACTTA    1260
TCAATCGCCA GTGGCTACTG GACAACCTCC ATCATATTTA CCACAAACTC CAGCCAGTGC    1320
TCCACCACCA CAAGTTGGTA GTGGCCTTCC AACATGCACG GCTTTATACG ATTATACTGC    1380
ACAAGCCCAG GGTGACTTGA CTTTCCCTGC AGGAGCTGTT ATTGAAATTA TACAAAGAAC    1440
CGAAGATGCC AACGGATGGT GGACTGGTAA ATACAATGGT CAAACCGGTG TGTTCCCTGG    1500
TAATTATGTG CAATTATAG                                                 1519
```

(2) INFORMATION FOR SEQ ID NO: 66:

(i) SEQUENCE CHARACTERISTICS:
(A) LENGTH: 440 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 66:

```
Met Ser Phe Lys Gly Phe Lys Lys Gly Val Leu Arg Ala Pro Gln Thr
1               5                   10                  15

Met Arg Gln Lys Phe Asn Met Gly Glu Ile Thr Gln Asp Ala Val Tyr
            20                  25                  30

Leu Asp Ala Glu Arg Arg Phe Lys Glu Ile Glu Thr Glu Thr Lys Lys
        35                  40                  45

Leu Ser Glu Glu Ser Lys Lys Tyr Phe Asn Ala Val Asn Gly Met Leu
    50                  55                  60

Asp Glu Gln Ile Asp Phe Ala Lys Ala Val Ala Glu Ile Tyr Lys Pro
65                  70                  75                  80

Ile Ser Gly Arg Leu Ser Asp Pro Ser Ala Thr Val Pro Glu Asp Asn
                85                  90                  95

Pro Gln Gly Ile Glu Ala Ser Glu Ser Tyr Gln Ala Val Val Lys Asp
            100                 105                 110

Leu Lys Asp Thr Leu Lys Pro Asp Leu Glu Leu Ile Glu Lys Arg Ile
        115                 120                 125

Val Glu Pro Ala Gln Glu Leu Leu Lys Ile Ile Gln Ala Ile Arg Lys
    130                 135                 140

Met Ser Val Lys Arg Asp His Lys Gln Leu Asp Leu Asp Arg His Lys
145                 150                 155                 160

Arg Asn Phe Ser Lys Tyr Glu Ser Lys Lys Glu Arg Thr Val Lys Asp
                165                 170                 175

Glu Glu Lys Met Phe Ser Ala Gln Xaa Glu Val Glu Ile Ala Gln Gln
            180                 185                 190

Glu Tyr Asp Tyr Tyr Asn Asp Leu Leu Lys Asn Glu Leu Pro Val Leu
        195                 200                 205

Phe Gln Met Gln Ser Asp Phe Ile Lys Pro Leu Phe Val Ser Phe Tyr
    210                 215                 220

Tyr Met Gln Leu Asn Ile Phe Tyr Thr Leu Tyr Thr Arg Met Glu Glu
225                 230                 235                 240

Leu Lys Ile Pro Tyr Phe Asp Leu Ser Thr Asp Ile Val Glu Ala Tyr
                245                 250                 255

Thr Ala Lys Lys Gly Asn Ile Glu Glu Gln Thr Asp Ala Ile Gly Ile
            260                 265                 270

Thr His Phe Lys Val Gly His Ala Lys Ser Lys Leu Glu Ala Thr Lys
        275                 280                 285

Arg Arg His Ala Ala Met Asn Ser Pro Pro Pro Thr Gly Ala Ser Ser
    290                 295                 300

Ile Ala Ser Thr Gly Thr Gly Gly Glu Leu Pro Ala Tyr Ser Pro Gly
305                 310                 315                 320

Gly Tyr Asn Gln Pro Tyr Gly Asp Ser Lys Tyr Gln Pro Pro Ser Ser
                325                 330                 335

Pro Ala Thr Tyr Gln Ser Pro Val Val Ala Ala Thr Ala Gln Ser Pro
            340                 345                 350

Ala Thr Tyr Gln Ser Pro Val Ala Thr Gly Gln Pro Pro Ser Tyr Leu
        355                 360                 365

Pro Gln Thr Pro Ala Ser Ala Pro Pro Pro Gln Val Gly Ser Gly Leu
```

```
            370                 375                 380

        Pro Thr Cys Thr Ala Leu Tyr Asp Tyr Thr Ala Gln Ala Gln Gly Asp
        385                 390                 395                 400

        Leu Thr Phe Pro Ala Gly Ala Val Ile Glu Ile Ile Gln Arg Thr Glu
                        405                 410                 415

        Asp Ala Asn Gly Trp Trp Thr Gly Lys Tyr Asn Gly Gln Thr Gly Val
                    420                 425                 430

        Phe Pro Gly Asn Tyr Val Gln Leu
                435                 440
```

(2) INFORMATION FOR SEQ ID NO: 67:

(i) SEQUENCE CHARACTERISTICS:
(A) LENGTH: 855 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(xi) SEQUENCE DESCRIPTION: SEQ ID NO: 67:

```
ATAATTTTCA GAAAGAGACT AGATTCTGAT AGAAATATAG ACGCATCACT ATATTTTGGA      60

AATATAGATC CACAAGTTAC GGAGTTGTTA ATGTATGAGT TGTTCATCCA ATTTGGTCCC     120

GTCAAATCAA TCAATATGCC AAAGGATCGT ATATTGAAAA CACACCAGGG GTATGGATTT     180

GTCGAATTTA AAAACTCAGC AGATGCCAAA TATACTATGG AAATACTACG AGGAATAAGA     240

CTTTATGGAA AAGCATTGAA ATTGAAACGA ATTGATGCCA AGTCTCAGTC ATCAACAAAC     300

AACCCAAATA ATCAAACAAT AGGAACATTT GTACAATCAG ATTTGATCAA TCCAAATTAC     360

ATAGATGTTG GAGCTAAACT ATTTATCAAC AATCTTAATC CATTGGTCGA TGAATCCTTT     420

TTAATGGATA CGTTTAGTAA GTTTGGAACC CTTATAAGAA ACCCAATAAT TAGACGTGAT     480

TCAGAGGGAC ACTCTTTGGG ATACGGATTT CTTACGTACG ATGACTTTGA AAGTAGTGAT     540

TTATGCATAC AAAAAATGAA CAACACGATT TTGATGAATA ACAAAATTGC TATCAGTTAT     600

GCATTCAAGG ATCTGAGTGT TGATGGGAAG AAATCCCGGC ATGGAGATCA AGTGGAGCGG     660

AAATTGGCTG AAAGTGCCAA AAAGAATAAT TTGTTGGTAA CGAAAACTTC TAAGGCAGGT     720

ACGACGAAGG GAAATAAAAG GAAGAATAAA CCACATAAAG TGACCAAACC GTGAGACAAT     780

GAGTTAGCTC CCCCTTTCAA AATAAGTAGA GTATCACCAT AGTTTATGAA ACAATTGATA     840

TATTAAGCTT CTCTG                                                      855
```

(2) INFORMATION FOR SEQ ID NO: 68:

(i) SEQUENCE CHARACTERISTICS:
(A) LENGTH: 257 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 68:

```
Ile Ile Phe Arg Lys Arg Leu Asp Ser Asp Arg Asn Ile Asp Ala Ser
1               5                   10                  15

Leu Tyr Phe Gly Asn Ile Asp Pro Gln Val Thr Glu Leu Leu Met Tyr
            20                  25                  30

Glu Leu Phe Ile Gln Phe Gly Pro Val Lys Ser Ile Asn Met Pro Lys
        35                  40                  45

Asp Arg Ile Leu Lys Thr His Gln Gly Tyr Gly Phe Val Glu Phe Lys
    50                  55                  60

Asn Ser Ala Asp Ala Lys Tyr Thr Met Glu Ile Leu Arg Gly Ile Arg
65                  70                  75                  80

Leu Tyr Gly Lys Ala Leu Lys Leu Lys Arg Ile Asp Ala Lys Ser Gln
                85                  90                  95

Ser Ser Thr Asn Asn Pro Asn Asn Gln Thr Ile Gly Thr Phe Val Gln
            100                 105                 110

Ser Asp Leu Ile Asn Pro Asn Tyr Ile Asp Val Gly Ala Lys Leu Phe
        115                 120                 125

Ile Asn Asn Leu Asn Pro Leu Val Asp Glu Ser Phe Leu Met Asp Thr
    130                 135                 140

Phe Ser Lys Phe Gly Thr Leu Ile Arg Asn Pro Ile Ile Arg Arg Asp
145                 150                 155                 160

Ser Glu Gly His Ser Leu Gly Tyr Gly Phe Leu Thr Tyr Asp Asp Phe
                165                 170                 175

Glu Ser Ser Asp Leu Cys Ile Gln Lys Met Asn Asn Thr Ile Leu Met
            180                 185                 190

Asn Asn Lys Ile Ala Ile Ser Tyr Ala Phe Lys Asp Ser Ser Val Asp
        195                 200                 205

Gly Lys Lys Ser Arg His Gly Asp Gln Val Glu Arg Lys Leu Ala Glu
    210                 215                 220

Ser Ala Lys Lys Asn Asn Leu Leu Val Thr Lys Thr Ser Lys Ala Gly
225                 230                 235                 240

Thr Thr Lys Gly Asn Lys Arg Lys Asn Lys Pro His Lys Val Thr Lys
                245                 250                 255

Pro
```

(2) INFORMATION FOR SEQ ID NO: 69:

(i) SEQUENCE CHARACTERISTICS:
- (A) LENGTH: 1685 base pairs
- (B) TYPE: nucleic acid
- (C) STRANDEDNESS: single
- (D) TOPOLOGY: linear

(ii) MOLECULE TYPE: cDNA

(iii) HYPOTHETICAL: NO

(xi) SEQUENCE DESCRIPTION: SEQ ID NO: 69:

```
CTGTTTATTA AATGGATATA TGTTAAACCA TGAACTTCGG TTTATCAGAA AAATTGGTGC      60
TGGTACCTAT GGTTTGATTT ACCTTGTGGA AAATATCTAC ACTAAACAAC AATTTGCTGC     120
TAAAATGGTT CTTGAACAGC CATTACTCAA ACAAAAGCAA CAACAACAAC AAAGTCATCA     180
TGGACATAAA GGAGAATCTA GTATGAACAA ACAAATAATA CTGCAAGAAT TTTATCAATA     240
TTTTTTAAAC AATAGTATGC CACAACCACG AAATTTGGAC TTGAATTACC TTCGAGACAA     300
CGGACATGAT TGCCCCTTTT TGACTGAAAT CTCATTACAT TTAAAAGTAC ATCAACACCC     360
AAACATAGCG ACTATTCATC AAGTATTAAA CATTGAAGAT TTTGCCATAA TAATATTGAT     420
GGATCATTTT GAGCAAGGAG ATTTGTTCAC TAATATCATT GATAGACAAA TATTCACCAA     480
TAATAGTCAT AGAAAAGTTC CAAGAACAGA TTTTGAAACC CAATTATTAA TGAAGAATGC     540
CATGTTACAA TTGATAGAAG CCATTGAATA TTGTCACGAA AATAATATTT ACCATTGTGA     600
TTTAAAACCA GAAAACATTA TGGTTAGATA TAATCCATAC TATGTTCGTC CAACTATCAA     660
TAACAATAAT AACAATGGAG AAGATGATTT ATGCTATGCC AACAGTATTA TTGACTATAA     720
TGAATTACAC CTCGTGTTGA TTGATTTTGG TTTAGCTATG GACTCTGCTA CCATTTGTTG     780
TAATTCATGT CGTGGATCGT CATTTTACAT GGCACCAGAA AGAACCACCA ATTATAACAC     840
CCATCGTTTA ATCAACCAAT TAATTGATAT GAATCAATAT GAGTCAATTG AAATCAATGG     900
GACAACAGTG ACAAAATCAA ACTGTAAATA TTTACCTACA TTGGCTGGGG ATATTTGGTC     960
ATTGGGAGTA TTGTTCATTA ATATCACTTG TTCAAGAAAC CCATGGCCCA TTGCATCATT    1020
TGATAATAAT CAAAATAATG AAGTGTTTAA GAATTATATG TTGAATAATA ACAAGGCTGT    1080
TTTGAGCAAA ATCTTACCCA TTTCCTCACA ATTTAATCGC TTATTAGATA GAATTTTCAA    1140
ATTGAATCCT AATGATAGAA TAGATTTACC AACTTTATAC AAAGAAGTTA TTCGTTGTGA    1200
TTTCTTCAAA GATGATCATT ACTACTATGC CCAACATCAA CATCATCACA ATCACAATCA    1260
AATCAATAAT GCTTACAATC ACTATCAGAA ACAACCTAAT CAAGCAAGAC CTACTGCAAA    1320
CCAACAATTG TATACACCAC CGGAAACCAC CACTTATAAT TCATACGCTA GTGATATGGA    1380
AGAAGATGAA ATTAGTGATG ATGAGTTTTA TTCTGATGAA GAAGATGAAG ATATTGAAGA    1440
CTATGAAGAG GAAGAGGAAG AGTATTTTGG TAATGAGCAA CAACAACAAC AGCAAGTCAC    1500
AACAGTGAAT GGTAATTTTG GTCAAGTTAA AGGTACCTGT TATTACGATA CCAAAACCAA    1560
AACAACTACA TATATAAAAC CACCAGCTGC ATATACTTTA GAGACGCCTA GTCAAAGTGT    1620
TGAATACTGT TAAGTTGTAC ACATAAATAA TTAATGACAA TTAATAATAA CGATTAATAA    1680
TATAG                                                                1685
```

(2) INFORMATION FOR SEQ ID NO: 70:

(i) SEQUENCE CHARACTERISTICS:
(A) LENGTH: 537 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 70:

```
Met Leu Asn His Glu Leu Arg Phe Ile Arg Lys Ile Gly Ala Gly Thr
1               5                   10                  15

Tyr Gly Leu Ile Tyr Leu Val Glu Asn Ile Tyr Thr Lys Gln Gln Phe
            20                  25                  30

Ala Ala Lys Met Val Leu Glu Gln Pro Leu Leu Lys Gln Lys Gln Gln
        35                  40                  45

Gln Gln Gln Ser His His Gly His Lys Gly Glu Ser Ser Met Asn Lys
    50                  55                  60

Gln Ile Ile Ser Gln Glu Phe Tyr Gln Tyr Phe Leu Asn Asn Ser Met
65                  70                  75                  80

Pro Gln Pro Arg Asn Leu Asp Leu Asn Tyr Leu Arg Asp Asn Gly His
                85                  90                  95

Asp Cys Pro Phe Leu Thr Glu Ile Ser Leu His Leu Lys Val His Gln
            100                 105                 110

His Pro Asn Ile Ala Thr Ile His Gln Val Leu Asn Ile Glu Asp Phe
        115                 120                 125

Ala Ile Ile Ile Leu Met Asp His Phe Glu Gln Gly Asp Leu Phe Thr
    130                 135                 140

Asn Ile Ile Asp Arg Gln Ile Phe Thr Asn Asn Ser His Arg Lys Val
145                 150                 155                 160

Pro Arg Thr Asp Phe Glu Thr Gln Leu Leu Met Lys Asn Ala Met Leu
                165                 170                 175

Gln Leu Ile Glu Ala Ile Glu Tyr Cys His Glu Asn Asn Ile Tyr His
            180                 185                 190

Cys Asp Leu Lys Pro Glu Asn Ile Met Val Arg Tyr Asn Pro Tyr Tyr
        195                 200                 205

Val Arg Pro Thr Ile Asn Asn Asn Asn Asn Asn Gly Glu Asp Asp Leu
    210                 215                 220

Cys Tyr Ala Asn Ser Ile Ile Asp Tyr Asn Glu Leu His Leu Val Leu
225                 230                 235                 240

Ile Asp Phe Gly Leu Ala Met Asp Ser Ala Thr Ile Cys Cys Asn Ser
                245                 250                 255

Cys Arg Gly Ser Ser Phe Tyr Met Ala Pro Glu Arg Thr Thr Asn Tyr
            260                 265                 270

Asn Thr His Arg Leu Ile Asn Gln Leu Ile Asp Met Asn Gln Tyr Glu
        275                 280                 285

Ser Ile Glu Ile Asn Gly Thr Thr Val Thr Lys Ser Asn Cys Lys Tyr
    290                 295                 300

Leu Pro Thr Leu Ala Gly Asp Ile Trp Ser Leu Gly Val Leu Phe Ile
305                 310                 315                 320

Asn Ile Thr Cys Ser Arg Asn Pro Trp Pro Ile Ala Ser Phe Asp Asn
                325                 330                 335

Asn Gln Asn Asn Glu Val Phe Lys Asn Tyr Met Leu Asn Asn Asn Lys
            340                 345                 350

Ala Val Leu Ser Lys Ile Leu Pro Ile Ser Ser Gln Phe Asn Arg Leu
```

```
            355                 360                 365

        Leu Asp Arg Ile Phe Lys Leu Asn Pro Asn Asp Arg Ile Asp Leu Pro
            370                 375                 380

        Thr Leu Tyr Lys Glu Val Ile Arg Cys Asp Phe Phe Lys Asp Asp His
        385                 390                 395                 400

        Tyr Tyr Tyr Ala Gln His Gln His His His Asn His Asn Gln Ile Asn
                        405                 410                 415

        Asn Ala Tyr Asn His Tyr Gln Lys Gln Pro Asn Gln Ala Arg Pro Thr
                    420                 425                 430

        Ala Asn Gln Gln Leu Tyr Thr Pro Pro Glu Thr Thr Thr Tyr Asn Ser
                435                 440                 445

        Tyr Ala Ser Asp Met Glu Glu Asp Glu Ile Ser Asp Asp Glu Phe Tyr
            450                 455                 460

        Ser Asp Glu Glu Asp Glu Asp Ile Glu Asp Tyr Glu Glu Glu Glu Glu
        465                 470                 475                 480

        Glu Tyr Phe Gly Asn Glu Gln Gln Gln Gln Gln Gln Val Thr Thr Val
                        485                 490                 495

        Asn Gly Asn Phe Gly Gln Val Lys Gly Thr Cys Tyr Tyr Asp Thr Lys
                    500                 505                 510

        Thr Lys Thr Thr Thr Tyr Ile Lys Pro Pro Ala Ala Tyr Thr Leu Glu
                515                 520                 525

        Thr Pro Ser Gln Ser Val Glu Tyr Cys
            530                 535
```

(2) INFORMATION FOR SEQ ID NO: 71:

(i) SEQUENCE CHARACTERISTICS:
- (A) LENGTH: 848 base pairs
- (B) TYPE: nucleic acid
- (C) STRANDEDNESS: single
- (D) TOPOLOGY: linear

(ii) MOLECULE TYPE: cDNA

(iii) HYPOTHETICAL: NO

(xi) SEQUENCE DESCRIPTION: SEQ ID NO: 71:

```
AACCAATTTT AGAAACAATG GCTCGTCAAT TTTTCGTAGG TGGTAACTTC AAAGCTAACG    60
GTACCAAACA ACAAATCACT TCAATCATCG ACAACTTGAA CAAGGCTGAT TTACCAAAGG   120
ATGTCGAAGT TGTCATTTGT CCACCCGCCC TTTACCTTGG TTTAGCTGTA GAGCAAAACA   180
AACAACCAAC TGTTGCCATT GGTGCTCAAA ATGTTTTTGA CAAGTCATGT GGTGCTTTCA   240
CTGGTGAAAC CTGTGCTTCT CAAATCTTGG ATGTTGGTGC CAGCTGGACT TTAACTGGTC   300
ACAGTGAAAG AAGAACCATT ATCAAAGAAT CCGATGAATT CATTGCTGAA AAAACCAAGT   360
TTGCCTTGGA CACTGGTGTC AAAGTTATTT TATGTATTGG TGAAACCTTA GAGGAAAGAA   420
AAGGTGGTGT CACTTTGGAT GTTTGTGCCA GACAATTGGA TGCTGTTTCC AAGATTGTTT   480
CTGATTGGTC AAACATTGTT GTTGCTTACG AACCTGTTTG GGCAATTGGT ACTGGTTTAG   540
CCGCTACCCC AGAAGATGCT GAAGAAACCC ACAAAGGTAT TAGAGCTCAT TTGGCCAAGA   600
```

CCATTGGTGC CGAACAAGCT GAAAAAACCA GAATCTTGTA CGGTGGTTCA GTTAACGGTA 660

AGAACGCTAA GGATTTCAAA GACAAAGCAA ATGTTGATGG TTTCTTAGTC GGTGGTGCTT 720

CATTAAAACC AGAATTTGTT GATATCATCA AATCTAGATT ATAAACAGTA TATTAAAAAC 780

TATATGCCTA TAGAATTTAG CATGTTGTTG TGAATTTGTA ATGAATCTAT AAAAATGTGC 840

TCATGAAC 848

(2) INFORMATION FOR SEQ ID NO: 72:

(i) SEQUENCE CHARACTERISTICS:
(A) LENGTH: 248 amino acids
(B) TYPE: amino acid
(C) STRANDEDNESS:
(D) TOPOLOGY: unknown

(ii) MOLECULE TYPE: peptide

(iii) HYPOTHETICAL: NO

(xi) SEQUENCE DESCRIPTION: SEQ ID NO: 72:

```
Met Ala Arg Gln Phe Phe Val Gly Gly Asn Phe Lys Ala Asn Gly Thr
1               5                   10                  15

Lys Gln Gln Ile Thr Ser Ile Ile Asp Asn Leu Asn Lys Ala Asp Leu
            20                  25                  30

Pro Lys Asp Val Glu Val Val Ile Cys Pro Pro Ala Leu Tyr Leu Gly
        35                  40                  45

Leu Ala Val Glu Gln Asn Lys Gln Pro Thr Val Ala Ile Gly Ala Gln
    50                  55                  60

Asn Val Phe Asp Lys Ser Cys Gly Ala Phe Thr Gly Glu Thr Cys Ala
65                  70                  75                  80

Ser Gln Ile Leu Asp Val Gly Ala Ser Trp Thr Leu Thr Gly His Ser
                85                  90                  95

Glu Arg Arg Thr Ile Ile Lys Glu Ser Asp Glu Phe Ile Ala Glu Lys
            100                 105                 110

Thr Lys Phe Ala Leu Asp Thr Gly Val Lys Val Ile Leu Cys Ile Gly
        115                 120                 125

Glu Thr Leu Glu Glu Arg Lys Gly Gly Val Thr Leu Asp Val Cys Ala
    130                 135                 140

Arg Gln Leu Asp Ala Val Ser Lys Ile Val Ser Asp Trp Ser Asn Ile
145                 150                 155                 160

Val Val Ala Tyr Glu Pro Val Trp Ala Ile Gly Thr Gly Leu Ala Ala
                165                 170                 175

Thr Pro Glu Asp Ala Glu Glu Thr His Lys Gly Ile Arg Ala His Leu
            180                 185                 190

Ala Lys Thr Ile Gly Ala Glu Gln Ala Glu Lys Thr Arg Ile Leu Tyr
        195                 200                 205

Gly Gly Ser Val Asn Gly Lys Asn Ala Lys Asp Phe Lys Asp Lys Ala
    210                 215                 220

Asn Val Asp Gly Phe Leu Val Gly Gly Ala Ser Leu Lys Pro Glu Phe
225                 230                 235                 240
```

Val Asp Ile Ile Lys Ser Arg Leu
245

## Description

The present invention is concerned with the identification of genes or functional fragments thereof from *Candida albicans* which are critical for growth and cell division and which genes may be used as selective drug targets to treat *Candida albicans* associated infections. Novel nucleic acid sequences from *Candida albicans* are also provided and which encode the polypeptides which are critical for growth of *Candida albicans*.

Opportunistic infections in immunocompromised hosts represent an increasingly common cause of mortality and morbidity. *Candida* species are among the most commonly identified fungal pathogens associated with such opportunistic infections, with *Candida albicans* being the most common species. Such fungal infections are thus problematical in, for example, AIDS populations in addition to normal healthy women where *Candida albicans* yeasts represent the most common cause of vulvovaginitis.

Although compounds do exist for treating such disorders, such as for example, amphotericin, these drugs are generally limited in their treatment because of their toxicity and side effects. Therefore, there exists a need for new compounds which may be used to treat *Candida* associated infections in addition to compounds which are selective in their action against *Candida albicans*.

Classical approaches for identifying anti-fungal compounds have relied almost exclusively on inhibition of fungal or yeast growth as an endpoint. Libraries of natural products, semi-synthetic, or synthetic chemicals are screened for their ability to kill or arrest growth of the target pathogen or a related nonpathogenic model organism. These tests are cumbersome and provide no information about a compounds mechanism of action. The promising lead compounds that emerge from such screens must then be tested for possible host-toxicity and detailed mechanism of action studies must subsequently be conducted to identify the affected molecular target.

The present inventors have now identified a range of nucleic acid sequences from *Candida albicans* which encode polypeptides which are critical for its survival and growth. These sequences represent novel targets which can be incorporated into an assay to selectively identify compounds capable of inhibiting expression of such polypeptides and their potential use in alleviating diseases or conditions associated with *Candida albicans* infection.

Therefore, according to a first aspect of the invention there is provided a nucleic acid molecule encoding a polypeptide which is critical for survival and growth of the yeast *Candida albicans* and which nucleic acid molecule comprises any of the sequences of nucleotides in Sequence ID Numbers 1 to 3, 5, 6, 8 to 11, 13, 15, 16, 18, 20, 21, 23, 25 to 29, 31, 35, 37, 39, 41, 43, 45, 47, 49, 51, 53, 55, 57, 59, 61, 63, 65, 67, 69 and 71.

A further aspect of the invention comprises a nucleic acid molecule encoding a polypeptide which is critical for survival and growth of the yeast *Candida albicans* and which nucleic acid molecule comprises any of the sequences of Sequence ID Numbers 1, 28, 35, 37 and 39 and fragments or derivatives of said nucleic acid molecules.

Also provided by the present invention is a nucleic acid molecule encoding a polypeptide which is critical for survival and growth of the yeast *Candida albicans* and which polypeptide has an amino acid sequence according to the sequence of any of Sequence ID Numbers 4, 7, 12, 14, 17, 19, 22, 24, 30, 32 to 34, 36, 38, 40, 42, 44, 46, 48, 50, 52, 54, 56, 58, 60, 62, 64, 66, 68, 70 and 72.

Letters utilised in the nucleic acid sequences according to the invention which are not recognisable as letters of the genetic code signify a position in the nucleic acid sequence where one or more of bases A, G, C or T can occupy the nucleotide position. Representative letters used to identify the range of bases which can be used are as follows:

M: A or C
R: A or G
W: A or T
S: C or G
Y: C or T
K: G or T
V: A or C or G
H: A or C or T
D: A or G or T
B: C or G or T
N: G or A or T or C

In one embodiment of the above identified aspects of the invention the nucleic acid may comprise a mRNA molecule or alternatively a DNA and preferably a cDNA molecule.

Also provided by the present invention is a nucleic acid molecule capable of hybridising to the nucleic acid molecules according to the invention under high stringency conditions.

Stringency of hybridisation as used herein refers to conditions under which polynucleic acids are stable. The stability of hybrids is reflected in the melting temperature (Tm) of the hybrids. Tm can be approximated by the formula:

$$81.5°C+16.6(1og_{10}[Na^+]+0.41 (\%G\&C)-6001/l$$

wherein l is the length of the hybrids in nucleotides. Tm decreases approximately by 1-1.5°C with every 1% decrease in sequence homology.

The nucleic acid capable of hybridising to nucleic acid molecules according to the invention will generally be at least 70%, preferably at least 80 or 90% and more preferably at least 95% homologous to the nucleotide sequences according to the invention.

The DNA molecules according to the invention may, advantageously, be included in a suitable expression vector to express polypeptides encoded therefrom in a suitable host.

The present invention also comprises within its scope proteins or polypeptides encoded by the nucleic acid molecules according to the invention or a functional equivalent, derivative or bioprecursor thereof.

Therefore, according to a further aspect of the invention there is provided a polypeptide having an amino acid sequence of any of Sequence ID Numbers 4, 7, 12, 14, 17, 19, 22, 24, 30, 32 to 34, 36, 38, 40, 42, 44, 46, 48, 50, 52, 54, 56, 58, 60, 62, 64, 66, 68, 70 and 72. A polypeptide encoded by the nucleic acid molecule according to the invention is also provided, which polypeptide preferably comprises an amino acid sequence of having the sequence of any of Sequence ID Numbers 4, 7, 12, 14, 17, 19, 22, 24, 30, 32 to 34, 36, 38, 40, 42, 44, 46, 48, 50, 52, 54, 56, 58, 60, 62, 64, 66, 68, 70 and 72.

An expression vector according to the invention includes a vector having a nucleic acid according to the invention operably linked to regulatory sequences, such as promoter regions, that are capable of effecting expression of said DNA fragments. The term "operably linked" refers to a juxta position wherein the components described are in a relationship permitting them to function in their intended manner. Such vectors may be transformed into a suitable host cell to provide for expression of a polypeptide according to the invention. Thus, in a further aspect, the invention provides a process for preparing polypeptides according to the invention which comprises cultivating a host cell, transformed or transfected with an expression vector as described above under conditions to provide for expression by the vector of a coding sequence encoding the polypeptides, and recovering the expressed polypeptides.

The vectors may be, for example, plasmid, virus or phage vectors provided with an origin of replication, optionally a promoter for the expression of said nucleotide and optionally a regulator of the promoter. The vectors may contain one or more selectable markers, such as, for example, ampicillin resistance.

Polynucleotides according to the invention may be inserted into the vectors described in an antisense orientation in order to provide for the production of antisense RNA. Antisense RNA or other antisense nucleic acids may be produced by synthetic means.

In accordance with the present invention, a defined nucleic acid includes not only the identical nucleic acid but also any minor base variations including in particular, substitutions in bases which result in a synonymous codon (a different codon specifying the same amino acid residue) due to the degenerate code in conservative amino acid substitutions. The term "nucleic acid sequence" also includes the complementary sequence to any single stranded sequence given regarding base variations.

The present invention also advantageously provides nucleic acid sequences of at least approximately 10 contiguous nucleotides of a nucleic acid according to the invention and preferably from 10 to 50 nucleotides. These sequences may, advantageously be used as probes or primers to initiate replication, or the like. Such nucleic acid sequences may be produced according to techniques well known in the art, such as by recombinant or synthetic means. They may also be used in diagnostic kits or the like for detecting the presence of a nucleic acid according to the invention. These tests generally comprise contacting the probe with the sample under hybridising conditions and detecting for the presence of any duplex or triplex formation between the probe and any nucleic acid in the sample.

According to the present invention these probes may be anchored to a solid support. Preferably, they are present on an array so that multiple probes can simultaneously hybridize to a single biological sample. The probes can be spotted onto the array or synthesised *in situ* on the array. (See Lockhart *et al*., Nature Biotechnology, vol. 14, December 1996 "Expression monitoring by hybridisation to high density oligonucleotide arrays". A single array can contain more than 100, 500 or even 1,000 different probes in discrete locations.

Advantageously, the nucleic acid sequences, according to the invention may be produced using such recombinant or synthetic means, such as for example using PCR cloning mechanisms which generally involve making a pair of primers, which may be from approximately 10 to 50 nucleotides to a region of the gene which is desired to be cloned, bringing the primers into contact with mRNA, CDNA, or genomic DNA from a human cell, performing a polymerase chain reaction under conditions which bring about amplification of the desired region, isolating the amplified region or fragment and recovering the amplified DNA. Generally, such techniques as defined herein are well known in the art, such as described in Sambrook *et al* (Molecular Cloning: a Laboratory Manual, 1989).

The nucleic acids or oligonucleotides according to the invention may carry a revealing label. Suitable labels include radioisotopes such as $^{32}P$ or $^{39}S$, enzyme labels or other protein labels such as biotin or fluorescent markers. Such labels may be added to the nucleic acids or oligonucleotides of the invention and may be detected using known techniques *per se*.

The polypeptide or protein according to the invention includes all possible amino acid variants encoded by the nucleic acid molecule according to the invention including a polypeptide encoded by said molecule and having conservative amino acid changes. Polypeptides according to the invention further include variants of such sequences, including naturally occurring allelic variants which are substantially homologous to said polypeptides. In this context, substantial homology is regarded as a sequence which has at least 70%, preferably 80 or 90% amino acid homology with the polypeptides encoded by the nucleic acid molecules according to the invention.

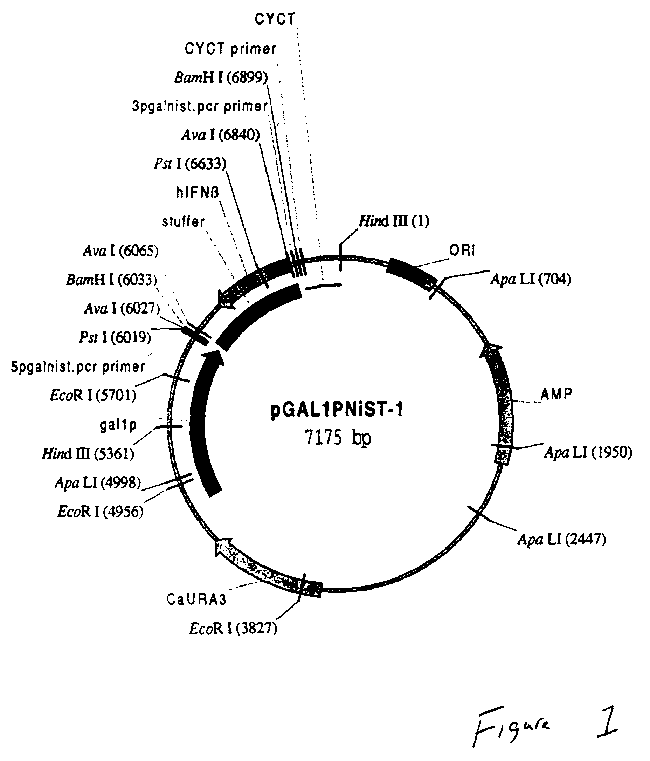

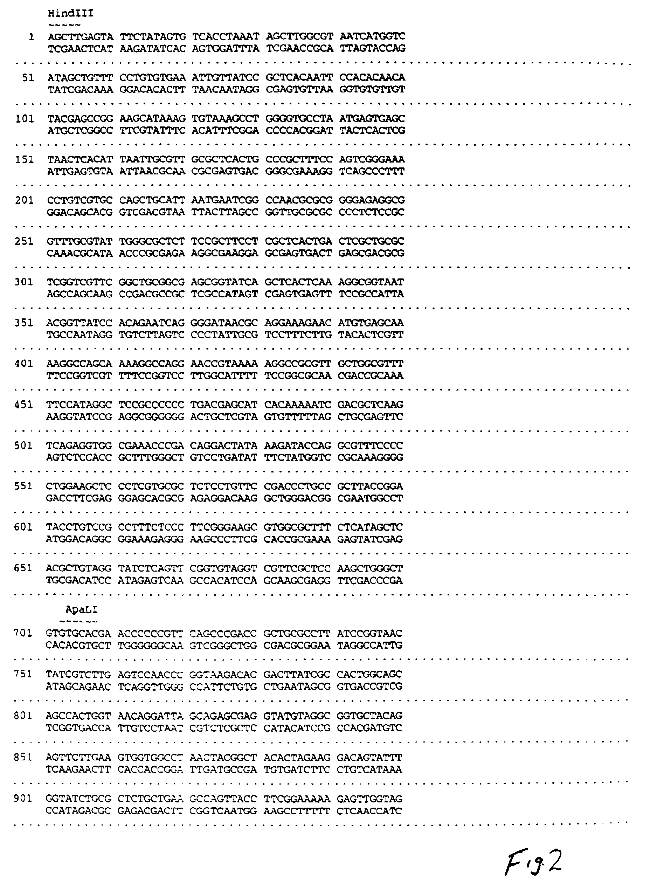

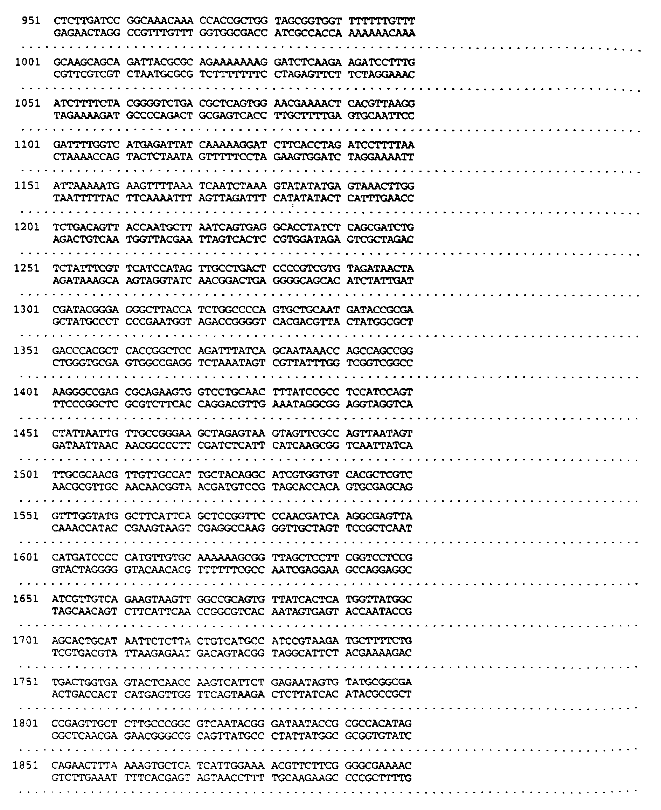

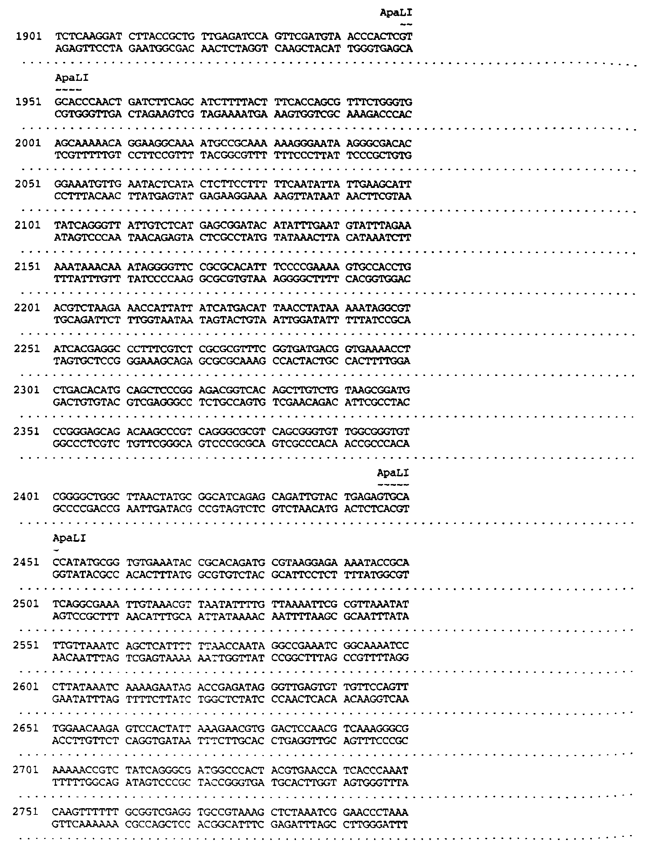

A nucleic acid which is particularly advantageous is one comprising the sequences of nucleotides illustrated in Figures 1 which is specific to *Candida albicans* with no functionally related sequences in other prokaryotic or eukaryotic organism as yet identified from the respective genomic databases.

Nucleotide sequences according to the invention are particularly advantageous for selective therapeutic targets for treating *Candida albicans* associated infections. For example, an antisense nucleic acid capable of binding to the nucleic acid sequences according to the invention may be used to selectively inhibit expression of the corresponding polypeptides, leading to impaired growth of the *Candida albicans* with reductions of associated illnesses or diseases.

The nucleic acid molecule or the polypeptide according to the invention may be used as a medicament, or in the preparation of a medicament, for treating diseases or conditions associated with *Candida albicans* infection.

Advantageously, the nucleic acid molecule or the polypeptide according to the invention may be provided in a pharmaceutical composition together with a pharmaceutically acceptable carrier, diluent or excipient therefor.

Antibodies to the protein or polypeptide of the present invention may, advantageously, be prepared by techniques which are known in the art. For example, polyclonal antibodies may be prepared by inoculating a host animal, such as a mouse, with the polypeptide according to the invention or an epitope thereof and recovering immune serum. Monoclonal antibodies may be prepared according to known techniques such as described by Kohler R. and Milstein C., Nature (1975) 256, 495-497.

Antibodies according to the invention may also be used in a method of detecting for the presence of a polypeptide according to the invention, which method comprises reacting the antibody with a sample and identifying any protein bound to said antibody. A kit may also be provided for performing said method which comprises an antibody according to the invention and means for reacting the antibody with said sample.

Proteins which interact with the polypeptide of the invention may be identified by investigating protein-protein interactions using the two-hybrid vector system first proposed by Chien *et al* (1991).

This technique is based on functional reconstitution in vivo of a transcription factor which activates a reporter gene. More particularly the technique comprises providing an appropriate host cell with a DNA construct comprising a reporter gene under the control of a promoter regulated by a transcription factor having a DNA binding domain and an activating domain, expressing in the host cell a first hybrid DNA sequence encoding a first fusion of a fragment or all of a nucleic acid sequence according to the invention and either said DNA binding domain or said activating domain of the transcription factor, expressing in the host at least one second hybrid DNA sequence, such as a library or the like, encoding putative binding proteins to be investigated together with the DNA binding or activating domain of the transcription factor which is not incorporated in the first fusion; detecting any binding of the proteins to be investigated with a protein according to the invention by detecting for the presence of any reporter gene product in the host cell; optionally isolating second hybrid DNA sequences encoding the binding protein.

An example of such a technique utilises the GAL4 protein in yeast. GAL4 is a transcriptional activator of galactose metabolism in yeast and has a separate domain for binding to activators upstream of the galactose metabolising genes as well as a protein binding domain. Nucleotide vectors may be constructed, one of which comprises the nucleotide residues encoding the DNA binding domain of GAL4. These binding domain residues may be fused to a known protein encoding sequence, such as for example the nucleic acids according to the invention. The other vector comprises the residues encoding the protein binding domain of GAL4. These residues are fused to residues encoding a test protein. Any interaction between polypeptides encoded by the nucleic acid according to the invention and the protein to be tested leads to transcriptional activation of a reporter molecule in a GAL-4 transcription deficient yeast cell into which the vectors have been transformed. Preferably, a reporter molecule such as β-galactosidase is activated upon restoration of transcription of the yeast galactose metabolism genes.

Further provided by the present invention is one or more *Candida albicans* cells comprising an induced mutation in the DNA sequence encoding the polypeptide according to the invention.

A further aspect of the invention provides a method of identifying compounds which selectively inhibit or interfere with the expression, or the functionality of polypeptides expressed from the nucleotides sequences according to the invention or the metabolic pathways in which these polypeptides are involved and which are critical for growth and survival of *Candida albicans*, which method comprises (a) contacting a compound to be tested with one or more *Candida albicans* cells having a mutation in a nucleic acid molecule according to the invention which mutation results in overexpression or underexpression of said polypeptides in addition to one or more wild type *Candida cells*, (b) monitoring the growth and/or activity of said mutated cell compared to said wild type wherein differential growth or activity of said one or more mutated *Candida cells* provides an indication of selective action of said compound on said polypeptide or another polypeptide in the same or a parallel pathway.

Compounds identifiable or identified using the method according to the invention, may advantageously be used as a medicament, or in the preparation of a medicament to treat diseases or conditions associated with *Candida albicans* infection. These compounds may also advantageously be included in a pharmaceutical composition together with a pharmaceutically acceptable carrier, diluent or excipient therefor.

A further aspect of the invention provides a method of identifying DNA sequences from a cell or organism which DNA encodes polypeptides which are critical for growth or survival, which method comprises (a) preparing a cDNA or genomic library from said cell or organism in a suitable expression vector which vector is such that it can either integrate into the genome in said cell or that it permits transcription of antisense RNA from the nucleotide sequences in said cDNA or genomic library, (b) selecting transformants exhibiting impaired growth and determining the nucleotide sequence of the cDNA or genomic sequence from the library included in the vector from said transformant. Preferably, the cell or organism may be any yeast or filamentous fungi, such as, for example, *Saccharomyces cervisiae*, *Saccharomyces pombe* or *Candida albicans*.

A further aspect of the invention provides a pharmaceutical composition comprising a compound according to the invention together with a pharmaceutically acceptable carrier, diluent or excipient therefor.

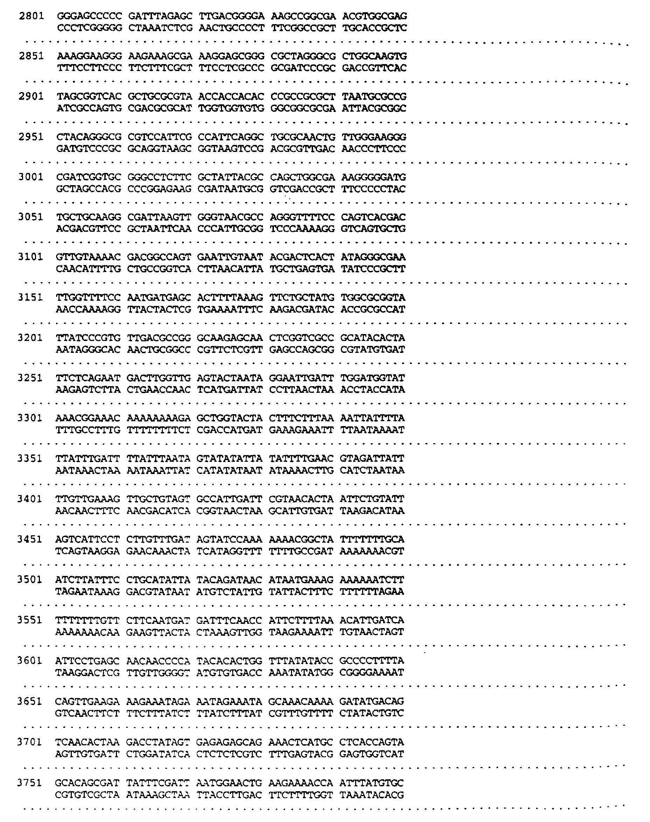

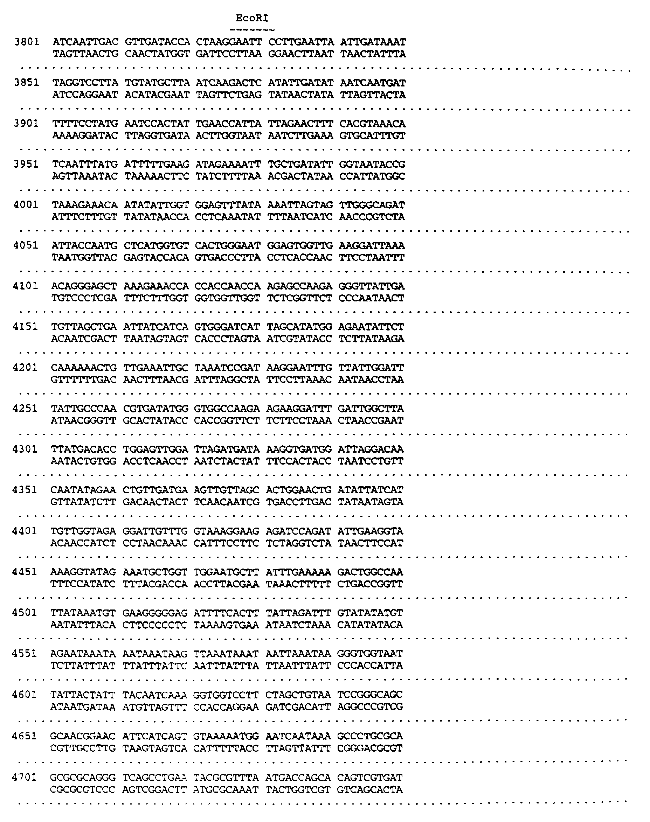

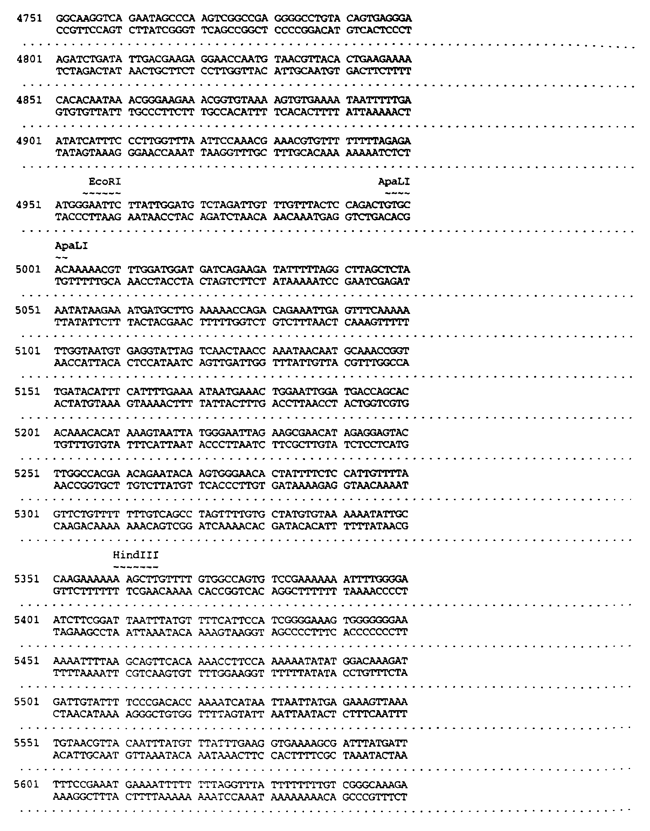

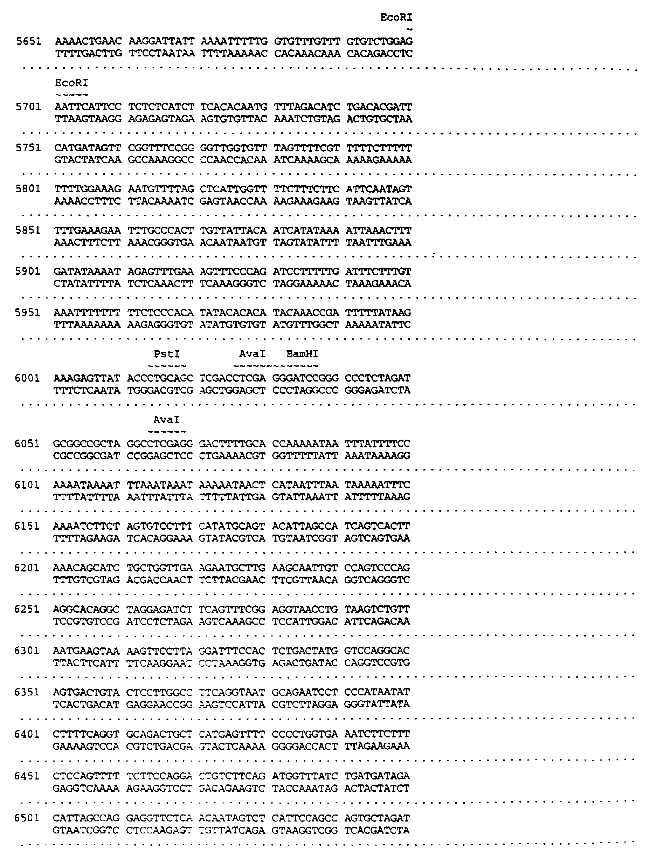

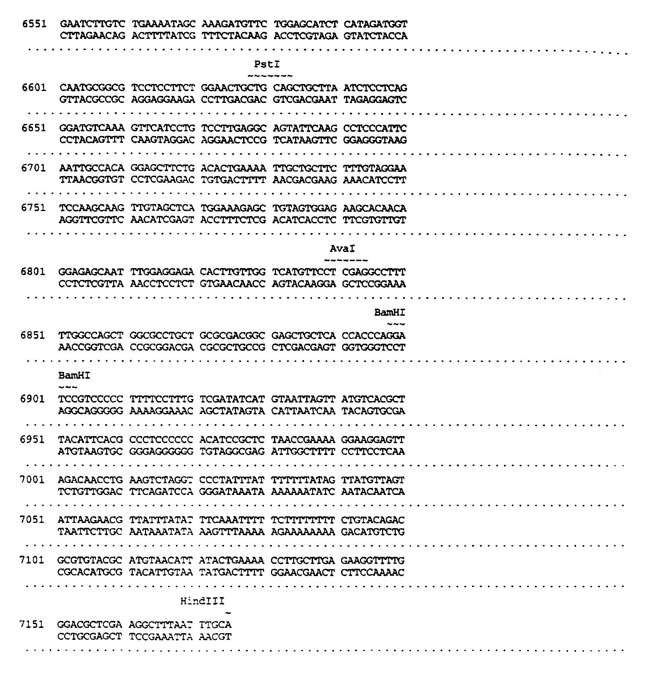

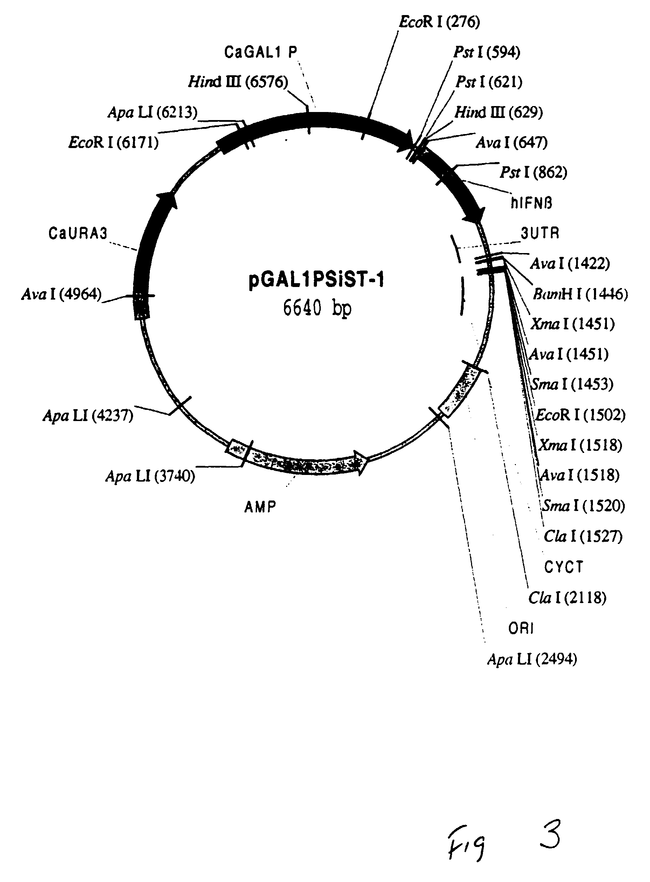

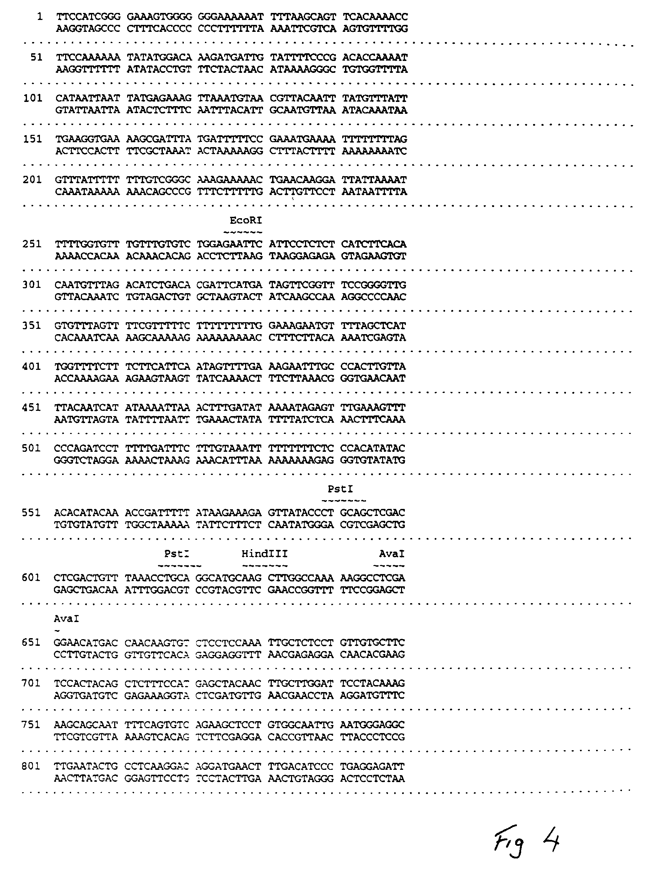

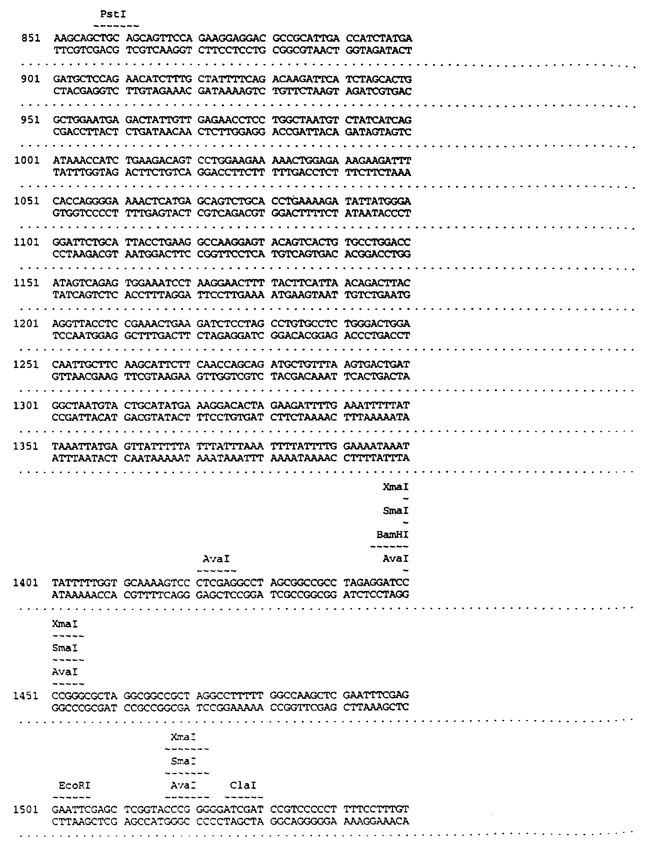

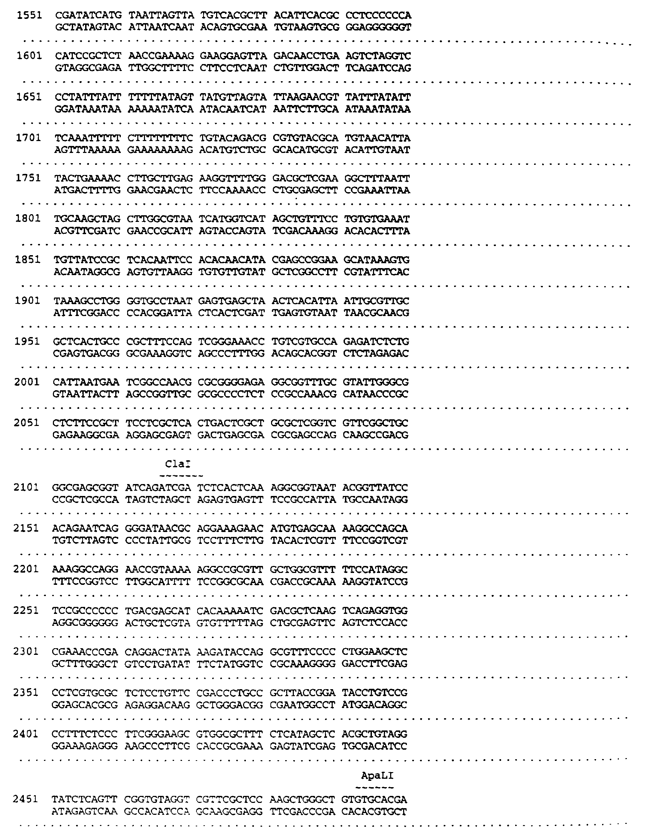

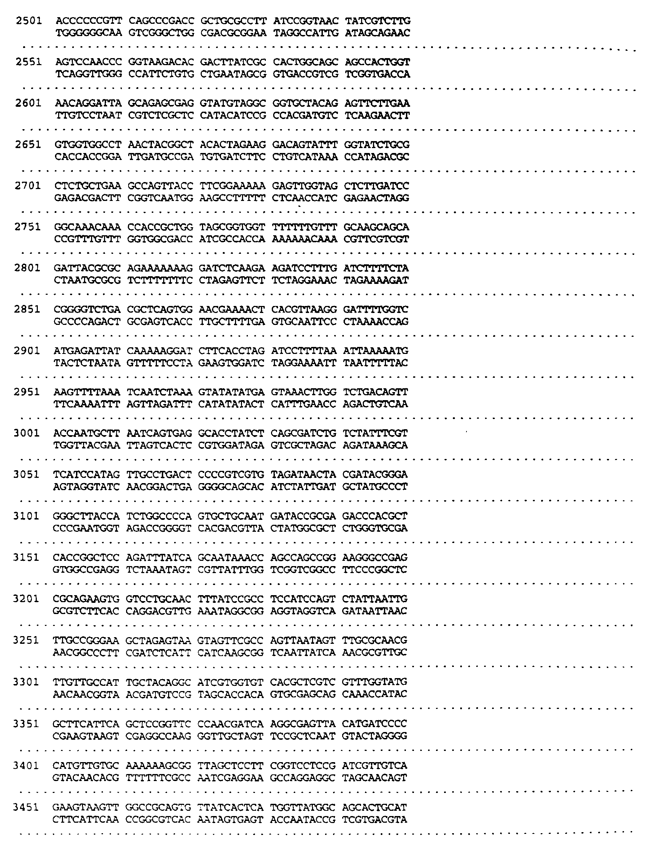

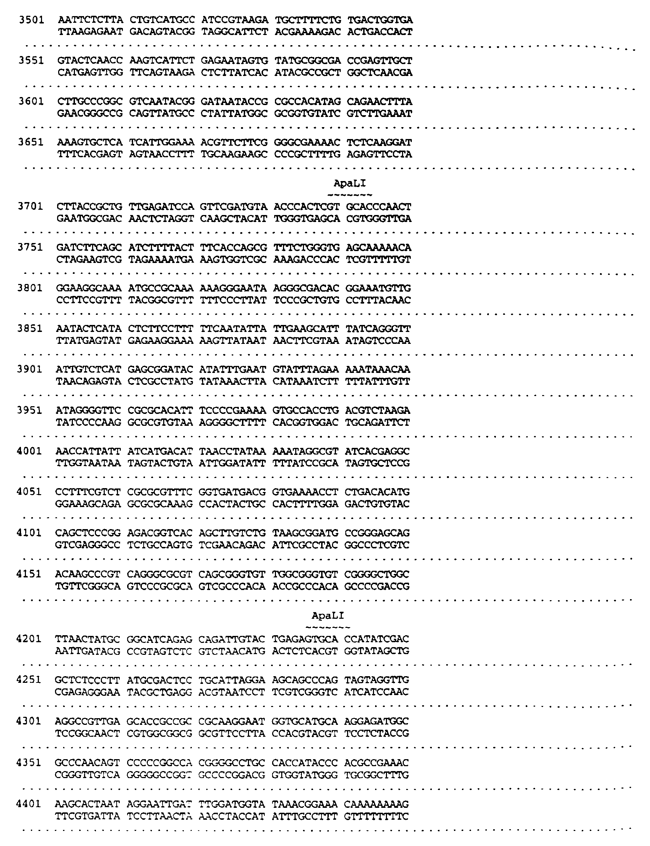

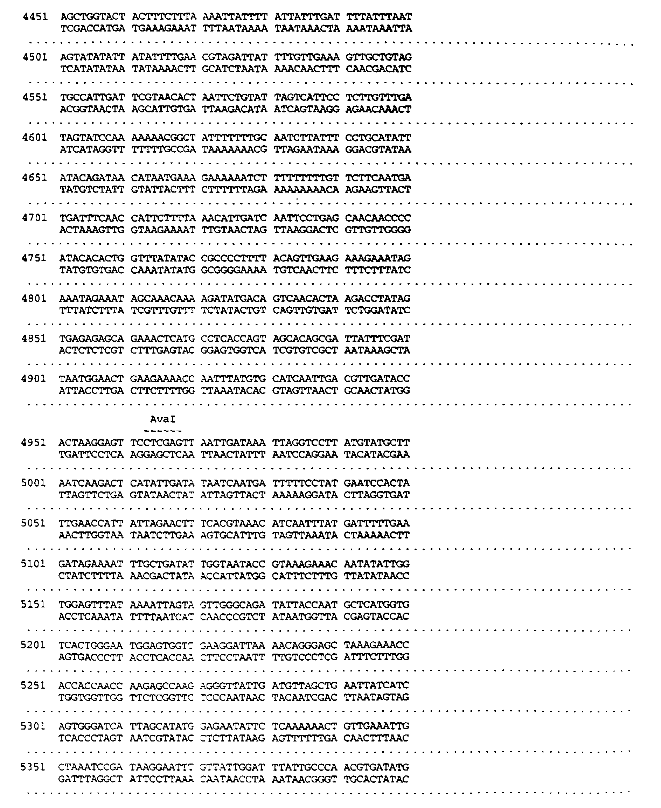

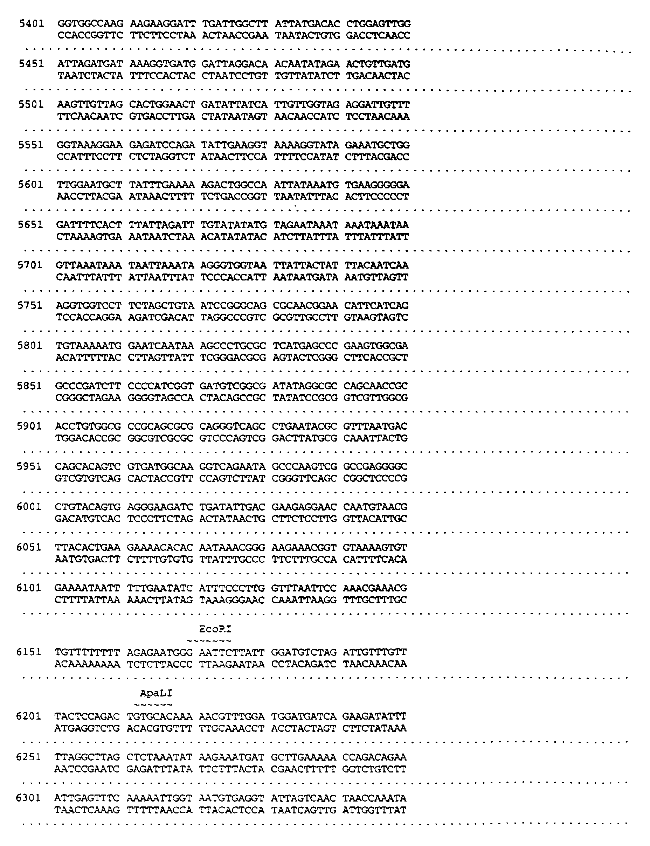

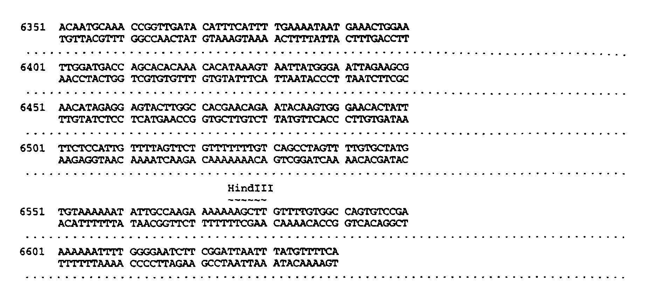

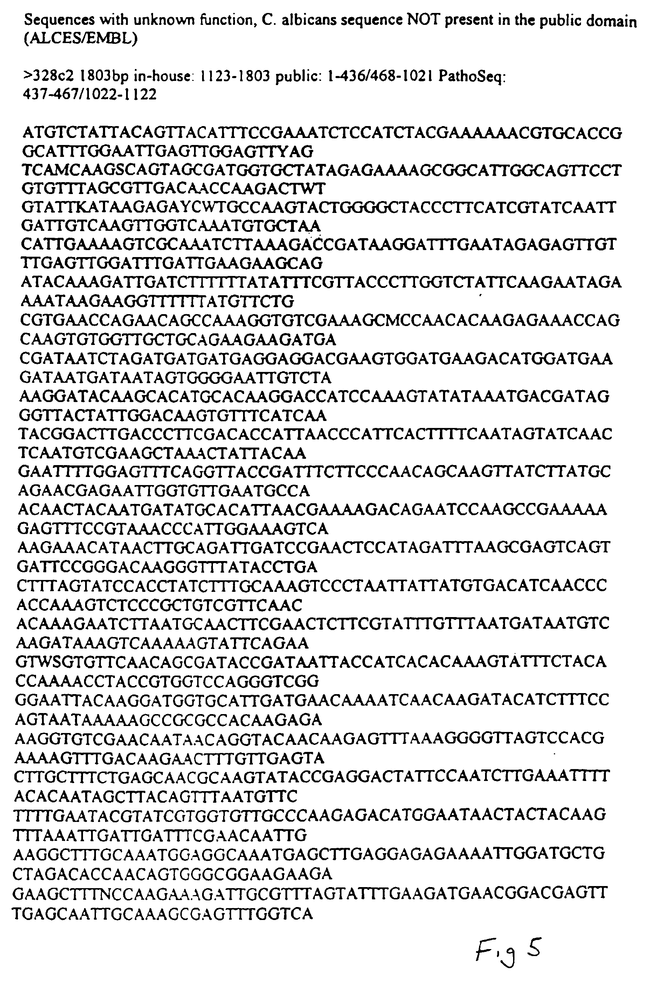

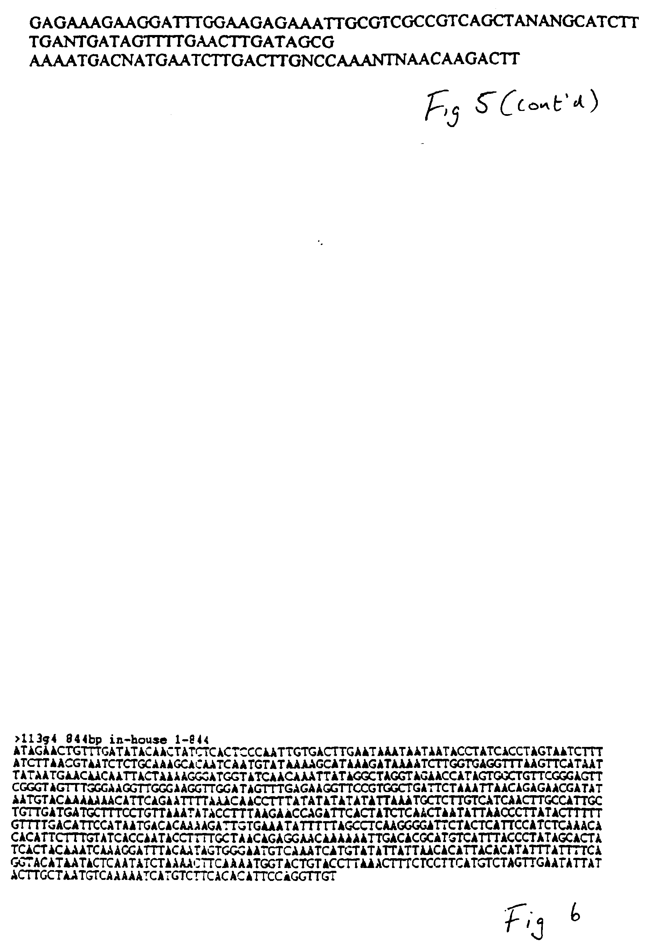

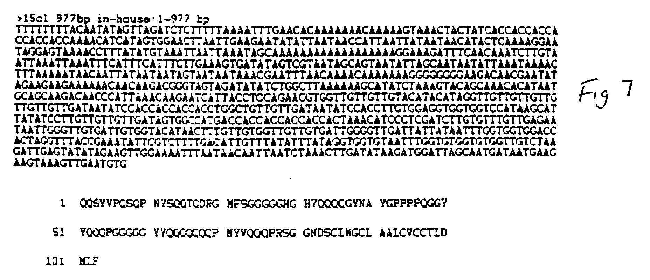

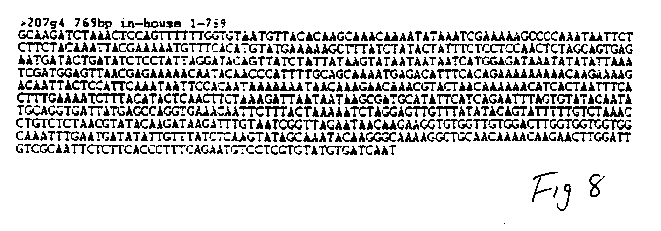

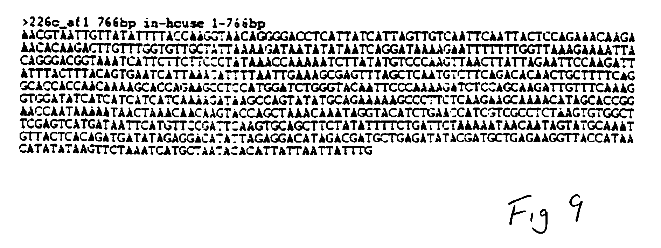

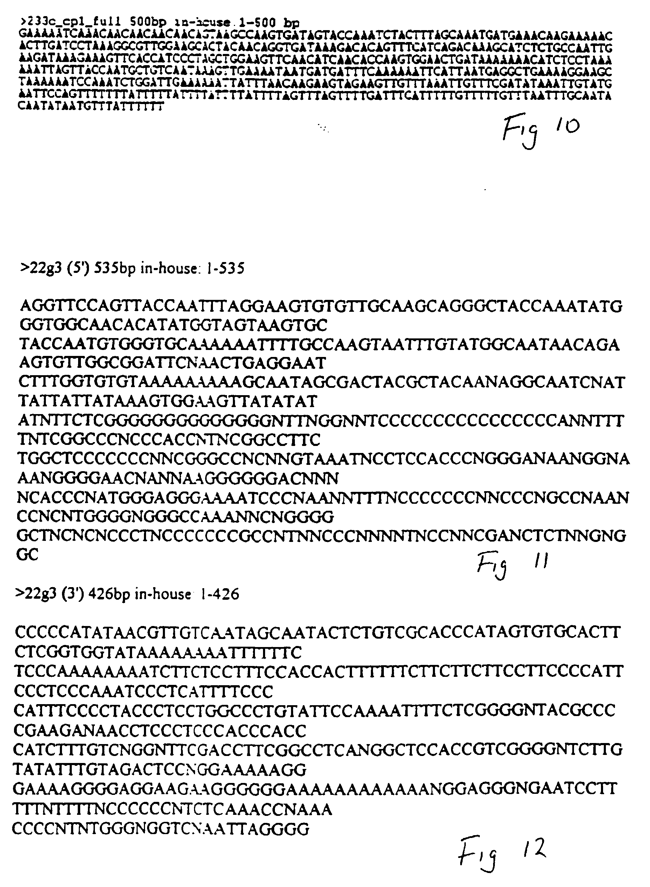

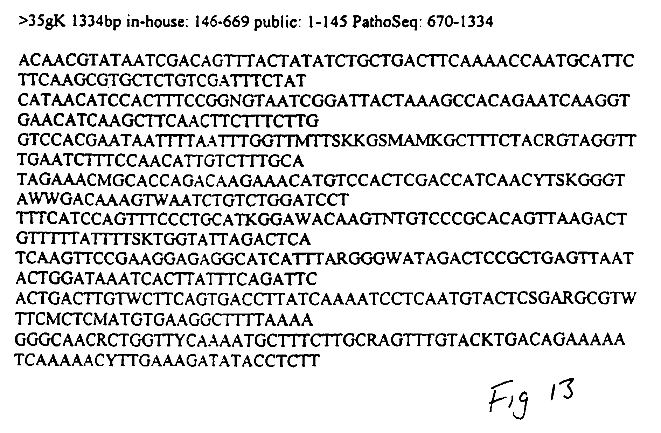

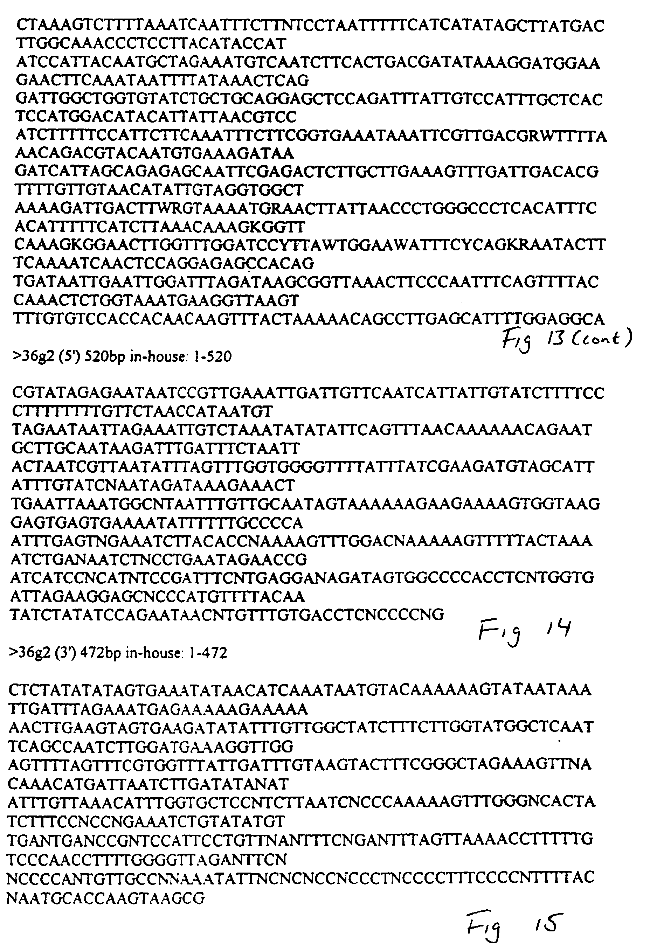

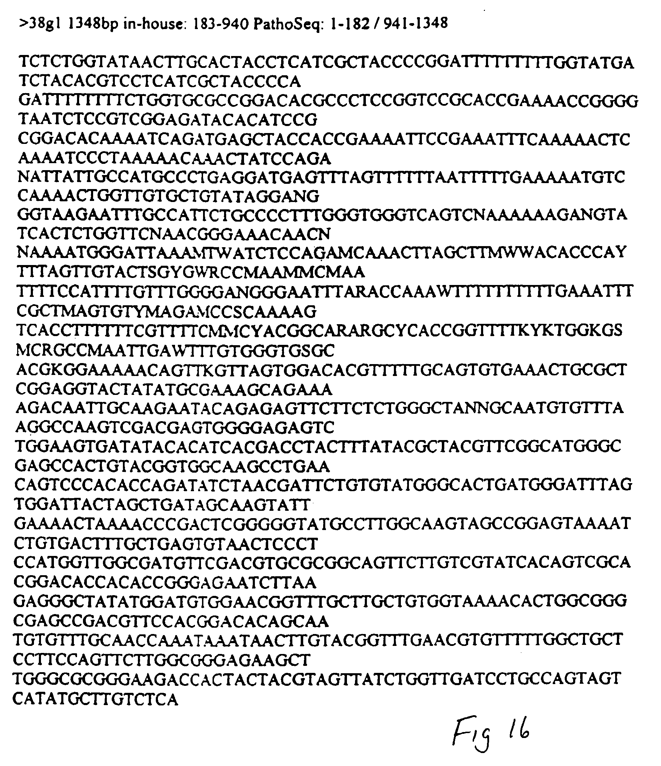

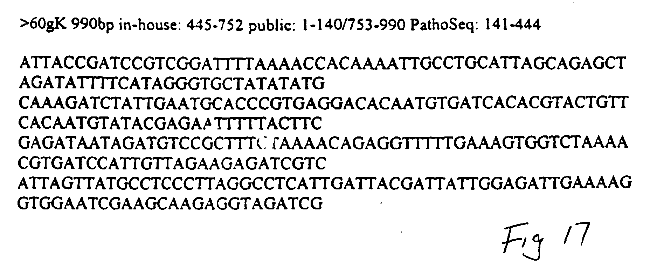

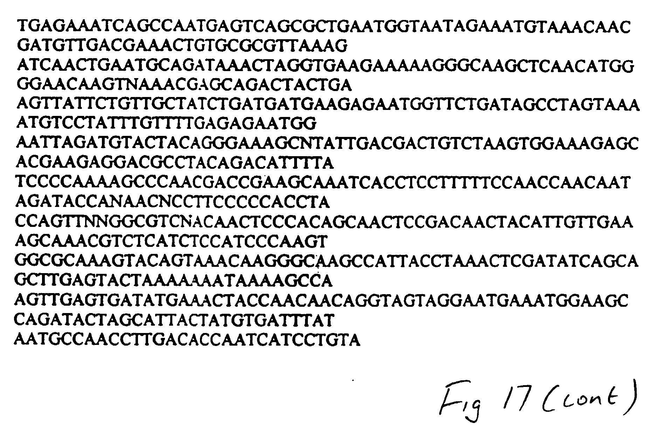

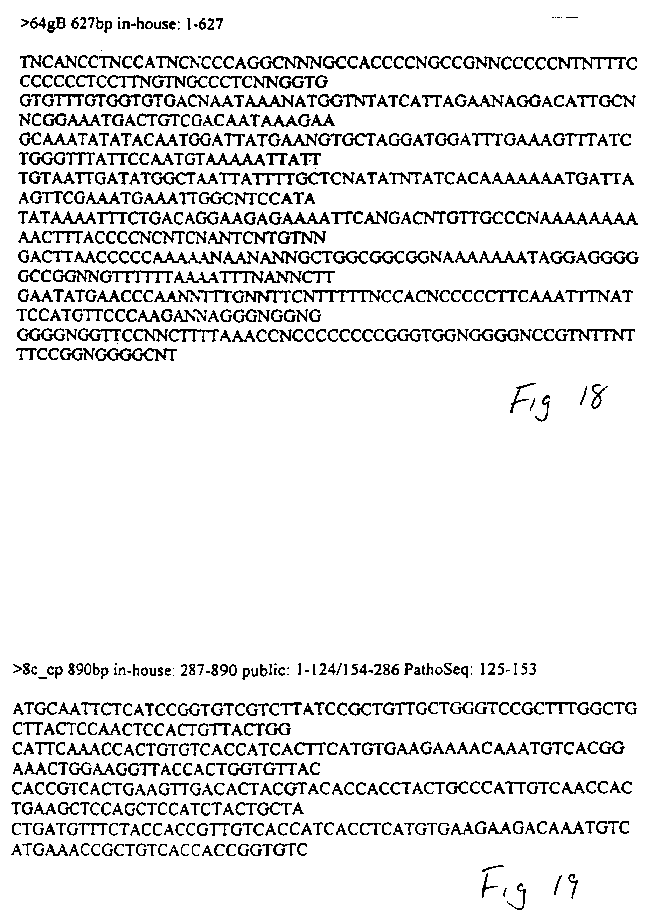

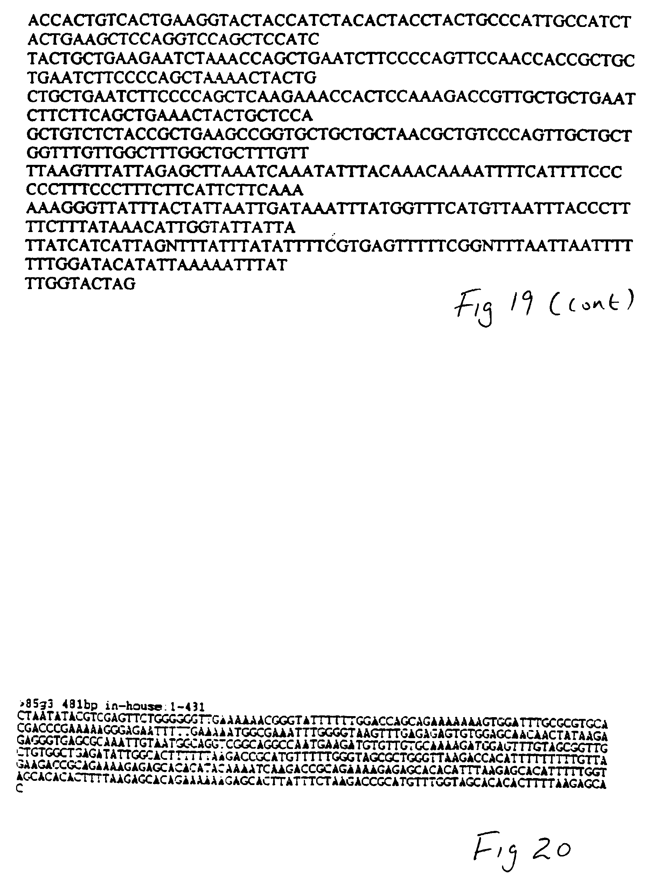

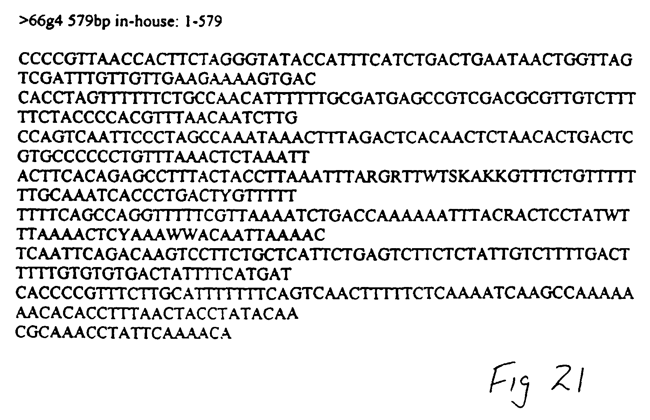

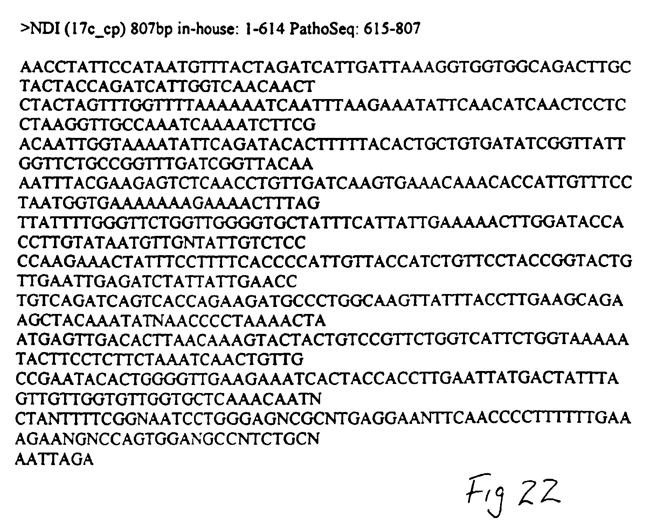

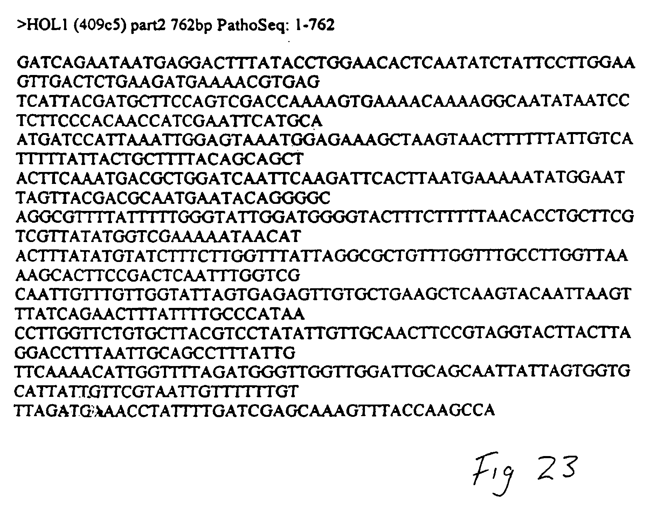

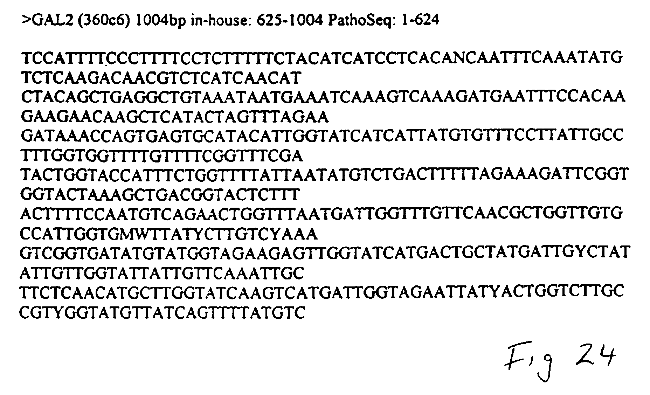

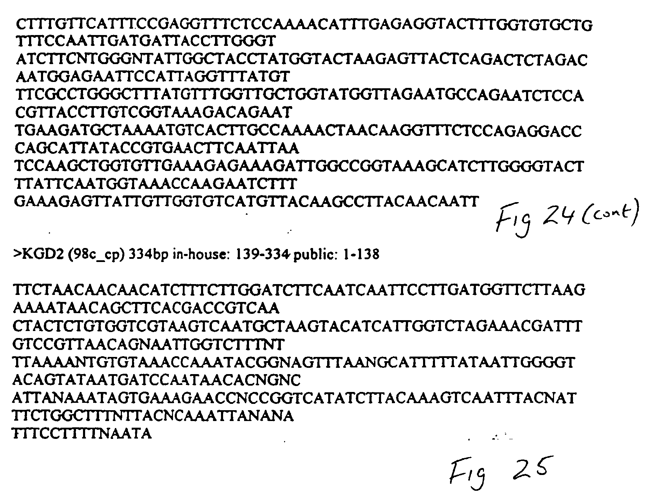

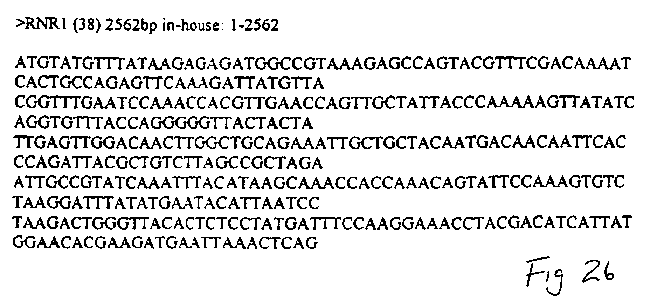

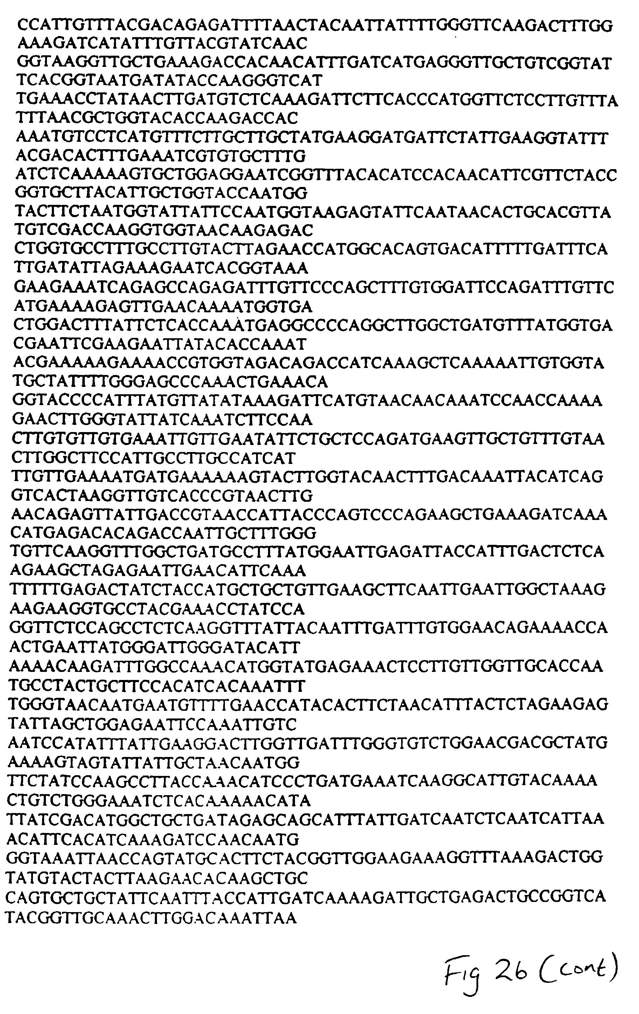

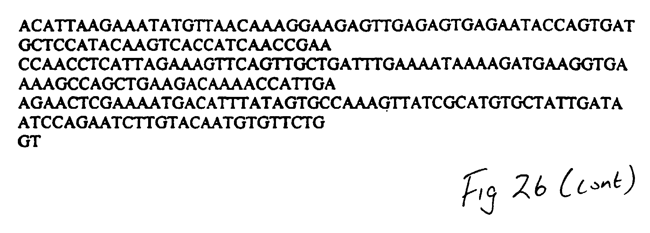

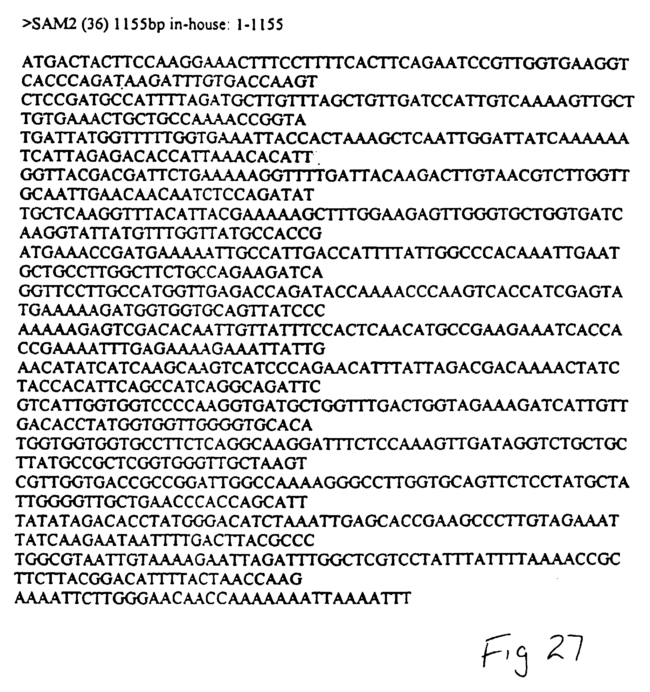

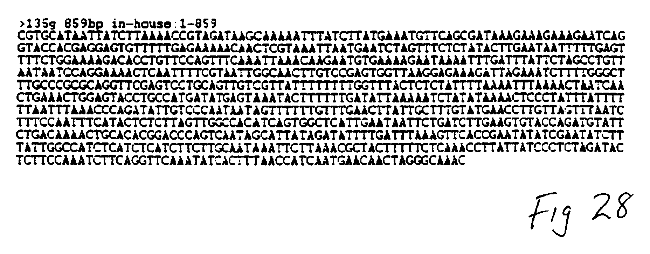

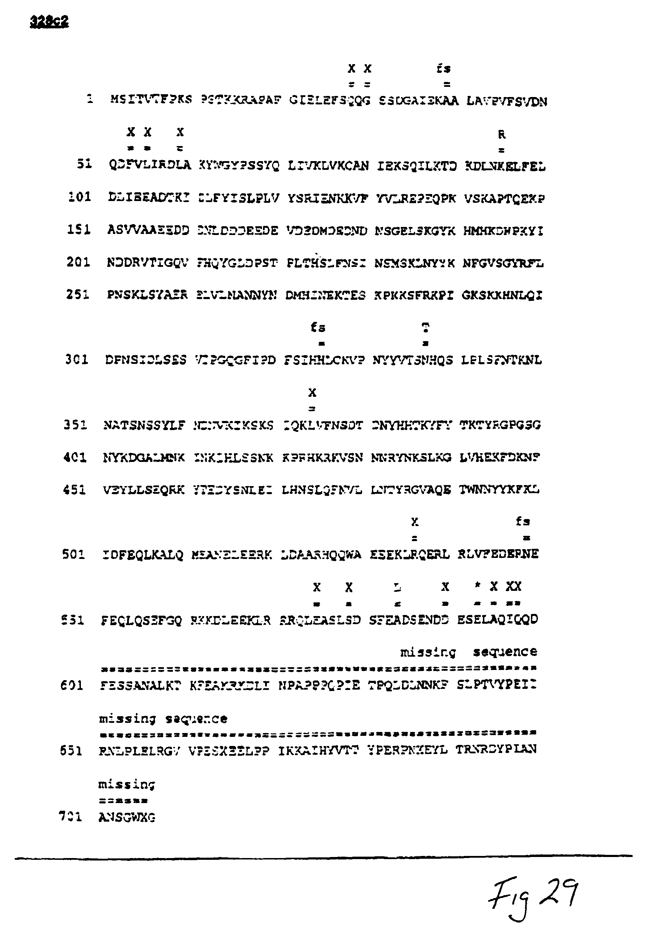

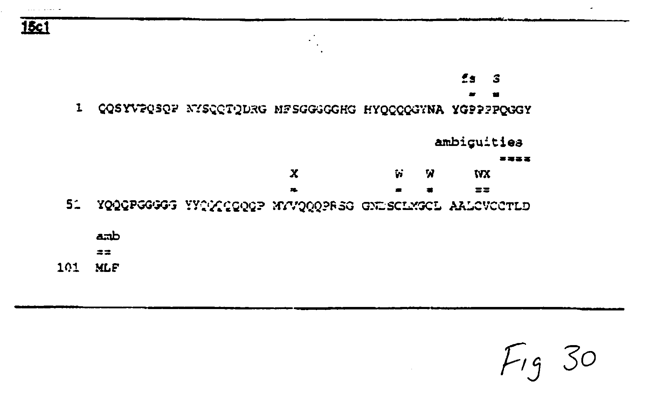

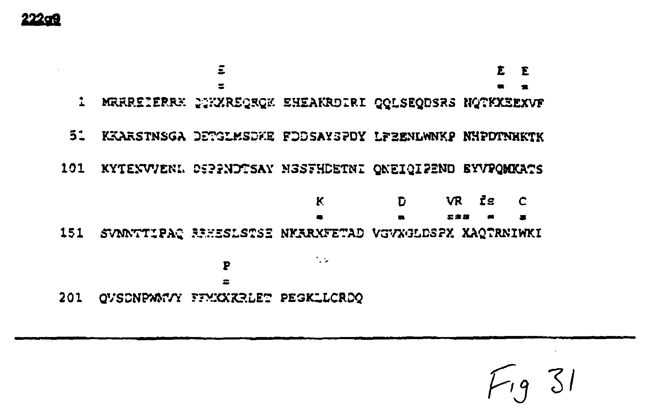

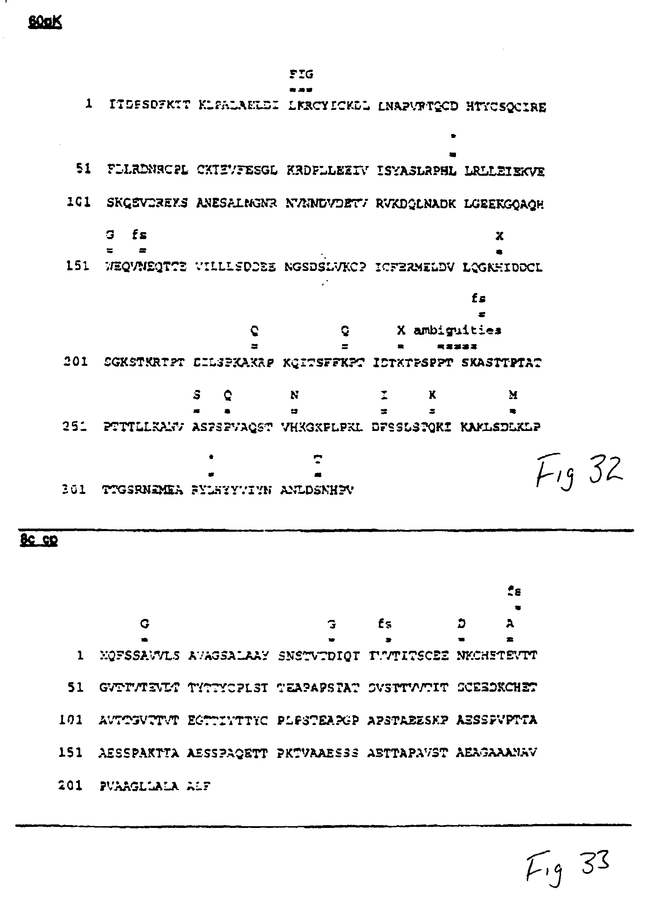

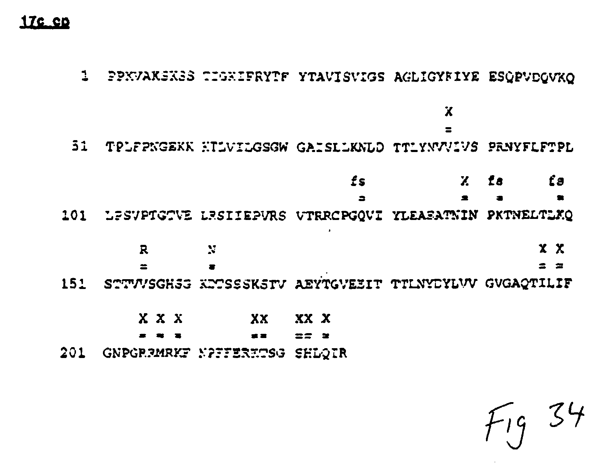

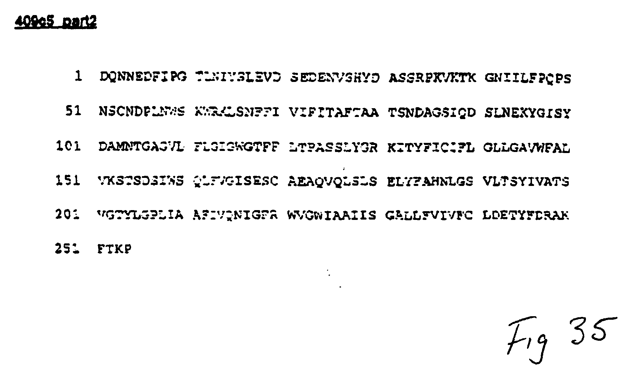

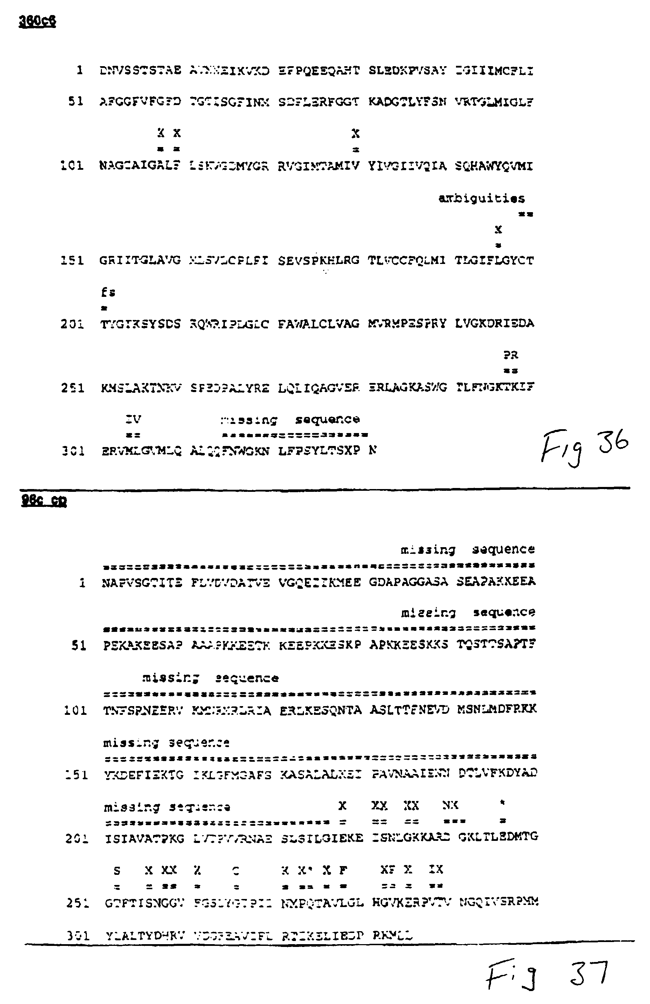

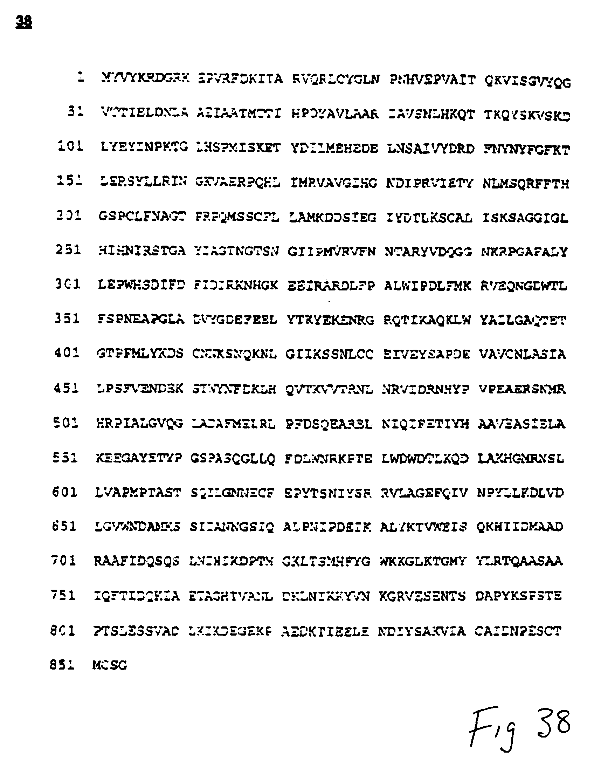

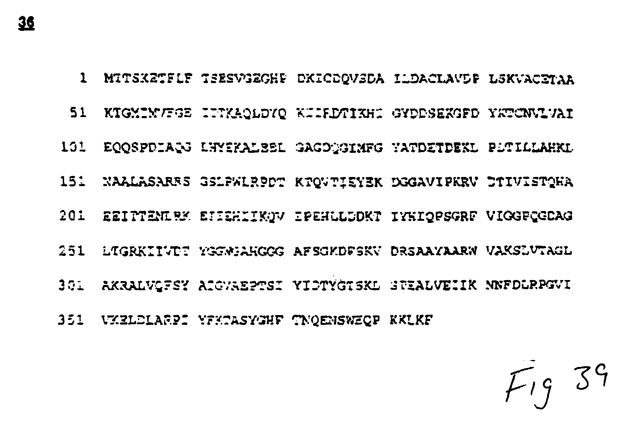

A further aspect of the invention comprises nucleic acid molecules encoding proteins which are critical for survival and growth of *Candida albicans*, which nucleic acid molecules comprise any of the sequences illustrated in Figures 5 to 29. Polypeptides which are critical for survival and growth of *Candida albicans* are also encompassed within the present invention, and which polypeptides comprise any of the amino acid sequences illustrated in Figures 29 to 39.

The present invention may be more clearly understood with reference to the accompanying example, which is purely exemplary, with reference to the accompanying drawings wherein:

| | |
|---|---|
| Figure 1: | is a diagrammatic representation of plasmid pGAL1PNiST-1. |
| Figure 2: | is a nucleotide sequence of plasmid pGAL1PNiST-1 of Figure 1. |
| Figure 3: | is a diagrammatic representation of plasmid pGAL1PSiST-1. |
| Figure 4: | is a nucleotide sequence of plasmid pGAL1PSiST-1 of Figure 3. |
| Figures 5 to 28: | illustrate the nucleotide sequences of oligonucleotides encoding polypeptides of previously unknown function isolated from *Candida albicans* which are critical for its survival and growth, according to the invention. |
| Figures 29 to 39: | illustrate the amino acid sequences of polypeptides from *Candida albicans* which are critical for its survival and growth, according to the invention. |

## Example 1

### Identification of novel drug targets in *C. albicans* by anti-sense and disruptive integration

The principle of the approach is based on the fact that when a particular *C. albicans* mRNA is inhibited by producing the complementary anti-sense RNA, the corresponding protein will decrease. If this protein is critical for growth or survival, the cell producing the anti-sense RNA will grow more slowly or will die.

Since anti-sense inhibition occurs at mRNA level, the gene copy number is irrelevant, thus allowing applications of the strategy even in diploid organisms.

Anti-sense RNA is endogenously produced from an integrative or episomal plasmid with an inducible promoter; induction of the promoter leads to the production of a RNA encoded by the insert of the plasmid. This insert will differ from one plasmid to another in the library. The inserts will be derived from genomic DNA fragments or from cDNA to cover-to the extent possible- the entire genome.

The vector is a proprietary vector allowing integration by homologous recombination at either the homologous insert or promoter sequence in the *Candida* genome. After introducing plasmids from cDNA or genomic libraries into *C. albicans*, transformants are screened for impaired growth after promoter (& thus anti-sense) induction in the presence of lithium acetate. Lithium acetate prolongs the G1 phase and thus allows anti-sense to act during a prolonged period of time during the cell cycle. Transformants which show impaired growth in both induced and non-induced media, thus showing a growth defect due to integrative disruption, are selected as well.

Transformants showing impaired growth are supposed to contain plasmids which produce anti-sense RNA to mRNAs critical for growth or survival. Growth is monitored by measuring growth-curves over a period of time in a device (Bioscreen Analyzer, Labsystems) which allows simultaneous measurement of growth-curves of 200 transformants.

Subsequently plasmids can be recovered from the transformants and the sequence of their inserts determined, thus revealing which mRNA they inhibit. In order to be able to recover the genomic or cDNA insert which has integrated into the *Candida* genome, genomic DNA is isolated, cut with an enzyme which cuts only once into the library vector (and estimated approx. every 4096 bp in the genome) and religated. PCR with primers flanking the insert will yield (partial) genomic or cDNA inserts as PCR fragments which can directly be sequenced. This PCR analysis (on ligation reaction) will also show us how many integrations occurred. Alternatively the ligation reaction is transformed to E. coli and PCR analysis is performed on colonies or on plasmid DNA derived thereof.

This method is employed for a genome wide search for novel *C. albicans* genes which are important for growth or survival.

<u>**Materials & Methods**</u>

**Construction of pGal1PNiST-1**

The backbone of the pGAL1PNiST-1 vector (integrative anti-sense S*fi*I-N*ot*I vector) is pGEM11Zf(+) (Promega Inc.). First, the CaMAL2 *Eco*RI/*Sal*I promoter fragment from pDBV50 (D.H. Brown *et al*.) was ligated into *Eco*RI/*Sal*I-opened pGEM11Zf(+) resulting in the intermediate construct pGEMMAL2P-1. Into the latter (*Msc*I/CIP) the CaURA3 selection marker was cloned as a E*co*47III/*Xmn*I fragment derived from pRM2. The resulting pGEMMAL2P-2 vector was *Not*I/*Hin*dIII opened in order to accept the *Not*I-stuffer-*Sfi*I cassette from pPCK1NiSCYCT-1 (*Eag*I/*Hind*III fragment): pMAL2PNiST-1. Finally, the plasmid pGAL1PNiST-1 was constructed by exchanging the *Sal*I/*Ecl*136II MAL2 promoter in pMAL2PNiST-1 by the *Xho*I/*Sma*I GAL1 promoter fragment derived from pRM2GAL1P.

**Construction of pGal1PSiST-1**

The vector pGAL1PSiST-1 was created for cloning the small genomic DNA fragments (flanked by *Sfi*I sites) behind the GAL1 promoter. The only difference with pGAL1PNiST-1 is that the hIFNβ (stuffer fragment) insert fragment in pGAL1PSiST-1 is flanked by two *Sfi*I sites in stead of a *Sfi*I and a *Not*I site as in pGAL1PNiST-1. To construct pGAL1PSiST-1 the *Eco*RI-*Hind*III fragment, containing hIFNβ flanked by a *Sfi*I and a *Not*I site, of pMAL2pHiET-3 (unpublished) was exchanged by the *Eco*RI-*Hind*III fragment, containing hIFNβ flanked by two *Sfi*I sites, from YCp50S-S (an *E. coli / S. cerevisiae* shuttle vector derived from the plasmid YCp50, which is deposited in the ATCC collection (number 37419; Thrash *et al*., 1985); an *Eco*RI-*Hind*III fragment, containing the gene hIFNβ, which is flanked by two *Sfi*I sites, was inserted in YCp50, creating YCp50S-S), resulting into plasmid pMAL2PSiST-1. The *mal*2 promoter from pMAL2PSiST-1 (by a *Nae*I-*Fsp*I digest) was further replaced by the *gal*1 promoter from pGAL1PNiST-1 (via a *Xho*I-*Sal*I digest), creating the vector pGAL1PSiST-1.

***Candida albicans* genomic library**

** Preparation of the genomic DNA fragments*

A *Candida albicans* genomic DNA library with small DNA fragments (400 to 1,000 bp) was prepared. Genomic DNA of *Candida albicans* B2630 was isolated following a modified protocol of Blin and Stafford (1976). The quality of the isolated genomic DNA was checked by gel electrophoresis. Undigested DNA was located on the gel above the marker band of 26,282 bp. A little smear, caused by fragmentation of the DNA, was present. To obtain enrichment for genomic DNA fragments of the desired size, the genomic DNA was partially digested. Several restriction enzymes (*Alu*I, *Hae*III and *Rsa*I; all creating blunt ends) were tried out. The appropriate digest conditions have been determined by titration of the enzyme. Enrichment of small DNA fragments was obtained with 70 units of *Alu*I on 10 µg of genomic DNA for 20 min. T4 DNA polymerase (Boehringer) and dNTPs (Boehringer) were added to polish the DNA ends. After extraction with phenolchloroform the digest was size-fractionated on an agarose gel. The genomic DNA fragments with a length of 500 to 1,250 bp were eluted from the gel by centrifugal filtration (Zhu *et al*., 1985). *Sfi*I adaptors (5' GTTGGCCTTTT) or (5' AGGCCAAC) were attached to the DNA ends (blunt) to facilitate cloning of the fragments into the vector. Therefore, a 8-mer and 11-mer oligonucleotide (comprising the *Sfi*I site) were kinated and annealed. After ligation of these adaptors to the DNA fragments a second size-fractionation was performed on an agarose gel. The DNA fragments of 400 to 1150 bp were eluted from the gel by centrifugal filtration.

** Preparation of the pGAL1PSiST-1 vector fragment*

The small genomic DNA fragments were cloned after the GAL1 promoter in the vector pGAL1PSIST-1. Qiagen-purified pGAL1PSiST-1 plasmid DNA was digested with *Sfi*I and the largest vector fragment eluted from the gel by centrifugal filtration (Zhu *et al*., 1985). Ligation with a control DNA fragment, flanked by *Sfi*I sites, was performed as a control. The ligation mix was electroporated to MC1061 *E. coli* cells. Plasmid DNA of 24 clones was analyzed. In all cases the control fragment was inserted in the pGAL1PSiST-1 vector fragment.

**** Upscaling***

All genomic DNA fragments (450 ng) were ligated into the pGAL1PSiST-1 vector (20 ng). After electroporation at 2500V, 40μF circa 400,000 clones were obtained. These clones were pooled into three groups and stored as glycerol slants. Also Qiagen-purified DNA was prepared from these clones. A clone analysis showed an average insert length of 600 bp and a percentage of 91 for clones with an insert. The size of the library corresponds to 5 times the diploid genome. The genomic DNA inserts are sense or anti-sense orientated in the vector.

***Candida albicans cDNA library***

Total RNA was extracted from *Candida albicans* B2630 grown on respectively minimal (SD) and rich (YPD) medium as described by Chirgwin *et al* in Sambrook *et al*. mRNA was prepared from total RNA using the Invitrogen Fast Track procedure.

First strand cDNA is synthesised with the Superscript Reverse Transcriptase (BRL) and with an oligo dT-*Not*I Primer adapter. After second strand synthesis, cDNA is polished with Klenow enzyme and purified over a Sephacryl S-400 spun column. Phosphorylated *Sfi*I adapters are then ligated to the cDNA, followed by digestion with the *Not*I restriction enzyme. The *Sfi*I/*Not*I cDNA is then purified and sized on a Biogel column A150M.

First fraction contains approximately 38,720 clones by transformation, the second fraction only 1540 clones. Clone analysis:

Fr. I: 22/24 inserts, 16 ≥ 1000 bp, 4 ≥ 2000 bp, average size: 1500 bp.
Fr. II: 9/12 inserts, 3 ≥ 1000 bp, average size: 960 bp cDNA was ligated in a *Not*I/*Sfi*I opened pGAL1PNiST-1 vector (anti-sense)

***Candida transformation***

The host strain used for transformation is a *C. albicans* ura3 mutant, CAI-4, which contains a deletion in orotidine-5'-phosphate decarboxylase and was obtained from William Fonzi, Georgetown University (Fonzi and Irwin). CAI-4 was transformed with the above described cDNA library or genomic library using the Pichia spheroplast module (Invitrogen). Resulting transformants were plated on minimal medium supplemented with glucose (SD, 0.67% or 1.34% Yeast Nitrogen base w/o amino acids + 2% glucose) plates and incubated for 2-3 days at 30°C.

**Screening for mutants**

Starter cultures were set up by inoculating each colony in 1 ml SD medium and incubating overnight at 30°C and 300 rpm. Cell densities were determined using a Coulter counter (Coulter Z1; Coulter electronics limited). 250.000 cells/ml were inoculated in 1 ml SD medium and cultures were incubated for 24 hours at 30°C and 300 rpm. Cultures were washed in minimal medium without glucose (S) and the pellet resuspended in 650 μl S medium. 8 μl of this culture is used for inoculating 400 μl cultures in a Honeywell-100 plate (Bioscreen analyzer; Labsystems). Each transformant was grown during three days in *S* medium containing LiAc; pH 6.0, with 2% glucose/2% maltose or 2% galactose/2% maltose respectively while shaking every 3 minutes for 20 seconds. Optical densities were measured every hour during three consecutive days and growth curves were generated (Bioscreen analyzer; Labsystems).

Growth curves of transformants grown in respectively anti-sense non-inducing (glucose/maltose) and inducing (galactose/maltose) medium are compared and those transformants showing impaired growth upon anti-sense induction are selected for further analysis. Transformants showing impaired growth by virtue of integration into a critical gene are also selected.

**Isolation of genomic or cDNA inserts**

Putatively interesting transformants are grown in 1.5 ml SD overnight and genomic DNA is isolated using the Nucleon MI Yeast kit (Clontech). Concentration of genomic DNA is estimated by analyzing a sample on an agarose gel.

20 ng of genomic DNA is digested for three hours with an enzyme that cuts uniquely in the library vector (SacI for the genomic library; PstI for the cDNA library) and treated with RNAse. Samples are phenol/chloroform extracted and precipitated using NaOAc/ethanol.

The resulting pellet is resuspended in 500 µl ligation mixture (1 x ligation buffer and 4 units of T4 DNA ligase; both from Boehringer) and incubated overnight at 16°C.

After denaturation (20 min 65°C), purification (phenol/chloroform extraction) and precipitation (NaOAc/ethanol) the pellet is resuspended in 10 µl MilliQ (Millipore) water.

**PCR analysis**

Inverse PCR is performed on 1 µl of the precipitated ligation reaction using library vector specific primers (oligo23 5' TGC-AGC-TCG-ACC-TCG-ACT-G 3' and oligo25 5' GCG-TGA-ATG-TAA-GCG-TGA-C 3' for the genomic library; 3pGALNistPCR primer :5'TGAGCAGCTCGCCGTCGCGC 3' and 5pGALNistPCR primer: 5'GAGTTATACCCTGCAGCTCGAC 3' for the cDNA library; both from Eurogentec) for 30 cycles each consisting of (a) 1 min at 95 °C, (b) 1 min at 57 °C, and (c) 3 min at 72 °C. In the reaction mixture 2.5 units of Taq polymerase (Boehringer) with TaqStart antibody (Clontech) (1:1) were used, and the final concentrations were 0.2 µM of each primer, 3 mM MgCl2 (Perkin Elmer Cetus) and 200 µM dNTPs (Perkin Elmer Cetus). PCR was performed in a Robocycler (Stratagene).

**Sequence determination**

Resulting PCR products were purified using PCR purification kit (Qiagen) and were quantified by comparison of band intensity on EtBr stained agarose gel with the intensity of DNA marker bands. The amount of PCR product (expressed in ng) used in the sequencing reaction is calculated as the length of the PCR product in basepairs divided by 10. Sequencing reactions were performed using the ABI Prism BigDye Terminator Cycle Sequencing Ready Reaction Kit according to the instructions of the manufacturer (PE Applied Biosystems, Foster City, CA) except for the following modifications.

The total reaction volume was reduced to 15 µl. Reaction volume of individual reagents were changed accordingly. 6.0 µl Terminator Ready Reaction Mix was replaced by a mixture of 3.0 µl Terminator Ready Reaction Mix + 3.0 µl Half Term (GENPAK Limited, Brighton, UK). After cycle sequencing, reaction mixtures were purified over Sephadex G50 columns prepared on Multiscreen HV opaque microtiter plates (Millipore, Molsheim, Fr) and were dried in a speed-Vac. Reaction products were resuspended in 3 µl loading buffer. Following denaturation for 2 min at 95°C, 1 µl of sample was applied on a 5% Long Ranger Gel (36 cm well-to-read) prepared from Singel Packs according to the supplier's instructions (FMC BioProducts, Rockland, ME). Samples were run for 7 hours 2X run on a ABI 377XL DNA sequencer. Data collection version 2.0 and Sequence analysis version 3.0 (for basecalling) software packages are from PE Applied Biosystems. Resulting sequence text files were copied onto a server for further analysis.

**Sequence analysis**

Nucleotide sequences were imported in the VectorNTI software package (InforMax Inc, North Bethesda, MD, USA), and the vector and insert regions of the sequences were identified. Sequence similarity searches against public and commercial sequence databases were performed with the BLAST software package (Altschul et al., 1990) version 1.4. Both the original nucleotide sequence and the six-frame conceptual translations of the insert region were used as query sequences. The used public databases were the EMBL nucleotide sequence database (Stoesser et al., 1998), the SWISS-PROT protein sequence database and its supplement TrEMBL (Bairoch and Apweiler, 1998), and the ALCES *Candida albicans* sequence database (Stanford University, University of Minnesota). The commercial sequence databases used were the LifeSeq® human and PathoSeq™ microbial genomic databases (Incyte Pharmaceuticals Inc., Palo Alto, CA, USA), and the GENESEQ patent sequence database (Derwent, London, UK). Three major results were obtained on the basis of the sequence similarity searches: function, novelty, and specificity. A putative function was deduced on the basis of the similarity with sequences with a known function, the novelty was based on the absence or presence of the sequences in public databases, and the specificity was based on the similarity with vertebrate homologues.

**Methods**

Blastx of the nucleic acid sequences against the appropriate protein databases: Swiss-Prot for clones of which the complete sequence is present in the public domain, and paorfp (PathoSeq™)for clones of which the complete sequences is not present in the public domain.

The protein to which the translated nucleic acid sequence corresponds to is used as a starting point. The differences between this protein and our translated nucleic acid sequences are marked with a double line and annotated above the protein sequence. The following symbols are used: a one-letter amino acid code or the ambiguity

code X is used if our translated nucleic acid sequence has another amino acid on a certain position,
the stop codon sign *is used if our translated nucleic acid sequence has a stop codon on a certain position,
The letters fs (frame shift) are used if a frame shift occurs in our translated nucleic acid sequence, and another reading frame is used,
the words ambiguity or ambiguities are used if a part of our translated nucleic acid sequence is present in the proteins, but not visible in the alignments of the blast results,
The phrase missing sequence is used if the translated nucleic acid sequence does not comprise that part of the protein.
Blastx: compares the six-frame conceptual translation products of a nucleotide query sequence (both strands) against a protein sequence database.

**Screening for compounds modulating expression of polypeptides critical for growth and survival of *C. albicans***

The method proposed is based on observations (Sandbaken *et al*., 1990; Hinnebusch and Liebman 1991; Ribogene PCT WO 95/11969, 1995) suggesting that underexpression or overexpression of any component of a process (e.g. translation) could lead to altered sensitivity to an inhibitor of a relevant step in that process. Such an inhibitor should be more potent against a cell limited by a deficiency in the macromolecule catalyzing that step and/or less potent against a cell containing an excess of that macromolecule, as compared to the wild type (WT) cell.

Mutant yeast strains, for example, have shown that some steps of translation are sensitive to the stoichiometry of macromolecules involved. (Sandbaken *et al*.). Such strains are more sensitive to compounds which specifically perturb translation (by acting on a component that participates in translation) but are equally sensitive to compounds with other mechanisms of action.

This method thus not only provides a means to identify whether a test compound perturbs a certain process but also an indication of the site at which it exerts its effect. The component which is present in altered form or amount in a cell whose growth is affected by a test compound is potentially the site of action of the test compound.

The assay to be set up involves measurement of growth of an isogenic strain which has been modified only in a certain specific allele, relative to a wild type (WT) *C. albicans* strain, in the presence of R-compounds. Strains can be ones in which the expression of a specific essential protein is impaired upon induction of anti-sense or strains which carry disruptions in an essential gene. An in silico approach to finding novel essential genes in *C. albicans* will be performed. A number of essential genes identified in this way will be disrupted (in one allele) and the resulting strains can be used for comparative growth screening.

**Assay for High Throughput screening for drugs**

35 µl minimal medium (S medium + 2% galactose + 2% maltose) is transferred in a transparent flat-bottomed 96 well plate using an automated pipetting system (Multidrop, Labsystems). A 96-channel pipettor (Hydra, Robbins Scientific) transfers 2.5 µl of R-compound at $10^{-3}$ M in DMSO from a stock plate into the assay plate.

The selected *C. albicans* strains (mutant and parent (CAI-4) strain) are stored as glycerol stocks (15%) at -70ºC. The strains are streaked out on selective plates (SD medium) and incubated for two days at 30ºC. For the parent strain, CAI-4, the medium is always supplemented with 20 µg/ml uridine. A single colony is scooped up and resuspended in 1 ml minimal medium (S medium + 2% galactose + 2% maltose). Cells are incubated at 30ºC for 8 hours while shaking at 250 rpm. A 10 ml culture is inoculated at 250.000 cells/ml. Cultures are incubated at 30ºC for 24 hours while shaking at 250 rpm. Cells are counted in Coulter counter and the final culture (S medium + 2% galactose + 2% maltose) is inoculated at 20.000 to 50.000 cells/ml. Cultures are grown at 30ºC while shaking at 250 rpm until a final OD of 0.24 (+/- 0.04) 6nM is reached.

200 µl of this yeast suspension is added to all wells of MW96 plates containing R-compounds in a 450 µl total volume. MW96 plates are incubated (static) at 30ºC for 48 hours.

Optical densities are measured after 48 hours.

Test growth is expressed as a percentage of positive control growth for both mutant (x) and wild type (y) strains. The ratio (x/y) of these derived variables is calculated.

<u>**References**</u>

Thrash C., Bankier A. T., Barrell B. G. and Sternglanz R. (1985) *Proc. Natl. Acad. Sci.* USA **82**: 4374-4378.

Blin N. and Stafford D. W. (1976) *Nucleic Acids Res.* **3**: 2303-2308.

Zhu J., Kempenaers W., Van Der Straeten D., Contreras R., and Fiers W. (1985) *Bio/Technology* **3**: 1014-1016.

Sambrook J., Fritsch E.F. and Maniatis T. (1989) Molecular Cloning: A Laboratory Manual, 2nd Ed., Cold Spring Harbor Laboratory, Cold Spring Harbor, NY.

Fonzi W. and Irwin H. (1993) *Genetics* **134**:717-728. Altschul S.F, Gish W., Miller W., Myers E.W., Lipman D.J. (1990) *J. Mol. Biol*. **215**(3):403-410.

Bairoch A. and Apweiler R. (1998) *Nucleic Acids Res.* **26**(1):38-42.

Stoesser G., Moseley M.A., Sleep J., McGowran M., Garcia-Pastor M., Sterk P. (1998) *Nucleic Acids Res.* **26**(1):8-15.

*Chien et al*., (1991) Proc. Natl. Acad. Sci USA 88, 9578-9582.

Sandbaken M.G. Lupisella J.A., DiDomenico B. and Chakraburtty K., *J. Biol. Chem*. **265**:15838-15844, 1990.

Hinnebusch A.G. and Liebman S.W., in : The Molecular Biology of the Yeast *Saccharomyces*, Broach J.R., Pringle J.R. and Jones E.W., eds., CSH Laboratory Press; NY 1991.

Patent application RiboGene Inc., PCT WO 95./11969, 1995.

Brown, D.H. *et al*., Molecular General Genetics 251 (1) 75-80, 1996.

## Claims

1. A nucleic acid molecule encoding a polypeptide which is critical for survival and growth of the yeast *Candida albicans* and which nucleic acid molecule comprises any of the sequences of nucleotides in Sequence ID Numbers 1 to 3, 5, 6, 8 to 11, 13, 15, 16, 18, 20, 21, 23, 25 to 29, 31, 35, 37, 39, 41, 43, 45, 47, 49, 51, 53, 55, 57, 59, 61, 63, 65, 67, 69 and 71.

2. A nucleic acid molecule encoding a polypeptide which is critical for survival and growth of the yeast *Candida albicans* and which nucleic acid molecule comprises any of the sequences of nucleotides in Sequence ID Numbers 28, 35, 37 and 39 and fragments or derivatives of said nucleic acid molecules.

3. A nucleic acid molecule encoding a polypeptide which is critical for survival and growth of the yeast *Candida albicans* and which polypeptide has an amino acid sequence according to the sequence of any of Sequence ID Numbers 4, 7, 12, 14, 17, 19, 22, 24, 30, 32 to 34, 36, 38, 40, 42, 44, 46, 48, 50, 52, 54, 56, 58, 60, 62, 64, 66, 68, 70 and 72.

4. A nucleic acid molecule according to any of claims 1 to 3 which is mRNA.

5. A nucleic acid molecule according to any of claims 1 to 3 which is DNA.

6. A nucleic acid molecule according to claim 5 which is cDNA.

7. A nucleic acid molecule capable of hybridising to the molecules according to any of claims 1 to 5 under high stringency conditions.

8. A polypeptide having the amino acid sequences of any of Sequence ID Numbers 4, 7, 12, 14, 17, 19, 22, 24, 30, 32 to 34, 36, 38, 40, 42, 44, 46, 48, 50, 52, 54, 56, 58, 60, 62, 64, 66, 68, 70 and 72.

9. A polypeptide encoded by the nucleic acid molecule according to any of claims 1 to 6.

10. A polypeptide according to claim 9 having an amino acid sequence of any of Sequence ID Numbers 4, 7, 12, 14, 17, 19, 22, 24, 30, 32 to 34, 36, 38, 40, 42, 44, 46, 48, 50, 52, 54, 56, 58, 60, 62, 64, 66, 68, 70 and 72.

11. An expression vector comprising a nucleic acid molecule according to claim 5 or 6.

12. An expression vector according to claim 11 which comprises an inducible promoter.

13. An expression vector according to claim 11 or 12 which comprises a sequence encoding a reporter molecule.

14. A nucleic acid molecule according to any of claims 1 to 7 for use as a medicament.

15. Use of a nucleic acid molecule according to any of claims 1 to 7 in the preparation of a medicament for treating *Candida albicans* associated diseases.

16. A polypeptide according to any of claims 8 or 10 for use as a medicament.

17. Use of a polypeptide according to any of claims 8 to 10 in the preparation of a medicament for treating *Candida albicans* associated infections.

18. A pharmaceutical composition comprising a nucleic acid molecule according to any of claims 1 to 7 or a polypeptide according to any of claims 8 to 10 together with a pharmaceutically acceptable carrier diluent or excipient therefor.

**19.** A *Candida albicans* cell comprising an induced mutation in the DNA sequence encoding the polypeptide according to any of claims 8 to 10.

**20.** A method of identifying compounds which selectively modulate expression of polypeptides which are crucial for growth and survival of *Candida albicans*, which method comprises:

(a) contacting a compound to be tested with one or more *Candida albicans* cells having a mutation in a nucleic acid molecule according to any of claims 1 to 6 which mutation results in overexpression or underexpression of said polypeptides in addition to contacting one or more wild type *Candida albicans* cells with said compound,
(b) monitoring the growth and/or activity of said mutated cell compared to said wild type; wherein differential growth or activity of said one or more mutated Candida cells is indicative of selective action of said compound on a polypeptide or another polypeptide in the same or a parallel pathway.

**21.** A compound identifiable according to the method of claim 20.

**22.** A compound according to claim 21 for use as a medicament.

**23.** Use of a compound according to claim 21 in the preparation of a medicament for treating *Candida albicans* associated diseases.

**24.** A pharmaceutical composition comprising a compound according to claim 21 together with a pharmaceutically acceptable carrier, diluent or excipient therefor.

**25.** A method of identifying DNA sequences from a cell or organism which DNA encodes polypeptides which are critical for growth or survival of said cell or organism, which method comprises:

(a) preparing a cDNA or genomic library from said cell or organism in a suitable expression vector which vector is such that it can either integrate into the genome in said cell or that it permits transcription of antisense RNA from the nucleotide sequences in said cDNA or genomic library,
(b) selecting transformants exhibiting impaired growth and determining the nucleotide sequence of the cDNA or genomic sequence from the library included in the vector from said transformant.

**26.** A method according to claim 25 wherein said cell or organism is a yeast or filamentous fungi.

**27.** A method according to claim 25 or 26 wherein said cell or organism is any of *Saccharomyces cervisiae*, *Saccharomyces pombe* or *Candida albicans*.

**28.** Plasmid pGAL1PSiST-1 having the sequence of nucleotides illustrated in Figure 2.

**29.** Plasmid pGAL1PNiST-1 having the sequence of nucleotides illustrated in Figure 4.

**30.** An antibody capable of binding to a polypeptide according to any of claims 8 or 10.

**31.** An oligonucleotide comprising a fragment of from 10 to 50 contiguous nucleic acid sequences of a nucleic acid molecule according to any of claims 1 to 7.

**32.** A nucleic acid molecule encoding a polypeptide which is critical for survival and growth of the yeast *Candida albicans*, said nucleic acid molecule comprising the sequences of any of the nucleotide sequences illustrated in Figures 5 to 28.

**33.** A polypeptide which is critical for survival and growth of the yeast *Candida albicans*, said polypeptide comprising the amino acid sequences of any of the sequences illustrated in Figures 29 to 39.

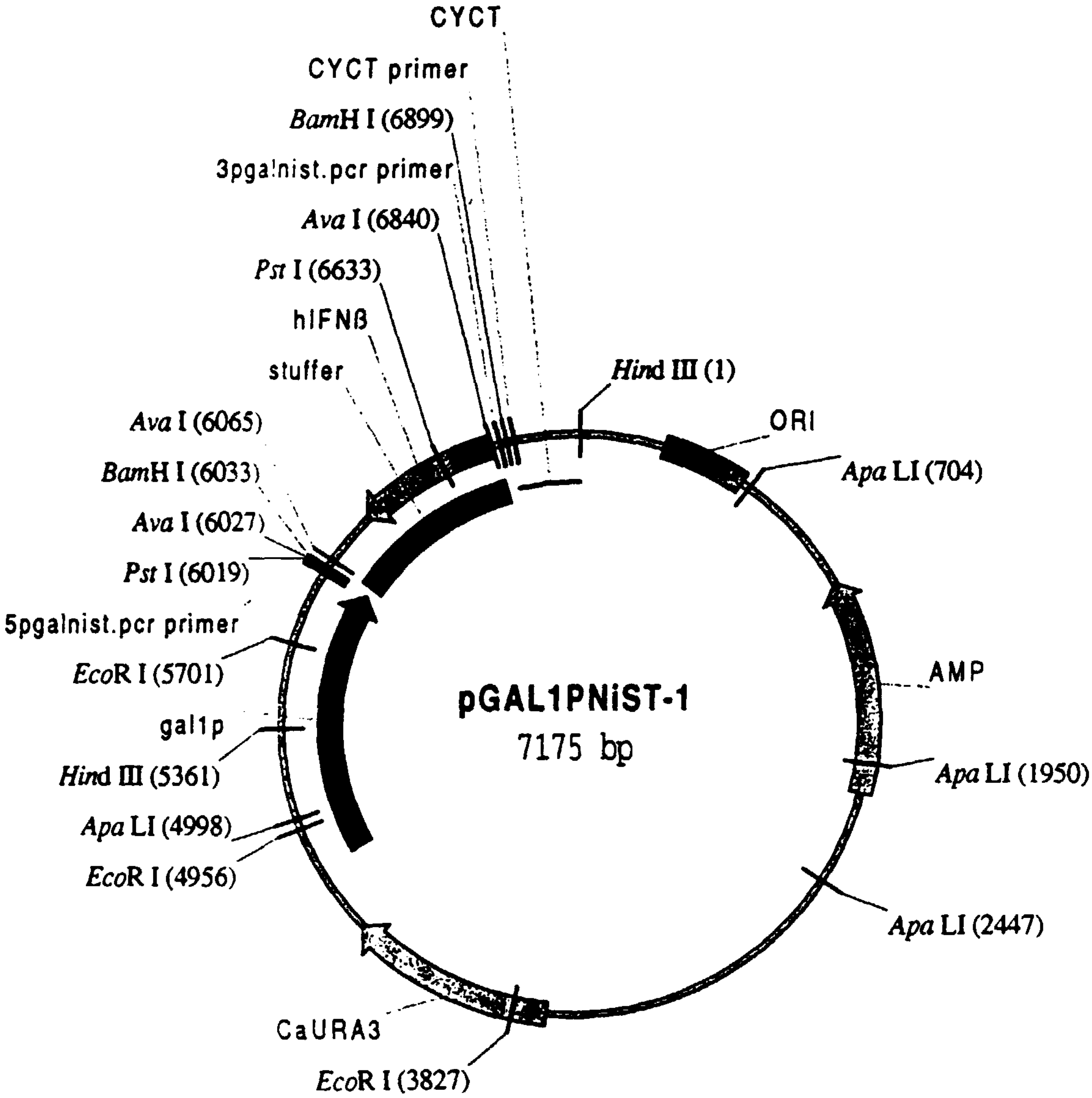
CYCT
CYCT primer
BamH I (6899)
3pgalnist.pcr primer
Ava I (6840)
Pst I (6633)
hIFNß
stuffer
Ava I (6065)
BamH I (6033)
Ava I (6027)
Pst I (6019)
5pgalnist.pcr primer
EcoR I (5701)
gal1p
Hind III (5361)
Apa LI (4998)
EcoR I (4956)
CaURA3
EcoR I (3827)
Hind III (1)
ORI
Apa LI (704)
AMP
Apa LI (1950)
Apa LI (2447)
pGAL1PNiST-1
7175 bp

Figure 1

```
     HindIII
     ~~~~~
  1  AGCTTGAGTA TTCTATAGTG TCACCTAAAT AGCTTGGCGT AATCATGGTC
     TCGAACTCAT AAGATATCAC AGTGGATTTA TCGAACCGCA TTAGTACCAG

 51  ATAGCTGTTT CCTGTGTGAA ATTGTTATCC GCTCACAATT CCACACAACA
     TATCGACAAA GGACACACTT TAACAATAGG CGAGTGTTAA GGTGTGTTGT

101  TACGAGCCGG AAGCATAAAG TGTAAAGCCT GGGGTGCCTA ATGAGTGAGC
     ATGCTCGGCC TTCGTATTTC ACATTTCGGA CCCCACGGAT TACTCACTCG

151  TAACTCACAT TAATTGCGTT GCGCTCACTG CCCGCTTTCC AGTCGGGAAA
     ATTGAGTGTA ATTAACGCAA CGCGAGTGAC GGGCGAAAGG TCAGCCCTTT

201  CCTGTCGTGC CAGCTGCATT AATGAATCGG CCAACGCGCG GGGAGAGGCG
     GGACAGCACG GTCGACGTAA TTACTTAGCC GGTTGCGCGC CCCTCTCCGC

251  GTTTGCGTAT TGGGCGCTCT TCCGCTTCCT CGCTCACTGA CTCGCTGCGC
     CAAACGCATA ACCCGCGAGA AGGCGAAGGA GCGAGTGACT GAGCGACGCG

301  TCGGTCGTTC GGCTGCGGCG AGCGGTATCA GCTCACTCAA AGGCGGTAAT
     AGCCAGCAAG CCGACGCCGC TCGCCATAGT CGAGTGAGTT TCCGCCATTA

351  ACGGTTATCC ACAGAATCAG GGGATAACGC AGGAAAGAAC ATGTGAGCAA
     TGCCAATAGG TGTCTTAGTC CCCTATTGCG TCCTTTCTTG TACACTCGTT

401  AAGGCCAGCA AAAGGCCAGG AACCGTAAAA AGGCCGCGTT GCTGGCGTTT
     TTCCGGTCGT TTTCCGGTCC TTGGCATTTT TCCGGCGCAA CGACCGCAAA

451  TTCCATAGGC TCCGCCCCCC TGACGAGCAT CACAAAAATC GACGCTCAAG
     AAGGTATCCG AGGCGGGGGG ACTGCTCGTA GTGTTTTTAG CTGCGAGTTC

501  TCAGAGGTGG CGAAACCCGA CAGGACTATA AAGATACCAG GCGTTTCCCC
     AGTCTCCACC GCTTTGGGCT GTCCTGATAT TTCTATGGTC CGCAAAGGGG

551  CTGGAAGCTC CCTCGTGCGC TCTCCTGTTC CGACCCTGCC GCTTACCGGA
     GACCTTCGAG GGAGCACGCG AGAGGACAAG GCTGGGACGG CGAATGGCCT

601  TACCTGTCCG CCTTTCTCCC TTCGGGAAGC GTGGCGCTTT CTCATAGCTC
     ATGGACAGGC GGAAAGAGGG AAGCCCTTCG CACCGCGAAA GAGTATCGAG

651  ACGCTGTAGG TATCTCAGTT CGGTGTAGGT CGTTCGCTCC AAGCTGGGCT
     TGCGACATCC ATAGAGTCAA GCCACATCCA GCAAGCGAGG TTCGACCCGA

      ApaLI
      ~~~~~~
701  GTGTGCACGA ACCCCCCGTT CAGCCCGACC GCTGCGCCTT ATCCGGTAAC
     CACACGTGCT TGGGGGGCAA GTCGGGCTGG CGACGCGGAA TAGGCCATTG

751  TATCGTCTTG AGTCCAACCC GGTAAGACAC GACTTATCGC CACTGGCAGC
     ATAGCAGAAC TCAGGTTGGG CCATTCTGTG CTGAATAGCG GTGACCGTCG

801  AGCCACTGGT AACAGGATTA GCAGAGCGAG GTATGTAGGC GGTGCTACAG
     TCGGTGACCA TTGTCCTAAT CGTCTCGCTC CATACATCCG CCACGATGTC

851  AGTTCTTGAA GTGGTGGCCT AACTACGGCT ACACTAGAAG GACAGTATTT
     TCAAGAACTT CACCACCGGA TTGATGCCGA TGTGATCTTC CTGTCATAAA

901  GGTATCTGCG CTCTGCTGAA GCCAGTTACC TTCGGAAAAA GAGTTGGTAG
     CCATAGACGC GAGACGACTT CGGTCAATGG AAGCCTTTTT CTCAACCATC
```

Fig 2

```
951  CTCTTGATCC GGCAAACAAA CCACCGCTGG TAGCGGTGGT TTTTTTGTTT
     GAGAACTAGG CCGTTTGTTT GGTGGCGACC ATCGCCACCA AAAAAACAAA

1001 GCAAGCAGCA GATTACGCGC AGAAAAAAAG GATCTCAAGA AGATCCTTTG
     CGTTCGTCGT CTAATGCGCG TCTTTTTTTC CTAGAGTTCT TCTAGGAAAC

1051 ATCTTTTCTA CGGGGTCTGA CGCTCAGTGG AACGAAAACT CACGTTAAGG
     TAGAAAAGAT GCCCCAGACT GCGAGTCACC TTGCTTTTGA GTGCAATTCC

1101 GATTTTGGTC ATGAGATTAT CAAAAAGGAT CTTCACCTAG ATCCTTTTAA
     CTAAAACCAG TACTCTAATA GTTTTTCCTA GAAGTGGATC TAGGAAAATT

1151 ATTAAAAATG AAGTTTTAAA TCAATCTAAA GTATATATGA GTAAACTTGG
     TAATTTTTAC TTCAAAATTT AGTTAGATTT CATATATACT CATTTGAACC

1201 TCTGACAGTT ACCAATGCTT AATCAGTGAG GCACCTATCT CAGCGATCTG
     AGACTGTCAA TGGTTACGAA TTAGTCACTC CGTGGATAGA GTCGCTAGAC

1251 TCTATTTCGT TCATCCATAG TTGCCTGACT CCCCGTCGTG TAGATAACTA
     AGATAAAGCA AGTAGGTATC AACGGACTGA GGGGCAGCAC ATCTATTGAT

1301 CGATACGGGA GGGCTTACCA TCTGGCCCCA GTGCTGCAAT GATACCGCGA
     GCTATGCCCT CCCGAATGGT AGACCGGGGT CACGACGTTA CTATGGCGCT

1351 GACCCACGCT CACCGGCTCC AGATTTATCA GCAATAAACC AGCCAGCCGG
     CTGGGTGCGA GTGGCCGAGG TCTAAATAGT CGTTATTTGG TCGGTCGGCC

1401 AAGGGCCGAG CGCAGAAGTG GTCCTGCAAC TTTATCCGCC TCCATCCAGT
     TTCCCGGCTC GCGTCTTCAC CAGGACGTTG AAATAGGCGG AGGTAGGTCA

1451 CTATTAATTG TTGCCGGGAA GCTAGAGTAA GTAGTTCGCC AGTTAATAGT
     GATAATTAAC AACGGCCCTT CGATCTCATT CATCAAGCGG TCAATTATCA

1501 TTGCGCAACG TTGTTGCCAT TGCTACAGGC ATCGTGGTGT CACGCTCGTC
     AACGCGTTGC AACAACGGTA ACGATGTCCG TAGCACCACA GTGCGAGCAG

1551 GTTTGGTATG GCTTCATTCA GCTCCGGTTC CCAACGATCA AGGCGAGTTA
     CAAACCATAC CGAAGTAAGT CGAGGCCAAG GGTTGCTAGT TCCGCTCAAT

1601 CATGATCCCC CATGTTGTGC AAAAAAGCGG TTAGCTCCTT CGGTCCTCCG
     GTACTAGGGG GTACAACACG TTTTTTCGCC AATCGAGGAA GCCAGGAGGC

1651 ATCGTTGTCA GAAGTAAGTT GGCCGCAGTG TTATCACTCA TGGTTATGGC
     TAGCAACAGT CTTCATTCAA CCGGCGTCAC AATAGTGAGT ACCAATACCG

1701 AGCACTGCAT AATTCTCTTA CTGTCATGCC ATCCGTAAGA TGCTTTTCTG
     TCGTGACGTA TTAAGAGAAT GACAGTACGG TAGGCATTCT ACGAAAAGAC

1751 TGACTGGTGA GTACTCAACC AAGTCATTCT GAGAATAGTG TATGCGGCGA
     ACTGACCACT CATGAGTTGG TTCAGTAAGA CTCTTATCAC ATACGCCGCT

1801 CCGAGTTGCT CTTGCCCGGC GTCAATACGG GATAATACCG CGCCACATAG
     GGCTCAACGA GAACGGGCCG CAGTTATGCC CTATTATGGC GCGGTGTATC

1851 CAGAACTTTA AAAGTGCTCA TCATTGGAAA ACGTTCTTCG GGGCGAAAAC
     GTCTTGAAAT TTTCACGAGT AGTAACCTTT TGCAAGAAGC CCCGCTTTTG
```

```
                                                        ApaLI
                                                           ~~
1901  TCTCAAGGAT CTTACCGCTG TTGAGATCCA GTTCGATGTA ACCCACTCGT
      AGAGTTCCTA GAATGGCGAC AACTCTAGGT CAAGCTACAT TGGGTGAGCA

      ApaLI
      ----
1951  GCACCCAACT GATCTTCAGC ATCTTTTACT TTCACCAGCG TTTCTGGGTG
      CGTGGGTTGA CTAGAAGTCG TAGAAAATGA AAGTGGTCGC AAAGACCCAC

2001  AGCAAAAACA GGAAGGCAAA ATGCCGCAAA AAAGGGAATA AGGGCGACAC
      TCGTTTTTGT CCTTCCGTTT TACGGCGTTT TTTCCCTTAT TCCCGCTGTG

2051  GGAAATGTTG AATACTCATA CTCTTCCTTT TTCAATATTA TTGAAGCATT
      CCTTTACAAC TTATGAGTAT GAGAAGGAAA AAGTTATAAT AACTTCGTAA

2101  TATCAGGGTT ATTGTCTCAT GAGCGGATAC ATATTTGAAT GTATTTAGAA
      ATAGTCCCAA TAACAGAGTA CTCGCCTATG TATAAACTTA CATAAATCTT

2151  AAATAAACAA ATAGGGGTTC CGCGCACATT TCCCCGAAAA GTGCCACCTG
      TTTATTTGTT TATCCCCAAG GCGCGTGTAA AGGGGCTTTT CACGGTGGAC

2201  ACGTCTAAGA AACCATTATT ATCATGACAT TAACCTATAA AAATAGGCGT
      TGCAGATTCT TTGGTAATAA TAGTACTGTA ATTGGATATT TTTATCCGCA

2251  ATCACGAGGC CCTTTCGTCT CGCGCGTTTC GGTGATGACG GTGAAAACCT
      TAGTGCTCCG GGAAAGCAGA GCGCGCAAAG CCACTACTGC CACTTTTGGA

2301  CTGACACATG CAGCTCCCGG AGACGGTCAC AGCTTGTCTG TAAGCGGATG
      GACTGTGTAC GTCGAGGGCC TCTGCCAGTG TCGAACAGAC ATTCGCCTAC

2351  CCGGGAGCAG ACAAGCCCGT CAGGGCGCGT CAGCGGGTGT TGGCGGGTGT
      GGCCCTCGTC TGTTCGGGCA GTCCCGCGCA GTCGCCCACA ACCGCCCACA

                                                        ApaLI
                                                        -----
2401  CGGGGCTGGC TTAACTATGC GGCATCAGAG CAGATTGTAC TGAGAGTGCA
      GCCCCGACCG AATTGATACG CCGTAGTCTC GTCTAACATG ACTCTCACGT

      ApaLI
      -
2451  CCATATGCGG TGTGAAATAC CGCACAGATG CGTAAGGAGA AAATACCGCA
      GGTATACGCC ACACTTTATG GCGTGTCTAC GCATTCCTCT TTTATGGCGT

2501  TCAGGCGAAA TTGTAAACGT TAATATTTTG TTAAAATTCG CGTTAAATAT
      AGTCCGCTTT AACATTTGCA ATTATAAAAC AATTTTAAGC GCAATTTATA

2551  TTGTTAAATC AGCTCATTTT TTAACCAATA GGCCGAAATC GGCAAAATCC
      AACAATTTAG TCGAGTAAAA AATTGGTTAT CCGGCTTTAG CCGTTTTAGG

2601  CTTATAAATC AAAAGAATAG ACCGAGATAG GGTTGAGTGT TGTTCCAGTT
      GAATATTTAG TTTTCTTATC TGGCTCTATC CCAACTCACA ACAAGGTCAA

2651  TGGAACAAGA GTCCACTATT AAAGAACGTG GACTCCAACG TCAAAGGGCG
      ACCTTGTTCT CAGGTGATAA TTTCTTGCAC CTGAGGTTGC AGTTTCCCGC

2701  AAAAACCGTC TATCAGGGCG ATGGCCCACT ACGTGAACCA TCACCCAAAT
      TTTTTGGCAG ATAGTCCCGC TACCGGGTGA TGCACTTGGT AGTGGGTTTA

2751  CAAGTTTTTT GCGGTCGAGG TGCCGTAAAG CTCTAAATCG GAACCCTAAA
      GTTCAAAAAA CGCCAGCTCC ACGGCATTTC GAGATTTAGC CTTGGGATTT
```

```
2801 GGGAGCCCCC GATTTAGAGC TTGACGGGGA AAGCCGGCGA ACGTGGCGAG
     CCCTCGGGGG CTAAATCTCG AACTGCCCCT TTCGGCCGCT TGCACCGCTC

2851 AAAGGAAGGG AAGAAAGCGA AAGGAGCGGG CGCTAGGGCG CTGGCAAGTG
     TTTCCTTCCC TTCTTTCGCT TTCCTCGCCC GCGATCCCGC GACCGTTCAC

2901 TAGCGGTCAC GCTGCGCGTA ACCACCACAC CCGCCGCGCT TAATGCGCCG
     ATCGCCAGTG CGACGCGCAT TGGTGGTGTG GGCGGCGCGA ATTACGCGGC

2951 CTACAGGGCG CGTCCATTCG CCATTCAGGC TGCGCAACTG TTGGGAAGGG
     GATGTCCCGC GCAGGTAAGC GGTAAGTCCG ACGCGTTGAC AACCCTTCCC

3001 CGATCGGTGC GGGCCTCTTC GCTATTACGC CAGCTGGCGA AAGGGGGATG
     GCTAGCCACG CCCGGAGAAG CGATAATGCG GTCGACCGCT TTCCCCCTAC

3051 TGCTGCAAGG CGATTAAGTT GGGTAACGCC AGGGTTTTCC CAGTCACGAC
     ACGACGTTCC GCTAATTCAA CCCATTGCGG TCCCAAAAGG GTCAGTGCTG

3101 GTTGTAAAAC GACGGCCAGT GAATTGTAAT ACGACTCACT ATAGGGCGAA
     CAACATTTTG CTGCCGGTCA CTTAACATTA TGCTGAGTGA TATCCCGCTT

3151 TTGGTTTTCC AATGATGAGC ACTTTTAAAG TTCTGCTATG TGGCGCGGTA
     AACCAAAAGG TTACTACTCG TGAAAATTTC AAGACGATAC ACCGCGCCAT

3201 TTATCCCGTG TTGACGCCGG GCAAGAGCAA CTCGGTCGCC GCATACACTA
     AATAGGGCAC AACTGCGGCC CGTTCTCGTT GAGCCAGCGG CGTATGTGAT

3251 TTCTCAGAAT GACTTGGTTG AGTACTAATA GGAATTGATT TGGATGGTAT
     AAGAGTCTTA CTGAACCAAC TCATGATTAT CCTTAACTAA ACCTACCATA

3301 AAACGGAAAC AAAAAAAAGA GCTGGTACTA CTTTCTTTAA AATTATTTTA
     TTTGCCTTTG TTTTTTTTCT CGACCATGAT GAAAGAAATT TTAATAAAAT

3351 TTATTTGATT TTATTTAATA GTATATATTA TATTTTGAAC GTAGATTATT
     AATAAACTAA AATAAATTAT CATATATAAT ATAAAACTTG CATCTAATAA

3401 TTGTTGAAAG TTGCTGTAGT GCCATTGATT CGTAACACTA ATTCTGTATT
     AACAACTTTC AACGACATCA CGGTAACTAA GCATTGTGAT TAAGACATAA

3451 AGTCATTCCT CTTGTTTGAT AGTATCCAAA AAAACGGCTA TTTTTTTGCA
     TCAGTAAGGA GAACAAACTA TCATAGGTTT TTTTGCCGAT AAAAAAACGT

3501 ATCTTATTTC CTGCATATTA TACAGATAAC ATAATGAAAG AAAAAATCTT
     TAGAATAAAG GACGTATAAT ATGTCTATTG TATTACTTTC TTTTTTAGAA

3551 TTTTTTTGTT CTTCAATGAT GATTTCAACC ATTCTTTTAA ACATTGATCA
     AAAAAAACAA GAAGTTACTA CTAAAGTTGG TAAGAAAATT TGTAACTAGT

3601 ATTCCTGAGC AACAACCCCA TACACACTGG TTTATATACC GCCCCTTTTA
     TAAGGACTCG TTGTTGGGGT ATGTGTGACC AAATATATGG CGGGGAAAAT

3651 CAGTTGAAGA AAGAAATAGA AATAGAAATA GCAAACAAAA GATATGACAG
     GTCAACTTCT TTCTTTATCT TTATCTTTAT CGTTTGTTTT CTATACTGTC

3701 TCAACACTAA GACCTATAGT GAGAGAGCAG AAACTCATGC CTCACCAGTA
     AGTTGTGATT CTGGATATCA CTCTCTCGTC TTTGAGTACG GAGTGGTCAT

3751 GCACAGCGAT TATTTCGATT AATGGAACTG AAGAAAACCA ATTTATGTGC
     CGTGTCGCTA ATAAAGCTAA TTACCTTGAC TTCTTTTGGT TAAATACACG
```

```
                                 EcoRI
                                 ~~~~~~~
3801  ATCAATTGAC GTTGATACCA CTAAGGAATT CCTTGAATTA ATTGATAAAT
      TAGTTAACTG CAACTATGGT GATTCCTTAA GGAACTTAAT TAACTATTTA
.....................................................................
3851  TAGGTCCTTA TGTATGCTTA ATCAAGACTC ATATTGATAT AATCAATGAT
      ATCCAGGAAT ACATACGAAT TAGTTCTGAG TATAACTATA TTAGTTACTA
.....................................................................
3901  TTTTCCTATG AATCCACTAT TGAACCATTA TTAGAACTTT CACGTAAACA
      AAAAGGATAC TTAGGTGATA ACTTGGTAAT AATCTTGAAA GTGCATTTGT
.....................................................................
3951  TCAATTTATG ATTTTTGAAG ATAGAAAATT TGCTGATATT GGTAATACCG
      AGTTAAATAC TAAAAACTTC TATCTTTTAA ACGACTATAA CCATTATGGC
.....................................................................
4001  TAAAGAAACA ATATATTGGT GGAGTTTATA AAATTAGTAG TTGGGCAGAT
      ATTTCTTTGT TATATAACCA CCTCAAATAT TTTAATCATC AACCCGTCTA
.....................................................................
4051  ATTACCAATG CTCATGGTGT CACTGGGAAT GGAGTGGTTG AAGGATTAAA
      TAATGGTTAC GAGTACCACA GTGACCCTTA CCTCACCAAC TTCCTAATTT
.....................................................................
4101  ACAGGGAGCT AAAGAAACCA CCACCAACCA AGAGCCAAGA GGGTTATTGA
      TGTCCCTCGA TTTCTTTGGT GGTGGTTGGT TCTCGGTTCT CCCAATAACT
.....................................................................
4151  TGTTAGCTGA ATTATCATCA GTGGGATCAT TAGCATATGG AGAATATTCT
      ACAATCGACT TAATAGTAGT CACCCTAGTA ATCGTATACC TCTTATAAGA
.....................................................................
4201  CAAAAAACTG TTGAAATTGC TAAATCCGAT AAGGAATTTG TTATTGGATT
      GTTTTTTGAC AACTTTAACG ATTTAGGCTA TTCCTTAAAC AATAACCTAA
.....................................................................
4251  TATTGCCCAA CGTGATATGG GTGGCCAAGA AGAAGGATTT GATTGGCTTA
      ATAACGGGTT GCACTATACC CACCGGTTCT TCTTCCTAAA CTAACCGAAT
.....................................................................
4301  TTATGACACC TGGAGTTGGA TTAGATGATA AAGGTGATGG ATTAGGACAA
      AATACTGTGG ACCTCAACCT AATCTACTAT TTCCACTACC TAATCCTGTT
.....................................................................
4351  CAATATAGAA CTGTTGATGA AGTTGTTAGC ACTGGAACTG ATATTATCAT
      GTTATATCTT GACAACTACT TCAACAATCG TGACCTTGAC TATAATAGTA
.....................................................................
4401  TGTTGGTAGA GGATTGTTTG GTAAAGGAAG AGATCCAGAT ATTGAAGGTA
      ACAACCATCT CCTAACAAAC CATTTCCTTC TCTAGGTCTA TAACTTCCAT
.....................................................................
4451  AAAGGTATAG AAATGCTGGT TGGAATGCTT ATTTGAAAAA GACTGGCCAA
      TTTCCATATC TTTACGACCA ACCTTACGAA TAAACTTTTT CTGACCGGTT
.....................................................................
4501  TTATAAATGT GAAGGGGGAG ATTTTCACTT TATTAGATTT GTATATATGT
      AATATTTACA CTTCCCCCTC TAAAAGTGAA ATAATCTAAA CATATATACA
.....................................................................
4551  AGAATAAATA AATAAATAAG TTAAATAAAT AATTAAATAA GGGTGGTAAT
      TCTTATTTAT TTATTTATTC AATTTATTTA TTAATTTATT CCCACCATTA
.....................................................................
4601  TATTACTATT TACAATCAAA GGTGGTCCTT CTAGCTGTAA TCCGGGCAGC
      ATAATGATAA ATGTTAGTTT CCACCAGGAA GATCGACATT AGGCCCGTCG
.....................................................................
4651  GCAACGGAAC ATTCATCAGT GTAAAAATGG AATCAATAAA GCCCTGCGCA
      CGTTGCCTTG TAAGTAGTCA CATTTTTACC TTAGTTATTT CGGGACGCGT
.....................................................................
4701  GCGCGCAGGG TCAGCCTGAA TACGCGTTTA ATGACCAGCA CAGTCGTGAT
      CGCGCGTCCC AGTCGGACTT ATGCGCAAAT TACTGGTCGT GTCAGCACTA
.....................................................................
```

```
4751  GGCAAGGTCA GAATAGCCCA AGTCGGCCGA GGGGCCTGTA CAGTGAGGGA
      CCGTTCCAGT CTTATCGGGT TCAGCCGGCT CCCCGGACAT GTCACTCCCT

4801  AGATCTGATA TTGACGAAGA GGAACCAATG TAACGTTACA CTGAAGAAAA
      TCTAGACTAT AACTGCTTCT CCTTGGTTAC ATTGCAATGT GACTTCTTTT

4851  CACACAATAA ACGGGAAGAA ACGGTGTAAA AGTGTGAAAA TAATTTTTGA
      GTGTGTTATT TGCCCTTCTT TGCCACATTT TCACACTTTT ATTAAAAACT

4901  ATATCATTTC CCTTGGTTTA ATTCCAAACG AAACGTGTTT TTTTTAGAGA
      TATAGTAAAG GGAACCAAAT TAAGGTTTGC TTTGCACAAA AAAAATCTCT

            EcoRI                                    ApaLI
          ~~~~~~                                      ~~~~
4951  ATGGGAATTC TTATTGGATG TCTAGATTGT TTGTTTACTC CAGACTGTGC
      TACCCTTAAG AATAACCTAC AGATCTAACA AACAAATGAG GTCTGACACG

      ApaLI
      ~~
5001  ACAAAAACGT TTGGATGGAT GATCAGAAGA TATTTTTAGG CTTAGCTCTA
      TGTTTTTGCA AACCTACCTA CTAGTCTTCT ATAAAAATCC GAATCGAGAT

5051  AATATAAGAA ATGATGCTTG AAAAACCAGA CAGAAATTGA GTTTCAAAAA
      TTATATTCTT TACTACGAAC TTTTTGGTCT GTCTTTAACT CAAAGTTTTT

5101  TTGGTAATGT GAGGTATTAG TCAACTAACC AAATAACAAT GCAAACCGGT
      AACCATTACA CTCCATAATC AGTTGATTGG TTTATTGTTA CGTTTGGCCA

5151  TGATACATTT CATTTTGAAA ATAATGAAAC TGGAATTGGA TGACCAGCAC
      ACTATGTAAA GTAAAACTTT TATTACTTTG ACCTTAACCT ACTGGTCGTG

5201  ACAAACACAT AAAGTAATTA TGGGAATTAG AAGCGAACAT AGAGGAGTAC
      TGTTTGTGTA TTTCATTAAT ACCCTTAATC TTCGCTTGTA TCTCCTCATG

5251  TTGGCCACGA ACAGAATACA AGTGGGAACA CTATTTTCTC CATTGTTTTA
      AACCGGTGCT TGTCTTATGT TCACCCTTGT GATAAAAGAG GTAACAAAAT

5301  GTTCTGTTTT TTTGTCAGCC TAGTTTTGTG CTATGTGTAA AAAATATTGC
      CAAGACAAAA AAACAGTCGG ATCAAAACAC GATACACATT TTTTATAACG

                HindIII
                ~~~~~~~
5351  CAAGAAAAAA AGCTTGTTTT GTGGCCAGTG TCCGAAAAAA ATTTTGGGGA
      GTTCTTTTTT TCGAACAAAA CACCGGTCAC AGGCTTTTTT TAAAACCCCT

5401  ATCTTCGGAT TAATTTATGT TTTCATTCCA TCGGGGAAAG TGGGGGGGAA
      TAGAAGCCTA ATTAAATACA AAAGTAAGGT AGCCCCTTTC ACCCCCCCTT

5451  AAAATTTTAA GCAGTTCACA AAACCTTCCA AAAAATATAT GGACAAAGAT
      TTTTAAAATT CGTCAAGTGT TTTGGAAGGT TTTTTATATA CCTGTTTCTA

5501  GATTGTATTT TCCCGACACC AAAATCATAA TTAATTATGA GAAAGTTAAA
      CTAACATAAA AGGGCTGTGG TTTTAGTATT AATTAATACT CTTTCAATTT

5551  TGTAACGTTA CAATTTATGT TTATTTGAAG GTGAAAAGCG ATTTATGATT
      ACATTGCAAT GTTAAATACA AATAAACTTC CACTTTTCGC TAAATACTAA

5601  TTTCCGAAAT GAAAATTTTT TTTAGGTTTA TTTTTTTTGT CGGGCAAAGA
      AAAGGCTTTA CTTTTAAAAA AAATCCAAAT AAAAAAAACA GCCCGTTTCT
```

```
                                                     EcoRI
                                                         ~
5651  AAAACTGAAC AAGGATTATT AAAATTTTTG GTGTTTGTTT GTGTCTGGAG
      TTTTGACTTG TTCCTAATAA TTTTAAAAAC CACAAACAAA CACAGACCTC

      EcoRI
      ~~~~~
5701  AATTCATTCC TCTCTCATCT TCACACAATG TTTAGACATC TGACACGATT
      TTAAGTAAGG AGAGAGTAGA AGTGTGTTAC AAATCTGTAG ACTGTGCTAA

5751  CATGATAGTT CGGTTTCCGG GGTTGGTGTT TAGTTTTCGT TTTTCTTTTT
      GTACTATCAA GCCAAAGGCC CCAACCACAA ATCAAAAGCA AAAAGAAAAA

5801  TTTTGGAAAG AATGTTTTAG CTCATTGGTT TTCTTTCTTC ATTCAATAGT
      AAAACCTTTC TTACAAAATC GAGTAACCAA AAGAAAGAAG TAAGTTATCA

5851  TTTGAAAGAA TTTGCCCACT TGTTATTACA ATCATATAAA ATTAAACTTT
      AAACTTTCTT AAACGGGTGA ACAATAATGT TAGTATATTT TAATTTGAAA

5901  GATATAAAAT AGAGTTTGAA AGTTTCCCAG ATCCTTTTTG ATTTCTTTGT
      CTATATTTTA TCTCAAACTT TCAAAGGGTC TAGGAAAAAC TAAAGAAACA

5951  AAATTTTTTT TTCTCCCACA TATACACACA TACAAACCGA TTTTTATAAG
      TTTAAAAAAA AAGAGGGTGT ATATGTGTGT ATGTTTGGCT AAAAATATTC

                  PstI         AvaI   BamHI
                 ~~~~~~       ~~~~~~~~~~~~~
6001  AAAGAGTTAT ACCCTGCAGC TCGACCTCGA GGGATCCGGG CCCTCTAGAT
      TTTCTCAATA TGGGACGTCG AGCTGGAGCT CCCTAGGCCC GGGAGATCTA

                  AvaI
                 ~~~~~~
6051  GCGGCCGCTA GGCCTCGAGG GACTTTTGCA CCAAAAATAA TTTATTTTCC
      CGCCGGCGAT CCGGAGCTCC CTGAAAACGT GGTTTTTATT AAATAAAAGG

6101  AAAATAAAAT TTAAATAAAT AAAAATAACT CATAATTTAA TAAAAATTTC
      TTTTATTTTA AATTTATTTA TTTTTATTGA GTATTAAATT ATTTTTAAAG

6151  AAAATCTTCT AGTGTCCTTT CATATGCAGT ACATTAGCCA TCAGTCACTT
      TTTTAGAAGA TCACAGGAAA GTATACGTCA TGTAATCGGT AGTCAGTGAA

6201  AAACAGCATC TGCTGGTTGA AGAATGCTTG AAGCAATTGT CCAGTCCCAG
      TTTGTCGTAG ACGACCAACT TCTTACGAAC TTCGTTAACA GGTCAGGGTC

6251  AGGCACAGGC TAGGAGATCT TCAGTTTCGG AGGTAACCTG TAAGTCTGTT
      TCCGTGTCCG ATCCTCTAGA AGTCAAAGCC TCCATTGGAC ATTCAGACAA

6301  AATGAAGTAA AAGTTCCTTA GGATTTCCAC TCTGACTATG GTCCAGGCAC
      TTACTTCATT TTCAAGGAAT CCTAAAGGTG AGACTGATAC CAGGTCCGTG

6351  AGTGACTGTA CTCCTTGGCC TTCAGGTAAT GCAGAATCCT CCCATAATAT
      TCACTGACAT GAGGAACCGG AAGTCCATTA CGTCTTAGGA GGGTATTATA

6401  CTTTTCAGGT GCAGACTGCT CATGAGTTTT CCCCTGGTGA AATCTTCTTT
      GAAAAGTCCA CGTCTGACGA GTACTCAAAA GGGGACCACT TTAGAAGAAA

6451  CTCCAGTTTT TCTTCCAGGA CTGTCTTCAG ATGGTTTATC TGATGATAGA
      GAGGTCAAAA AGAAGGTCCT GACAGAAGTC TACCAAATAG ACTACTATCT

6501  CATTAGCCAG GAGGTTCTCA ACAATAGTCT CATTCCAGCC AGTGCTAGAT
      GTAATCGGTC CTCCAAGAGT TGTTATCAGA GTAAGGTCGG TCACGATCTA
```

```
6551  GAATCTTGTC TGAAAATAGC AAAGATGTTC TGGAGCATCT CATAGATGGT
      CTTAGAACAG ACTTTTATCG TTTCTACAAG ACCTCGTAGA GTATCTACCA
. . . . . . . . . . . . . . . . . . . . . . . . . . . . . . . . . . . . . . . . . . . . . . . . . . . . . . . .
                                   PstI
                                  ~~~~~~~
6601  CAATGCGGCG TCCTCCTTCT GGAACTGCTG CAGCTGCTTA ATCTCCTCAG
      GTTACGCCGC AGGAGGAAGA CCTTGACGAC GTCGACGAAT TAGAGGAGTC
. . . . . . . . . . . . . . . . . . . . . . . . . . . . . . . . . . . . . . . . . . . . . . . . . . . . . . . .
6651  GGATGTCAAA GTTCATCCTG TCCTTGAGGC AGTATTCAAG CCTCCCATTC
      CCTACAGTTT CAAGTAGGAC AGGAACTCCG TCATAAGTTC GGAGGGTAAG
. . . . . . . . . . . . . . . . . . . . . . . . . . . . . . . . . . . . . . . . . . . . . . . . . . . . . . . .
6701  AATTGCCACA GGAGCTTCTG ACACTGAAAA TTGCTGCTTC TTTGTAGGAA
      TTAACGGTGT CCTCGAAGAC TGTGACTTTT AACGACGAAG AAACATCCTT
. . . . . . . . . . . . . . . . . . . . . . . . . . . . . . . . . . . . . . . . . . . . . . . . . . . . . . . .
6751  TCCAAGCAAG TTGTAGCTCA TGGAAAGAGC TGTAGTGGAG AAGCACAACA
      AGGTTCGTTC AACATCGAGT ACCTTTCTCG ACATCACCTC TTCGTGTTGT
. . . . . . . . . . . . . . . . . . . . . . . . . . . . . . . . . . . . . . . . . . . . . . . . . . . . . . . .
                                                AvaI
                                               ~~~~~~~
6801  GGAGAGCAAT TTGGAGGAGA CACTTGTTGG TCATGTTCCT CGAGGCCTTT
      CCTCTCGTTA AACCTCCTCT GTGAACAACC AGTACAAGGA GCTCCGGAAA
. . . . . . . . . . . . . . . . . . . . . . . . . . . . . . . . . . . . . . . . . . . . . . . . . . . . . . . .
                                                        BamHI
                                                          ~~~
6851  TTGGCCAGCT GGCGCCTGCT GCGCGACGGC GAGCTGCTCA CCACCCAGGA
      AACCGGTCGA CCGCGGACGA CGCGCTGCCG CTCGACGAGT GGTGGGTCCT
. . . . . . . . . . . . . . . . . . . . . . . . . . . . . . . . . . . . . . . . . . . . . . . . . . . . . . . .
      BamHI
      ~~~
6901  TCCGTCCCCC TTTTCCTTTG TCGATATCAT GTAATTAGTT ATGTCACGCT
      AGGCAGGGGG AAAAGGAAAC AGCTATAGTA CATTAATCAA TACAGTGCGA
. . . . . . . . . . . . . . . . . . . . . . . . . . . . . . . . . . . . . . . . . . . . . . . . . . . . . . . .
6951  TACATTCACG CCCTCCCCCC ACATCCGCTC TAACCGAAAA GGAAGGAGTT
      ATGTAAGTGC GGGAGGGGGG TGTAGGCGAG ATTGGCTTTT CCTTCCTCAA
. . . . . . . . . . . . . . . . . . . . . . . . . . . . . . . . . . . . . . . . . . . . . . . . . . . . . . . .
7001  AGACAACCTG AAGTCTAGGT CCCTATTTAT TTTTTTATAG TTATGTTAGT
      TCTGTTGGAC TTCAGATCCA GGGATAAATA AAAAAATATC AATACAATCA
. . . . . . . . . . . . . . . . . . . . . . . . . . . . . . . . . . . . . . . . . . . . . . . . . . . . . . . .
7051  ATTAAGAACG TTATTTATAT TTCAAATTTT TCTTTTTTTT CTGTACAGAC
      TAATTCTTGC AATAAATATA AAGTTTAAAA AGAAAAAAAA GACATGTCTG
. . . . . . . . . . . . . . . . . . . . . . . . . . . . . . . . . . . . . . . . . . . . . . . . . . . . . . . .
7101  GCGTGTACGC ATGTAACATT ATACTGAAAA CCTTGCTTGA GAAGGTTTTG
      CGCACATGCG TACATTGTAA TATGACTTTT GGAACGAACT CTTCCAAAAC
. . . . . . . . . . . . . . . . . . . . . . . . . . . . . . . . . . . . . . . . . . . . . . . . . . . . . . . .
                         HindIII
                               ~
7151  GGACGCTCGA AGGCTTTAAT TTGCA
      CCTGCGAGCT TCCGAAATTA AACGT
. . . . . . . . . . . . . . . . . . . . . . . . . . . . . . . . . . . . . . . . . . . . . . . . . . . . . . . .
```

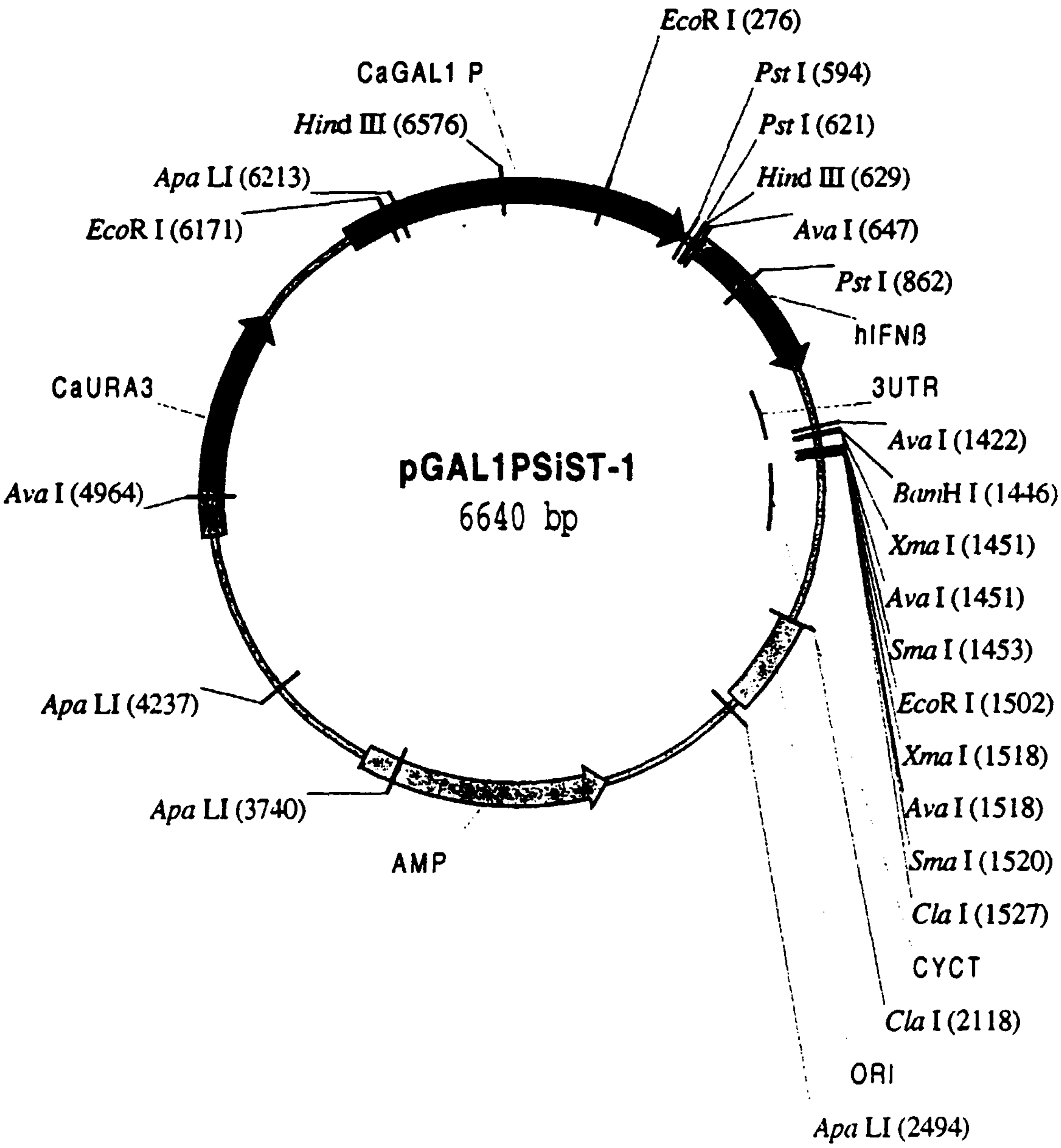
EcoR I (276)
CaGAL1 P
Pst I (594)
Hind III (6576)
Pst I (621)
Hind III (629)
Apa LI (6213)
EcoR I (6171)
Ava I (647)
Pst I (862)
hIFNß
CaURA3
3UTR
Ava I (1422)
pGAL1PSiST-1
6640 bp
Ava I (4964)
BamH I (1446)
Xma I (1451)
Ava I (1451)
Sma I (1453)
Apa LI (4237)
EcoR I (1502)
Xma I (1518)
Apa LI (3740)
Ava I (1518)
Sma I (1520)
AMP
Cla I (1527)
CYCT
Cla I (2118)
ORI
Apa LI (2494)

Fig 3

```
  1  TTCCATCGGG GAAAGTGGGG GGGAAAAAAT TTTAAGCAGT TCACAAAACC
     AAGGTAGCCC CTTTCACCCC CCCTTTTTTA AAATTCGTCA AGTGTTTTGG

 51  TTCCAAAAAA TATATGGACA AAGATGATTG TATTTTCCCG ACACCAAAAT
     AAGGTTTTTT ATATACCTGT TTCTACTAAC ATAAAAGGGC TGTGGTTTTA

101  CATAATTAAT TATGAGAAAG TTAAATGTAA CGTTACAATT TATGTTTATT
     GTATTAATTA ATACTCTTTC AATTTACATT GCAATGTTAA ATACAAATAA

151  TGAAGGTGAA AAGCGATTTA TGATTTTTCC GAAATGAAAA TTTTTTTTAG
     ACTTCCACTT TTCGCTAAAT ACTAAAAAGG CTTTACTTTT AAAAAAAATC

201  GTTTATTTTT TTTGTCGGGC AAAGAAAAAC TGAACAAGGA TTATTAAAAT
     CAAATAAAAA AAACAGCCCG TTTCTTTTTG ACTTGTTCCT AATAATTTTA

                               EcoRI
                              ~~~~~~
251  TTTTGGTGTT TGTTTGTGTC TGGAGAATTC ATTCCTCTCT CATCTTCACA
     AAAACCACAA ACAAACACAG ACCTCTTAAG TAAGGAGAGA GTAGAAGTGT

301  CAATGTTTAG ACATCTGACA CGATTCATGA TAGTTCGGTT TCCGGGGTTG
     GTTACAAATC TGTAGACTGT GCTAAGTACT ATCAAGCCAA AGGCCCCAAC

351  GTGTTTAGTT TTCGTTTTTC TTTTTTTTTG GAAAGAATGT TTTAGCTCAT
     CACAAATCAA AAGCAAAAAG AAAAAAAAAC CTTTCTTACA AAATCGAGTA

401  TGGTTTTCTT TCTTCATTCA ATAGTTTTGA AAGAATTTGC CCACTTGTTA
     ACCAAAAGAA AGAAGTAAGT TATCAAAACT TTCTTAAACG GGTGAACAAT

451  TTACAATCAT ATAAAATTAA ACTTTGATAT AAAATAGAGT TTGAAAGTTT
     AATGTTAGTA TATTTTAATT TGAAACTATA TTTTATCTCA AACTTTCAAA

501  CCCAGATCCT TTTTGATTTC TTTGTAAATT TTTTTTTCTC CCACATATAC
     GGGTCTAGGA AAAACTAAAG AAACATTTAA AAAAAAAGAG GGTGTATATG

                                            PstI
                                          ~~~~~~~
551  ACACATACAA ACCGATTTTT ATAAGAAAGA GTTATACCCT GCAGCTCGAC
     TGTGTATGTT TGGCTAAAAA TATTCTTTCT CAATATGGGA CGTCGAGCTG

                    PstI        HindIII              AvaI
                  ~~~~~~~       ~~~~~~~             ~~~~~
601  CTCGACTGTT TAAACCTGCA GGCATGCAAG CTTGGCCAAA AAGGCCTCGA
     GAGCTGACAA ATTTGGACGT CCGTACGTTC GAACCGGTTT TTCCGGAGCT

     AvaI
     ~
651  GGAACATGAC CAACAAGTGT CTCCTCCAAA TTGCTCTCCT GTTGTGCTTC
     CCTTGTACTG GTTGTTCACA GAGGAGGTTT AACGAGAGGA CAACACGAAG

701  TCCACTACAG CTCTTTCCAT GAGCTACAAC TTGCTTGGAT TCCTACAAAG
     AGGTGATGTC GAGAAAGGTA CTCGATGTTG AACGAACCTA AGGATGTTTC

751  AAGCAGCAAT TTTCAGTGTC AGAAGCTCCT GTGGCAATTG AATGGGAGGC
     TTCGTCGTTA AAAGTCACAG TCTTCGAGGA CACCGTTAAC TTACCCTCCG

801  TTGAATACTG CCTCAAGGAC AGGATGAACT TTGACATCCC TGAGGAGATT
     AACTTATGAC GGAGTTCCTG TCCTACTTGA AACTGTAGGG ACTCCTCTAA
```

Fig 4

```
        PstI
        ~~~~~~~
 851  AAGCAGCTGC AGCAGTTCCA GAAGGAGGAC GCCGCATTGA CCATCTATGA
      TTCGTCGACG TCGTCAAGGT CTTCCTCCTG CGGCGTAACT GGTAGATACT
.......................................................................
 901  GATGCTCCAG AACATCTTTG CTATTTTCAG ACAAGATTCA TCTAGCACTG
      CTACGAGGTC TTGTAGAAAC GATAAAAGTC TGTTCTAAGT AGATCGTGAC
.......................................................................
 951  GCTGGAATGA GACTATTGTT GAGAACCTCC TGGCTAATGT CTATCATCAG
      CGACCTTACT CTGATAACAA CTCTTGGAGG ACCGATTACA GATAGTAGTC
.......................................................................
1001  ATAAACCATC TGAAGACAGT CCTGGAAGAA AAACTGGAGA AAGAAGATTT
      TATTTGGTAG ACTTCTGTCA GGACCTTCTT TTTGACCTCT TTCTTCTAAA
.......................................................................
1051  CACCAGGGGA AAACTCATGA GCAGTCTGCA CCTGAAAAGA TATTATGGGA
      GTGGTCCCCT TTTGAGTACT CGTCAGACGT GGACTTTTCT ATAATACCCT
.......................................................................
1101  GGATTCTGCA TTACCTGAAG GCCAAGGAGT ACAGTCACTG TGCCTGGACC
      CCTAAGACGT AATGGACTTC CGGTTCCTCA TGTCAGTGAC ACGGACCTGG
.......................................................................
1151  ATAGTCAGAG TGGAAATCCT AAGGAACTTT TACTTCATTA ACAGACTTAC
      TATCAGTCTC ACCTTTAGGA TTCCTTGAAA ATGAAGTAAT TGTCTGAATG
.......................................................................
1201  AGGTTACCTC CGAAACTGAA GATCTCCTAG CCTGTGCCTC TGGGACTGGA
      TCCAATGGAG GCTTTGACTT CTAGAGGATC GGACACGGAG ACCCTGACCT
.......................................................................
1251  CAATTGCTTC AAGCATTCTT CAACCAGCAG ATGCTGTTTA AGTGACTGAT
      GTTAACGAAG TTCGTAAGAA GTTGGTCGTC TACGACAAAT TCACTGACTA
.......................................................................
1301  GGCTAATGTA CTGCATATGA AAGGACACTA GAAGATTTTG AAATTTTTAT
      CCGATTACAT GACGTATACT TTCCTGTGAT CTTCTAAAAC TTTAAAAATA
.......................................................................
1351  TAAATTATGA GTTATTTTTA TTTATTTAAA TTTTATTTTG GAAAATAAAT
      ATTTAATACT CAATAAAAAT AAATAAATTT AAAATAAAAC CTTTTATTTA
.......................................................................
                                                    XmaI
                                                       ~
                                                    SmaI
                                                       ~
                                                   BamHI
                                                  ~~~~~~
                            AvaI                    AvaI
                           ~~~~~~                      ~
1401  TATTTTTGGT GCAAAAGTCC CTCGAGGCCT AGCGGCCGCC TAGAGGATCC
      ATAAAAACCA CGTTTTCAGG GAGCTCCGGA TCGCCGGCGG ATCTCCTAGG
.......................................................................
      XmaI
      ~~~~~
      SmaI
      ~~~~~
      AvaI
      ~~~~~
1451  CCGGGCGCTA GGCGGCCGCT AGGCCTTTTT GGCCAAGCTC GAATTTCGAG
      GGCCCGCGAT CCGCCGGCGA TCCGGAAAAA CCGGTTCGAG CTTAAAGCTC
.......................................................................
                        XmaI
                       ~~~~~~~
                        SmaI
                       ~~~~~~~
       EcoRI            AvaI     ClaI
      ~~~~~~           ~~~~~~~  ~~~~~~
1501  GAATTCGAGC TCGGTACCCG GGGATCGAT CCGTCCCCCT TTTCCTTTGT
      CTTAAGCTCG AGCCATGGGC CCCCTAGCTA GGCAGGGGGA AAAGGAAACA
.......................................................................
```

```
1551  CGATATCATG TAATTAGTTA TGTCACGCTT ACATTCACGC CCTCCCCCCA
      GCTATAGTAC ATTAATCAAT ACAGTGCGAA TGTAAGTGCG GGAGGGGGGT

1601  CATCCGCTCT AACCGAAAAG GAAGGAGTTA GACAACCTGA AGTCTAGGTC
      GTAGGCGAGA TTGGCTTTTC CTTCCTCAAT CTGTTGGACT TCAGATCCAG

1651  CCTATTTATT TTTTTATAGT TATGTTAGTA TTAAGAACGT TATTTATATT
      GGATAAATAA AAAAATATCA ATACAATCAT AATTCTTGCA ATAAATATAA

1701  TCAAATTTTT CTTTTTTTTC TGTACAGACG CGTGTACGCA TGTAACATTA
      AGTTTAAAAA GAAAAAAAAG ACATGTCTGC GCACATGCGT ACATTGTAAT

1751  TACTGAAAAC CTTGCTTGAG AAGGTTTTGG GACGCTCGAA GGCTTTAATT
      ATGACTTTTG GAACGAACTC TTCCAAAACC CTGCGAGCTT CCGAAATTAA

1801  TGCAAGCTAG CTTGGCGTAA TCATGGTCAT AGCTGTTTCC TGTGTGAAAT
      ACGTTCGATC GAACCGCATT AGTACCAGTA TCGACAAAGG ACACACTTTA

1851  TGTTATCCGC TCACAATTCC ACACAACATA CGAGCCGGAA GCATAAAGTG
      ACAATAGGCG AGTGTTAAGG TGTGTTGTAT GCTCGGCCTT CGTATTTCAC

1901  TAAAGCCTGG GGTGCCTAAT GAGTGAGCTA ACTCACATTA ATTGCGTTGC
      ATTTCGGACC CCACGGATTA CTCACTCGAT TGAGTGTAAT TAACGCAACG

1951  GCTCACTGCC CGCTTTCCAG TCGGGAAACC TGTCGTGCCA GAGATCTCTG
      CGAGTGACGG GCGAAAGGTC AGCCCTTTGG ACAGCACGGT CTCTAGAGAC

2001  CATTAATGAA TCGGCCAACG CGCGGGGAGA GGCGGTTTGC GTATTGGGCG
      GTAATTACTT AGCCGGTTGC GCGCCCCTCT CCGCCAAACG CATAACCCGC

2051  CTCTTCCGCT TCCTCGCTCA CTGACTCGCT GCGCTCGGTC GTTCGGCTGC
      GAGAAGGCGA AGGAGCGAGT GACTGAGCGA CGCGAGCCAG CAAGCCGACG

                          ClaI
                         -------
2101  GGCGAGCGGT ATCAGATCGA TCTCACTCAA AGGCGGTAAT ACGGTTATCC
      CCGCTCGCCA TAGTCTAGCT AGAGTGAGTT TCCGCCATTA TGCCAATAGG

2151  ACAGAATCAG GGGATAACGC AGGAAAGAAC ATGTGAGCAA AAGGCCAGCA
      TGTCTTAGTC CCCTATTGCG TCCTTTCTTG TACACTCGTT TTCCGGTCGT

2201  AAAGGCCAGG AACCGTAAAA AGGCCGCGTT GCTGGCGTTT TTCCATAGGC
      TTTCCGGTCC TTGGCATTTT TCCGGCGCAA CGACCGCAAA AAGGTATCCG

2251  TCCGCCCCCC TGACGAGCAT CACAAAAATC GACGCTCAAG TCAGAGGTGG
      AGGCGGGGGG ACTGCTCGTA GTGTTTTTAG CTGCGAGTTC AGTCTCCACC

2301  CGAAACCCGA CAGGACTATA AAGATACCAG GCGTTTCCCC CTGGAAGCTC
      GCTTTGGGCT GTCCTGATAT TTCTATGGTC CGCAAAGGGG GACCTTCGAG

2351  CCTCGTGCGC TCTCCTGTTC CGACCCTGCC GCTTACCGGA TACCTGTCCG
      GGAGCACGCG AGAGGACAAG GCTGGGACGG CGAATGGCCT ATGGACAGGC

2401  CCTTTCTCCC TTCGGGAAGC GTGGCGCTTT CTCATAGCTC ACGCTGTAGG
      GGAAAGAGGG AAGCCCTTCG CACCGCGAAA GAGTATCGAG TGCGACATCC

                                                ApaLI
                                               ------
2451  TATCTCAGTT CGGTGTAGGT CGTTCGCTCC AAGCTGGGCT GTGTGCACGA
      ATAGAGTCAA GCCACATCCA GCAAGCGAGG TTCGACCCGA CACACGTGCT
```

```
2501  ACCCCCCGTT CAGCCCGACC GCTGCGCCTT ATCCGGTAAC TATCGTCTTG
      TGGGGGGCAA GTCGGGCTGG CGACGCGGAA TAGGCCATTG ATAGCAGAAC

2551  AGTCCAACCC GGTAAGACAC GACTTATCGC CACTGGCAGC AGCCACTGGT
      TCAGGTTGGG CCATTCTGTG CTGAATAGCG GTGACCGTCG TCGGTGACCA

2601  AACAGGATTA GCAGAGCGAG GTATGTAGGC GGTGCTACAG AGTTCTTGAA
      TTGTCCTAAT CGTCTCGCTC CATACATCCG CCACGATGTC TCAAGAACTT

2651  GTGGTGGCCT AACTACGGCT ACACTAGAAG GACAGTATTT GGTATCTGCG
      CACCACCGGA TTGATGCCGA TGTGATCTTC CTGTCATAAA CCATAGACGC

2701  CTCTGCTGAA GCCAGTTACC TTCGGAAAAA GAGTTGGTAG CTCTTGATCC
      GAGACGACTT CGGTCAATGG AAGCCTTTTT CTCAACCATC GAGAACTAGG

2751  GGCAAACAAA CCACCGCTGG TAGCGGTGGT TTTTTTGTTT GCAAGCAGCA
      CCGTTTGTTT GGTGGCGACC ATCGCCACCA AAAAAACAAA CGTTCGTCGT

2801  GATTACGCGC AGAAAAAAAG GATCTCAAGA AGATCCTTTG ATCTTTTCTA
      CTAATGCGCG TCTTTTTTTC CTAGAGTTCT TCTAGGAAAC TAGAAAAGAT

2851  CGGGGTCTGA CGCTCAGTGG AACGAAAACT CACGTTAAGG GATTTTGGTC
      GCCCCAGACT GCGAGTCACC TTGCTTTTGA GTGCAATTCC CTAAAACCAG

2901  ATGAGATTAT CAAAAAGGAT CTTCACCTAG ATCCTTTTAA ATTAAAAATG
      TACTCTAATA GTTTTTCCTA GAAGTGGATC TAGGAAAATT TAATTTTTAC

2951  AAGTTTTAAA TCAATCTAAA GTATATATGA GTAAACTTGG TCTGACAGTT
      TTCAAAATTT AGTTAGATTT CATATATACT CATTTGAACC AGACTGTCAA

3001  ACCAATGCTT AATCAGTGAG GCACCTATCT CAGCGATCTG TCTATTTCGT
      TGGTTACGAA TTAGTCACTC CGTGGATAGA GTCGCTAGAC AGATAAAGCA

3051  TCATCCATAG TTGCCTGACT CCCCGTCGTG TAGATAACTA CGATACGGGA
      AGTAGGTATC AACGGACTGA GGGGCAGCAC ATCTATTGAT GCTATGCCCT

3101  GGGCTTACCA TCTGGCCCCA GTGCTGCAAT GATACCGCGA GACCCACGCT
      CCCGAATGGT AGACCGGGGT CACGACGTTA CTATGGCGCT CTGGGTGCGA

3151  CACCGGCTCC AGATTTATCA GCAATAAACC AGCCAGCCGG AAGGGCCGAG
      GTGGCCGAGG TCTAAATAGT CGTTATTTGG TCGGTCGGCC TTCCCGGCTC

3201  CGCAGAAGTG GTCCTGCAAC TTTATCCGCC TCCATCCAGT CTATTAATTG
      GCGTCTTCAC CAGGACGTTG AAATAGGCGG AGGTAGGTCA GATAATTAAC

3251  TTGCCGGGAA GCTAGAGTAA GTAGTTCGCC AGTTAATAGT TTGCGCAACG
      AACGGCCCTT CGATCTCATT CATCAAGCGG TCAATTATCA AACGCGTTGC

3301  TTGTTGCCAT TGCTACAGGC ATCGTGGTGT CACGCTCGTC GTTTGGTATG
      AACAACGGTA ACGATGTCCG TAGCACCACA GTGCGAGCAG CAAACCATAC

3351  GCTTCATTCA GCTCCGGTTC CCAACGATCA AGGCGAGTTA CATGATCCCC
      CGAAGTAAGT CGAGGCCAAG GGTTGCTAGT TCCGCTCAAT GTACTAGGGG

3401  CATGTTGTGC AAAAAAGCGG TTAGCTCCTT CGGTCCTCCG ATCGTTGTCA
      GTACAACACG TTTTTTCGCC AATCGAGGAA GCCAGGAGGC TAGCAACAGT

3451  GAAGTAAGTT GGCCGCAGTG TTATCACTCA TGGTTATGGC AGCACTGCAT
      CTTCATTCAA CCGGCGTCAC AATAGTGAGT ACCAATACCG TCGTGACGTA
```

```
3501  AATTCTCTTA CTGTCATGCC ATCCGTAAGA TGCTTTTCTG TGACTGGTGA
      TTAAGAGAAT GACAGTACGG TAGGCATTCT ACGAAAAGAC ACTGACCACT

3551  GTACTCAACC AAGTCATTCT GAGAATAGTG TATGCGGCGA CCGAGTTGCT
      CATGAGTTGG TTCAGTAAGA CTCTTATCAC ATACGCCGCT GGCTCAACGA

3601  CTTGCCCGGC GTCAATACGG GATAATACCG CGCCACATAG CAGAACTTTA
      GAACGGGCCG CAGTTATGCC CTATTATGGC GCGGTGTATC GTCTTGAAAT

3651  AAAGTGCTCA TCATTGGAAA ACGTTCTTCG GGGCGAAAAC TCTCAAGGAT
      TTTCACGAGT AGTAACCTTT TGCAAGAAGC CCCGCTTTTG AGAGTTCCTA

                                           ApaLI
                                          ~~~~~~~
3701  CTTACCGCTG TTGAGATCCA GTTCGATGTA ACCCACTCGT GCACCCAACT
      GAATGGCGAC AACTCTAGGT CAAGCTACAT TGGGTGAGCA CGTGGGTTGA

3751  GATCTTCAGC ATCTTTTACT TTCACCAGCG TTTCTGGGTG AGCAAAAACA
      CTAGAAGTCG TAGAAAATGA AAGTGGTCGC AAAGACCCAC TCGTTTTTGT

3801  GGAAGGCAAA ATGCCGCAAA AAAGGGAATA AGGGCGACAC GGAAATGTTG
      CCTTCCGTTT TACGGCGTTT TTTCCCTTAT TCCCGCTGTG CCTTTACAAC

3851  AATACTCATA CTCTTCCTTT TTCAATATTA TTGAAGCATT TATCAGGGTT
      TTATGAGTAT GAGAAGGAAA AAGTTATAAT AACTTCGTAA ATAGTCCCAA

3901  ATTGTCTCAT GAGCGGATAC ATATTTGAAT GTATTTAGAA AAATAAACAA
      TAACAGAGTA CTCGCCTATG TATAAACTTA CATAAATCTT TTTATTTGTT

3951  ATAGGGGTTC CGCGCACATT TCCCCGAAAA GTGCCACCTG ACGTCTAAGA
      TATCCCCAAG GCGCGTGTAA AGGGGCTTTT CACGGTGGAC TGCAGATTCT

4001  AACCATTATT ATCATGACAT TAACCTATAA AAATAGGCGT ATCACGAGGC
      TTGGTAATAA TAGTACTGTA ATTGGATATT TTTATCCGCA TAGTGCTCCG

4051  CCTTTCGTCT CGCGCGTTTC GGTGATGACG GTGAAAACCT CTGACACATG
      GGAAAGCAGA GCGCGCAAAG CCACTACTGC CACTTTTGGA GACTGTGTAC

4101  CAGCTCCCGG AGACGGTCAC AGCTTGTCTG TAAGCGGATG CCGGGAGCAG
      GTCGAGGGCC TCTGCCAGTG TCGAACAGAC ATTCGCCTAC GGCCCTCGTC

4151  ACAAGCCCGT CAGGGCGCGT CAGCGGGTGT TGGCGGGTGT CGGGGCTGGC
      TGTTCGGGCA GTCCCGCGCA GTCGCCCACA ACCGCCCACA GCCCCGACCG

                                      ApaLI
                                     ~~~~~~~
4201  TTAACTATGC GGCATCAGAG CAGATTGTAC TGAGAGTGCA CCATATCGAC
      AATTGATACG CCGTAGTCTC GTCTAACATG ACTCTCACGT GGTATAGCTG

4251  GCTCTCCCTT ATGCGACTCC TGCATTAGGA AGCAGCCCAG TAGTAGGTTG
      CGAGAGGGAA TACGCTGAGG ACGTAATCCT TCGTCGGGTC ATCATCCAAC

4301  AGGCCGTTGA GCACCGCCGC CGCAAGGAAT GGTGCATGCA AGGAGATGGC
      TCCGGCAACT CGTGGCGGCG GCGTTCCTTA CCACGTACGT TCCTCTACCG

4351  GCCCAACAGT CCCCCGGCCA CGGGGCCTGC CACCATACCC ACGCCGAAAC
      CGGGTTGTCA GGGGGCCGGT GCCCCGGACG GTGGTATGGG TGCGGCTTTG

4401  AAGCACTAAT AGGAATTGAT TTGGATGGTA TAAACGGAAA CAAAAAAAAG
      TTCGTGATTA TCCTTAACTA AACCTACCAT ATTTGCCTTT GTTTTTTTTC
```

```
4451  AGCTGGTACT ACTTTCTTTA AAATTATTTT ATTATTTGAT TTTATTTAAT
      TCGACCATGA TGAAAGAAAT TTTAATAAAA TAATAAACTA AAATAAATTA

4501  AGTATATATT ATATTTTGAA CGTAGATTAT TTTGTTGAAA GTTGCTGTAG
      TCATATATAA TATAAAACTT GCATCTAATA AAACAACTTT CAACGACATC

4551  TGCCATTGAT TCGTAACACT AATTCTGTAT TAGTCATTCC TCTTGTTTGA
      ACGGTAACTA AGCATTGTGA TTAAGACATA ATCAGTAAGG AGAACAAACT

4601  TAGTATCCAA AAAAACGGCT ATTTTTTTGC AATCTTATTT CCTGCATATT
      ATCATAGGTT TTTTTGCCGA TAAAAAAACG TTAGAATAAA GGACGTATAA

4651  ATACAGATAA CATAATGAAA GAAAAAATCT TTTTTTTTGT TCTTCAATGA
      TATGTCTATT GTATTACTTT CTTTTTTAGA AAAAAAAACA AGAAGTTACT

4701  TGATTTCAAC CATTCTTTTA AACATTGATC AATTCCTGAG CAACAACCCC
      ACTAAAGTTG GTAAGAAAAT TTGTAACTAG TTAAGGACTC GTTGTTGGGG

4751  ATACACACTG GTTTATATAC CGCCCCTTTT ACAGTTGAAG AAAGAAATAG
      TATGTGTGAC CAAATATATG GCGGGGAAAA TGTCAACTTC TTTCTTTATC

4801  AAATAGAAAT AGCAAACAAA AGATATGACA GTCAACACTA AGACCTATAG
      TTTATCTTTA TCGTTTGTTT TCTATACTGT CAGTTGTGAT TCTGGATATC

4851  TGAGAGAGCA GAAACTCATG CCTCACCAGT AGCACAGCGA TTATTTCGAT
      ACTCTCTCGT CTTTGAGTAC GGAGTGGTCA TCGTGTCGCT AATAAAGCTA

4901  TAATGGAACT GAAGAAAACC AATTTATGTG CATCAATTGA CGTTGATACC
      ATTACCTTGA CTTCTTTTGG TTAAATACAC GTAGTTAACT GCAACTATGG

                  AvaI
                 ~~~~~~
4951  ACTAAGGAGT TCCTCGAGTT AATTGATAAA TTAGGTCCTT ATGTATGCTT
      TGATTCCTCA AGGAGCTCAA TTAACTATTT AATCCAGGAA TACATACGAA

5001  AATCAAGACT CATATTGATA TAATCAATGA TTTTTCCTAT GAATCCACTA
      TTAGTTCTGA GTATAACTAT ATTAGTTACT AAAAAGGATA CTTAGGTGAT

5051  TTGAACCATT ATTAGAACTT TCACGTAAAC ATCAATTTAT GATTTTTGAA
      AACTTGGTAA TAATCTTGAA AGTGCATTTG TAGTTAAATA CTAAAAACTT

5101  GATAGAAAAT TTGCTGATAT TGGTAATACC GTAAAGAAAC AATATATTGG
      CTATCTTTTA AACGACTATA ACCATTATGG CATTTCTTTG TTATATAACC

5151  TGGAGTTTAT AAAATTAGTA GTTGGGCAGA TATTACCAAT GCTCATGGTG
      ACCTCAAATA TTTTAATCAT CAACCCGTCT ATAATGGTTA CGAGTACCAC

5201  TCACTGGGAA TGGAGTGGTT GAAGGATTAA AACAGGGAGC TAAAGAAACC
      AGTGACCCTT ACCTCACCAA CTTCCTAATT TTGTCCCTCG ATTTCTTTGG

5251  ACCACCAACC AAGAGCCAAG AGGGTTATTG ATGTTAGCTG AATTATCATC
      TGGTGGTTGG TTCTCGGTTC TCCCAATAAC TACAATCGAC TTAATAGTAG

5301  AGTGGGATCA TTAGCATATG GAGAATATTC TCAAAAAACT GTTGAAATTG
      TCACCCTAGT AATCGTATAC CTCTTATAAG AGTTTTTTGA CAACTTTAAC

5351  CTAAATCCGA TAAGGAATTT GTTATTGGAT TTATTGCCCA ACGTGATATG
      GATTTAGGCT ATTCCTTAAA CAATAACCTA AATAACGGGT TGCACTATAC
```

```
5401  GGTGGCCAAG AAGAAGGATT TGATTGGCTT ATTATGACAC CTGGAGTTGG
      CCACCGGTTC TTCTTCCTAA ACTAACCGAA TAATACTGTG GACCTCAACC

5451  ATTAGATGAT AAAGGTGATG GATTAGGACA ACAATATAGA ACTGTTGATG
      TAATCTACTA TTTCCACTAC CTAATCCTGT TGTTATATCT TGACAACTAC

5501  AAGTTGTTAG CACTGGAACT GATATTATCA TTGTTGGTAG AGGATTGTTT
      TTCAACAATC GTGACCTTGA CTATAATAGT AACAACCATC TCCTAACAAA

5551  GGTAAAGGAA GAGATCCAGA TATTGAAGGT AAAAGGTATA GAAATGCTGG
      CCATTTCCTT CTCTAGGTCT ATAACTTCCA TTTTCCATAT CTTTACGACC

5601  TTGGAATGCT TATTTGAAAA AGACTGGCCA ATTATAAATG TGAAGGGGGA
      AACCTTACGA ATAAACTTTT TCTGACCGGT TAATATTTAC ACTTCCCCCT

5651  GATTTTCACT TTATTAGATT TGTATATATG TAGAATAAAT AAATAAATAA
      CTAAAAGTGA AATAATCTAA ACATATATAC ATCTTATTTA TTTATTTATT

5701  GTTAAATAAA TAATTAAATA AGGGTGGTAA TTATTACTAT TTACAATCAA
      CAATTTATTT ATTAATTTAT TCCCACCATT AATAATGATA AATGTTAGTT

5751  AGGTGGTCCT TCTAGCTGTA ATCCGGGCAG CGCAACGGAA CATTCATCAG
      TCCACCAGGA AGATCGACAT TAGGCCCGTC GCGTTGCCTT GTAAGTAGTC

5801  TGTAAAAATG GAATCAATAA AGCCCTGCGC TCATGAGCCC GAAGTGGCGA
      ACATTTTTAC CTTAGTTATT TCGGGACGCG AGTACTCGGG CTTCACCGCT

5851  GCCCGATCTT CCCCATCGGT GATGTCGGCG ATATAGGCGC CAGCAACCGC
      CGGGCTAGAA GGGGTAGCCA CTACAGCCGC TATATCCGCG GTCGTTGGCG

5901  ACCTGTGGCG CCGCAGCGCG CAGGGTCAGC CTGAATACGC GTTTAATGAC
      TGGACACCGC GGCGTCGCGC GTCCCAGTCG GACTTATGCG CAAATTACTG

5951  CAGCACAGTC GTGATGGCAA GGTCAGAATA GCCCAAGTCG GCCGAGGGGC
      GTCGTGTCAG CACTACCGTT CCAGTCTTAT CGGGTTCAGC CGGCTCCCCG

6001  CTGTACAGTG AGGGAAGATC TGATATTGAC GAAGAGGAAC CAATGTAACG
      GACATGTCAC TCCCTTCTAG ACTATAACTG CTTCTCCTTG GTTACATTGC

6051  TTACACTGAA GAAAACACAC AATAAACGGG AAGAAACGGT GTAAAAGTGT
      AATGTGACTT CTTTTGTGTG TTATTTGCCC TTCTTTGCCA CATTTTCACA

6101  GAAAATAATT TTTGAATATC ATTTCCCTTG GTTTAATTCC AAACGAAACG
      CTTTTATTAA AAACTTATAG TAAAGGGAAC CAAATTAAGG TTTGCTTTGC

                                 EcoRI
                                 ~~~~~~~
6151  TGTTTTTTTT AGAGAATGGG AATTCTTATT GGATGTCTAG ATTGTTTGTT
      ACAAAAAAAA TCTCTTACCC TTAAGAATAA CCTACAGATC TAACAAACAA

                       ApaLI
                       ~~~~~~
6201  TACTCCAGAC TGTGCACAAA AACGTTTGGA TGGATGATCA GAAGATATTT
      ATGAGGTCTG ACACGTGTTT TTGCAAACCT ACCTACTAGT CTTCTATAAA

6251  TTAGGCTTAG CTCTAAATAT AAGAAATGAT GCTTGAAAAA CCAGACAGAA
      AATCCGAATC GAGATTTATA TTCTTTACTA CGAACTTTTT GGTCTGTCTT

6301  ATTGAGTTTC AAAAATTGGT AATGTGAGGT ATTAGTCAAC TAACCAAATA
      TAACTCAAAG TTTTTAACCA TTACACTCCA TAATCAGTTG ATTGGTTTAT
```

```
6351  ACAATGCAAA CCGGTTGATA CATTTCATTT TGAAAATAAT GAAACTGGAA
      TGTTACGTTT GGCCAACTAT GTAAAGTAAA ACTTTTATTA CTTTGACCTT
 ......................................................................
6401  TTGGATGACC AGCACACAAA CACATAAAGT AATTATGGGA ATTAGAAGCG
      AACCTACTGG TCGTGTGTTT GTGTATTTCA TTAATACCCT TAATCTTCGC
 ......................................................................
6451  AACATAGAGG AGTACTTGGC CACGAACAGA ATACAAGTGG GAACACTATT
      TTGTATCTCC TCATGAACCG GTGCTTGTCT TATGTTCACC CTTGTGATAA
 ......................................................................
6501  TTCTCCATTG TTTTAGTTCT GTTTTTTTGT CAGCCTAGTT TTGTGCTATG
      AAGAGGTAAC AAAATCAAGA CAAAAAAACA GTCGGATCAA AACACGATAC
 ......................................................................
                                  HindIII
                                  ~~~~~~
6551  TGTAAAAAAT ATTGCCAAGA AAAAAAGCTT GTTTTGTGGC CAGTGTCCGA
      ACATTTTTTA TAACGGTTCT TTTTTTCGAA CAAAACACCG GTCACAGGCT
 ......................................................................
6601  AAAAAATTTT GGGGAATCTT CGGATTAATT TATGTTTTCA
      TTTTTTAAAA CCCCTTAGAA GCCTAATTAA ATACAAAAGT
 ......................................................................
```

Sequences with unknown function, C. albicans sequence NOT present in the public domain (ALCES/EMBL)

>328c2 1803bp in-house: 1123-1803 public: 1-436/468-1021 PathoSeq: 437-467/1022-1122

ATGTCTATTACAGTTACATTTCCGAAATCTCCATCTACGAAAAAACGTGCACCG
GCATTTGGAATTGAGTTGGAGTTYAG
TCAMCAAGSCAGTAGCGATGGTGCTATAGAGAAAAGCGGCATTGGCAGTTCCT
GTGTTTAGCGTTGACAACCAAGACTWT
GTATTKATAAGAGAYCWTGCCAAGTACTGGGGCTACCCTTCATCGTATCAATT
GATTGTCAAGTTGGTCAAATGTGCTAA
CATTGAAAAGTCGCAAATCTTAAAGACCGATAAGGATTTGAATAGAGAGTTGT
TTGAGTTGGATTTGATTGAAGAAGCAG
ATACAAAGATTGATCTTTTTTATATTTCGTTACCCTTGGTCTATTCAAGAATAGA
AAATAAGAAGGTTTTTTATGTTCTG
CGTGAACCAGAACAGCCAAAGGTGTCGAAAGCMCCAACACAAGAGAAACCAG
CAAGTGTGGTTGCTGCAGAAGAAGATGA
CGATAATCTAGATGATGATGAGGAGGACGAAGTGGATGAAGACATGGATGAA
GATAATGATAATAGTGGGGAATTGTCTA
AAGGATACAAGCACATGCACAAGGACCATCCAAAGTATATAAATGACGATAG
GGTTACTATTGGACAAGTGTTTCATCAA
TACGGACTTGACCCTTCGACACCATTAACCCATTCACTTTTCAATAGTATCAAC
TCAATGTCGAAGCTAAACTATTACAA
GAATTTTGGAGTTTCAGGTTACCGATTTCTTCCCAACAGCAAGTTATCTTATGC
AGAACGAGAATTGGTGTTGAATGCCA
ACAACTACAATGATATGCACATTAACGAAAAGACAGAATCCAAGCCGAAAAA
GAGTTTCCGTAAACCCATTGGAAAGTCA
AAGAAACATAACTTGCAGATTGATCCGAACTCCATAGATTTAAGCGAGTCAGT
GATTCCGGGACAAGGGTTTATACCTGA
CTTTAGTATCCACCTATCTTTGCAAAGTCCCTAATTATTATGTGACATCAACCC
ACCAAAGTCTCCCGCTGTCGTTCAAC
ACAAAGAATCTTAATGCAACTTCGAACTCTTCGTATTTGTTTAATGATAATGTC
AAGATAAAGTCAAAAAGTATTCAGAA
GTWSGTGTTCAACAGCGATACCGATAATTACCATCACACAAAGTATTTCTACA
CCAAAACCTACCGTGGTCCAGGGTCGG
GGAATTACAAGGATGGTGCATTGATGAACAAAATCAACAAGATACATCTTTCC
AGTAATAAAAAGCCGCGCCACAAGAGA
AAGGTGTCGAACAATAACAGGTACAACAAGAGTTTAAAGGGGTTAGTCCACG
AAAAGTTTGACAAGAACTTTGTTGAGTA
CTTGCTTTCTGAGCAACGCAAGTATACCGAGGACTATTCCAATCTTGAAATTTT
ACACAATAGCTTACAGTTTAATGTTC
TTTTGAATACGTATCGTGGTGTTGCCCAAGAGACATGGAATAACTACTACAAG
TTTAAATTGATTGATTTCGAACAATTG
AAGGCTTTGCAAATGGAGGCAAATGAGCTTGAGGAGAGAAAATTGGATGCTG
CTAGACACCAACAGTGGGCGGAAGAAGA
GAAGCTTTNCCAAGAAAGATTGCGTTTAGTATTTGAAGATGAACGGACGAGTT
TGAGCAATTGCAAAGCGAGTTTGGTCA

Fig 5

GAGAAAGAAGGATTTGGAAGAGAAATTGCGTCGCCGTCAGCTANANGCATCTT
TGANTGATAGTTTTGAACTTGATAGCG
AAAATGACNATGAATCTTGACTTGNCCAAANTNAACAAGACTT

Fig 5 (cont'd)

>113g4 844bp in-house 1-844
ATAGAACTGTTTGATATACAACTATCTCACTCCCAATTGTGACTTGAATAAATAATAATACCTATCACCTAGTAATCTTT
ATCTTAACGTAATCTCTGCAAAGCACAATCAATGTATAAAAGCATAAAGATAAAATCTTGGTGAGGTTTAAGTTCATAAT
TATAATGAACAACAATTACTAAAAGGGATGGTATCAACAAATTATAGGCTAGGTAGAACCATAGTGGCTGTTCGGGAGTT
CGGGTAGTTTGGGAAGGTTGGGAAGGTTGGATAGTTTGAGAAGGTTCCGTGGCTGATTCTAAATTAACAGAGAACGATAT
AATGTACAAAAAAACATTCAGAATTTTAAACAACCTTTATATATATATATTAAATGCTCTTGTCATCAACTTGCCATTGC
TGTTGATGATGCTTTCCTGTTAAATATACCTTTAAGAACCAGATTCACTATCTCAACTAATATTAACCCTTATACTTTTT
GTTTTGACATTCCATAATGACACAAAAGATTGTGAAATATTTTTAGCCTCAAGGGGATTCTACTCATTCCATCTCAAACA
CACATTCTTTGTATCACCAATACCTTTTGCTAACAGAGGAACAAAAAATTGACACGCATGTCATTTACCCTATAGCACTA
TCACTACAAATCAAAGGATTTACAATAGTGGGAATGTCAAATCATGTATATTATTAACACATTACACATATTTATTTTCA
GGTACATAATACTCAATATCTAAAACTTCAAAATGGTACTGTACCTTAAACTTTCTCCTTCATGTCTAGTTGAATATTAT
ACTTGCTAATGTCAAAAATCATGTCTTCACACATTCCAGGTTGT

Fig 6

>15c1 977bp in-house:1-977 bp
TTTTTTTTACAATATAGTTAGATCTCTTTTTAAAATTTGAACACAAAAAAACAAAAAGTAAACTACTATCACCACCACCA
CCACCACCAAAACATCATAGTGGAACTTAATTGAAGAATATATTAATAACCATTAATTATAATAACATACTCAAAAGGAA
TAGGAGTAAAACCTTTATATGTAAATTAATTAAATAGCAAAAAAAAAAAAAAAAAAAGGAAAGATTTCAACAAATCTTGTA
ATTAAATTAAATTTCATTTCATTTCTTGAAAGTGATATAGTCGTAATAGCAGTAATATTAGCAATAATATTAAATAAAAC
TTTAAAAATAACAATTATAATAATAGTAATAATAAACGAATTTAACAAAACAAAAAAAGGGGGGGAAGACAACGAATAT
AGAAGAAGAAAAAACAACAAGACGGGTAGTAGATATATCTGGCTTAAAAAAGCATATCTAAAGTACAGCAAACACATAAT
GCAGCAAGACAACCCATTAAACAAGAATCATTACCTCCAGAACGTGGTTGTTGTTGTACATACATAGGTTGTTGTTGTTG
TTGTTGTTGATAATATCCACCACCACCACCTGGCTGTTGTTGATAATATCCACCTTGTGGAGGTGGTGGTCCATAAGCAT
TATATCCTTGTTGTTGTTGATAGTGGCCATGACCACCACCACCACCACTAAACATCCCTCGATCTTGTGTTTGTTGAGAA
TAATTGGGTTGTGATTGTGGTACATAACTTTGTTGTGGTTGTTGTGATTGGGGTTGATTATTATAATTTGGTGGTGGACC
ACTAGGTTTACCGAAATATTCGTCTTTTGACATTGTTTATATTTATAGGTGGTGTAATTTGGTGTGGTGTGGTTGTCTAA
GATTGAGTATATAGAAGTTGGAAAATTTAATAACAATTAATCTAAACTTGATATAAGATGGATTAGCAATGATAATGAAG
AAGTAAAGTTGAATGTG

Fig 7

1 QQSYVPQSQP NYSQQTQDRG MFSGGGGGHG HYQQQQGYNA YGPPPPQGGY

51 YQQQPGGGGG YYQQQQQQQP MYVQQQPRSG GNDSCIMGCL AAICVCCTLD

101 MLF

>207g4 769bp in-house 1-769
GCAAGATCTAAACTCCAGTTTTTTGGTGTAATGTTACACAAGCAAACAAAATATAAATCGAAAAAGCCCCAAATAATTCT
CTTCTACAAATTACGAAAAATGTTTCACATGTATGAAAAAGCTTTATCTATACTATTTCTCCTCCAACTCTAGCAGTGAG
AATGATACTGATATCTCCTATTAGGATACAGTTATCTATTATAAGTATAATAATAATCATGGAGATAAATATATATTAAA
TCGATGGAGTTAACGAGAAAAACAATACAACCCATTTTGCAGCAAAATGAGACATTTCACAGAAAAAAAAAACAAGAAAAG
ACAATTACTCCATTCAAATAATTCCACAATAAAAAAAATAACAAAGAACAAACGTACTAACAAAAAACATCACTAATTTCA
CTTTGAAAATCTTTACATACTCAACTTCTAAAGATTAATAATAAGCGATGCATATTCATCAGAATTTAGTGTATACAATA
TGCAGGTGATTATGAGCCAGGTGAAACAATTCTTTACTAAAAATCTAGGAGTTGTTTATATACAGTATTTTTGTCTAAAC
CTGTCTCTAACGTATACAAGATAAGATTTGTAATCGGTTAGAATAACAAGAAGGTGTGGTTGTGGACTTGGTGGTGGTGG
CAAATTTGAATGATATATTGTTTATCTCAAGTATAGCAAATACAAGGGCAAAAGGCTGCAACAAAACAAGAACTTGGATT
GTCGCAATTCTCTTCACCCTTTCAGAATGTCCTCGTGTATGTGATCAAT

Fig 8

>226c_af1 766bp in-house 1-766bp
AACGTAATTGTTATATTTTACCAAGGTAACAGGGGACCTCATTATCATTAGTTGTCAATTCAATTACTCCAGAAACAAGA
AACACAAGACTTGTTTGGTGTTGCTATTAAAAGATAATATATAATCAGGATAAAAGAATTTTTTTGGTTAAAGAAAATTA
CAGGGACGGTAAATCATTCTTCTTCCCTATAAACCAAAAATCTTATATGTCCCAAGTTAACTTATTAGAATTCCAAGATT
ATTTACTTTACAGTGAATCATTAAACATTTTAATTGAAAGCGAGTTTAGCTCAATGTCTTCAGACACAACTGCTTTTCAG
GCACCACCAACAAAAGCACCAGAAGCCTCCATGGATCTGGGTACAATTCCCAAAAGATCTCCAGCAAGATTGTTTCAAAG
GTGGATATCATCATCATCATCAAAAGATAAGCCAGTATATGCAGAAAAAGCCCTTCTCAAGAAGCAAAACATAGCACCGG
AACCAATAAAAATAACTAAACAACAAGTACCAGCTAAACAAATAGGTACATCTGAACCATCGTCGCCTCTAAGTGTGGCT
TCGAGTCATGATAATTCATGTTCCGATTCAAGTGCAGCTTCTATATTTTTCTGATTCTAAAAATAACAATAGTATGCAAAT
GTTACTCACAGATGATATAGAGGACATATTAGAGGACATAGACGATGCTGAGATATACGATGCTGAGAAGGTTACCATAA
CATATATAAGTTCTAAATCATGCTAATACACATTATTAATTATTTG

Fig 9

>233c_cp1_full 500bp in-house 1-500 bp
GAAAAATCAAACAACAACAACAACAGTAAGCCAAGTGATAGTACCAAATCTACTTTAGCAAATGATGAAACAAGAAAAAC
ACTTGATCCTAAAGGCGTTGGAAGCACTACAACAGGTGATAAAGACACAGTTTCATCAGACAAAGCATCTCTGCCAATTG
AAGATAAAGAAAGTTCACCATCCCTAGCTGGAAGTTCAACATCAACACCAAGTGGAACTGATAAAAAAACATCTCCTAAA
AAATTAGTTACCAATGCTGTCAATAAAGTTGAAAATAATGATGATTTCAAAAAATTCATTAATGAGGCTGAAAAGGAAGC
TAAAAAATCCAAATCTGGATTGAAAAAATTATTTAACAAGAAGTAGAAGTTGTTTAAATTGTTTCGATATAAATTGTATG
AATTCCAGTTTTTTTATTTTTATTTTATTTTATTTTAGTTTAGTTTTGATTTCATTTTTGTTTTTGTTTAATTTGCAATA
CAATATAATGTTTATTTTTT

Fig 10

>22g3 (5') 535bp in-house: 1-535

AGGTTCCAGTTACCAATTTAGGAAGTGTGTTGCAAGCAGGGCTACCAAATATG
GGTGGCAACACATATGGTAGTAAGTGC
TACCAATGTGGGTGCAAAAAATTTTGCCAAGTAATTTGTATGGCAATAACAGA
AGTGTTGGCGGATTCNAACTGAGGAAT
CTTTGGTGTGTAAAAAAAAAGCAATAGCGACTACGCTACAANAGGCAATCNAT
TATTATTATAAAGTGGAAGTTATATAT
ATNTTCTCGGGGGGGGGGGGGGGNTTNGGNNTCCCCCCCCCCCCCCCANNTTT
TNTCGGCCCNCCCACCNTNCGGCCTTC
TGGCTCCCCCCCNNCGGGCCNCNNGTAAATNCCTCCACCCNGGGANAANGGNA
AANGGGGAACNANNAAGGGGGGACNNN
NCACCCNATGGGAGGGAAAATCCCNAANNTTTNCCCCCCCNNCCCNGCCNAAN
CCNCNTGGGGNGGGCCAAANNCNGGGG
GCTNCNCNCCCTNCCCCCCCGCCNTNNCCCNNNNTNCCNNCGANCTCTNNGNG
GC

Fig 11

>22g3 (3') 426bp in-house 1-426

CCCCCATATAACGTTGTCAATAGCAATACTCTGTCGCACCCATAGTGTGCACTT
CTCGGTGGTATAAAAAAAATTTTTTC
TCCCAAAAAAAATCTTCTCCTTTCCACCACTTTTTTCTTCTTCTTCCTTCCCCATT
CCCTCCCAAATCCCTCATTTTCCC
CATTTCCCCTACCCTCCTGGCCCTGTATTCCAAAATTTTCTCGGGGNTACGCCC
CGAAGANAACCTCCCTCCCACCCACC
CATCTTTGTCNGGNTTCGACCTTCGGCCTCANGGCTCCACCGTCGGGGNTCTTG
TATATTTGTAGACTCCNGGAAAAAGG
GAAAAGGGGAGGAAGAAGGGGGGAAAAAAAAAAAANGGAGGGNGAATCCTT
TTTNTTTTNCCCCCCNTCTCAAACCNAAA
CCCCNTNTGGGNGGTCNAATTAGGGG

Fig 12

>35gK 1334bp in-house: 146-669 public: 1-145 PathoSeq: 670-1334

ACAACGTATAATCGACAGTTTACTATATCTGCTGACTTCAAAACCAATGCATTC
TTCAAGCGTGCTCTGTCGATTTCTAT
CATAACATCCACTTTCCGGNGTAATCGGATTACTAAAGCCACAGAATCAAGGT
GAACATCAAGCTTCAACTTCTTTCTTG
GTCCACGAATAATTTTAATTTGGTTMTTSKKGSMAMKGCTTTCTACRGTAGGTT
TGAATCTTTCCAACATTGTCTTTGCA
TAGAAACMGCACCAGACAAGAAACATGTCCACTCGACCATCAACYTSKGGGT
AWWGACAAAGTWAATCTGTCTGGATCCT
TTTCATCCAGTTTCCCTGCATKGGAWACAAGTNTGTCCCGCACAGTTAAGACT
GTTTTTATTTTSKTGGTATTAGACTCA
TCAAGTTCCGAAGGAGAGGCATCATTTARGGGWATAGACTCCGCTGAGTTAAT
ACTGGATAAATCACTTATTTCAGATTC
ACTGACTTGTWCTTCAGTGACCTTATCAAAATCCTCAATGTACTCSGARGCGTW
TTCMCTCMATGTGAAGGCTTTTAAAA
GGGCAACRCTGGTTYCAAAATGCTTTCTTGCRAGTTTGTACKTGACAGAAAAA
TCAAAAACYTTGAAAGATATACCTCTT

Fig 13

CTAAAGTCTTTTAAATCAATTTCTTNTCCTAATTTTTCATCATATAGCTTATGAC
TTGGCAAACCCTCCTTACATACCAT
ATCCATTACAATGCTAGAAATGTCAATCTTCACTGACGATATAAAGGATGGAA
GAACTTCAAATAATTTTATAAACTCAG
GATTGGCTGGTGTATCTGCTGCAGGAGCTCCAGATTTATTGTCCATTTGCTCAC
TCCATGGACATACATTATTAACGTCC
ATCTTTTTCCATTCTTCAAATTTCTTCGGTGAAATAAATTCGTTGACGRWTTTTA
AACAGACGTACAATGTGAAAGATAA
GATCATTAGCAGAGAGCAATTCGAGACTCTTGCTTGAAAGTTTGATTGACACG
TTTTGTTGTAACATATTGTAGGTGGCT
AAAAGATTGACTTWRGTAAAATGRAACTTATTAACCCTGGGCCCTCACATTTC
ACATTTTTCATCTTAAACAAAGKGGTT
CAAAGKGGAACTTGGTTTGGATCCYTTAWTGGAAWATTTCYCAGKRAATACTT
TCAAAATCAACTCCAGGAGAGCCACAG
TGATAATTGAATTGGATTTAGATAAGCGGTTAAACTTCCCAATTTCAGTTTTAC
CAAACTCTGGTAAATGAAGGTTAAGT
TTTGTGTCCACCACAACAAGTTTACTAAAAACAGCCTTGAGCATTTTGGAGGCA

Fig 13 (cont)

>36g2 (5') 520bp in-house: 1-520

CGTATAGAGAATAATCCGTTGAAATTGATTGTTCAATCATTATTGTATCTTTTCC
CTTTTTTTTGTTCTAACCATAATGT
TAGAATAATTAGAAATTGTCTAAATATATATTCAGTTTAACAAAAAACAGAAT
GCTTGCAATAAGATTTGATTTCTAATT
ACTAATCGTTAATATTTAGTTTGGTGGGGTTTTATTTATCGAAGATGTAGCATT
ATTTGTATCNAATAGATAAAGAAACT
TGAATTAAATGGCNTAATTTGTTGCAATAGTAAAAAAGAAGAAAAGTGGTAAG
GAGTGAGTGAAAATATTTTTTGCCCCA
ATTTGAGTNGAAATCTTACACCNAAAAGTTTGGACNAAAAAGTTTTTACTAAA
ATCTGANAATCTNCCTGAATAGAACCG
ATCATCCNCATNTCCGATTTCNTGAGGANAGATAGTGGCCCCACCTCNTGGTG
ATTAGAAGGAGCNCCCATGTTTTACAA
TATCTATATCCAGAATAACNTGTTTGTGACCTCNCCCCNG

Fig 14

>36g2 (3') 472bp in-house: 1-472

CTCTATATATAGTGAAATATAACATCAAATAATGTACAAAAAAGTATAATAAA
TTGATTTAGAAATGAGAAAAAGAAAAA
AACTTGAAGTAGTGAAGATATATTTGTTGGCTATCTTTCTTGGTATGGCTCAAT
TCAGCCAATCTTGGATGAAAGGTTGG
AGTTTTAGTTTCGTGGTTTATTGATTTGTAAGTACTTTCGGGCTAGAAAGTTNA
CAAACATGATTAATCTTGATATANAT
ATTTGTTAAACATTTGGTGCTCCNTCTTAATCNCCCAAAAAGTTTGGGNCACTA
TCTTTCCNCCNGAAATCTGTATATGT
TGANTGANCCGNTCCATTCCTGTTNANTTTCNGANTTTAGTTAAAACCTTTTTG
TCCCAACCTTTTGGGGTTAGANTTCN
NCCCCANTGTTGCCNNAAATATTNCNCNCCNCCCTNCCCCTTTCCCCNTTTTAC
NAATGCACCAAGTAAGCG

Fig 15

>38g1 1348bp in-house: 183-940 PathoSeq: 1-182 / 941-1348

TCTCTGGTATAACTTGCACTACCTCATCGCTACCCCGGATTTTTTTTTGGTATGA
TCTACACGTCCTCATCGCTACCCCA
GATTTTTTTTTCTGGTGCGCCGGACACGCCCTCCGGTCCGCACCGAAAACCGGGG
TAATCTCCGTCGGAGATACACATCCG
CGGACACAAAATCAGATGAGCTACCACCGAAAATTCCGAAATTTCAAAAACTC
AAAATCCCTAAAAACAAACTATCCAGA
NATTATTGCCATGCCCTGAGGATGAGTTTAGTTTTTTAATTTTTGAAAAATGTC
CAAAACTGGTTGTGCTGTATAGGANG
GGTAAGAATTTGCCATTCTGCCCCTTTGGGTGGGTCAGTCNAAAAAAGANGTA
TCACTCTGGTTCNAACGGGAAACAACN
NAAAATGGGATTAAAMTWATCTCCAGAMCAAACTTAGCTTMWWACACCCAY
TTTAGTTGTACTSGYGWRCCMAAMMCMAA
TTTTCCATTTTGTTTGGGGANGGGAATTTARACCAAAWTTTTTTTTTTGAAATTT
CGCTMAGTGTYMAGAMCCSCAAAAG
TCACCTTTTTTCGTTTTCMMCYACGGCARARGCYCACCGGTTTTKYKTGGKGS
MCRGCCMAATTGAWTTTGTGGGTGSGC
ACGKGGAAAAACAGTTKGTTAGTGGACACGTTTTTGCAGTGTGAAACTGCGCT
CGGAGGTACTATATGCGAAAGCAGAAA
AGACAATTGCAAGAATACAGAGAGTTCTTCTCTGGGCTANNGCAATGTGTTTA
AGGCCAAGTCGACGAGTGGGGAGAGTC
TGGAAGTGATATACACATCACGACCTACTTTATACGCTACGTTCGGCATGGGC
GAGCCACTGTACGGTGGCAAGCCTGAA
CAGTCCCACACCAGATATCTAACGATTCTGTGTATGGGCACTGATGGGATTTAG
TGGATTACTAGCTGATAGCAAGTATT
GAAAACTAAAACCCGACTCGGGGGTATGCCTTGGCAAGTAGCCGGAGTAAAAT
CTGTGACTTTGCTGAGTGTAACTCCCT
CCATGGTTGGCGATGTTCGACGTGCGCGGCAGTTCTTGTCGTATCACAGTCGCA
CGGACACCACACCGGGAGAATCTTAA
GAGGGCTATATGGATGTGGAACGGTTTGCTTGCTGTGGTAAAACACTGGCGGG
CGAGCCGACGTTCCACGGACACAGCAA
TGTGTTTGCAACCAAATAAATAACTTGTACGGTTTGAACGTGTTTTTGGCTGCT
CCTTCCAGTTCTTGGCGGGAGAAGCT
TGGGCGCGGGAAGACCACTACTACGTAGTTATCTGGTTGATCCTGCCAGTAGT
CATATGCTTGTCTCA

Fig 16

>60gK 990bp in-house: 445-752 public: 1-140/753-990 PathoSeq: 141-444

ATTACCGATCCGTCGGATTTTAAAACCACAAAATTGCCTGCATTAGCAGAGCT
AGATATTTTCATAGGGTGCTATATATG
CAAAGATCTATTGAATGCACCCGTGAGGACACAATGTGATCACACGTACTGTT
CACAATGTATACGAGAATTTTTACTTC
GAGATAATAGATGTCCGCTTTCTAAAACAGAGGTTTTTGAAAGTGGTCTAAAA
CGTGATCCATTGTTAGAAGAGATCGTC
ATTAGTTATGCCTCCCTTAGGCCTCATTGATTACGATTATTGGAGATTGAAAAG
GTGGAATCGAAGCAAGAGGTAGATCG

Fig 17

TGAGAAATCAGCCAATGAGTCAGCGCTGAATGGTAATAGAAATGTAAACAAC
GATGTTGACGAAACTGTGCGCGTTAAAG
ATCAACTGAATGCAGATAAACTAGGTGAAGAAAAAGGGCAAGCTCAACATGG
GGAACAAGTNAAACGAGCAGACTACTGA
AGTTATTCTGTTGCTATCTGATGATGAAGAGAATGGTTCTGATAGCCTAGTAAA
ATGTCCTATTTGTTTTGAGAGAATGG
AATTAGATGTACTACAGGGAAAGCNTATTGACGACTGTCTAAGTGGAAAGAGC
ACGAAGAGGACGCCTACAGACATTTTA
TCCCCAAAAGCCCAACGACCGAAGCAAATCACCTCCTTTTTCCAACCAACAAT
AGATACCANAACNCCTTCCCCCACCTA
CCAGTTNNGGCGTCNACAACTCCCACAGCAACTCCGACAACTACATTGTTGAA
AGCAAACGTCTCATCTCCATCCCAAGT
GGCGCAAAGTACAGTAAACAAGGGCAAGCCATTACCTAAACTCGATATCAGCA
GCTTGAGTACTAAAAAAATAAAAGCCA
AGTTGAGTGATATGAAACTACCAACAACAGGTAGTAGGAATGAAATGGAAGC
CAGATACTAGCATTACTATGTGATTTAT
AATGCCAACCTTGACACCAATCATCCTGTA

Fig 17 (cont)

>64gB 627bp in-house: 1-627

TNCANCCTNCCATNCNCCCAGGCNNNGCCACCCCNGCCGNNCCCCCNTNTTTC
CCCCCCTCCTTNGTNGCCCTCNNGGTG
GTGTTTGTGGTGTGACNAATAAANATGGTNTATCATTAGAANAGGACATTGCN
NCGGAAATGACTGTCGACAATAAAGAA
GCAAATATATACAATGGATTATGAANGTGCTAGGATGGATTTGAAAGTTTATC
TGGGTTTATTCCAATGTAAAAATTATT
TGTAATTGATATGGCTAATTATTTTGCTCNATATNTATCACAAAAAAATGATTA
AGTTCGAAATGAAATTGGCNTCCATA
TATAAAATTTCTGACAGGAAGAGAAAATTCANGACNTGTTGCCCNAAAAAAAA
AACTTTACCCCNCNTCNANTCNTGTNN
GACTTAACCCCCAAAAANAANANNGCTGGCGGCGGNAAAAAAATAGGAGGGG
GCCGGNNGTTTTTTAAAATTTNANNCTT
GAATATGAACCCAANNTTTGNNTTCNTTTTTNCCACNCCCCCTTCAAATTTNAT
TCCATGTTCCCAAGANNAGGGNGGNG
GGGGNGGTTCCNNCTTTTAAACCNCCCCCCCCGGGTGGNGGGGNCCGTNTTNT
TTCCGGNGGGGCNT

Fig 18

>8c_cp 890bp in-house: 287-890 public: 1-124/154-286 PathoSeq: 125-153

ATGCAATTCTCATCCGGTGTCGTCTTATCCGCTGTTGCTGGGTCCGCTTTGGCTG
CTTACTCCAACTCCACTGTTACTGG
CATTCAAACCACTGTGTCACCATCACTTCATGTGAAGAAAACAAATGTCACGG
AAACTGGAAGGTTACCACTGGTGTTAC
CACCGTCACTGAAGTTGACACTACGTACACCACCTACTGCCCATTGTCAACCAC
TGAAGCTCCAGCTCCATCTACTGCTA
CTGATGTTTCTACCACCGTTGTCACCATCACCTCATGTGAAGAAGACAAATGTC
ATGAAACCGCTGTCACCACCGGTGTC

Fig 19

ACCACTGTCACTGAAGGTACTACCATCTACACTACCTACTGCCCATTGCCATCT
ACTGAAGCTCCAGGTCCAGCTCCATC
TACTGCTGAAGAATCTAAACCAGCTGAATCTTCCCCAGTTCCAACCACCGCTGC
TGAATCTTCCCCAGCTAAAACTACTG
CTGCTGAATCTTCCCCAGCTCAAGAAACCACTCCAAAGACCGTTGCTGCTGAAT
CTTCTTCAGCTGAAACTACTGCTCCA
GCTGTCTCTACCGCTGAAGCCGGTGCTGCTGCTAACGCTGTCCCAGTTGCTGCT
GGTTTGTTGGCTTTGGCTGCTTTGTT
TTAAGTTTATTAGAGCTTAAATCAAATATTTACAAACAAAATTTTCATTTTCCC
CCCTTTCCCTTTCTTCATTCTTCAAA
AAAGGGTTATTTACTATTAATTGATAAATTTATGGTTTCATGTTAATTTACCCTT
TTCTTTATAAACATTGGTATTATTA
TTATCATCATTAGNTTTATTTATATTTTCGTGAGTTTTTCGGNTTTAATTAATTTT
TTTGGATACATATTAAAAATTTAT
TTGGTACTAG

Fig 19 (cont)

>85g3 481bp in-house:1-431
CTAATATACGTCGAGTTCTGGGGGGTTGAAAAAACGGGTATTTTTTGGACCAGCAGAAAAAAAGTGGATTTGCGCGTGCA
CGACCCGAAAAAGGGAGAATTTTTGAAAAATGGCGAAATTTGGGGTAAGTTTGAGAGAGTGTGGAGCAACAACTATAAGA
GAGGGTGAGCGCAAATTGTAATGGCAGGTCGGCAGGCCAATGAAGATGTGTTGTGCAAAAGATGGAGTTTGTAGCGGTTG
CTGTGGCTGAGATATTGGCACTTTTTTAAGACCGCATGTTTTTGGGTAGCGCTGGGTTAAGACCACATTTTTTTTTGTTA
GAAGACCGCAGAAAAGAGAGCACACATACAAAATCAAGACCGCAGAAAAGAGAGCACACATTTAAGAGCACATTTTTGGT
AGCACACACTTTTAAGAGCACAGAAAAAAGAGCACTTATTTCTAAGACCGCATGTTTGGTAGCACACACTTTTAAGAGCA
C

Fig 20

>66g4 579bp in-house: 1-579

CCCCGTTAACCACTTCTAGGGTATACCATTTCATCTGACTGAATAACTGGTTAG
TCGATTTGTTGTTGAAGAAAAGTGAC
CACCTAGTTTTTTCTGCCAACATTTTTTGCGATGAGCCGTCGACGCGTTGTCTTT
TTCTACCCCACGTTTAACAATCTTG
CCAGTCAATTCCCTAGCCAAATAAACTTTAGACTCACAACTCTAACACTGACTC
GTGCCCCCCTGTTTAAACTCTAAATT
ACTTCACAGAGCCTTTACTACCTTAAATTTARGRTTWTSKAKKGTTTCTGTTTTT
TTGCAAATCACCCTGACTYGTTTTT
TTTTCAGCCAGGTTTTTCGTTAAAATCTGACCAAAAAATTTACRACTCCTATWT
TTAAAACTCYAAAWWACAATTAAAAC
TCAATTCAGACAAGTCCTTCTGCTCATTCTGAGTCTTCTCTATTGTCTTTTGACT
TTTTGTGTGTGACTATTTTCATGAT
CACCCCGTTTCTTGCATTTTTTTCAGTCAACTTTTTCTCAAAATCAAGCCAAAAA
AACACACCTTTAACTACCTATACAA
CGCAAACCTATTCAAAACA

Fig 21

>NDI (17c_cp) 807bp in-house: 1-614 PathoSeq: 615-807

AACCTATTCCATAATGTTTACTAGATCATTGATTAAAGGTGGTGGCAGACTTGC
TACTACCAGATCATTGGTCAACAACT
CTACTAGTTTGGTTTTAAAAAATCAATTTAAGAAATATTCAACATCAACTCCTC
CTAAGGTTGCCAAATCAAAATCTTCG
ACAATTGGTAAAATATTCAGATACACTTTTTACACTGCTGTGATATCGGTTATT
GGTTCTGCCGGTTTGATCGGTTACAA
AATTTACGAAGAGTCTCAACCTGTTGATCAAGTGAAACAAACACCATTGTTTCC
TAATGGTGAAAAAAAGAAAACTTTAG
TTATTTTGGGTTCTGGTTGGGGTGCTATTTCATTATTGAAAAACTTGGATACCA
CCTTGTATAATGTTGNTATTGTCTCC
CCAAGAAACTATTTCCTTTTCACCCCATTGTTACCATCTGTTCCTACCGGTACTG
TTGAATTGAGATCTATTATTGAACC
TGTCAGATCAGTCACCAGAAGATGCCCTGGCAAGTTATTTACCTTGAAGCAGA
AGCTACAAATATNAACCCCTAAAACTA
ATGAGTTGACACTTAACAAAGTACTACTGTCCGTTCTGGTCATTCTGGTAAAAA
TACTTCCTCTTCTAAATCAACTGTTG
CCGAATACACTGGGGTTGAAGAAATCACTACCACCTTGAATTATGACTATTTA
GTTGTTGGTGTTGGTGCTCAAACAATN
CTANTTTTCGGNAATCCTGGGAGNCGCNTGAGGAANTTCAACCCCTTTTTTGAA
AGAANGNCCAGTGGANGCCNTCTGCN
AATTAGA

Fig 22

>HOL1 (409c5) part2 762bp PathoSeq: 1-762

GATCAGAATAATGAGGACTTTATACCTGGAACACTCAATATCTATTCCTTGGAA
GTTGACTCTGAAGATGAAAACGTGAG
TCATTACGATGCTTCCAGTCGACCAAAAGTGAAAACAAAAGGCAATATAATCC
TCTTCCCACAACCATCGAATTCATGCA
ATGATCCATTAAATTGGAGTAAATGGAGAAAGCTAAGTAACTTTTTTATTGTCA
TTTTTATTACTGCTTTTACAGCAGCT
ACTTCAAATGACGCTGGATCAATTCAAGATTCACTTAATGAAAAATATGGAAT
TAGTTACGACGCAATGAATACAGGGGC
AGGCGTTTTATTTTTGGGTATTGGATGGGGTACTTTCTTTTTAACACCTGCTTCG
TCGTTATATGGTCGAAAAATAACAT
ACTTTATATGTATCTTTCTTGGTTTATTAGGCGCTGTTTGGTTTGCCTTGGTTAA
AAGCACTTCCGACTCAATTTGGTCG
CAATTGTTTGTTGGTATTAGTGAGAGTTGTGCTGAAGCTCAAGTACAATTAAGT
TTATCAGAACTTTATTTTGCCCATAA
CCTTGGTTCTGTGCTTACGTCCTATATTGTTGCAACTTCCGTAGGTACTTACTTA
GGACCTTTAATTGCAGCCTTTATTG
TTCAAAACATTGGTTTTAGATGGGTTGGTTGGATTGCAGCAATTATTAGTGGTG
CATTATTGTTCGTAATTGTTTTTTGT
TTAGATGAAACCTATTTTGATCGAGCAAAGTTTACCAAGCCA

Fig 23

>GAL2 (360c6) 1004bp in-house: 625-1004 PathoSeq: 1-624

TCCATTTTCCCTTTTCCTCTTTTTCTACATCATCCTCACANCAATTTCAAATATG
TCTCAAGACAACGTCTCATCAACAT
CTACAGCTGAGGCTGTAAATAATGAAATCAAAGTCAAAGATGAATTTCCACAA
GAAGAACAAGCTCATACTAGTTTAGAA
GATAAACCAGTGAGTGCATACATTGGTATCATCATTATGTGTTTCCTTATTGCC
TTTGGTGGTTTTGTTTTCGGTTTCGA
TACTGGTACCATTTCTGGTTTTATTAATATGTCTGACTTTTTAGAAAGATTCGGT
GGTACTAAAGCTGACGGTACTCTTT
ACTTTTCCAATGTCAGAACTGGTTTAATGATTGGTTTGTTCAACGCTGGTTGTG
CCATTGGTGMWTTATYCTTGTCYAAA
GTCGGTGATATGTATGGTAGAAGAGTTGGTATCATGACTGCTATGATTGYCTAT
ATTGTTGGTATTATTGTTCAAATTGC
TTCTCAACATGCTTGGTATCAAGTCATGATTGGTAGAATTATYACTGGTCTTGC
CGTYGGTATGTTATCAGTTTTATGTC

Fig 24

CTTTGTTCATTTCCGAGGTTTCTCCAAAACATTTGAGAGGTACTTTGGTGTGCTG
TTTCCAATTGATGATTACCTTGGGT
ATCTTCNTGGGNTATTGGCTACCTATGGTACTAAGAGTTACTCAGACTCTAGAC
AATGGAGAATTCCATTAGGTTTATGT
TTCGCCTGGGCTTTATGTTTGGTTGCTGGTATGGTTAGAATGCCAGAATCTCCA
CGTTACCTTGTCGGTAAAGACAGAAT
TGAAGATGCTAAAATGTCACTTGCCAAAACTAACAAGGTTTCTCCAGAGGACC
CAGCATTATACCGTGAACTTCAATTAA
TCCAAGCTGGTGTTGAAAGAGAAAGATTGGCCGGTAAAGCATCTTGGGGTACT
TTATTCAATGGTAAACCAAGAATCTTT
GAAAGAGTTATTGTTGGTGTCATGTTACAAGCCTTACAACAATT

Fig 24 (cont)

>KGD2 (98c_cp) 334bp in-house: 139-334 public: 1-138

TTCTAACAACAACATCTTTCTTGGATCTTCAATCAATTCCTTGATGGTTCTTAAG
AAAATAACAGCTTCACGACCGTCAA
CTACTCTGTGGTCGTAAGTCAATGCTAAGTACATCATTGGTCTAGAAACGATTT
GTCCGTTAACAGNAATTGGTCTTTNT
TTAAAANTGTGTAAACCAAATACGGNAGTTTAANGCATTTTTATAATTGGGGT
ACAGTATAATGATCCAATAACACNGNC
ATTANAAATAGTGAAAGAACCNCCGGTCATATCTTACAAAGTCAATTTACNAT
TTCTGGCTTTNTTACNCAAATTANANA
TTTCCTTTTNAATA

Fig 25

>RNR1 (38) 2562bp in-house: 1-2562

ATGTATGTTTATAAGAGAGATGGCCGTAAAGAGCCAGTACGTTTCGACAAAAT
CACTGCCAGAGTTCAAAGATTATGTTA
CGGTTTGAATCCAAACCACGTTGAACCAGTTGCTATTACCCAAAAAGTTATATC
AGGTGTTTACCAGGGGGTTACTACTA
TTGAGTTGGACAACTTGGCTGCAGAAATTGCTGCTACAATGACAACAATTCAC
CCAGATTACGCTGTCTTAGCCGCTAGA
ATTGCCGTATCAAATTTACATAAGCAAACCACCAAACAGTATTCCAAAGTGTC
TAAGGATTTATATGAATACATTAATCC
TAAGACTGGGTTACACTCTCCTATGATTTCCAAGGAAACCTACGACATCATTAT
GGAACACGAAGATGAATTAAACTCAG

Fig 26

CCATTGTTTACGACAGAGATTTTAACTACAATTATTTTGGGTTCAAGACTTTGG
AAAGATCATATTTGTTACGTATCAAC
GGTAAGGTTGCTGAAAGACCACAACATTTGATCATGAGGGTTGCTGTCGGTAT
TCACGGTAATGATATACCAAGGGTCAT
TGAAACCTATAACTTGATGTCTCAAAGATTCTTCACCCATGGTTCTCCTTGTTTA
TTTAACGCTGGTACACCAAGACCAC
AAATGTCCTCATGTTTCTTGCTTGCTATGAAGGATGATTCTATTGAAGGTATTT
ACGACACTTTGAAATCGTGTGCTTTG
ATCTCAAAAAGTGCTGGAGGAATCGGTTTACACATCCACAACATTCGTTCTACC
GGTGCTTACATTGCTGGTACCAATGG
TACTTCTAATGGTATTATTCCAATGGTAAGAGTATTCAATAACACTGCACGTTA
TGTCGACCAAGGTGGTAACAAGAGAC
CTGGTGCCTTTGCCTTGTACTTAGAACCATGGCACAGTGACATTTTTGATTTCA
TTGATATTAGAAAGAATCACGGTAAA
GAAGAAATCAGAGCCAGAGATTTGTTCCCAGCTTTGTGGATTCCAGATTTGTTC
ATGAAAAGAGTTGAACAAAATGGTGA
CTGGACTTTATTCTCACCAAATGAGGCCCCAGGCTTGGCTGATGTTTATGGTGA
CGAATTCGAAGAATTATACACCAAAT
ACGAAAAAGAAAACCGTGGTAGACAGACCATCAAAGCTCAAAAATTGTGGTA
TGCTATTTTGGGAGCCCAAACTGAAACA
GGTACCCCATTTATGTTATATAAAGATTCATGTAACAACAAATCCAACCAAAA
GAACTTGGGTATTATCAAATCTTCCAA
CTTGTGTTGTGAAATTGTTGAATATTCTGCTCCAGATGAAGTTGCTGTTTGTAA
CTTGGCTTCCATTGCCTTGCCATCAT
TTGTTGAAAATGATGAAAAAAGTACTTGGTACAACTTTGACAAATTACATCAG
GTCACTAAGGTTGTCACCCGTAACTTG
AACAGAGTTATTGACCGTAACCATTACCCAGTCCCAGAAGCTGAAAGATCAAA
CATGAGACACAGACCAATTGCTTTGGG
TGTTCAAGGTTTGGCTGATGCCTTTATGGAATTGAGATTACCATTTGACTCTCA
AGAAGCTAGAGAATTGAACATTCAAA
TTTTTGAGACTATCTACCATGCTGCTGTTGAAGCTTCAATTGAATTGGCTAAAG
AAGAAGGTGCCTACGAAACCTATCCA
GGTTCTCCAGCCTCTCAAGGTTTATTACAATTTGATTTGTGGAACAGAAAACCA
ACTGAATTATGGGATTGGGATACATT
AAAACAAGATTTGGCCAAACATGGTATGAGAAACTCCTTGTTGGTTGCACCAA
TGCCTACTGCTTCCACATCACAAATTT
TGGGTAACAATGAATGTTTTGAACCATACACTTCTAACATTTACTCTAGAAGAG
TATTAGCTGGAGAATTCCAAATTGTC
AATCCATATTTATTGAAGGACTTGGTTGATTTGGGTGTCTGGAACGACGCTATG
AAAAGTAGTATTATTGCTAACAATGG
TTCTATCCAAGCCTTACCAAACATCCCTGATGAAATCAAGGCATTGTACAAAA
CTGTCTGGGAAATCTCACAAAAACATA
TTATCGACATGGCTGCTGATAGAGCAGCATTTATTGATCAATCTCAATCATTAA
ACATTCACATCAAAGATCCAACAATG
GGTAAATTAACCAGTATGCACTTCTACGGTTGGAAGAAAGGTTTAAAGACTGG
TATGTACTACTTAAGAACACAAGCTGC
CAGTGCTGCTATTCAATTTACCATTGATCAAAAGATTGCTGAGACTGCCGGTCA
TACGGTTGCAAACTTGGACAAATTAA

Fig 26 (cont)

ACATTAAGAAATATGTTAACAAAGGAAGAGTTGAGAGTGAGAATACCAGTGAT
GCTCCATACAAGTCACCATCAACCGAA
CCAACCTCATTAGAAAGTTCAGTTGCTGATTTGAAAATAAAAGATGAAGGTGA
AAAGCCAGCTGAAGACAAAACCATTGA
AGAACTCGAAAATGACATTTATAGTGCCAAAGTTATCGCATGTGCTATTGATA
ATCCAGAATCTTGTACAATGTGTTCTG
GT

Fig 26 (cont)

>SAM2 (36) 1155bp in-house: 1-1155

ATGACTACTTCCAAGGAAACTTTCCTTTTCACTTCAGAATCCGTTGGTGAAGGT
CACCCAGATAAGATTTGTGACCAAGT
CTCCGATGCCATTTTAGATGCTTGTTTAGCTGTTGATCCATTGTCAAAAGTTGCT
TGTGAAACTGCTGCCAAAACCGGTA
TGATTATGGTTTTTGGTGAAATTACCACTAAAGCTCAATTGGATTATCAAAAAA
TCATTAGAGACACCATTAAACACATT
GGTTACGACGATTCTGAAAAAGGTTTTGATTACAAGACTTGTAACGTCTTGGTT
GCAATTGAACAACAATCTCCAGATAT
TGCTCAAGGTTTACATTACGAAAAAGCTTTGGAAGAGTTGGGTGCTGGTGATC
AAGGTATTATGTTTGGTTATGCCACCG
ATGAAACCGATGAAAAATTGCCATTGACCATTTTATTGGCCCACAAATTGAAT
GCTGCCTTGGCTTCTGCCAGAAGATCA
GGTTCCTTGCCATGGTTGAGACCAGATACCAAAACCCAAGTCACCATCGAGTA
TGAAAAAGATGGTGGTGCAGTTATCCC
AAAAAGAGTCGACACAATTGTTATTTCCACTCAACATGCCGAAGAAATCACCA
CCGAAAATTTGAGAAAAGAAATTATTG
AACATATCATCAAGCAAGTCATCCCAGAACATTTATTAGACGACAAAACTATC
TACCACATTCAGCCATCAGGCAGATTC
GTCATTGGTGGTCCCCAAGGTGATGCTGGTTTGACTGGTAGAAAGATCATTGTT
GACACCTATGGTGGTTGGGGTGCACA
TGGTGGTGGTGCCTTCTCAGGCAAGGATTTCTCCAAAGTTGATAGGTCTGCTGC
TTATGCCGCTCGGTGGGTTGCTAAGT
CGTTGGTGACCGCCGGATTGGCCAAAAGGGCCTTGGTGCAGTTCTCCTATGCTA
TTGGGGTTGCTGAACCCACCAGCATT
TATATAGACACCTATGGGACATCTAAATTGAGCACCGAAGCCCTTGTAGAAAT
TATCAAGAATAATTTTGACTTACGCCC
TGGCGTAATTGTAAAAGAATTAGATTTGGCTCGTCCTATTTATTTTAAAACCGC
TTCTTACGGACATTTTACTAACCAAG
AAAATTCTTGGGAACAACCAAAAAAATTAAAATTT

Fig 27

>135g 859bp in-house:1-859
CGTGCATAATTATCTTAAAACCGTAGATAAGCAAAAATTTATCTTATGAAATGTTCAGCGATAAAGAAAGAAAGAATCAG
GTACCACGAGGAGTGTTTTTGAGAAAAACAACTCGTAAATTAATGAATCTAGTTTCTCTATACTTGAATAATTTTTGAGT
TTTCTGGAAAAGACACCTGTTCCAGTTTCAAATTAAACAAGAATGTGAAAAGAATAAAATTTGATTTATTCTAGCCTGTT
AATAATCCAGGAAAACTCAATTTTCGTAATTGGCAACTTGTCCGAGTGGTTAAGGAGAAAGATTAGAAATCTTTTGGGCT
TTGCCCGCGCAGGTTCGAGTCCTGCAGTTGTCGTTATTTTTTTTGGTTTACTCTCTATTTTAAAATTTAAAACTAATCAA
CTGAAACTGGAGTACCTGCCATGATATGAGTAAATACTTTTTTGATATTAAAAATCTATATAAAACTCCCTATTTATTTT
TTAATTTAAACCCAGATATTGTCCCAATAATAGTTTTTTGTTTGAACTTATTGCTTTGTATGAACCTTGTTAGTTTAATC
TTTCCAATTTCATACTCTCTTAGTTGGCCACATCAGTGGCTCATTGAATAATTCTGATCTTGAAGTGTACCAGATGTATT
CTGACAAAACTGCACACGGACCCAGTCAATAGCATTATAGATATTTTGATTTAAAGTTCACCGAATATATCGAATATCTT
TATTGGCCATCTCATCTCATCTTCTTGCAATAAATTCTTAAACGCTACTTTTTCTCAAACCTTATTATCCCTCTAGATAC
TCTTCCAAATCTTCAGGTTCAAATATCACTTTAACCATCAATGAACAACTAGGGCAAAC

Fig 28

**328c2**

```
                                            X X          fs
                                            = =          =
  1  MSITVTFPKS PSTKKRAPAF GIELEFSQQG SSDGAIEKAA LAVPVFSVDN

      X X   X                                        R
      = =   =                                        =
 51  QDFVLIRDLA KYNGYPSSYQ LIVKLVKCAN IEKSQILKTD KDLNKELFEL

101  DLIEEADTKI DLFYISLPLV YSRIENKKVF YVLREPEQPK VSKAPTQEKP

151  ASVVAAEEDD DNLDDDEEDE VDEDMDEDND NSGELSKGYK HMHKDHPKYI

201  NDDRVTIGQV FHQYGLDPST FLTHSLFNSI NSMSKLNYYK NFGVSGYRFL

251  PNSKLSYAER ELVLNANNYN DMHINEKTES KPKKSFRKPI GKSKKHNLQI

                                  fs           T
                                  =            =
301  DFNSIDLSES VIPGQGFIPD FSIHHLCKVP NYYVTSNHQS LPLSFNTKNL

                                 X
                                 =
351  NATSNSSYLF NDIVKIKSKS IQKLVFNSDT DNYHHTKYFY TKTYEGPGSG

401  NYKDGALMNK INKIHLSSNK KPFHKRKVSN NNRYNKSLKG LVHEKFDKNP

451  VEYLLSEQRK YTEDYSNLEI LHNSLQFNVL LNTYRGVAQE TWNNYYKFKL

                                               X          fs
                                               =          =
501  IDFEQLKALQ MEANELEERK LDAARHQQWA EEEKLRQERL RLVPEDEPNE

                                X    X      L      X    * X XX
                                =    =      =      =    = = ==
551  FEQLQSEFGQ RKKDLEEKLR ERQLEASLSD SFEADSENDD ESELAQIQQD

                                              missing  sequence
     ==================================================
601  FESSANALKT KFEAKRKDLI NPAPPPQPIE TPQLDLNNKP SLPTVYPEII

     missing sequence
     ==================================================
651  RNLPLELRGV VPESKEELPP IKKAIHYVTT YPERPNKEYL TRNRDYPIAN

     missing
     ======
701  ANSGWKG
```

Fig 29

**15c1**

```
                                                           fs   S
                                                           =    =
  1  QQSYVPQSQP NYSQQTQDRG MFSGGGGHG HYQQQQGYNA YGPPPPQGGY

                                                        ambiguities
                                                               ====
                           X              W   W      WX
                           =              =   =      ==
 51  YQQQPGGGGG YYQQQQQQQP MYVQQQPRSG GNDSCLYGCL AALCVCCTLD

     amb
     ==
101  MLF
```

Fig 30

**222g9**

```
                     E                                       E  E
                     =                                       =  =
  1  MRRREIERRK QQKXREQRQK EHEAKRDIRI QQLSEQDSRS NQTKXEEXVF

 51  KKARSTNSGA DETGLMSDKE FDDSAYSPDY LFEENLWNKP NHPDTNHKTK

101  KYTEXVVENL DSPPNDTSAY NSSFHDETNI QNEIQIPEND EYVPQMKATS

                                 K          D       VR  fs   C
                                 =          =       ===  =   =
151  SVNNTTIPAQ RRHESLSTSE NKRRXFETAD VGVXGLDSPX XAQTRNIWKI

                     P
                     =
201  QVSDNPWNVY FFMKXKRLET PEGKLLCRDQ
```

Fig 31

**60gK**

```
                                    FIG
                                    ===
  1  ITDFSDFKTT KLPALAELDI LKRCYICKDL LNAPVRTQCD HTYCSQCIRE
                                                   *
                                                   =
 51  FLLRDNRCPL CKTEVFESGL KRDPLLEEIV ISYASLRPHL LRLLEIEKVE

101  SKQEVDREYS ANESALNGNR NVNNDVDETV RVKDQLNADK LGEEKGQAQH

     G  fs                                              X
     =  =                                               =
151  WEQVNEQTTE VILLLSDDEE NGSDSLVKCP ICFERMELDV LQGKHIDDCL

                                                    fs
                                                    =
                           Q              Q     X ambiguities
                           =              =     =   =====
201  SGKSTKRTPT DILSPKAKRP KQITSFFKPT IDTKTPSPPT SKASTTPTAT

                   S   Q        N          I     K       M
                   =   =        =          =     =       =
251  PTTTLLKANV ASPSPVAQST VHKGKPLPKL DPSSLSTQKI KAKLSDLKLP

                    *            T
                    =            =
301  TTGSRNEMEA PYLHYYVIYN ANLDSNHPV
```

Fig 32

**8c cp**

```
                                                            fs
                                                            =
          G                          G      fs        D     A
          =                          =      =         =     =
  1  MQPSSAVVLS AVAGSALAAY SNSTVTDIQT TVVTITSCEE NKCHETEVTT

 51  GVETVTEVLT TVTTYCPLST TEAPAPSTAT DVSTTVVTIT DCEEDKCHET

101  AVTTGVTTVT EGTTIYTTYC PLPSTEAPGP APSTAEESKP AESSPVPTTA

151  AESSPAKTTA AESSPAQETT PKTVAAESSS ABTTAPAVST AEAGAAANAV

201  PVAAGLLALA ALF
```

Fig 33

**17c cp**

```
  1  PPKVAKSKSS TIGKIFRYTF YTAVISVIGS AGLIGYFIYE ESQPVDQVKQ

                                                X
                                                =
 51  TPLFPNGEKK KTLVILGSGW GAISLLKNLD TTLYNVVIVS PRNYFLFTPL

                                    fs           X  fs     fs
                                     =           =  =       =
101  LPSVPTGTVE LRSIIEPVRS VTRRCPGQVI YLEASATNIN PKTNELTLKQ

          R       N                                       X X
          =       =                                       = =
151  STTVVSGHSG KDTSSSKSTV AEYTGVEEIT TTLNYDYLVV GVGAQTILIF

          X X X         XX   XX X
          = = =         ==   == =
201  GNPGRPMRKF NPFFERKTSG SHLQIR
```

Fig 34

**409c5 part2**

1 DQNNEDFIPG TLNIYGLEVD SEDENVSHYD ASSRPKVKTK GNIILFPQPS

51 NSCNDPLNWS KWRALSNFFI VIFITAFTAA TSNDAGSIQD SLNEXYGISY

101 DAMNTGAGVL FLGIGWGTFF LTPASSLYGR KITYFICIPL GLLGAVWFAL

151 VKSTSDSINS QLFVGISESC AEAQVQLSLS ELYFAHNLGS VLTSYIVATS

201 VGTYLGPLIA AFIVQNIGFR WVGWIAAIIS GALLFVIVFC LDETYFDRAK

251 FTKP

Fig 35

**360c6**

```
1   DNVSSTSTAE A...EIKVKD EFPQEEQAHT SLEDKPVSAY IGIIIMCFLI

51  AFGGFVFGFD TGTISGFINM SDFLERFGGT KADGTLYFSN VRTGLMIGLF

        X X                    X
        = =                    =
101 NAGIAIGALF LSKVGIMYGR RVGINTAMIV YIVGIIVQIA SQHAWYQVMI

                                                   ambiguities
                                                            ==
                                                         X
                                                         =
151 GRIITGLAVG NLSVLCPLFI SEVSPKHLRG TLVCCFQLMI TLGIFLGYCT

    fs
    =
201 TYGTKSYSDS RQWRIPLGLC FAWALCLVAG MVRMPESPRY LVGKDRIEDA

                                                          PR
                                                          ==
251 KMSLAKTNKV SPEDPALYRE LQLIQAGVER ERLAGKASWG TLFNGKTKIF

      IV          missing sequence
      ==          =================
301 ERVMLGVMLQ ALQQFNWGKN LFPSYLTSXP N
```

Fig 36

**98c cp**

```
                                                 missing sequence
    ===========================================================
1   NAPVSGTITE FLVDVDATVE VGQEIIKMEE GDAPAGGASA SEAPAKKEEA

                                                 missing sequence
    ===========================================================
51  PEKAKEESAP AAAPKKEETK KEEPKKEEKP APKKEESKKS TQSTTSAPTP

        missing sequence
    ===========================================================
101 TNFSPNEERV KMSRMRLRIA ERLKESQNTA ASLTTFNEVD MSNLMDFRKK

    missing sequence
    ===========================================================
151 VKDEFIEKTG IKLGFMSAFS KASALALKEI PAVNAAIENN DTLVFKDYAD

    missing sequence               X    XX  XX   NK     *
    ============================== =    ==  ==   ===    =
201 ISIAVATPKG LVTPVVRNAE SLSILGIEKE ISNLGKKARD GKLTLEDMTG

     S   X XX  X    C     X X* X F    XF X  IX
     =   = ==  =    =     = == = =    == =  ==
251 GTFTISNGGV FGSLYGTPII NMPQTAVLGL HGVKERPVTV NGQIVSRPMM

301 YLALTYDHRV VDGREAVIFL RTIKELIEDP RKMLL
```

Fig 37

**38**

1 MYVYKRDGRK EPVRFDKITA RVQRLCYGLN PNHVEPVAIT QKVISGVYQG
51 VTTIELDNLA AEIAATMTTI HPDYAVLAAR IAVSNLHKQT TKQYSKVSKD
101 LYEYINPKTG LHSPMISKET YDIIMEHEDE LNSAIVYDRD FNYNYFGFKT
151 LERSYLLRIN GKVAERPQHL IMRVAVGIHG NDIPRVIETY NLMSQRFFTH
201 GSPCLFNAGT PRPQMSSCFL LAMKDDSIEG IYDTLKSCAL ISKSAGGIGL
251 HIHNIRSTGA YIAGTNGTSN GIIPMVRVFN NTARYVDQGG NKRPGAFALY
301 LEPWHSDIFD FIDIRKNHGK EEIRARDLFP ALWIPDLFMK RVEQNGDWTL
351 FSPNEAPGLA DVYGDEFEEL YTKYEKENRG RQTIKAQKLW YAILGAQTET
401 GTPFMLYKDS CNRKSNQKNL GIIKSSNLCC EIVEYSAPDE VAVCNLASIA
451 LPSFVENDEK STNYNFDKLH QVTKVVTRNL NRVIDRNHYP VPEAERSNMR
501 HRPIALGVQG LADAFMELRL PFDSQEARBL NIQIFETIYH AAVEASIELA
551 KEEGAYETYP GSPASQGLLQ FDLWNRKPTE LWDWDTLKQD LAKHGMRNSL
601 LVAPMPTAST SQILGNNECF EPYTSNIYSR RVLAGEFQIV NPYLLKDLVD
651 LGVWNDAMKS SIIANNGSIQ ALPNIPDEIK ALYKTVWEIS QKHIIDMAAD
701 RAAFIDQSQS LNIHIKDPTM GKLTSMHFYG WKKGLKTGMY YLRTQAASAA
751 IQFTIDQKIA ETAGHTVANL DKLNIKKYVN KGRVESENTS DAPYKSFSTE
801 PTSLESSVAD LKIKDEGEKP AEDKTIEELE NDIYSAKVIA CAIDNPESCT
851 MCSG

Fig 38

**<u>36</u>**

1 MTTSKETFLF TSESVGEGHP DKICDQVSDA ILDACLAVDP LSKVACETAA

51 KTGMIMVFGE ITTKAQLDYQ KIIRDTIKHI GYDDSEKGFD YKTCNVLVAI

101 EQQSPDIAQG LHYEKALEEL GAGDQGIMFG YATDETDEKL PLTILLAHKL

151 NAALASARRS GSLPWLRPDT KTQVTIEYEK DGGAVIPKRV DTIVISTQHA

201 EEIPTENLRK EIIEHIIKQV IPEHLLDDKT IYHIQPSGRF VIGGPQGDAG

251 LTGRKIIVDT YGGWGAHGGG AFSGKDPSKV DRSAAYAARW VAKSLVTAGL

301 AKRALVQFSY AIGVAEPTSI YIDTYGTSKL STEALVEIIK NNFDLRPGVI

351 VKELDLARPI YFKTASYGHF TNQENSWEQP KKLKF

Fig 39